# EUROPEAN PATENT APPLICATION

(11) **EP 4 768 599 A2**
(43) Date of publication of application: **01.07.2026**
(21) Application number: 26165883.5
(22) Date of filing: 30.03.2022
(51) Int. Cl.: C12Q 1/6855

(54) **COMPOSITIONS AND METHODS FOR SIMULTANEOUS GENETIC ANALYSIS OF MULTIPLE LIBRARIES**

(30) Priority: 30.03.2021 US 202163167914 P; 16.02.2022 US 202263311041 P
(62) Divisional of application: 22782137.8
(71) Applicant: Resolution Bioscience, Inc., Madison, WI 53719 (US)
(72) Inventor: GUO, Jiannan, Madison, WI 53719 (US); HUTCHEON, Carolyn, Madison, WI 53719 (US); LI, Mark, Madison, WI 53719 (US); LIM, Lee, Madison, WI 53719 (US); PEKKER, Ira, Madison, WI 53719 (US); TSAI, Chen-Hsun, Madison, WI 53719 (US)
(74) Representative: Forresters IP LLP

(57) **Abstract**

The disclosure provides compositions and methods that combine the benefits of different genetic analysis methods by performing simultaneous genetic analysis on a single pool of different libraries that were generated for different approaches, such as a combined library comprising a LPWG library and a target-enriched library.

## Description

### CROSS REFERENCE TO RELATED APPLICATIONS

This application claims priority to U.S. Provisional Application Serial No. 63/311,041, filed on February 16, 2022, and U.S. Provisional Application Serial No. 63/167,914, filed on March 30, 2021, both of which are incorporated by reference herein in their entirety for all purposes.

### FIELD OF THE INVENTION

The present invention relates to compositions and methods for genetic analysis.

### SEQUENCE LISTING

The instant application contains a Sequence Listing which has been submitted electronically in ASCII format and is hereby incorporated by reference in its entirety. Said ASCII copy, created on March 28, 2022, is named CLFK_007_02WO_SeqList_ST25 and is about 21.4 KB in size.

### BACKGROUND

Next generation sequencing (NGS) is rapidly becoming more prevalent in clinical settings to identify genetic changes. With a growing number of druggable gene aberrations and predictive biomarkers, NGS technologies have increasingly substituted conventional techniques, such as single-gene testing, with simultaneous analysis of a broad spectrum of genomic alterations. Current applications of NGS approaches include targeted gene panels and whole genome sequencing, with most applications choosing to utilize only one of the two approaches, or proceeding with both approaches in parallel. The ability to combine libraries specific to different NGS approaches in a single sequencing analysis will consolidate the benefits of each approach, thereby improving data quality, cost, and turnaround time. Therefore, compositions and methods for simultaneous genetic analysis of multiple libraries are highly desired.

### SUMMARY

In some embodiments of the kits of the disclosure, the kit comprises one or more capture probe modules, wherein each capture probe module comprises a tail sequence and a capture probe sequence capable of hybridizing to a target sequence in a test sample, wherein the one or more capture probe modules have passed a probe Quality Control (QC) process. In some embodiments of the kits of the disclosure, the kit further comprises a set of adaptors, wherein each adaptor comprises an adaptor module. In some embodiments of the kits of the disclosure, the set of adaptors have passed an adaptor QC process.

In some embodiments of the kits of the disclosure, the kit comprises a set of adaptors, wherein each adaptor comprises an adaptor module, wherein the set of adaptors have passed an adaptor QC process. In some embodiments of the kits of the disclosure, the kit further comprises one or more capture probe modules, wherein each capture probe module comprises a tail sequence and a capture probe sequence capable of hybridizing to a target sequence in a test sample.

In some embodiments of the kits of the disclosure, the adaptor QC process comprises a test for adaptor ligation, wherein the test for adaptor ligation comprises (a) ligating the set of adaptors to a pre-determined amount of end-repaired DNA fragments to generate a library of adaptor-tagged DNA fragments (LIBS); and (b) amplifying the LIBS to generate a Library Post Amplification (LPA); wherein the set of adaptors is considered to have passed the test for adaptor ligation when the concentration of the LPA is higher than a pre-determined concentration.

In some embodiments of the kits of the disclosure, the adaptor QC process comprises a test for adaptor distribution comprising (a) ligating the set of adaptors to a pre-determined amount of end-repaired DNA fragments to generate a library of adaptor-tagged DNA fragments (LIBS); (b) amplifying the LIBS using a primer pair comprising at least one primer comprising an index sequence to generate a Library Post Index Amplification (LPIA); and (c) performing a quantitative genetic analysis on the LPIA, wherein the set of adaptors is considered to have passed the test for adaptor distribution when one or more pre-determined acceptance criteria for the quantitative genetic analysis has been met.

In some embodiments of the kits of the disclosure, the pre-determined acceptance criteria for the adaptor QC process comprise one or more of: (a) Barcode Crosstalk is present in no more than 0.05%-5% of reads; (b) unknown adaptors are present in no more than 1%-50% of reads; (c) no more than 10%-80% of unique adaptor sequences have a number of reads that is 0% to 50% of the average number of reads for all unique adaptor sequences; (d) at least 60%-99.9% of all unique adaptor sequences are present; and (e) no more than 5%-50% of unique adaptor sequences have reads greater than twice the average number of reads for all unique adaptor sequences.

In some embodiments of the kits of the disclosure, the set of adaptors is considered to have passed the adaptor QC process when it has passed the test for adaptor ligation and/or the test for adaptor distribution. In some embodiments of the kits of the disclosure, the set of adaptors is considered to have passed the adaptor QC process when it has passed the test for adaptor ligation. In some embodiments of the kits of the disclosure, the set of adaptors is considered to have passed the adaptor QC process when it has passed the test for adaptor distribution. In some embodiments of the kits of the disclosure, the set of adaptors is considered to have passed the adaptor QC process when it has passed the test for adaptor ligation and the test for adaptor distribution.

In some embodiments of the kits of the disclosure, the probe QC process comprises a test for capture probe modules comprising: (a) ligating a set of adaptors to a DNA sample comprising end-repaired DNA fragments to generate a library of adaptor-tagged DNA fragments (LIBS); (b) amplifying the LIBS to generate a Library Post Amplification (LPA); (c) splitting or diluting the LPA to generate a Target Capture LPA (TC LPA) and a Whole-Genome LPA (WG LPA); (d) amplifying the WG LPA to generate a Whole-Genome Library Amplified (WGLA); (e) hybridizing the one or more capture probe modules to be tested to the TC LPA to form adaptor-tagged DNA fragment-capture probe module complexes; (f) isolating the adaptor-tagged DNA fragment-capture probe module complexes to form isolated adaptor-tagged DNA fragment-capture probe module complexes; (g) enzymatically processing the isolated adaptor-tagged DNA fragment-capture probe module complexes to generate Hybrid Molecules, wherein each Hybrid Molecule comprises the capture probe module and a complement of the adaptor-tagged DNA fragment; (h) amplifying the Hybrid Molecules to generate a Target Capture Library Amplified (TCLA); (i) combining the WGLA and the TCLA to form a Sequence-Ready Library (SRL); and
(j) performing a quantitative genetic analysis on the SRL; wherein the DNA sample comprises a plurality of single nucleotide polymorphisms (SNPs); and wherein the one or more capture probe modules are considered to have passed the probe QC process if one or more pre-determined acceptance criteria for the quantitative genetic analysis have been met.

In some embodiments of the kits of the disclosure, the pre-determined acceptance criteria for the probe QC process comprise one or more of: (a) at least 60%-99.9% of capture probes have at least 1 total reads; (b) at least 60%-99.9% of capture probes have at least 10 to at least 200 on-target total reads; and (c) at least 60%-99.9% of expected SNPs within the DNA sample are detected.

In some embodiments of the kits of the disclosure, the kit comprises a first primer pair comprising a first F primer and a first R primer, wherein each adaptor module comprises an amplification region; wherein the first F primer comprises an amplification region binding region and a sequencing primer binding region; wherein the first R primer comprises a tail sequence binding region and a sequencing primer binding region.

In some embodiments of the kits of the disclosure, the kit comprises a second primer pair comprising a second F primer and a second R primer, wherein each adaptor module comprises an amplification region; wherein each of the second F primer and the second R primer comprises an amplification region binding region and a sequencing primer binding region.

In some embodiments of the kits of the disclosure, the tail sequence of each capture probe module comprises a Library Tag.

In some embodiments of the kits of the disclosure, the kit comprises (a) a set of adaptors, wherein each adaptor comprises an adaptor module comprising an amplification region; (b) one or more capture probe modules, wherein each capture probe module comprises a tail sequence and a capture probe sequence capable of hybridizing to a target sequence in a test sample, wherein the tail sequence of each capture probe module comprises a Library Tag; (c) a first primer pair comprising a first F primer and a first R primer, wherein the first F primer comprises an amplification region binding region and a sequencing primer binding region; wherein the first R primer comprises a Library Tag binding region and a sequencing primer binding region; (d) a second primer pair comprising a second F primer and a second R primer, wherein each of the second F primer and the second R primer comprises an amplification region binding region and a sequencing primer binding region, wherein none of the primers of the second primer pair bind to the Library Tag.

In some embodiments of the kits of the disclosure, the Library Tag comprises a nucleic acid sequence or an amino acid sequence.

In some embodiments of the kits of the disclosure, the first primer pair is used to generate a first modified library and the second primer pair is used to generate a second modified library, wherein the first modified library and the second modified library are configured to be combined into a Sequence-Ready Library (SRL).

In some embodiments of the kits of the disclosure, both the first modified library and the second modified library are generated from the test sample.

In some embodiments of the kits of the disclosure, the first modified library or the second modified library comprises a Library Tag, wherein the Library Tag is configured to distinguish the first modified library from the second modified library.

In some embodiments of the kits of the disclosure, each library fragment of the first modified library is an adaptor-tagged DNA fragment comprising an adaptor, a capture probe module, and at least a portion of a DNA sequence of the test sample; and each library fragment of the second modified library is an adaptor-tagged DNA fragment comprising an adaptor and at least a portion of a DNA sequence of the test sample, wherein none of the adaptor-tagged DNA fragments of the second modified library comprises a capture probe module.

In some embodiments of the kits of the disclosure, the kit comprises one or more capture probe modules, wherein each capture probe module comprises a tail sequence and a capture probe sequence capable of hybridizing to a target sequence in a test sample.

In some embodiments of the kits of the disclosure, the kit comprises one or more capture probe modules, wherein each capture probe module comprises a tail sequence and a capture probe sequence capable of hybridizing to a target sequence in a test sample, wherein the one or more capture probe modules have passed a probe Quality Control (QC) process.

In some embodiments of the kits of the disclosure, the kit comprises a set of adaptors, wherein each adaptor comprises an adaptor module.

In some embodiments of the kits of the disclosure, the kit comprises a set of adaptors, wherein each adaptor comprises an adaptor module, wherein the set of adaptors have passed an adaptor Quality Control (QC) process.

In some embodiments of the kits of the disclosure, the kit comprises (a) one or more capture probe modules, wherein each capture probe module comprises a tail sequence and a capture probe sequence capable of hybridizing to a target sequence in a test sample; and (b) a set of adaptors, wherein each adaptor comprises an adaptor module.

In some embodiments of the kits of the disclosure, the kit comprises (a) one or more capture probe modules, wherein each capture probe module comprises a tail sequence and a capture probe sequence capable of hybridizing to a target sequence in a test sample; and (b) a set of adaptors, wherein each adaptor comprises an adaptor module, wherein the set of adaptors have passed an adaptor Quality Control (QC) process.

In some embodiments of the kits of the disclosure, the kit comprises (a) one or more capture probe modules, wherein each capture probe module comprises a tail sequence and a capture probe sequence capable of hybridizing to a target sequence in a test sample, wherein the one or more capture probe modules have passed a probe Quality Control (QC) process; and (b) a set of adaptors, wherein each adaptor comprises an adaptor module.

In some embodiments of the kits of the disclosure, the kit comprises (a) one or more capture probe modules, wherein each capture probe module comprises a tail sequence and a capture probe sequence capable of hybridizing to a target sequence in a test sample, wherein the one or more capture probe modules have passed a probe Quality Control (QC) process; and (b) a set of adaptors, wherein each adaptor comprises an adaptor module, wherein the set of adaptors have passed an adaptor Quality Control (QC) process.

In some embodiments of the kits of the disclosure, the adaptor QC process comprises a test for adaptor ligation, wherein the test for adaptor ligation comprises (a) ligating the set of adaptors to a pre-determined amount of end-repaired DNA fragments to generate a library of adaptor-tagged DNA fragments (LIBS); and (b) amplifying the LIBS to generate a Library Post Amplification (LPA); wherein the set of adaptors is considered to have passed the test for adaptor ligation when the concentration of the LPA is higher than a pre-determined concentration.

In some embodiments of the kits of the disclosure, the end-repaired DNA fragments of step (a) of the test for adaptor ligation is generated from a DNA sample. In some embodiments, said DNA sample is a blended DNA sample of at least two different cell lines. In some embodiments, said DNA sample is a blended DNA sample of at two different cell lines. In some embodiments, the two different cell lines are blended at a 50:50 ratio. In some embodiments, the two different cell lines are NA09596 and NA12878. In some embodiments, said DNA sample is the 50:50 blended sample described in Example 15.

In some embodiments of the kits of the disclosure, the DNA sample used in the test for adaptor ligation is different from the DNA sample used in the test for adaptor distribution.

In some embodiments of the kits of the disclosure, the pre-determined amount of end-repaired DNA fragments is about 5 ng. In some embodiments of the kits of the disclosure, the pre-determined amount of end-repaired DNA fragments is about 10 ng. In some embodiments of the kits of the disclosure, the pre-determined amount of end-repaired DNA fragments is about 15 ng. In some embodiments of the kits of the disclosure, the pre-determined amount of end-repaired DNA fragments is about 20 ng. In some embodiments of the kits of the disclosure, the pre-determined amount of end-repaired DNA fragments is about 25 ng. In some embodiments of the kits of the disclosure, the pre-determined amount of end-repaired DNA fragments is about 30 ng. In some embodiments of the kits of the disclosure, the pre-determined amount of end-repaired DNA fragments is about 35 ng. In some embodiments of the kits of the disclosure, the pre-determined amount of end-repaired DNA fragments is about 40 ng. In some embodiments of the kits of the disclosure, the pre-determined amount of end-repaired DNA fragments is about 50 ng.

In some embodiments of the kits of the disclosure, the pre-determined amount of end-repaired DNA fragments is 5 ng. In some embodiments of the kits of the disclosure, the pre-determined amount of end-repaired DNA fragments is 10 ng. In some embodiments of the kits of the disclosure, the pre-determined amount of end-repaired DNA fragments is 15 ng. In some embodiments of the kits of the disclosure, the pre-determined amount of end-repaired DNA fragments is 20 ng. In some embodiments of the kits of the disclosure, the pre-determined amount of end-repaired DNA fragments is 25 ng. In some embodiments of the kits of the disclosure, the pre-determined amount of end-repaired DNA fragments is 30 ng. In some embodiments of the kits of the disclosure, the pre-determined amount of end-repaired DNA fragments is 35 ng. In some embodiments of the kits of the disclosure, the pre-determined amount is 40 ng. In some embodiments of the kits of the disclosure, the pre-determined amount of end-repaired DNA fragments is 50 ng.

In some embodiments of the kits of the disclosure, the pre-determined amount of end-repaired DNA fragments is about 1 ng to about 100 ng. In some embodiments of the kits of the disclosure, the pre-determined amount of end-repaired DNA fragments is about 1 ng to about 50 ng. In some embodiments of the kits of the disclosure, the pre-determined amount of end-repaired DNA fragments is about 50 ng to about 100 ng. In some embodiments of the kits of the disclosure, the pre-determined amount of end-repaired DNA fragments is about 5 ng to about 90 ng. In some embodiments of the kits of the disclosure, the pre-determined amount of end-repaired DNA fragments is about 10 ng to about 80 ng. In some embodiments of the kits of the disclosure, the pre-determined amount of end-repaired DNA fragments is about 20 ng to about 60 ng. In some embodiments of the kits of the disclosure, the pre-determined amount of end-repaired DNA fragments is about 20 ng to about 50 ng. In some embodiments of the kits of the disclosure, the pre-determined amount of end-repaired DNA fragments is about 20 ng to about 40 ng. In some embodiments of the kits of the disclosure, the pre-determined amount of end-repaired DNA fragments is about 30 ng to about 50 ng. In some embodiments of the kits of the disclosure, the pre-determined amount of end-repaired DNA fragments is about 40 ng to about 50 ng. In some embodiments of the kits of the disclosure, the pre-determined amount of end-repaired DNA fragments is about 30 ng to about 40 ng. In some embodiments of the kits of the disclosure, the pre-determined amount of end-repaired DNA fragments is about 20 ng to about 30 ng. In some embodiments of the kits of the disclosure, the pre-determined amount of end-repaired DNA fragments is greater than about 100 ng.

In some embodiments of the kits of the disclosure, the pre-determined amount of end-repaired DNA fragments is 1 ng to 100 ng. In some embodiments of the kits of the disclosure, the pre-determined amount of end-repaired DNA fragments is 1 ng to 50 ng. In some embodiments of the kits of the disclosure, the pre-determined amount of end-repaired DNA fragments is 50 ng to 100 ng. In some embodiments of the kits of the disclosure, the pre-determined amount of end-repaired DNA fragments is 5 ng to 90 ng. In some embodiments of the kits of the disclosure, the pre-determined amount of end-repaired DNA fragments is 10 ng to 80 ng. In some embodiments of the kits of the disclosure, the pre-determined amount of end-repaired DNA fragments is 20 ng to 60 ng. In some embodiments of the kits of the disclosure, the pre-determined amount of end-repaired DNA fragments is 20 ng to 50 ng. In some embodiments of the kits of the disclosure, the pre-determined amount of end-repaired DNA fragments is 20 ng to 40 ng. In some embodiments of the kits of the disclosure, the pre-determined amount of end-repaired DNA fragments is 30 ng to 50 ng. In some embodiments of the kits of the disclosure, the pre-determined amount of end-repaired DNA fragments is 40 ng to 50 ng. In some embodiments of the kits of the disclosure, the pre-determined amount of end-repaired DNA fragments is 30 ng to 40 ng. In some embodiments of the kits of the disclosure, the pre-determined amount of end-repaired DNA fragments is 20 ng to 30 ng. In some embodiments of the kits of the disclosure, the pre-determined amount of end-repaired DNA fragments is greater than 100 ng.

In some embodiments of the kits of the disclosure, the pre-determined concentration of the LPA is about 1 ng/µL. In some embodiments of the kits of the disclosure, the pre-determined concentration of the LPA is about 5 ng/µL. In some embodiments of the kits of the disclosure, the pre-determined concentration of the LPA is about 10 ng/µL. In some embodiments of the kits of the disclosure, the pre-determined concentration of the LPA is about 20 ng/µL. In some embodiments of the kits of the disclosure, the pre-determined concentration of the LPA is about 30 ng/µL. In some embodiments of the kits of the disclosure, the pre-determined concentration of the LPA is about 40 ng/µL. In some embodiments of the kits of the disclosure, the pre-determined concentration of the LPA is about 50 ng/µL. In some embodiments of the kits of the disclosure, the pre-determined concentration of the LPA is about 60 ng/µL. In some embodiments of the kits of the disclosure, the pre-determined concentration of the LPA is about 70 ng/µL. In some embodiments of the kits of the disclosure, the pre-determined concentration of the LPA is about 80 ng/µL. In some embodiments of the kits of the disclosure, the pre-determined concentration of the LPA is about 90 ng/µL. In some embodiments of the kits of the disclosure, the pre-determined concentration of the LPA is about 100 ng/µL. In some embodiments of the kits of the disclosure, the pre-determined concentration of the LPA is about 150 ng/µL. In some embodiments of the kits of the disclosure, the pre-determined concentration of the LPA is about 200 ng/µL.

In some embodiments of the kits of the disclosure, the pre-determined concentration of the LPA is 1 ng/µL. In some embodiments of the kits of the disclosure, the pre-determined concentration of the LPA is 5 ng/µL. In some embodiments of the kits of the disclosure, the pre-determined concentration of the LPA is 10 ng/µL. In some embodiments of the kits of the disclosure, the pre-determined concentration of the LPA is 20 ng/µL. In some embodiments of the kits of the disclosure, the pre-determined concentration of the LPA is 30 ng/µL. In some embodiments of the kits of the disclosure, the pre-determined concentration of the LPA is 40 ng/µL. In some embodiments of the kits of the disclosure, the pre-determined concentration of the LPA is 50 ng/µL. In some embodiments of the kits of the disclosure, the pre-determined concentration of the LPA is 60 ng/µL. In some embodiments of the kits of the disclosure, the pre-determined concentration of the LPA is 70 ng/µL. In some embodiments of the kits of the disclosure, the pre-determined concentration of the LPA is 80 ng/µL. In some embodiments of the kits of the disclosure, the pre-determined concentration of the LPA is 90 ng/µL. In some embodiments of the kits of the disclosure, the pre-determined concentration of the LPA is 100 ng/µL. In some embodiments of the kits of the disclosure, the pre-determined concentration of the LPA is 150 ng/µL. In some embodiments of the kits of the disclosure, the pre-determined concentration of the LPA is 200 ng/µL.

In some embodiments of the kits of the disclosure, the pre-determined concentration of the LPA is about 1 ng/µL to about 200 ng/µL. In some embodiments of the kits of the disclosure, the pre-determined concentration of the LPA is greater than about 200 ng/µL. In some embodiments of the kits of the disclosure, the pre-determined concentration of the LPA is about 10 ng/µL to about 100 ng/µL. In some embodiments of the kits of the disclosure, the pre-determined concentration of the LPA is about 10 ng/µL to about 90 ng/µL. In some embodiments of the kits of the disclosure, the pre-determined concentration of the LPA is about 20 ng/µL to about 80 ng/µL. In some embodiments of the kits of the disclosure, the pre-determined concentration of the LPA is about 10 ng/µL to about 80 ng/µL. In some embodiments of the kits of the disclosure, the pre-determined concentration of the LPA is about 30 ng/µL to about 80 ng/µL. In some embodiments of the kits of the disclosure, the pre-determined concentration of the LPA is about 40 ng/µL to about 80 ng/µL. In some embodiments of the kits of the disclosure, the pre-determined concentration of the LPA is about 50 ng/µL to about 80 ng/µL. In some embodiments of the kits of the disclosure, the pre-determined concentration of the LPA is about 50 ng/µL to about 70 ng/µL. In some embodiments of the kits of the disclosure, the pre-determined concentration of the LPA is about 60 ng/µL to about 70 ng/µL. In some embodiments of the kits of the disclosure, the pre-determined concentration of the LPA is about 50 ng/µL to about 60 ng/µL.

In some embodiments of the kits of the disclosure, the pre-determined concentration of the LPA is 1 ng/µL to 200 ng/µL. In some embodiments of the kits of the disclosure, the pre-determined concentration of the LPA is 10 ng/µL to 100 ng/µL. In some embodiments of the kits of the disclosure, the pre-determined concentration of the LPA is 10 ng/µL to 90 ng/µL. In some embodiments of the kits of the disclosure, the pre-determined concentration of the LPA is 20 ng/µL to 80 ng/µL. In some embodiments of the kits of the disclosure, the pre-determined concentration of the LPA is 30 ng/µL to 80 ng/µL. In some embodiments of the kits of the disclosure, the pre-determined concentration of the LPA is 10 ng/µL to 80 ng/µL. In some embodiments of the kits of the disclosure, the pre-determined concentration of the LPA is 40 ng/µL to 80 ng/µL. In some embodiments of the kits of the disclosure, the pre-determined concentration of the LPA is 50 ng/µL to 80 ng/µL. In some embodiments of the kits of the disclosure, the pre-determined concentration of the LPA is 50 ng/µL to 70 ng/µL. In some embodiments of the kits of the disclosure, the pre-determined concentration of the LPA is 60 ng/µL to 70 ng/µL. In some embodiments of the kits of the disclosure, the pre-determined concentration of the LPA is 50 ng/µL to 60 ng/µL.

In some embodiments of the kits of the disclosure, the pre-determined concentration of the LPA is greater than about 200 ng/µL. In some embodiments of the kits of the disclosure, the pre-determined concentration of the LPA is greater than 200 ng/µL.

In some embodiments of the kits of the disclosure, the set of adaptors is considered to have passed the adaptor QC process when it has passed the test for adaptor ligation.

In some embodiments of the kits of the disclosure, the adaptor QC process comprises a test for adaptor distribution comprising (a) ligating the set of adaptors to a pre-determined amount of end-repaired DNA fragments to generate a library of adaptor-tagged DNA fragments (LIBS); (b) amplifying the LIBS using a primer pair comprising at least one primer comprising an index sequence to generate a Library Post Index Amplification (LPIA); and (c) performing a quantitative genetic analysis on the LPIA, wherein the set of adaptors is considered to have passed the test for adaptor distribution when one or more pre-determined acceptance criteria for the quantitative genetic analysis has been met.

In some embodiments of the kits of the disclosure, the end-repaired DNA fragments of step (a) of the test for adaptor distribution are generated from a DNA sample. In some embodiments, said DNA sample comprises wild-type (wt) cell-free DNA (cfDNA). In some embodiments, said DNA sample consists of wt cfDNA. In some embodiments, said DNA sample comprises wt cfDNA obtained from a healthy human. In some embodiments, said DNA sample is the wt cfDNA sample described in Example 16.

In some embodiments of the kits of the disclosure, the DNA sample used in the test for adaptor distribution is different from the DNA sample used in the test for adaptor ligation.

In some embodiments of the kits of the disclosure, the pre-determined acceptance criteria for the adaptor QC process comprise a criterion (a). In some embodiments, criterion (a) is Barcode Crosstalk is present in no more than about 0.05% of reads. In some embodiments, criterion (a) is Barcode Crosstalk is present in no more than about 0.1% of reads. In some embodiments, criterion (a) is Barcode Crosstalk is present in no more than about 0.2% of reads. In some embodiments, criterion (a) is Barcode Crosstalk is present in no more than about 0.3% of reads. In some embodiments, criterion (a) is Barcode Crosstalk is present in no more than about 0.4% of reads. In some embodiments, criterion (a) is Barcode Crosstalk is present in no more than about 0.5% of reads. In some embodiments, criterion (a) is Barcode Crosstalk is present in no more than about 0.6% of reads. In some embodiments, criterion (a) is Barcode Crosstalk is present in no more than about 0.7% of reads. In some embodiments, criterion (a) is Barcode Crosstalk is present in no more than about 0.8% of reads. In some embodiments, criterion (a) is Barcode Crosstalk is present in no more than about 0.9% of reads. In some embodiments, criterion (a) is Barcode Crosstalk present in no more than about 1% of reads. In some embodiments, criterion (a) is Barcode Crosstalk present in no more than about 2% of reads. In some embodiments, criterion (a) is Barcode Crosstalk present in no more than about 3% of reads. In some embodiments, criterion (a) is Barcode Crosstalk present in no more than about 4% of reads. In some embodiments, criterion (a) is Barcode Crosstalk present in no more than about 5% of reads.

In some embodiments, criterion (a) is Barcode Crosstalk is present in no more than 0.05% of reads. In some embodiments, criterion (a) is Barcode Crosstalk is present in no more than 0.1% of reads. In some embodiments, criterion (a) is Barcode Crosstalk is present in no more than 0.2% of reads. In some embodiments, criterion (a) is Barcode Crosstalk present in no more than 0.3% of reads. In some embodiments, criterion (a) is Barcode Crosstalk present in no more than 0.4% of reads. In some embodiments, criterion (a) is Barcode Crosstalk is present in no more than 0.5% of reads. In some embodiments, criterion (a) is Barcode Crosstalk is present in no more than 0.6% of reads. In some embodiments, criterion (a) is Barcode Crosstalk is present in no more than 0.7% of reads. In some embodiments, criterion (a) is Barcode Crosstalk is present in no more than 0.8% of reads. In some embodiments, criterion (a) is Barcode Crosstalk present in no more than 0.9% of reads. In some embodiments, criterion (a) is Barcode Crosstalk present in no more than 1% of reads. In some embodiments, criterion (a) is Barcode Crosstalk present in no more than 2% of reads. In some embodiments, criterion (a) is Barcode Crosstalk present in no more than 3% of reads. In some embodiments, criterion (a) is Barcode Crosstalk present in no more than 4% of reads. In some embodiments, criterion (a) is Barcode Crosstalk present in no more than 5% of reads.

In some embodiments, criterion (a) is Barcode Crosstalk is present in no more than about 0.05% to about 10% of reads. In some embodiments, criterion (a) is Barcode Crosstalk is present in no more than about 0.05% to about 5% of reads. In some embodiments, criterion (a) is Barcode Crosstalk is present in no more than about 0.1% to about 5% of reads. In some embodiments, criterion (a) is Barcode Crosstalk is present in no more than about 0.1% to about 2% of reads. In some embodiments, criterion (a) is Barcode Crosstalk is present in no more than about 0.05% to about 2% of reads. In some embodiments, criterion (a) is Barcode Crosstalk is present in no more than about 0.1% to about 1% of reads. In some embodiments, criterion (a) is Barcode Crosstalk is present in no more than about 0.05% to about 1% of reads. In some embodiments, criterion (a) is Barcode Crosstalk is present in no more than about 0.1% to about 0.5% of reads. In some embodiments, criterion (a) is Barcode Crosstalk is present in no more than about 0.1% to about 0.4% of reads. In some embodiments, criterion (a) is Barcode Crosstalk is present in no more than about 0.1% to about 0.3% of reads. In some embodiments, criterion (a) is Barcode Crosstalk is present in no more than about 0.2% to about 0.3% of reads. In some embodiments, criterion (a) is Barcode Crosstalk is present in no more than about 0.1% to about 0.2% of reads.

In some embodiments, criterion (a) is Barcode Crosstalk is present in no more than 0.05% to 10% of reads. In some embodiments, criterion (a) is Barcode Crosstalk is present in no more than 0.05% to 5% of reads. In some embodiments, criterion (a) is Barcode Crosstalk is present in no more than 0.1% to 5% of reads. In some embodiments, criterion (a) is Barcode Crosstalk is present in no more than 0.1% to 2% of reads. In some embodiments, criterion (a) is Barcode Crosstalk is present in no more than 0.05% to 2% of reads. In some embodiments, criterion (a) is Barcode Crosstalk is present in no more than 0.1% to 1% of reads. In some embodiments, criterion (a) is Barcode Crosstalk is present in no more than 0.05% to 1% of reads. In some embodiments, criterion (a) is Barcode Crosstalk is present in no more than 0.1% to 0.5% of reads. In some embodiments, criterion (a) is Barcode Crosstalk is present in no more than 0.1% to 0.4% of reads. In some embodiments, criterion (a) is Barcode Crosstalk is present in no more than 0.1% to 0.3% of reads. In some embodiments, criterion (a) is Barcode Crosstalk is present in no more than 0.2% to 0.3% of reads. In some embodiments, criterion (a) is Barcode Crosstalk is present in no more than 0.1% to 0.2% of reads.

In some embodiments of the kits of the disclosure, the pre-determined acceptance criteria for the adaptor QC process comprise a criterion (b). In some embodiments, criterion (b) is unknown adaptors are present in no more than about 1% of reads. In some embodiments, criterion (b) is unknown adaptors are present in no more than about 5% of reads. In some embodiments, criterion (b) is unknown adaptors are present in no more than about 10% of reads. In some embodiments, criterion (b) is unknown adaptors are present in no more than about 15% of reads. In some embodiments, criterion (b) is unknown adaptors are present in no more than about 20% of reads. In some embodiments, criterion (b) is unknown adaptors are present in no more than about 25% of reads. In some embodiments, criterion (b) is unknown adaptors are present in no more than about 30% of reads. In some embodiments, criterion (b) is unknown adaptors are present in no more than about 35% of reads. In some embodiments, criterion (b) is unknown adaptors are present in no more than about 40% of reads. In some embodiments, criterion (b) is unknown adaptors are present in no more than about 45% of reads. In some embodiments, criterion (b) is unknown adaptors are present in no more than about 50% of reads.

In some embodiments, criterion (b) is unknown adaptors are present in no more than 1% of reads. In some embodiments, criterion (b) is unknown adaptors are present in no more than 5% of reads. In some embodiments, criterion (b) is unknown adaptors are present in no more than 10% of reads. In some embodiments, criterion (b) is unknown adaptors are present in no more than 15% of reads. In some embodiments, criterion (b) is unknown adaptors are present in no more than 20% of reads. In some embodiments, criterion (b) is unknown adaptors are present in no more than 25% of reads. In some embodiments, criterion (b) is unknown adaptors are present in no more than 30% of reads. In some embodiments, criterion (b) is unknown adaptors are present in no more than 35% of reads. In some embodiments, criterion (b) is unknown adaptors are present in no more than 40% of reads. In some embodiments, criterion (b) is unknown adaptors are present in no more than 45% of reads. In some embodiments, criterion (b) is unknown adaptors are present in no more than 50% of reads.

In some embodiments, criterion (b) is unknown adaptors are present in no more than about 1% to about 50% of reads. In some embodiments, criterion (b) is unknown adaptors are present in no more than about 1% to about 20% of reads. In some embodiments, criterion (b) is unknown adaptors are present in no more than about 1% to about 15% of reads. In some embodiments, criterion (b) is unknown adaptors are present in no more than about 1% to about 10% of reads. In some embodiments, criterion (b) is unknown adaptors are present in no more than about 5% to about 20% of reads. In some embodiments, criterion (b) is unknown adaptors are present in no more than about 5% to about 15% of reads. In some embodiments, criterion (b) is unknown adaptors are present in no more than about 5% to about 10% of reads.

In some embodiments, criterion (b) is unknown adaptors are present in no more than 1% to 50% of reads. In some embodiments, criterion (b) is unknown adaptors are present in no more than 1% to 20% of reads. In some embodiments, criterion (b) is unknown adaptors are present in no more than 1% to 15% of reads. In some embodiments, criterion (b) is unknown adaptors are present in no more than 1% to 10% of reads. In some embodiments, criterion (b) is unknown adaptors are present in no more than 5% to 20% of reads. In some embodiments, criterion (b) is unknown adaptors are present in no more than 5% to 15% of reads. In some embodiments, criterion (b) is unknown adaptors are present in no more than 5% to 10% of reads.

In some embodiments of the kits of the disclosure, the pre-determined acceptance criteria for the adaptor QC process comprise a criterion (c). In some embodiments, criterion (c) is no more than about 10% of unique adaptor sequences have a number of reads that is 0% to 50% of the average number of reads for all unique adaptor sequences. In some embodiments, criterion (c) is no more than about 20% of unique adaptor sequences have a number of reads that is 0% to 50% of the average number of reads for all unique adaptor sequences. In some embodiments, criterion (c) is no more than about 30% of unique adaptor sequences have a number of reads that is 0% to 50% of the average number of reads for all unique adaptor sequences. In some embodiments, criterion (c) is no more than about 40% of unique adaptor sequences have a number of reads that is 0% to 50% of the average number of reads for all unique adaptor sequences. In some embodiments, criterion (c) is no more than about 50% of unique adaptor sequences have a number of reads that is 0% to 50% of the average number of reads for all unique adaptor sequences. In some embodiments, criterion (c) is no more than about 60% of unique adaptor sequences have a number of reads that is 0% to 50% of the average number of reads for all unique adaptor sequences. In some embodiments, criterion (c) is no more than about 70% of unique adaptor sequences have a number of reads that is 0% to 50% of the average number of reads for all unique adaptor sequences. In some embodiments, criterion (c) is no more than about 80% of unique adaptor sequences have a number of reads that is 0% to 50% of the average number of reads for all unique adaptor sequences. In some embodiments, criterion (c) is no more than about 90% of unique adaptor sequences have a number of reads that is 0% to 50% of the average number of reads for all unique adaptor sequences.

In some embodiments of the kits of the disclosure, the pre-determined acceptance criteria for the adaptor QC process comprise a criterion (c). In some embodiments, criterion (c) is no more than 10% of unique adaptor sequences have a number of reads that is 0% to 50% of the average number of reads for all unique adaptor sequences. In some embodiments, criterion (c) is no more than 20% of unique adaptor sequences have a number of reads that is 0% to 50% of the average number of reads for all unique adaptor sequences. In some embodiments, criterion (c) is no more than 30% of unique adaptor sequences have a number of reads that is 0% to 50% of the average number of reads for all unique adaptor sequences. In some embodiments, criterion (c) is no more than 40% of unique adaptor sequences have a number of reads that is 0% to 50% of the average number of reads for all unique adaptor sequences. In some embodiments, criterion (c) is no more than 50% of unique adaptor sequences have a number of reads that is 0% to 50% of the average number of reads for all unique adaptor sequences. In some embodiments, criterion (c) is no more than 60% of unique adaptor sequences have a number of reads that is 0% to 50% of the average number of reads for all unique adaptor sequences. In some embodiments, criterion (c) is no more than 70% of unique adaptor sequences have a number of reads that is 0% to 50% of the average number of reads for all unique adaptor sequences. In some embodiments, criterion (c) is no more than 80% of unique adaptor sequences have a number of reads that is 0% to 50% of the average number of reads for all unique adaptor sequences. In some embodiments, criterion (c) is no more than 90% of unique adaptor sequences have a number of reads that is 0% to 50% of the average number of reads for all unique adaptor sequences.

In some embodiments, criterion (c) is no more than about 10% to about 90% of unique adaptor sequences have a number of reads that is 0% to 50% of the average number of reads for all unique adaptor sequences. In some embodiments, criterion (c) is no more than about 10% to about 80% of unique adaptor sequences have a number of reads that is 0% to 50% of the average number of reads for all unique adaptor sequences. In some embodiments, criterion (c) is no more than about 20% to about 80% of unique adaptor sequences have a number of reads that is 0% to 50% of the average number of reads for all unique adaptor sequences. In some embodiments, criterion (c) is no more than about 30% to about 70% of unique adaptor sequences have a number of reads that is 0% to 50% of the average number of reads for all unique adaptor sequences. In some embodiments, criterion (c) is no more than about 40% to about 60% of unique adaptor sequences have a number of reads that is 0% to 50% of the average number of reads for all unique adaptor sequences. In some embodiments, criterion (c) is no more than about 40% to about 50% of unique adaptor sequences have a number of reads that is 0% to 50% of the average number of reads for all unique adaptor sequences. In some embodiments, criterion (c) is no more than about 50% to about 60% of unique adaptor sequences have a number of reads that is 0% to 50% of the average number of reads for all unique adaptor sequences.

In some embodiments, criterion (c) is no more than 10% to 90% of unique adaptor sequences have a number of reads that is 0% to 50% of the average number of reads for all unique adaptor sequences. In some embodiments, criterion (c) is no more than 10% to 80% of unique adaptor sequences have a number of reads that is 0% to 50% of the average number of reads for all unique adaptor sequences. In some embodiments, criterion (c) is no more than 20% to 80% of unique adaptor sequences have a number of reads that is 0% to 50% of the average number of reads for all unique adaptor sequences. In some embodiments, criterion (c) is no more than 30% to 70% of unique adaptor sequences have a number of reads that is 0% to 50% of the average number of reads for all unique adaptor sequences. In some embodiments, criterion (c) is no more than 40% to 60% of unique adaptor sequences have a number of reads that is 0% to 50% of the average number of reads for all unique adaptor sequences. In some embodiments, criterion (c) is no more than 40% to 50% of unique adaptor sequences have a number of reads that is 0% to 50% of the average number of reads for all unique adaptor sequences. In some embodiments, criterion (c) is no more than 50% to 60% of unique adaptor sequences have a number of reads that is 0% to 50% of the average number of reads for all unique adaptor sequences.

In some embodiments of the kits of the disclosure, the pre-determined acceptance criteria for the adaptor QC process comprise a criterion (d). In some embodiments, criterion (d) is at least about 60% of all unique adaptor sequences are present. In some embodiments, criterion (d) is at least about 65% of all unique adaptor sequences are present. In some embodiments, criterion (d) is at least about 70% of all unique adaptor sequences are present. In some embodiments, criterion (d) is at least about 75% of all unique adaptor sequences are present. In some embodiments, criterion (d) is at least about 80% of all unique adaptor sequences are present. In some embodiments, criterion (d) is at least about 85% of all unique adaptor sequences are present. In some embodiments, criterion (d) is at least about 90% of all unique adaptor sequences are present. In some embodiments, criterion (d) is at least about 95% of all unique adaptor sequences are present. In some embodiments, criterion (d) is at least about 96% of all unique adaptor sequences are present. In some embodiments, criterion (d) is at least about 97% of all unique adaptor sequences are present. In some embodiments, criterion (d) is at least about 98% of all unique adaptor sequences are present. In some embodiments, criterion (d) is at least about 99% of all unique adaptor sequences are present. In some embodiments, criterion (d) is at least about 99.5% of all unique adaptor sequences are present. In some embodiments, criterion (d) is at least about 99.9% of all unique adaptor sequences are present. In some embodiments, criterion (d) is about 100% of all unique adaptor sequences are present.

In some embodiments, criterion (d) is at least 60% of all unique adaptor sequences are present. In some embodiments, criterion (d) is at least 65% of all unique adaptor sequences are present. In some embodiments, criterion (d) is at least 70% of all unique adaptor sequences are present. In some embodiments, criterion (d) is at least 75% of all unique adaptor sequences are present. In some embodiments, criterion (d) is at least 80% of all unique adaptor sequences are present. In some embodiments, criterion (d) is at least 85% of all unique adaptor sequences are present. In some embodiments, criterion (d) is at least 90% of all unique adaptor sequences are present. In some embodiments, criterion (d) is at least 95% of all unique adaptor sequences are present. In some embodiments, criterion (d) is at least 96% of all unique adaptor sequences are present. In some embodiments, criterion (d) is at least 97% of all unique adaptor sequences are present. In some embodiments, criterion (d) is at least 98% of all unique adaptor sequences are present. In some embodiments, criterion (d) is at least 99% of all unique adaptor sequences are present. In some embodiments, criterion (d) is at least 99.5% of all unique adaptor sequences are present. In some embodiments, criterion (d) is at least 99.9% of all unique adaptor sequences are present. In some embodiments, criterion (d) is 100% of all unique adaptor sequences are present.

In some embodiments, criterion (d) is at least about 60% to about 99.9% of all unique adaptor sequences are present. In some embodiments, criterion (d) is at least about 70% to about 99.9% of all unique adaptor sequences are present. In some embodiments, criterion (d) is at least about 80% to about 99.9% of all unique adaptor sequences are present. In some embodiments, criterion (d) is at least about 90% to about 99.9% of all unique adaptor sequences are present. In some embodiments, criterion (d) is at least about 95% to about 99.9% of all unique adaptor sequences are present. In some embodiments, criterion (d) is at least about 96% to about 99.9% of all unique adaptor sequences are present. In some embodiments, criterion (d) is at least about 97% to about 99.9% of all unique adaptor sequences are present. In some embodiments, criterion (d) is at least about 98% to about 99.9% of all unique adaptor sequences are present. In some embodiments, criterion (d) is at least about 99% to about 99.9% of all unique adaptor sequences are present.

In some embodiments, criterion (d) is at least about 60% to about 100% of all unique adaptor sequences are present. In some embodiments, criterion (d) is at least about 70% to about 100% of all unique adaptor sequences are present. In some embodiments, criterion (d) is at least about 80% to about 100% of all unique adaptor sequences are present. In some embodiments, criterion (d) is at least about 90% to about 100% of all unique adaptor sequences are present. In some embodiments, criterion (d) is at least about 95% to about 100% of all unique adaptor sequences are present. In some embodiments, criterion (d) is at least about 96% to about 100% of all unique adaptor sequences are present. In some embodiments, criterion (d) is at least about 97% to about 100% of all unique adaptor sequences are present. In some embodiments, criterion (d) is at least about 98% to about 100% of all unique adaptor sequences are present. In some embodiments, criterion (d) is at least about 99% to about 100% of all unique adaptor sequences are present. In some embodiments, criterion (d) is at least about 99.9% to about 100% of all unique adaptor sequences are present.

In some embodiments, criterion (d) is at least 60% to 99.9% of all unique adaptor sequences are present. In some embodiments, criterion (d) is at least 70% to 99.9% of all unique adaptor sequences are present. In some embodiments, criterion (d) is at least 80% to 99.9% of all unique adaptor sequences are present. In some embodiments, criterion (d) is at least 90% to 99.9% of all unique adaptor sequences are present. In some embodiments, criterion (d) is at least 95% to 99.9% of all unique adaptor sequences are present. In some embodiments, criterion (d) is at least 96% to 99.9% of all unique adaptor sequences are present. In some embodiments, criterion (d) is at least 97% to 99.9% of all unique adaptor sequences are present. In some embodiments, criterion (d) is at least 98% to 99.9% of all unique adaptor sequences are present. In some embodiments, criterion (d) is at least 99% to about 99.9% of all unique adaptor sequences are present.

In some embodiments, criterion (d) is at least 60% to 100% of all unique adaptor sequences are present. In some embodiments, criterion (d) is at least 70% to 100% of all unique adaptor sequences are present. In some embodiments, criterion (d) is at least 80% to 100% of all unique adaptor sequences are present. In some embodiments, criterion (d) is at least 90% to 100% of all unique adaptor sequences are present. In some embodiments, criterion (d) is at least 95% to 100% of all unique adaptor sequences are present. In some embodiments, criterion (d) is at least 96% to 100% of all unique adaptor sequences are present. In some embodiments, criterion (d) is at least 97% to 100% of all unique adaptor sequences are present. In some embodiments, criterion (d) is at least 98% to 100% of all unique adaptor sequences are present. In some embodiments, criterion (d) is at least 99% to 100% of all unique adaptor sequences are present. In some embodiments, criterion (d) is at least 99.9% to 100% of all unique adaptor sequences are present.

In some embodiments of the kits of the disclosure, the pre-determined acceptance criteria for the adaptor QC process comprise a criterion (e). In some embodiments, criterion (e) is no more than about 1% of unique adaptor sequences have reads greater than twice the average number of reads for all unique adaptor sequences. In some embodiments, criterion (e) is no more than about 5% of unique adaptor sequences have reads greater than twice the average number of reads for all unique adaptor sequences. In some embodiments, criterion (e) is no more than about 10% of unique adaptor sequences have reads greater than twice the average number of reads for all unique adaptor sequences. In some embodiments, criterion (e) is no more than about 15% of unique adaptor sequences have reads greater than twice the average number of reads for all unique adaptor sequences. In some embodiments, criterion (e) is no more than about 20% of unique adaptor sequences have reads greater than twice the average number of reads for all unique adaptor sequences. In some embodiments, criterion (e) is no more than about 25% of unique adaptor sequences have reads greater than twice the average number of reads for all unique adaptor sequences. In some embodiments, criterion (e) is no more than about 30% of unique adaptor sequences have reads greater than twice the average number of reads for all unique adaptor sequences. In some embodiments, criterion (e) is no more than about 35% of unique adaptor sequences have reads greater than twice the average number of reads for all unique adaptor sequences. In some embodiments, criterion (e) is no more than about 40% of unique adaptor sequences have reads greater than twice the average number of reads for all unique adaptor sequences. In some embodiments, criterion (e) is no more than about 45% of unique adaptor sequences have reads greater than twice the average number of reads for all unique adaptor sequences. In some embodiments, criterion (e) is no more than about 50% of unique adaptor sequences have reads greater than twice the average number of reads for all unique adaptor sequences.

In some embodiments, criterion (e) is no more than 1% of unique adaptor sequences have reads greater than twice the average number of reads for all unique adaptor sequences. In some embodiments, criterion (e) is no more than 5% of unique adaptor sequences have reads greater than twice the average number of reads for all unique adaptor sequences. In some embodiments, criterion (e) is no more than 10% of unique adaptor sequences have reads greater than twice the average number of reads for all unique adaptor sequences. In some embodiments, criterion (e) is no more than 15% of unique adaptor sequences have reads greater than twice the average number of reads for all unique adaptor sequences. In some embodiments, criterion (e) is no more than 20% of unique adaptor sequences have reads greater than twice the average number of reads for all unique adaptor sequences. In some embodiments, criterion (e) is no more than 25% of unique adaptor sequences have reads greater than twice the average number of reads for all unique adaptor sequences. In some embodiments, criterion (e) is no more than 30% of unique adaptor sequences have reads greater than twice the average number of reads for all unique adaptor sequences. In some embodiments, criterion (e) is no more than 35% of unique adaptor sequences have reads greater than twice the average number of reads for all unique adaptor sequences. In some embodiments, criterion (e) is no more than 40% of unique adaptor sequences have reads greater than twice the average number of reads for all unique adaptor sequences. In some embodiments, criterion (e) is no more than 45% of unique adaptor sequences have reads greater than twice the average number of reads for all unique adaptor sequences. In some embodiments, criterion (e) is no more than 50% of unique adaptor sequences have reads greater than twice the average number of reads for all unique adaptor sequences.

In some embodiments, criterion (e) is no more than about 1% to about 50% of unique adaptor sequences have reads greater than twice the average number of reads for all unique adaptor sequences. In some embodiments, criterion (e) is no more than about 5% to about 50% of unique adaptor sequences have reads greater than twice the average number of reads for all unique adaptor sequences. In some embodiments, criterion (e) is no more than about 5% to about 40% of unique adaptor sequences have reads greater than twice the average number of reads for all unique adaptor sequences. In some embodiments, criterion (e) is no more than about 10% to about 30% of unique adaptor sequences have reads greater than twice the average number of reads for all unique adaptor sequences. In some embodiments, criterion (e) is no more than about 10% to about 20% of unique adaptor sequences have reads greater than twice the average number of reads for all unique adaptor sequences. In some embodiments, criterion (e) is no more than about 20% to about 40% of unique adaptor sequences have reads greater than twice the average number of reads for all unique adaptor sequences. In some embodiments, criterion (e) is no more than about 20% to about 30% of unique adaptor sequences have reads greater than twice the average number of reads for all unique adaptor sequences. In some embodiments, criterion (e) is no more than about 15% to about 25% of unique adaptor sequences have reads greater than twice the average number of reads for all unique adaptor sequences. In some embodiments, criterion (e) is no more than about 15% to about 20% of unique adaptor sequences have reads greater than twice the average number of reads for all unique adaptor sequences.

In some embodiments, criterion (e) is no more than 1% to 50% of unique adaptor sequences have reads greater than twice the average number of reads for all unique adaptor sequences. In some embodiments, criterion (e) is no more than 5% to 50% of unique adaptor sequences have reads greater than twice the average number of reads for all unique adaptor sequences. In some embodiments, criterion (e) is no more than 5% to 40% of unique adaptor sequences have reads greater than twice the average number of reads for all unique adaptor sequences. In some embodiments, criterion (e) is no more than 10% to 30% of unique adaptor sequences have reads greater than twice the average number of reads for all unique adaptor sequences. In some embodiments, criterion (e) is no more than 10% to 20% of unique adaptor sequences have reads greater than twice the average number of reads for all unique adaptor sequences. In some embodiments, criterion (e) is no more than 20% to 40% of unique adaptor sequences have reads greater than twice the average number of reads for all unique adaptor sequences. In some embodiments, criterion (e) is no more than 20% to 30% of unique adaptor sequences have reads greater than twice the average number of reads for all unique adaptor sequences. In some embodiments, criterion (e) is no more than 15% to 25% of unique adaptor sequences have reads greater than twice the average number of reads for all unique adaptor sequences. In some embodiments, criterion (e) is no more than 15% to 20% of unique adaptor sequences have reads greater than twice the average number of reads for all unique adaptor sequences.

In some embodiments of the kits of the disclosure, the pre-determined acceptance criteria for the adaptor QC process comprise a criterion (a) and a criterion (b). In some embodiments, the pre-determined acceptance criteria comprise a criterion (c), a criterion (d), and a criterion (e). In some embodiments, the pre-determined acceptance criteria comprise a criterion (a), a criterion (b), and a criterion (c). In some embodiments, the pre-determined acceptance criteria comprise a criterion (a), a criterion (b), and a criterion (d). In some embodiments, the pre-determined acceptance criteria comprise a criterion (a), a criterion (b), and a criterion (e). In some embodiments, the pre-determined acceptance criteria comprise a criterion (a), a criterion (b), a criterion (c), and a criterion (d). In some embodiments, the pre-determined acceptance criteria comprise a criterion (a), a criterion (b), a criterion (c), and a criterion (e). In some embodiments, the pre-determined acceptance criteria comprise a criterion (a), a criterion (b), a criterion (d), and a criterion (e). In some embodiments, the pre-determined acceptance criteria comprise a criterion (a), a criterion (b), a criterion (c), a criterion (d), and a criterion (e). In some embodiments, the pre-determined acceptance criteria comprise at least one criterion selected from criteria (a) (b) (c) (d) and (e).

In some embodiments of the kits of the disclosure, the set of adaptors is considered to have passed the adaptor QC process when it has passed the test for adaptor distribution.

In some embodiments of the kits of the disclosure, the set of adaptors is considered to have passed the adaptor QC process when it has passed both the test for adaptor ligation and the test for adaptor distribution.

In some embodiments of the kits of the disclosure, the probe QC process comprises a test for capture probe modules comprising (a) ligating a set of adaptors to a DNA sample comprising end-repaired DNA fragments to generate a library of adaptor-tagged DNA fragments (LIBS); (b) amplifying the LIBS to generate a Library Post Amplification (LPA); (c) splitting or diluting the LPA to generate a Target Capture LPA (TC LPA) and a Whole-Genome LPA (WG LPA); (d) amplifying the WG LPA to generate a Whole-Genome Library Amplified (WGLA); (e) hybridizing the one or more capture probe modules to be tested to the TC LPA to form adaptor-tagged DNA fragment-capture probe module complexes; (f) isolating the adaptor-tagged DNA fragment-capture probe module complexes to form isolated adaptor-tagged DNA fragment-capture probe module complexes; (g) enzymatically processing the isolated adaptor-tagged DNA fragment-capture probe module complexes to generate Hybrid Molecules, wherein each Hybrid Molecule comprises the capture probe module and a complement of the adaptor-tagged DNA fragment; (h) amplifying the Hybrid Molecules to generate a Target Capture Library Amplified (TCLA); (i) combining the WGLA and the TCLA to form a Sequence-Ready Library (SRL); (j) performing a quantitative genetic analysis on the SRL.

In some embodiments, the terms LPA and adaptor-tagged Parent Library are used interchangeably. In some embodiments, the terms WGLA and LPWG library are used interchangeably. In some embodiments, the terms SRL and combined library are used interchangeably.

In some embodiments of the kits of the disclosure, the TC LPA is at least about 1 ng/µL. In some embodiments of the kits of the disclosure, the TC LPA is at least about 5 ng/µL. In some embodiments of the kits of the disclosure, the TC LPA is at least about 10 ng/µL. In some embodiments of the kits of the disclosure, the TC LPA is at least about 15 ng/µL. In some embodiments of the kits of the disclosure, the TC LPA is at least about 20 ng/µL. In some embodiments of the kits of the disclosure, the TC LPA is at least about 25 ng/µL. In some embodiments of the kits of the disclosure, the TC LPA is at least about 30 ng/µL. In some embodiments of the kits of the disclosure, the TC LPA is at least about 35 ng/µL. In some embodiments of the kits of the disclosure, the TC LPA is at least about 40 ng/µL. In some embodiments of the kits of the disclosure, the TC LPA is at least about 45 ng/µL. In some embodiments of the kits of the disclosure, the TC LPA is at least about 50 ng/µL. In some embodiments of the kits of the disclosure, the TC LPA is at least about 55 ng/µL. In some embodiments of the kits of the disclosure, the TC LPA is at least about 60 ng/µL. In some embodiments of the kits of the disclosure, the TC LPA is at least about 65 ng/µL. In some embodiments of the kits of the disclosure, the TC LPA is at least about 70 ng/µL. In some embodiments of the kits of the disclosure, the TC LPA is at least about 75 ng/µL. In some embodiments of the kits of the disclosure, the TC LPA is at least about 80 ng/µL.

In some embodiments of the kits of the disclosure, the TC LPA is at least 1 ng/µL. In some embodiments of the kits of the disclosure, the TC LPA is at least 5 ng/µL. In some embodiments of the kits of the disclosure, the TC LPA is at least 10 ng/µL. In some embodiments of the kits of the disclosure, the TC LPA is at least 15 ng/µL. In some embodiments of the kits of the disclosure, the TC LPA is at least 20 ng/µL. In some embodiments of the kits of the disclosure, the TC LPA is at least 25 ng/µL. In some embodiments of the kits of the disclosure, the TC LPA is at least 30 ng/µL. In some embodiments of the kits of the disclosure, the TC LPA is at least 35 ng/µL. In some embodiments of the kits of the disclosure, the TC LPA is at least 40 ng/µL. In some embodiments of the kits of the disclosure, the TC LPA is at least 45 ng/µL. In some embodiments of the kits of the disclosure, the TC LPA is at least 50 ng/µL. In some embodiments of the kits of the disclosure, the TC LPA is at least 55 ng/µL. In some embodiments of the kits of the disclosure, the TC LPA is at least 60 ng/µL. In some embodiments of the kits of the disclosure, the TC LPA is at least 65 ng/µL. In some embodiments of the kits of the disclosure, the TC LPA is at least 70 ng/µL. In some embodiments of the kits of the disclosure, the TC LPA is at least 75 ng/µL. In some embodiments of the kits of the disclosure, the TC LPA is at least 80 ng/µL.

In some embodiments of the kits of the disclosure, the TC LPA is at least about 1 ng/µL to about 100 ng/µL. In some embodiments of the kits of the disclosure, the TC LPA is at least about 1 ng/µL to about 80 ng/µL. In some embodiments of the kits of the disclosure, the TC LPA is at least about 10 ng/µL to about 80 ng/µL. In some embodiments of the kits of the disclosure, the TC LPA is at least about 10 ng/µL to about 70 ng/µL. In some embodiments of the kits of the disclosure, the TC LPA is at least about 10 ng/µL to about 60 ng/µL. In some embodiments of the kits of the disclosure, the TC LPA is at least about 20 ng/µL to about 60 ng/µL. In some embodiments of the kits of the disclosure, the TC LPA is at least about 20 ng/µL to about 50 ng/µL. In some embodiments of the kits of the disclosure, the TC LPA is at least about 20 ng/µL to about 40 ng/µL. In some embodiments of the kits of the disclosure, the TC LPA is at least about 30 ng/µL to about 50 ng/µL. In some embodiments of the kits of the disclosure, the TC LPA is at least about 30 ng/µL to about 40 ng/µL. In some embodiments of the kits of the disclosure, the TC LPA is at least about 20 ng/µL to about 30 ng/µL.

In some embodiments of the kits of the disclosure, the TC LPA is at least 1 ng/µL to 100 ng/µL. In some embodiments of the kits of the disclosure, the TC LPA is at least 1 ng/µL to 80 ng/µL. In some embodiments of the kits of the disclosure, the TC LPA is at least 10 ng/µL to 80 ng/µL. In some embodiments of the kits of the disclosure, the TC LPA is at least 10 ng/µL to 70 ng/µL. In some embodiments of the kits of the disclosure, the TC LPA is at least 10 ng/µL to 60 ng/µL. In some embodiments of the kits of the disclosure, the TC LPA is at least 20 ng/µL to 60 ng/µL. In some embodiments of the kits of the disclosure, the TC LPA is at least 20 ng/µL to 50 ng/µL. In some embodiments of the kits of the disclosure, the TC LPA is at least 20 ng/µL to 40 ng/µL. In some embodiments of the kits of the disclosure, the TC LPA is at least 30 ng/µL to 50 ng/µL. In some embodiments of the kits of the disclosure, the TC LPA is at least 30 ng/µL to 40 ng/µL. In some embodiments of the kits of the disclosure, the TC LPA is at least 20 ng/µL to 30 ng/µL.

In some embodiments of the kits of the disclosure, the WG LPA is at least about 0.1 ng/µL. In some embodiments of the kits of the disclosure, the WG LPA is at least about 0.5 ng/µL. In some embodiments of the kits of the disclosure, the WG LPA is at least about 1 ng/µL. In some embodiments of the kits of the disclosure, the WG LPA is at least about 1.5 ng/µL. In some embodiments of the kits of the disclosure, the WG LPA is at least about 2 ng/µL. In some embodiments of the kits of the disclosure, the WG LPA is at least about 2.5 ng/µL. In some embodiments of the kits of the disclosure, the WG LPA is at least about 3 ng/µL. In some embodiments of the kits of the disclosure, the WG LPA is at least about 3.5 ng/µL. In some embodiments of the kits of the disclosure, the WG LPA is at least about 4 ng/µL. In some embodiments of the kits of the disclosure, the WG LPA is at least about 4.5 ng/µL. In some embodiments of the kits of the disclosure, the WG LPA is at least about 5 ng/µL. In some embodiments of the kits of the disclosure, the WG LPA is at least about 5.5 ng/µL. In some embodiments of the kits of the disclosure, the WG LPA is at least about 6 ng/µL. In some embodiments of the kits of the disclosure, the WG LPA is at least about 6.5 ng/µL. In some embodiments of the kits of the disclosure, the WG LPA is at least about 7 ng/µL. In some embodiments of the kits of the disclosure, the WG LPA is at least about 7.5 ng/µL. In some embodiments of the kits of the disclosure, the WG LPA is at least about 8 ng/µL.

In some embodiments of the kits of the disclosure, the WG LPA is at least 0.1 ng/µL. In some embodiments of the kits of the disclosure, the WG LPA is at least 0.5 ng/µL. In some embodiments of the kits of the disclosure, the WG LPA is at least 1 ng/µL. In some embodiments of the kits of the disclosure, the WG LPA is at least 1.5 ng/µL. In some embodiments of the kits of the disclosure, the WG LPA is at least 2 ng/µL. In some embodiments of the kits of the disclosure, the WG LPA is at least 2.5 ng/µL. In some embodiments of the kits of the disclosure, the WG LPA is at least 3 ng/µL. In some embodiments of the kits of the disclosure, the WG LPA is at least 3.5 ng/µL. In some embodiments of the kits of the disclosure, the WG LPA is at least 4 ng/µL. In some embodiments of the kits of the disclosure, the WG LPA is at least 4.5 ng/µL. In some embodiments of the kits of the disclosure, the WG LPA is at least 5 ng/µL. In some embodiments of the kits of the disclosure, the WG LPA is at least 5.5 ng/µL. In some embodiments of the kits of the disclosure, the WG LPA is at least 6 ng/µL. In some embodiments of the kits of the disclosure, the WG LPA is at least 6.5 ng/µL. In some embodiments of the kits of the disclosure, the WG LPA is at least 7 ng/µL. In some embodiments of the kits of the disclosure, the WG LPA is at least 7.5 ng/µL. In some embodiments of the kits of the disclosure, the WG LPA is at least 8 ng/µL.

In some embodiments of the kits of the disclosure, the WG LPA is at least about 0.1 ng/µL to about 10 ng/µL. In some embodiments of the kits of the disclosure, the WG LPA is at least about 0.1 ng/µL to about 8 ng/µL. In some embodiments of the kits of the disclosure, the WG LPA is at least about 1 ng/µL to about 10 ng/µL. In some embodiments of the kits of the disclosure, the WG LPA is at least about 1 ng/µL to about 5 ng/µL. In some embodiments of the kits of the disclosure, the WG LPA is at least about 2 ng/µL to about 10 ng/µL. In some embodiments of the kits of the disclosure, the WG LPA is at least about 2 ng/µL to about 6 ng/µL. In some embodiments of the kits of the disclosure, the WG LPA is at least about 3 ng/µL to about 5 ng/µL. In some embodiments of the kits of the disclosure, the WG LPA is at least about 3 ng/µL to about 4 ng/µL. In some embodiments of the kits of the disclosure, the WG LPA is at least about 2 ng/µL to about 3 ng/µL.

In some embodiments of the kits of the disclosure, the WG LPA is at least 0.1 ng/µL to 10 ng/µL. In some embodiments of the kits of the disclosure, the WG LPA is at least 0.1 ng/µL to 8 ng/µL. In some embodiments of the kits of the disclosure, the WG LPA is at least 1 ng/µL to 10 ng/µL. In some embodiments of the kits of the disclosure, the WG LPA is at least 1 ng/µL to 5 ng/µL. In some embodiments of the kits of the disclosure, the WG LPA is at least 2 ng/µL to 10 ng/µL. In some embodiments of the kits of the disclosure, the WG LPA is at least 2 ng/µL to 6 ng/µL. In some embodiments of the kits of the disclosure, the WG LPA is at least 3 ng/µL to 5 ng/µL. In some embodiments of the kits of the disclosure, the WG LPA is at least 3 ng/µL to 4 ng/µL. In some embodiments of the kits of the disclosure, the WG LPA is at least 2 ng/µL to 3 ng/µL.

In some embodiments of the kits of the disclosure, the TCLA is at least about 1.0 ng/µL. In some embodiments of the kits of the disclosure, the TCLA is at least about 1.5 ng/µL. In some embodiments of the kits of the disclosure, the TCLA is at least about 2.0 ng/µL. In some embodiments of the kits of the disclosure, the TCLA is at least about 2.5 ng/µL. In some embodiments of the kits of the disclosure, the TCLA is at least about 3.0 ng/µL. In some embodiments of the kits of the disclosure, the TCLA is at least about 3.5 ng/µL. In some embodiments of the kits of the disclosure, the TCLA is at least about 4.0 ng/µL. In some embodiments of the kits of the disclosure, the TCLA is at least about at least 4.5 ng/µL. In some embodiments of the kits of the disclosure, the TCLA is at least about 5.0 ng/µL. In some embodiments of the kits of the disclosure, the TCLA is at least about 5.5 ng/µL. In some embodiments of the kits of the disclosure, the TCLA is at least about 6.0 ng/µL. In some embodiments of the kits of the disclosure, the TCLA is at least about 6.5 ng/µL. In some embodiments of the kits of the disclosure, the TCLA is at least about 7.0 ng/µL. In some embodiments of the kits of the disclosure, the TCLA is at least about 7.5 ng/µL. In some embodiments of the kits of the disclosure, the TCLA is at least about 8.0 ng/µL. In some embodiments of the kits of the disclosure, the TCLA is at least about 8.5 ng/µL. In some embodiments of the kits of the disclosure, the TCLA is at least about 9.0 ng/µL. In some embodiments of the kits of the disclosure, the TCLA is at least about 9.5 ng/µL. In some embodiments of the kits of the disclosure, the TCLA is at least about 10 ng/µL. In some embodiments of the kits of the disclosure, the TCLA is at least about 15 ng/µL. In some embodiments of the kits of the disclosure, the TCLA is at least about 20 ng/µL. In some embodiments of the kits of the disclosure, the TCLA is at least about 25 ng/µL. In some embodiments of the kits of the disclosure, the TCLA is at least about 30 ng/µL. In some embodiments of the kits of the disclosure, the TCLA is at least about 35 ng/µL. In some embodiments of the kits of the disclosure, the TCLA is at least about 40 ng/µL. In some embodiments of the kits of the disclosure, the TCLA is at least about 45 ng/µL. In some embodiments of the kits of the disclosure, the TCLA is at least about 50 ng/µL. In some embodiments of the kits of the disclosure, the TCLA is at least about 55 ng/µL. In some embodiments of the kits of the disclosure, the TCLA is at least about 60 ng/µL. In some embodiments of the kits of the disclosure, the TCLA is at least about 65 ng/µL. In some embodiments of the kits of the disclosure, the TCLA is at least about 70 ng/µL. In some embodiments of the kits of the disclosure, the TCLA is at least about 75 ng/µL. In some embodiments of the kits of the disclosure, the TCLA is at least about 80 ng/µL.

In some embodiments of the kits of the disclosure, the TCLA is at least 1.0 ng/µL. In some embodiments of the kits of the disclosure, the TCLA is at least 1.5 ng/µL. In some embodiments of the kits of the disclosure, the TCLA is at least 2.0 ng/µL. In some embodiments of the kits of the disclosure, the TCLA is at least 2.5 ng/µL. In some embodiments of the kits of the disclosure, the TCLA is at least 3.0 ng/µL. In some embodiments of the kits of the disclosure, the TCLA is at least 3.5 ng/µL. In some embodiments of the kits of the disclosure, the TCLA is at least 4.0 ng/µL. In some embodiments of the kits of the disclosure, the TCLA is at least about at 4.5 ng/µL. In some embodiments of the kits of the disclosure, the TCLA is at least 5.0 ng/µL. In some embodiments of the kits of the disclosure, the TCLA is at least 5.5 ng/µL. In some embodiments of the kits of the disclosure, the TCLA is at least 6.0 ng/µL. In some embodiments of the kits of the disclosure, the TCLA is at least 6.5 ng/µL. In some embodiments of the kits of the disclosure, the TCLA is at least 7.0 ng/µL. In some embodiments of the kits of the disclosure, the TCLA is at least 7.5 ng/µL. In some embodiments of the kits of the disclosure, the TCLA is at least 8.0 ng/µL. In some embodiments of the kits of the disclosure, the TCLA is at least 8.5 ng/µL. In some embodiments of the kits of the disclosure, the TCLA is at least 9.0 ng/µL. In some embodiments of the kits of the disclosure, the TCLA is at least 9.5 ng/µL. In some embodiments of the kits of the disclosure, the TCLA is at least 10 ng/µL. In some embodiments of the kits of the disclosure, the TCLA is at least 15 ng/µL. In some embodiments of the kits of the disclosure, the TCLA is at least 20 ng/µL. In some embodiments of the kits of the disclosure, the TCLA is at least 25 ng/µL. In some embodiments of the kits of the disclosure, the TCLA is at least 30 ng/µL. In some embodiments of the kits of the disclosure, the TCLA is at least 35 ng/µL. In some embodiments of the kits of the disclosure, the TCLA is at least 40 ng/µL. In some embodiments of the kits of the disclosure, the TCLA is at least 45 ng/µL. In some embodiments of the kits of the disclosure, the TCLA is at least 50 ng/µL. In some embodiments of the kits of the disclosure, the TCLA is at least 55 ng/µL. In some embodiments of the kits of the disclosure, the TCLA is at least 60 ng/µL. In some embodiments of the kits of the disclosure, the TCLA is at least 65 ng/µL. In some embodiments of the kits of the disclosure, the TCLA is at least 70 ng/µL. In some embodiments of the kits of the disclosure, the TCLA is at least 75 ng/µL. In some embodiments of the kits of the disclosure, the TCLA is at least 80 ng/µL.

In some embodiments of the kits of the disclosure, the TCLA is at least about 1 ng/µL to about 200 ng/µL. In some embodiments of the kits of the disclosure, the TCLA is at least about 100 ng/µL to about 200 ng/µL. In some embodiments of the kits of the disclosure, the TCLA is at least about 1 ng/µL to about 100 ng/µL. In some embodiments of the kits of the disclosure, the TCLA is at least about 1 ng/µL to about 80 ng/µL. In some embodiments of the kits of the disclosure, the TCLA is at least about 1 ng/µL to about 50 ng/µL. In some embodiments of the kits of the disclosure, the TCLA is at least about 1 ng/µL to about 40 ng/µL. In some embodiments of the kits of the disclosure, the TCLA is at least about 1 ng/µL to about 30 ng/µL. In some embodiments of the kits of the disclosure, the TCLA is at least about 1 ng/µL to about 20 ng/µL. In some embodiments of the kits of the disclosure, the TCLA is at least about 1 ng/µL to about 10 ng/µL. In some embodiments of the kits of the disclosure, the TCLA is at least about 5 ng/µL to about 10 ng/µL.

In some embodiments of the kits of the disclosure, the TCLA is at least 1 ng/µL to 200 ng/µL. In some embodiments of the kits of the disclosure, the TCLA is at least 100 ng/µL to 200 ng/µL. In some embodiments of the kits of the disclosure, the TCLA is at least 1 ng/µL to 100 ng/µL. In some embodiments of the kits of the disclosure, the TCLA is at least 1 ng/µL to 80 ng/µL. In some embodiments of the kits of the disclosure, the TCLA is at least 1 ng/µL to 50 ng/µL. In some embodiments of the kits of the disclosure, the TCLA is at least 1 ng/µL to 40 ng/µL. In some embodiments of the kits of the disclosure, the TCLA is at least 1 ng/µL to 30 ng/µL. In some embodiments of the kits of the disclosure, the TCLA is at least 1 ng/µL to 20 ng/µL. In some embodiments of the kits of the disclosure, the TCLA is at least 1 ng/µL to 10 ng/µL. In some embodiments of the kits of the disclosure, the TCLA is at least 5 ng/µL to 10 ng/µL.

In some embodiments of the kits of the disclosure, the WGLA is at least about 1.0 ng/µL. In some embodiments of the kits of the disclosure, the WGLA is at least about 1.5 ng/µL. In some embodiments of the kits of the disclosure, the WGLA is at least about 2.0 ng/µL. In some embodiments of the kits of the disclosure, the WGLA is at least about 2.5 ng/µL. In some embodiments of the kits of the disclosure, the WGLA is at least about 3.0 ng/µL. In some embodiments of the kits of the disclosure, the WGLA is at least about 3.5 ng/µL. In some embodiments of the kits of the disclosure, the WGLA is at least about 4.0 ng/µL. In some embodiments of the kits of the disclosure, the WGLA is at least about at least 4.5 ng/µL. In some embodiments of the kits of the disclosure, the WGLA is at least about 5.0 ng/µL. In some embodiments of the kits of the disclosure, the WGLA is at least about 5.5 ng/µL. In some embodiments of the kits of the disclosure, the WGLA is at least about 6.0 ng/µL. In some embodiments of the kits of the disclosure, the WGLA is at least about 6.5 ng/µL. In some embodiments of the kits of the disclosure, the WGLA is at least about 7.0 ng/µL. In some embodiments of the kits of the disclosure, the WGLA is at least about 7.5 ng/µL. In some embodiments of the kits of the disclosure, the WGLA is at least about 8.0 ng/µL. In some embodiments of the kits of the disclosure, the WGLA is at least about 8.5 ng/µL. In some embodiments of the kits of the disclosure, the WGLA is at least about 9.0 ng/µL. In some embodiments of the kits of the disclosure, the WGLA is at least about 9.5 ng/µL. In some embodiments of the kits of the disclosure, the WGLA is at least about 10 ng/µL. In some embodiments of the kits of the disclosure, the WGLA is at least about 15 ng/µL. In some embodiments of the kits of the disclosure, the WGLA is at least about 20 ng/µL. In some embodiments of the kits of the disclosure, the WGLA is at least about 25 ng/µL. In some embodiments of the kits of the disclosure, the WGLA is at least about 30 ng/µL. In some embodiments of the kits of the disclosure, the WGLA is at least about 35 ng/µL. In some embodiments of the kits of the disclosure, the WGLA is at least about 40 ng/µL. In some embodiments of the kits of the disclosure, the WGLA is at least about 45 ng/µL. In some embodiments of the kits of the disclosure, the WGLA is at least about 50 ng/µL. In some embodiments of the kits of the disclosure, the WGLA is at least about 55 ng/µL. In some embodiments of the kits of the disclosure, the WGLA is at least about 60 ng/µL. In some embodiments of the kits of the disclosure, the WGLA is at least about 65 ng/µL. In some embodiments of the kits of the disclosure, the WGLA is at least about 70 ng/µL. In some embodiments of the kits of the disclosure, the WGLA is at least about 75 ng/µL. In some embodiments of the kits of the disclosure, the WGLA is at least about 80 ng/µL.

In some embodiments of the kits of the disclosure, the WGLA is at least 1.0 ng/µL. In some embodiments of the kits of the disclosure, the WGLA is at least 1.5 ng/µL. In some embodiments of the kits of the disclosure, the WGLA is at least 2.0 ng/µL. In some embodiments of the kits of the disclosure, the WGLA is at least 2.5 ng/µL. In some embodiments of the kits of the disclosure, the WGLA is at least 3.0 ng/µL. In some embodiments of the kits of the disclosure, the WGLA is at least 3.5 ng/µL. In some embodiments of the kits of the disclosure, the WGLA is at least 4.0 ng/µL. In some embodiments of the kits of the disclosure, the WGLA is at least about at 4.5 ng/µL. In some embodiments of the kits of the disclosure, the WGLA is at least 5.0 ng/µL. In some embodiments of the kits of the disclosure, the WGLA is at least 5.5 ng/µL. In some embodiments of the kits of the disclosure, the WGLA is at least 6.0 ng/µL. In some embodiments of the kits of the disclosure, the WGLA is at least 6.5 ng/µL. In some embodiments of the kits of the disclosure, the WGLA is at least 7.0 ng/µL. In some embodiments of the kits of the disclosure, the WGLA is at least 7.5 ng/µL. In some embodiments of the kits of the disclosure, the WGLA is at least 8.0 ng/µL. In some embodiments of the kits of the disclosure, the WGLA is at least 8.5 ng/µL. In some embodiments of the kits of the disclosure, the WGLA is at least 9.0 ng/µL. In some embodiments of the kits of the disclosure, the WGLA is at least 9.5 ng/µL. In some embodiments of the kits of the disclosure, the WGLA is at least 10 ng/µL. In some embodiments of the kits of the disclosure, the WGLA is at least 15 ng/µL. In some embodiments of the kits of the disclosure, the WGLA is at least 20 ng/µL. In some embodiments of the kits of the disclosure, the WGLA is at least 25 ng/µL. In some embodiments of the kits of the disclosure, the WGLA is at least 30 ng/µL. In some embodiments of the kits of the disclosure, the WGLA is at least 35 ng/µL. In some embodiments of the kits of the disclosure, the WGLA is at least 40 ng/µL. In some embodiments of the kits of the disclosure, the WGLA is at least 45 ng/µL. In some embodiments of the kits of the disclosure, the WGLA is at least 50 ng/µL. In some embodiments of the kits of the disclosure, the WGLA is at least 55 ng/µL. In some embodiments of the kits of the disclosure, the WGLA is at least 60 ng/µL. In some embodiments of the kits of the disclosure, the WGLA is at least 65 ng/µL. In some embodiments of the kits of the disclosure, the WGLA is at least 70 ng/µL. In some embodiments of the kits of the disclosure, the WGLA is at least 75 ng/µL. In some embodiments of the kits of the disclosure, the WGLA is at least 80 ng/µL.

In some embodiments of the kits of the disclosure, the WGLA is at least about 1 ng/µL to about 200 ng/µL. In some embodiments of the kits of the disclosure, the WGLA is at least about 100 ng/µL to about 200 ng/µL. In some embodiments of the kits of the disclosure, the WGLA is at least about 1 ng/µL to about 100 ng/µL. In some embodiments of the kits of the disclosure, the WGLA is at least about 1 ng/µL to about 80 ng/µL. In some embodiments of the kits of the disclosure, the WGLA is at least about 1 ng/µL to about 50 ng/µL. In some embodiments of the kits of the disclosure, the WGLA is at least about 1 ng/µL to about 40 ng/µL. In some embodiments of the kits of the disclosure, the WGLA is at least about 1 ng/µL to about 30 ng/µL. In some embodiments of the kits of the disclosure, the WGLA is at least about 1 ng/µL to about 20 ng/µL. In some embodiments of the kits of the disclosure, the WGLA is at least about 1 ng/µL to about 10 ng/µL. In some embodiments of the kits of the disclosure, the WGLA is at least about 5 ng/µL to about 10 ng/µL.

In some embodiments of the kits of the disclosure, the WGLA is at least 1 ng/µL to 200 ng/µL. In some embodiments of the kits of the disclosure, the WGLA is at least 100 ng/µL to 200 ng/µL. In some embodiments of the kits of the disclosure, the WGLA is at least 1 ng/µL to 100 ng/µL. In some embodiments of the kits of the disclosure, the WGLA is at least 1 ng/µL to 80 ng/µL. In some embodiments of the kits of the disclosure, the WGLA is at least 1 ng/µL to 50 ng/µL. In some embodiments of the kits of the disclosure, the WGLA is at least 1 ng/µL to 40 ng/µL. In some embodiments of the kits of the disclosure, the WGLA is at least 1 ng/µL to 30 ng/µL. In some embodiments of the kits of the disclosure, the WGLA is at least 1 ng/µL to 20 ng/µL. In some embodiments of the kits of the disclosure, the WGLA is at least 1 ng/µL to 10 ng/µL. In some embodiments of the kits of the disclosure, the WGLA is at least 5 ng/µL to 10 ng/µL.

In some embodiments of the kits of the disclosure, the one or more capture probe modules are considered to have passed the probe QC process if one or more pre-determined acceptance criteria for the quantitative genetic analysis has been met.

In some embodiments of the kits of the disclosure, the one or more capture probe modules are considered to have passed the probe QC process if one or more pre-determined acceptance criteria for the quantitative genetic analysis have been met.

In some embodiments of the kits of the disclosure, the pre-determined acceptance criteria for the probe QC process comprise a criterion (a). In some embodiments, criterion (a) is about 100% of capture probes have at least 1 total reads. In some embodiments, criterion (a) is at least about 99.9% of capture probes have at least 1 total reads. In some embodiments, criterion (a) is at least about 99.8% of capture probes have at least 1 total reads. In some embodiments, criterion (a) is at least about 99.7% of capture probes have at least 1 total reads. In some embodiments, criterion (a) is at least about 99.6% of capture probes have at least 1 total reads. In some embodiments, criterion (a) is at least about 99.5% of capture probes have at least 1 total reads. In some embodiments, criterion (a) is at least about 99.4% of capture probes have at least 1 total reads. In some embodiments, criterion (a) is at least about 99.3% of capture probes have at least 1 total reads. In some embodiments, criterion (a) is at least about 99.2% of capture probes have at least 1 total reads. In some embodiments, criterion (a) is at least about 99.1% of capture probes have at least 1 total reads. In some embodiments, criterion (a) is at least about 99% of capture probes have at least 1 total reads. In some embodiments, criterion (a) is at least about 98.9% of capture probes have at least 1 total reads. In some embodiments, criterion (a) is at least about 98.8% of capture probes have at least 1 total reads. In some embodiments, criterion (a) is at least about 98.7% of capture probes have at least 1 total reads. In some embodiments, criterion (a) is at least about 98.6% of capture probes have at least 1 total reads. In some embodiments, criterion (a) is at least about 98.5% of capture probes have at least 1 total reads. In some embodiments, criterion (a) is at least about 98.4% of capture probes have at least 1 total reads. In some embodiments, criterion (a) is at least about 98.3% of capture probes have at least 1 total reads. In some embodiments, criterion (a) is at least about 98.2% of capture probes have at least 1 total reads. In some embodiments, criterion (a) is at least about 98.1% of capture probes have at least 1 total reads. In some embodiments, criterion (a) is at least about 98% of capture probes have at least 1 total reads. In some embodiments, criterion (a) is at least about 97% of capture probes have at least 1 total reads. In some embodiments, criterion (a) is at least about 96% of capture probes have at least 1 total reads. In some embodiments of the kits of the disclosure, criterion (a) is at least about 95% of capture probes have at least 1 total reads. In some embodiments of the kits of the disclosure, criterion (a) is at least about 90% of capture probes have at least 1 total reads. In some embodiments of the kits of the disclosure, criterion (a) is at least about 85% of capture probes have at least 1 total reads. In some embodiments of the kits of the disclosure, criterion (a) is at least about 80% of capture probes have at least 1 total reads. In some embodiments of the kits of the disclosure, criterion (a) is at least about 75% of capture probes have at least 1 total reads. In some embodiments of the kits of the disclosure, criterion (a) is at least about 70% of capture probes have at least 1 total reads. In some embodiments of the kits of the disclosure, criterion (a) is at least about 65% of capture probes have at least 1 total reads. In some embodiments of the kits of the disclosure, criterion (a) is at least about 60% of capture probes have at least 1 total reads.

In some embodiments, criterion (a) is 100% of capture probes have at least 1 total reads. In some embodiments, criterion (a) is at least 99.9% of capture probes have at least 1 total reads. In some embodiments, criterion (a) is at least 99.8% of capture probes have at least 1 total reads. In some embodiments, criterion (a) is at least 99.7% of capture probes have at least 1 total reads. In some embodiments, criterion (a) is at least 99.6% of capture probes have at least 1 total reads. In some embodiments, criterion (a) is at least 99.5% of capture probes have at least 1 total reads. In some embodiments, criterion (a) is at least 99.4% of capture probes have at least 1 total reads. In some embodiments, criterion (a) is at least 99.3% of capture probes have at least 1 total reads. In some embodiments, criterion (a) is at least 99.2% of capture probes have at least 1 total reads. In some embodiments, criterion (a) is at least 99.1% of capture probes have at least 1 total reads. In some embodiments, criterion (a) is at least 99% of capture probes have at least 1 total reads. In some embodiments, criterion (a) is at least 98.9% of capture probes have at least 1 total reads. In some embodiments, criterion (a) is at least 98.8% of capture probes have at least 1 total reads. In some embodiments, criterion (a) is at least 98.7% of capture probes have at least 1 total reads. In some embodiments, criterion (a) is at least 98.6% of capture probes have at least 1 total reads. In some embodiments, criterion (a) is at least 98.5% of capture probes have at least 1 total reads. In some embodiments, criterion (a) is at least 98.4% of capture probes have at least 1 total reads. In some embodiments, criterion (a) is at least 98.3% of capture probes have at least 1 total reads. In some embodiments, criterion (a) is at least 98.2% of capture probes have at least 1 total reads. In some embodiments, criterion (a) is at least 98.1% of capture probes have at least 1 total reads. In some embodiments, criterion (a) is at least 98% of capture probes have at least 1 total reads. In some embodiments, criterion (a) is at least 97% of capture probes have at least 1 total reads. In some embodiments, criterion (a) is at least 96% of capture probes have at least 1 total reads. In some embodiments of the kits of the disclosure, criterion (a) is at least 95% of capture probes have at least 1 total reads. In some embodiments of the kits of the disclosure, criterion (a) is at least 90% of capture probes have at least 1 total reads. In some embodiments of the kits of the disclosure, criterion (a) is at least 85% of capture probes have at least 1 total reads. In some embodiments of the kits of the disclosure, criterion (a) is at least 80% of capture probes have at least 1 total reads. In some embodiments of the kits of the disclosure, criterion (a) is at least 75% of capture probes have at least 1 total reads. In some embodiments of the kits of the disclosure, criterion (a) is at least 70% of capture probes have at least 1 total reads. In some embodiments of the kits of the disclosure, criterion (a) is at least 65% of capture probes have at least 1 total reads. In some embodiments of the kits of the disclosure, criterion (a) is at least 60% of capture probes have at least 1 total reads.

In some embodiments, criterion (a) is at least about 60% to about 99.9% of capture probes have at least 1 total reads. In some embodiments, criterion (a) is at least about 70% to about 99.9% of capture probes have at least 1 total reads. In some embodiments, criterion (a) is at least about 80% to about 99.9% of capture probes have at least 1 total reads. In some embodiments, criterion (a) is at least about 90% to about 99.9% of capture probes have at least 1 total reads. In some embodiments, criterion (a) is at least about 95% to about 99.9% of capture probes have at least 1 total reads. In some embodiments, criterion (a) is at least about 96% to about 99.9% of capture probes have at least 1 total reads. In some embodiments, criterion (a) is at least about 97% to about 99.9% of capture probes have at least 1 total reads. In some embodiments, criterion (a) is at least about 98% to about 99.9% of capture probes have at least 1 total reads. In some embodiments, criterion (a) is at least about 99% to about 99.9% of capture probes have at least 1 total reads. In some embodiments, criterion (a) is at least about 99% to about 100% of capture probes have at least 1 total reads.

In some embodiments, criterion (a) is at least 60% to 99.9% of capture probes have at least 1 total reads. In some embodiments, criterion (a) is at least 70% to 99.9% of capture probes have at least 1 total reads. In some embodiments, criterion (a) is at least 80% to 99.9% of capture probes have at least 1 total reads. In some embodiments, criterion (a) is at least 90% to 99.9% of capture probes have at least 1 total reads. In some embodiments, criterion (a) is at least 95% to 99.9% of capture probes have at least 1 total reads. In some embodiments, criterion (a) is at least 96% to 99.9% of capture probes have at least 1 total reads. In some embodiments, criterion (a) is at least 97% to 99.9% of capture probes have at least 1 total reads. In some embodiments, criterion (a) is at least 98% to 99.9% of capture probes have at least 1 total reads. In some embodiments, criterion (a) is at least 99% to 99.9% of capture probes have at least 1 total reads. In some embodiments, criterion (a) is at least 99% to 100% of capture probes have at least 1 total reads.

In some embodiments, criterion (a) is at least about 60% to about 100% of capture probes have at least 1 total reads. In some embodiments, criterion (a) is at least about 70% to about 100% of capture probes have at least 1 total reads. In some embodiments, criterion (a) is at least about 80% to about 100% of capture probes have at least 1 total reads. In some embodiments, criterion (a) is at least about 90% to about 100% of capture probes have at least 1 total reads. In some embodiments, criterion (a) is at least about 95% to about 100% of capture probes have at least 1 total reads. In some embodiments, criterion (a) is at least about 96% to about 100% of capture probes have at least 1 total reads. In some embodiments, criterion (a) is at least about 97% to about 100% of capture probes have at least 1 total reads. In some embodiments, criterion (a) is at least about 98% to about 100% of capture probes have at least 1 total reads. In some embodiments, criterion (a) is at least about 99% to about 100% of capture probes have at least 1 total reads. In some embodiments, criterion (a) is at least about 99.5% to about 100% of capture probes have at least 1 total reads. In some embodiments, criterion (a) is at least about 99.9% to about 100% of capture probes have at least 1 total reads.

In some embodiments, criterion (a) is at least 60% to 100% of capture probes have at least 1 total reads. In some embodiments, criterion (a) is at least 70% to 100% of capture probes have at least 1 total reads. In some embodiments, criterion (a) is at least 80% to 100% of capture probes have at least 1 total reads. In some embodiments, criterion (a) is at least 90% to 100% of capture probes have at least 1 total reads. In some embodiments, criterion (a) is at least 95% to 100% of capture probes have at least 1 total reads. In some embodiments, criterion (a) is at least 96% to 100% of capture probes have at least 1 total reads. In some embodiments, criterion (a) is at least 97% to 100% of capture probes have at least 1 total reads. In some embodiments, criterion (a) is at least 98% to 100% of capture probes have at least 1 total reads. In some embodiments, criterion (a) is at least 99% to 100% of capture probes have at least 1 total reads. In some embodiments, criterion (a) is at least 99.5% to 100% of capture probes have at least 1 total reads. In some embodiments, criterion (a) is at least 99.9% to 100% of capture probes have at least 1 total reads.

In some embodiments of the kits of the disclosure, the pre-determined acceptance criteria for the probe QC process comprise a criterion (b). In some embodiments, criterion (b) is about 100% of capture probes have at least 10-200 on-target total reads. In some embodiments, criterion (b) is at least about 99.9% of capture probes have at least 10-200 on-target total reads. In some embodiments, criterion (b) is at least about 99.5% of capture probes have at least 10-200 on-target total reads. In some embodiments, criterion (b) is at least about 99% of capture probes have at least 10-200 on-target total reads. In some embodiments, criterion (b) is at least about 98.5% of capture probes have at least 10-200 on-target total reads. In some embodiments, criterion (b) is at least about 98% of capture probes have at least 10-200 on-target total reads. In some embodiments, criterion (b) is at least about 97.5% of capture probes have at least 10-200 on-target total reads. In some embodiments, criterion (b) is at least about 97% of capture probes have at least 10-200 on-target total reads. In some embodiments, criterion (b) is at least about 96.5% of capture probes have at least 10-200 on-target total reads. In some embodiments, criterion (b) is at least about 96% of capture probes have at least 10-200 on-target total reads. In some embodiments, criterion (b) is at least about 95.5% of capture probes have at least 10-200 on-target total reads. In some embodiments, criterion (b) is at least about 95% of capture probes have at least 10-200 on-target total reads. In some embodiments, the pre-determined acceptance criteria for the probe QC process comprise criterion (b) at least about 90% of capture probes have at least 10-200 on-target total reads. In some embodiments, criterion (b) is at least about 85% of capture probes have at least 10-200 on-target total reads. In some embodiments, criterion (b) is at least about 80% of capture probes have at least 10-200 on-target total reads. In some embodiments, criterion (b) is at least about 75% of capture probes have at least 10-200 on-target total reads. In some embodiments, criterion (b) is at least about 70% of capture probes have at least 10-200 on-target total reads. In some embodiments, criterion (b) is at least about 65% of capture probes have at least 10-200 on-target total reads. In some embodiments, criterion (b) is at least about 60% of capture probes have at least 10-200 on-target total reads.

In some embodiments, criterion (b) is 100% of capture probes have at least 10-200 on-target total reads. In some embodiments, criterion (b) is at least 99.9% of capture probes have at least 10-200 on-target total reads. In some embodiments, criterion (b) is at least 99.5% of capture probes have at least 10-200 on-target total reads. In some embodiments, criterion (b) is at least 99% of capture probes have at least 10-200 on-target total reads. In some embodiments, criterion (b) is at least 98.5% of capture probes have at least 10-200 on-target total reads. In some embodiments, criterion (b) is at least 98% of capture probes have at least 10-200 on-target total reads. In some embodiments, criterion (b) is at least 97.5% of capture probes have at least 10-200 on-target total reads. In some embodiments, criterion (b) is at least 97% of capture probes have at least 10-200 on-target total reads. In some embodiments, criterion (b) is at least 96.5% of capture probes have at least 10-200 on-target total reads. In some embodiments, criterion (b) is at least 96% of capture probes have at least 10-200 on-target total reads. In some embodiments, criterion (b) is at least 95.5% of capture probes have at least 10-200 on-target total reads. In some embodiments, criterion (b) is at least 95% of capture probes have at least 10-200 on-target total reads. In some embodiments of the kits of the disclosure, the pre-determined acceptance criteria for the probe QC process comprise criterion (b) at least 90% of capture probes have at least 10-200 on-target total reads. In some embodiments, criterion (b) is at least 85% of capture probes have at least 10-200 on-target total reads. In some embodiments, criterion (b) is at least 80% of capture probes have at least 10-200 on-target total reads. In some embodiments, criterion (b) is at least 75% of capture probes have at least 10-200 on-target total reads. In some embodiments, criterion (b) is at least 70% of capture probes have at least 10-200 on-target total reads. In some embodiments, criterion (b) is at least 65% of capture probes have at least 10-200 on-target total reads. In some embodiments, criterion (b) is at least 60% of capture probes have at least 10-200 on-target total reads.

In some embodiments, criterion (b) is about 100% of capture probes have at least 50 on-target total reads. In some embodiments, criterion (b) is at least about 99.9% of capture probes have at least 50 on-target total reads. In some embodiments, criterion (b) is at least about 99.5% of capture probes have at least 50 on-target total reads. In some embodiments, criterion (b) is at least about 99% of capture probes have at least 50 on-target total reads. In some embodiments, criterion (b) is at least about 98.5% of capture probes have at least 50 on-target total reads. In some embodiments, criterion (b) is at least about 98% of capture probes have at least 50 on-target total reads. In some embodiments, criterion (b) is at least about 97.5% of capture probes have at least 50 on-target total reads. In some embodiments, criterion (b) is at least about 97% of capture probes have at least 50 on-target total reads. In some embodiments, criterion (b) is at least about 96.5% of capture probes have at least 50 on-target total reads. In some embodiments, criterion (b) is at least about 96% of capture probes have at least 50 on-target total reads. In some embodiments, criterion (b) is at least about 95.5% of capture probes have at least 50 on-target total reads. In some embodiments, criterion (b) is at least about 95% of capture probes have at least 50 on-target total reads. In some embodiments, the pre-determined acceptance criteria for the probe QC process comprise criterion (b) at least about 90% of capture probes have at least 50 on-target total reads. In some embodiments, criterion (b) is at least about 85% of capture probes have at least 50 on-target total reads. In some embodiments, criterion (b) is at least about 80% of capture probes have at least 50 on-target total reads. In some embodiments, criterion (b) is at least about 75% of capture probes have at least 50 on-target total reads. In some embodiments, criterion (b) is at least about 70% of capture probes have at least 50 on-target total reads. In some embodiments, criterion (b) is at least about 65% of capture probes have at least 50 on-target total reads. In some embodiments, criterion (b) is at least about 60% of capture probes have at least 50 on-target total reads.

In some embodiments, criterion (b) is 100% of capture probes have at least 50 on-target total reads. In some embodiments, criterion (b) is at least 99.9% of capture probes have at least 50 on-target total reads. In some embodiments, criterion (b) is at least 99.5% of capture probes have at least 50 on-target total reads. In some embodiments, criterion (b) is at least 99% of capture probes have at least 50 on-target total reads. In some embodiments, criterion (b) is at least 98.5% of capture probes have at least 50 on-target total reads. In some embodiments, criterion (b) is at least 98% of capture probes have at least 50 on-target total reads. In some embodiments, criterion (b) is at least 97.5% of capture probes have at least 50 on-target total reads. In some embodiments, criterion (b) is at least 97% of capture probes have at least 50 on-target total reads. In some embodiments, criterion (b) is at least 96.5% of capture probes have at least 50 on-target total reads. In some embodiments, criterion (b) is at least 96% of capture probes have at least 50 on-target total reads. In some embodiments, criterion (b) is at least 95.5% of capture probes have at least 50 on-target total reads. In some embodiments, criterion (b) is at least 95% of capture probes have at least 50 on-target total reads. In some embodiments of the kits of the disclosure, the pre-determined acceptance criteria for the probe QC process comprise criterion (b) at least 90% of capture probes have at least 50 on-target total reads. In some embodiments, criterion (b) is at least 85% of capture probes have at least 50 on-target total reads. In some embodiments, criterion (b) is at least 80% of capture probes have at least 50 on-target total reads. In some embodiments, criterion (b) is at least 75% of capture probes have at least 50 on-target total reads. In some embodiments, criterion (b) is at least 70% of capture probes have at least 50 on-target total reads. In some embodiments, criterion (b) is at least 65% of capture probes have at least 50 on-target total reads. In some embodiments, criterion (b) is at least 60% of capture probes have at least 50 on-target total reads.

In some embodiments, criterion (b) is about 100% of capture probes have at least 60 on-target total reads. In some embodiments, criterion (b) is at least about 99.9% of capture probes have at least 60 on-target total reads. In some embodiments, criterion (b) is at least about 99.5% of capture probes have at least 60 on-target total reads. In some embodiments, criterion (b) is at least about 99% of capture probes have at least 60 on-target total reads. In some embodiments, criterion (b) is at least about 98.5% of capture probes have at least 60 on-target total reads. In some embodiments, criterion (b) is at least about 98% of capture probes have at least 60 on-target total reads. In some embodiments, criterion (b) is at least about 97.5% of capture probes have at least 60 on-target total reads. In some embodiments, criterion (b) is at least about 97% of capture probes have at least 60 on-target total reads. In some embodiments, criterion (b) is at least about 96.5% of capture probes have at least 60 on-target total reads. In some embodiments, criterion (b) is at least about 96% of capture probes have at least 60 on-target total reads. In some embodiments, criterion (b) is at least about 95.5% of capture probes have at least 60 on-target total reads. In some embodiments, criterion (b) is at least about 95% of capture probes have at least 60 on-target total reads. In some embodiments, the pre-determined acceptance criteria for the probe QC process comprise criterion (b) at least about 90% of capture probes have at least 60 on-target total reads. In some embodiments, criterion (b) is at least about 85% of capture probes have at least 60 on-target total reads. In some embodiments, criterion (b) is at least about 80% of capture probes have at least 60 on-target total reads. In some embodiments, criterion (b) is at least about 75% of capture probes have at least 60 on-target total reads. In some embodiments, criterion (b) is at least about 70% of capture probes have at least 60 on-target total reads. In some embodiments, criterion (b) is at least about 65% of capture probes have at least 60 on-target total reads. In some embodiments, criterion (b) is at least about 60% of capture probes have at least 60 on-target total reads.

In some embodiments, criterion (b) is 100% of capture probes have at least 60 on-target total reads. In some embodiments, criterion (b) is at least 99.9% of capture probes have at least 60 on-target total reads. In some embodiments, criterion (b) is at least 99.5% of capture probes have at least 60 on-target total reads. In some embodiments, criterion (b) is at least 99% of capture probes have at least 60 on-target total reads. In some embodiments, criterion (b) is at least 98.5% of capture probes have at least 60 on-target total reads. In some embodiments, criterion (b) is at least 98% of capture probes have at least 60 on-target total reads. In some embodiments, criterion (b) is at least 97.5% of capture probes have at least 60 on-target total reads. In some embodiments, criterion (b) is at least 97% of capture probes have at least 60 on-target total reads. In some embodiments, criterion (b) is at least 96.5% of capture probes have at least 60 on-target total reads. In some embodiments, criterion (b) is at least 96% of capture probes have at least 60 on-target total reads. In some embodiments, criterion (b) is at least 95.5% of capture probes have at least 60 on-target total reads. In some embodiments, criterion (b) is at least 95% of capture probes have at least 60 on-target total reads. In some embodiments of the kits of the disclosure, the pre-determined acceptance criteria for the probe QC process comprise criterion (b) at least 90% of capture probes have at least 60 on-target total reads. In some embodiments, criterion (b) is at least 85% of capture probes have at least 60 on-target total reads. In some embodiments, criterion (b) is at least 80% of capture probes have at least 60 on-target total reads. In some embodiments, criterion (b) is at least 75% of capture probes have at least 60 on-target total reads. In some embodiments, criterion (b) is at least 70% of capture probes have at least 60 on-target total reads. In some embodiments, criterion (b) is at least 65% of capture probes have at least 60 on-target total reads. In some embodiments, criterion (b) is at least 60% of capture probes have at least 60 on-target total reads.

In some embodiments, criterion (b) is about 100% of capture probes have at least 70 on-target total reads. In some embodiments, criterion (b) is at least about 99.9% of capture probes have at least 70 on-target total reads. In some embodiments, criterion (b) is at least about 99.5% of capture probes have at least 70 on-target total reads. In some embodiments, criterion (b) is at least about 99% of capture probes have at least 70 on-target total reads. In some embodiments, criterion (b) is at least about 98.5% of capture probes have at least 70 on-target total reads. In some embodiments, criterion (b) is at least about 98% of capture probes have at least 70 on-target total reads. In some embodiments, criterion (b) is at least about 97.5% of capture probes have at least 70 on-target total reads. In some embodiments, criterion (b) is at least about 97% of capture probes have at least 70 on-target total reads. In some embodiments, criterion (b) is at least about 96.5% of capture probes have at least 70 on-target total reads. In some embodiments, criterion (b) is at least about 96% of capture probes have at least 70 on-target total reads. In some embodiments, criterion (b) is at least about 95.5% of capture probes have at least 70 on-target total reads. In some embodiments, criterion (b) is at least about 95% of capture probes have at least 70 on-target total reads. In some embodiments, the pre-determined acceptance criteria for the probe QC process comprise criterion (b) at least about 90% of capture probes have at least 70 on-target total reads. In some embodiments, criterion (b) is at least about 85% of capture probes have at least 70 on-target total reads. In some embodiments, criterion (b) is at least about 80% of capture probes have at least 70 on-target total reads. In some embodiments, criterion (b) is at least about 75% of capture probes have at least 70 on-target total reads. In some embodiments, criterion (b) is at least about 70% of capture probes have at least 70 on-target total reads. In some embodiments, criterion (b) is at least about 65% of capture probes have at least 70 on-target total reads. In some embodiments, criterion (b) is at least about 60% of capture probes have at least 70 on-target total reads.

In some embodiments, criterion (b) is 100% of capture probes have at least 70 on-target total reads. In some embodiments, criterion (b) is at least 99.9% of capture probes have at least 70 on-target total reads. In some embodiments, criterion (b) is at least 99.5% of capture probes have at least 70 on-target total reads. In some embodiments, criterion (b) is at least 99% of capture probes have at least 70 on-target total reads. In some embodiments, criterion (b) is at least 98.5% of capture probes have at least 70 on-target total reads. In some embodiments, criterion (b) is at least 98% of capture probes have at least 70 on-target total reads. In some embodiments, criterion (b) is at least 97.5% of capture probes have at least 70 on-target total reads. In some embodiments, criterion (b) is at least 97% of capture probes have at least 70 on-target total reads. In some embodiments, criterion (b) is at least 96.5% of capture probes have at least 70 on-target total reads. In some embodiments, criterion (b) is at least 96% of capture probes have at least 70 on-target total reads. In some embodiments, criterion (b) is at least 95.5% of capture probes have at least 70 on-target total reads. In some embodiments, criterion (b) is at least 95% of capture probes have at least 70 on-target total reads. In some embodiments of the kits of the disclosure, the pre-determined acceptance criteria for the probe QC process comprise criterion (b) at least 90% of capture probes have at least 70 on-target total reads. In some embodiments, criterion (b) is at least 85% of capture probes have at least 70 on-target total reads. In some embodiments, criterion (b) is at least 80% of capture probes have at least 70 on-target total reads. In some embodiments, criterion (b) is at least 75% of capture probes have at least 70 on-target total reads. In some embodiments, criterion (b) is at least 70% of capture probes have at least 70 on-target total reads. In some embodiments, criterion (b) is at least 65% of capture probes have at least 70 on-target total reads. In some embodiments, criterion (b) is at least 60% of capture probes have at least 70 on-target total reads.

In some embodiments, criterion (b) is about 100% of capture probes have at least 80 on-target total reads. In some embodiments, criterion (b) is at least about 99.9% of capture probes have at least 80 on-target total reads. In some embodiments, criterion (b) is at least about 99.5% of capture probes have at least 80 on-target total reads. In some embodiments, criterion (b) is at least about 99% of capture probes have at least 80 on-target total reads. In some embodiments, criterion (b) is at least about 98.5% of capture probes have at least 80 on-target total reads. In some embodiments, criterion (b) is at least about 98% of capture probes have at least 80 on-target total reads. In some embodiments, criterion (b) is at least about 97.5% of capture probes have at least 80 on-target total reads. In some embodiments, criterion (b) is at least about 97% of capture probes have at least 80 on-target total reads. In some embodiments, criterion (b) is at least about 96.5% of capture probes have at least 80 on-target total reads. In some embodiments, criterion (b) is at least about 96% of capture probes have at least 80 on-target total reads. In some embodiments, criterion (b) is at least about 95.5% of capture probes have at least 80 on-target total reads. In some embodiments, criterion (b) is at least about 95% of capture probes have at least 80 on-target total reads. In some embodiments, the pre-determined acceptance criteria for the probe QC process comprise criterion (b) at least about 90% of capture probes have at least 80 on-target total reads. In some embodiments, criterion (b) is at least about 85% of capture probes have at least 80 on-target total reads. In some embodiments, criterion (b) is at least about 80% of capture probes have at least 80 on-target total reads. In some embodiments, criterion (b) is at least about 75% of capture probes have at least 80 on-target total reads. In some embodiments, criterion (b) is at least about 70% of capture probes have at least 80 on-target total reads. In some embodiments, criterion (b) is at least about 65% of capture probes have at least 80 on-target total reads. In some embodiments, criterion (b) is at least about 60% of capture probes have at least 80 on-target total reads.

In some embodiments, criterion (b) is 100% of capture probes have at least 80 on-target total reads. In some embodiments, criterion (b) is at least 99.9% of capture probes have at least 80 on-target total reads. In some embodiments, criterion (b) is at least 99.5% of capture probes have at least 80 on-target total reads. In some embodiments, criterion (b) is at least 99% of capture probes have at least 80 on-target total reads. In some embodiments, criterion (b) is at least 98.5% of capture probes have at least 80 on-target total reads. In some embodiments, criterion (b) is at least 98% of capture probes have at least 80 on-target total reads. In some embodiments, criterion (b) is at least 97.5% of capture probes have at least 80 on-target total reads. In some embodiments, criterion (b) is at least 97% of capture probes have at least 80 on-target total reads. In some embodiments, criterion (b) is at least 96.5% of capture probes have at least 80 on-target total reads. In some embodiments, criterion (b) is at least 96% of capture probes have at least 80 on-target total reads. In some embodiments, criterion (b) is at least 95.5% of capture probes have at least 80 on-target total reads. In some embodiments, criterion (b) is at least 95% of capture probes have at least 80 on-target total reads. In some embodiments of the kits of the disclosure, the pre-determined acceptance criteria for the probe QC process comprise criterion (b) at least 90% of capture probes have at least 80 on-target total reads. In some embodiments, criterion (b) is at least 85% of capture probes have at least 80 on-target total reads. In some embodiments, criterion (b) is at least 80% of capture probes have at least 80 on-target total reads. In some embodiments, criterion (b) is at least 75% of capture probes have at least 80 on-target total reads. In some embodiments, criterion (b) is at least 70% of capture probes have at least 80 on-target total reads. In some embodiments, criterion (b) is at least 65% of capture probes have at least 80 on-target total reads. In some embodiments, criterion (b) is at least 60% of capture probes have at least 80 on-target total reads.

In some embodiments, criterion (b) is at least about 60% to about 99.9% of capture probes have at least 10-200 on-target total reads. In some embodiments, criterion (b) is at least about 70% to about 99.9% of capture probes have at least 10-200 on-target total reads. In some embodiments, criterion (b) is at least about 80% to about 99.9% of capture probes have at least 10-200 on-target total reads. In some embodiments, criterion (b) is at least about 90% to about 99.9% of capture probes have at least 10-200 on-target total reads. In some embodiments, criterion (b) is at least about 95% to about 99.9% of capture probes have at least 10-200 on-target total reads. In some embodiments, criterion (b) is at least about 99% to about 99.9% of capture probes have at least 10-200 on-target total reads.

In some embodiments, criterion (b) is at least 60% to 99.9% of capture probes have at least 10-200 on-target total reads. In some embodiments, criterion (b) is at least 70% to 99.9% of capture probes have at least 10-200 on-target total reads. In some embodiments, criterion (b) is at least 80% to 99.9% of capture probes have at least 10-200 on-target total reads. In some embodiments, criterion (b) is at least 90% to 99.9% of capture probes have at least 10-200 on-target total reads. In some embodiments, criterion (b) is at least 95% to 99.9% of capture probes have at least 10-200 on-target total reads. In some embodiments, criterion (b) is at least 99% to 99.9% of capture probes have at least 10-200 on-target total reads.

In some embodiments, criterion (b) is at least about 60% to about 100% of capture probes have at least 10-200 on-target total reads. In some embodiments, criterion (b) is at least about 70% to about 100% of capture probes have at least 10-200 on-target total reads. In some embodiments, criterion (b) is at least about 80% to about 100% of capture probes have at least 10-200 on-target total reads. In some embodiments, criterion (b) is at least about 90% to about 100% of capture probes have at least 10-200 on-target total reads. In some embodiments, criterion (b) is at least about 95% to about 100% of capture probes have at least 10-200 on-target total reads. In some embodiments, criterion (b) is at least about 99% to about 100% of capture probes have at least 10-200 on-target total reads.

In some embodiments, criterion (b) is at least 60% to 100% of capture probes have at least 10-200 on-target total reads. In some embodiments, criterion (b) is at least 70% to 100% of capture probes have at least 10-200 on-target total reads. In some embodiments, criterion (b) is at least 80% to 100% of capture probes have at least 10-200 on-target total reads. In some embodiments, criterion (b) is at least 90% to 100% of capture probes have at least 10-200 on-target total reads. In some embodiments, criterion (b) is at least 95% to 100% of capture probes have at least 10-200 on-target total reads. In some embodiments, criterion (b) is at least 99% to 100% of capture probes have at least 10-200 on-target total reads.

In some embodiments, criterion (b) is at least about 60% to about 100% of capture probes have at least 50 on-target total reads. In some embodiments, criterion (b) is at least about 70% to about 100% of capture probes have at least 50 on-target total reads. In some embodiments, criterion (b) is at least about 80% to about 100% of capture probes have at least 50 on-target total reads. In some embodiments, criterion (b) is at least about 90% to about 100% of capture probes have at least 50 on-target total reads. In some embodiments, criterion (b) is at least about 95% to about 100% of capture probes have at least 50 on-target total reads. In some embodiments, criterion (b) is at least about 99% to about 100% of capture probes have at least 50 on-target total reads.

In some embodiments, criterion (b) is at least 60% to 100% of capture probes have at least 50 on-target total reads. In some embodiments, criterion (b) is at least 70% to 100% of capture probes have at least 50 on-target total reads. In some embodiments, criterion (b) is at least 80% to 100% of capture probes have at least 50 on-target total reads. In some embodiments, criterion (b) is at least 90% to 100% of capture probes have at least 50 on-target total reads. In some embodiments, criterion (b) is at least 95% to 100% of capture probes have at least 50 on-target total reads. In some embodiments, criterion (b) is at least 99% to 100% of capture probes have at least 50 on-target total reads.

In some embodiments, criterion (b) is at least about 60% to about 100% of capture probes have at least 60 on-target total reads. In some embodiments, criterion (b) is at least about 70% to about 100% of capture probes have at least 60 on-target total reads. In some embodiments, criterion (b) is at least about 80% to about 100% of capture probes have at least 60 on-target total reads. In some embodiments, criterion (b) is at least about 90% to about 100% of capture probes have at least 60 on-target total reads. In some embodiments, criterion (b) is at least about 95% to about 100% of capture probes have at least 60 on-target total reads. In some embodiments, criterion (b) is at least about 99% to about 100% of capture probes have at least 60 on-target total reads.

In some embodiments, criterion (b) is at least 60% to 100% of capture probes have at least 60 on-target total reads. In some embodiments, criterion (b) is at least 70% to 100% of capture probes have at least 60 on-target total reads. In some embodiments, criterion (b) is at least 80% to 100% of capture probes have at least 60 on-target total reads. In some embodiments, criterion (b) is at least 90% to 100% of capture probes have at least 60 on-target total reads. In some embodiments, criterion (b) is at least 95% to 100% of capture probes have at least 60 on-target total reads. In some embodiments, criterion (b) is at least 99% to 100% of capture probes have at least 60 on-target total reads.

In some embodiments, criterion (b) is at least about 60% to about 100% of capture probes have at least 70 on-target total reads. In some embodiments, criterion (b) is at least about 70% to about 100% of capture probes have at least 70 on-target total reads. In some embodiments, criterion (b) is at least about 80% to about 100% of capture probes have at least 70 on-target total reads. In some embodiments, criterion (b) is at least about 90% to about 100% of capture probes have at least 70 on-target total reads. In some embodiments, criterion (b) is at least about 95% to about 100% of capture probes have at least 70 on-target total reads. In some embodiments, criterion (b) is at least about 99% to about 100% of capture probes have at least 70 on-target total reads.

In some embodiments, criterion (b) is at least 60% to 100% of capture probes have at least 70 on-target total reads. In some embodiments, criterion (b) is at least 70% to 100% of capture probes have at least 70 on-target total reads. In some embodiments, criterion (b) is at least 80% to 100% of capture probes have at least 70 on-target total reads. In some embodiments, criterion (b) is at least 90% to 100% of capture probes have at least 70 on-target total reads. In some embodiments, criterion (b) is at least 95% to 100% of capture probes have at least 70 on-target total reads. In some embodiments, criterion (b) is at least 99% to 100% of capture probes have at least 70 on-target total reads.

In some embodiments, criterion (b) is at least about 60% to about 100% of capture probes have at least 80 on-target total reads. In some embodiments, criterion (b) is at least about 70% to about 100% of capture probes have at least 80 on-target total reads. In some embodiments, criterion (b) is at least about 80% to about 100% of capture probes have at least 80 on-target total reads. In some embodiments, criterion (b) is at least about 90% to about 100% of capture probes have at least 80 on-target total reads. In some embodiments, criterion (b) is at least about 95% to about 100% of capture probes have at least 80 on-target total reads. In some embodiments, criterion (b) is at least about 99% to about 100% of capture probes have at least 80 on-target total reads.

In some embodiments, criterion (b) is at least 60% to 100% of capture probes have at least 80 on-target total reads. In some embodiments, criterion (b) is at least 70% to 100% of capture probes have at least 80 on-target total reads. In some embodiments, criterion (b) is at least 80% to 100% of capture probes have at least 80 on-target total reads. In some embodiments, criterion (b) is at least 90% to 100% of capture probes have at least 80 on-target total reads. In some embodiments, criterion (b) is at least 95% to 100% of capture probes have at least 80 on-target total reads. In some embodiments, criterion (b) is at least 99% to 100% of capture probes have at least 80 on-target total reads.

In some embodiments of the kits of the disclosure, the pre-determined acceptance criteria for the probe QC process comprise a criterion (c). In some embodiments, criterion (c) is at least about 99.9% of expected SNPs within the DNA sample are detected. In some embodiments, criterion (c) is at least about 99.5% of expected SNPs within the DNA sample are detected. In some embodiments, criterion (c) is at least about 99% of expected SNPs within the DNA sample are detected. In some embodiments, criterion (c) is at least about 98% of expected SNPs within the DNA sample are detected. In some embodiments, criterion (c) is at least about 97% of expected SNPs within the DNA sample are detected. In some embodiments, criterion (c) is at least about 96% of expected SNPs within the DNA sample are detected. In some embodiments, criterion (c) is at least about 95% of expected SNPs within the DNA sample are detected. In some embodiments, criterion (c) is at least about 90% of expected SNPs within the DNA sample are detected. In some embodiments, criterion (c) is at least about 85% of expected SNPs within the DNA sample are detected. In some embodiments, criterion (c) is at least about 80% of expected SNPs within the DNA sample are detected. In some embodiments, criterion (c) is at least about 75% of expected SNPs within the DNA sample are detected. In some embodiments, criterion (c) is at least about 70% of expected SNPs within the DNA sample are detected. In some embodiments, criterion (c) is at least about 65% of expected SNPs within the DNA sample are detected. In some embodiments, criterion (c) is at least about 60% of expected SNPs within the DNA sample are detected.

In some embodiments, criterion (c) is at least 99.9% of expected SNPs within the DNA sample are detected. In some embodiments, criterion (c) is at least 99.5% of expected SNPs within the DNA sample are detected. In some embodiments, criterion (c) is at least 99% of expected SNPs within the DNA sample are detected. In some embodiments, criterion (c) is at least 98% of expected SNPs within the DNA sample are detected. In some embodiments, criterion (c) is at least 97% of expected SNPs within the DNA sample are detected. In some embodiments, criterion (c) is at least 96% of expected SNPs within the DNA sample are detected. In some embodiments, criterion (c) is at least 95% of expected SNPs within the DNA sample are detected. In some embodiments, criterion (c) is at least 90% of expected SNPs within the DNA sample are detected. In some embodiments, criterion (c) is at least 85% of expected SNPs within the DNA sample are detected. In some embodiments, criterion (c) is at least 80% of expected SNPs within the DNA sample are detected. In some embodiments, criterion (c) is at least 75% of expected SNPs within the DNA sample are detected. In some embodiments, criterion (c) is at least 70% of expected SNPs within the DNA sample are detected. In some embodiments, criterion (c) is at least 65% of expected SNPs within the DNA sample are detected. In some embodiments, criterion (c) is at least 60% of expected SNPs within the DNA sample are detected.

In some embodiments, criterion (c) is at least about 60% to about 99.9% of expected SNPs within the DNA sample are detected. In some embodiments, criterion (c) is at least about 70% to about 99.9% of expected SNPs within the DNA sample are detected. In some embodiments, criterion (c) is at least about 80% to about 99.9% of expected SNPs within the DNA sample are detected. In some embodiments, criterion (c) is at least about 90% to about 99.9% of expected SNPs within the DNA sample are detected. In some embodiments, criterion (c) is at least about 95% to about 99.9% of expected SNPs within the DNA sample are detected. In some embodiments, criterion (c) is at least about 96% to about 99.9% of expected SNPs within the DNA sample are detected. In some embodiments, criterion (c) is at least about 97% to about 99.9% of expected SNPs within the DNA sample are detected. In some embodiments, criterion (c) is at least about 98% to about 99.9% of expected SNPs within the DNA sample are detected. In some embodiments, criterion (c) is at least about 99% to about 99.9% of expected SNPs within the DNA sample are detected. In some embodiments, criterion (c) is at least about 100% of expected SNPs within the DNA sample are detected.

In some embodiments, criterion (c) is at least 60% to 99.9% of expected SNPs within the DNA sample are detected. In some embodiments, criterion (c) is at least 70% to 99.9% of expected SNPs within the DNA sample are detected. In some embodiments, criterion (c) is at least 80% to 99.9% of expected SNPs within the DNA sample are detected. In some embodiments, criterion (c) is at least 90% to 99.9% of expected SNPs within the DNA sample are detected. In some embodiments, criterion (c) is at least 95% to 99.9% of expected SNPs within the DNA sample are detected. In some embodiments, criterion (c) is at least 96% to 99.9% of expected SNPs within the DNA sample are detected. In some embodiments, criterion (c) is at least 97% to 99.9% of expected SNPs within the DNA sample are detected. In some embodiments, criterion (c) is at least 98% to 99.9% of expected SNPs within the DNA sample are detected. In some embodiments, criterion (c) is at least about 99% to 99.9% of expected SNPs within the DNA sample are detected. In some embodiments, criterion (c) is at least 100% of expected SNPs within the DNA sample are detected.

In some embodiments, criterion (c) is at least about 60% to about 100% of expected SNPs within the DNA sample are detected. In some embodiments, criterion (c) is at least about 70% to about 100% of expected SNPs within the DNA sample are detected. In some embodiments, criterion (c) is at least about 80% to about 100% of expected SNPs within the DNA sample are detected. In some embodiments, criterion (c) is at least about 90% to about 100% of expected SNPs within the DNA sample are detected. In some embodiments, criterion (c) is at least about 95% to about 100% of expected SNPs within the DNA sample are detected. In some embodiments, criterion (c) is at least about 96% to about 100% of expected SNPs within the DNA sample are detected.. In some embodiments, criterion (c) is at least about 97% to about 100% of expected SNPs within the DNA sample are detected. In some embodiments, criterion (c) is at least about 98% to about 100% of expected SNPs within the DNA sample are detected. In some embodiments, criterion (c) is at least about 99% to about 100% of expected SNPs within the DNA sample are detected. In some embodiments, criterion (c) is about 100% of expected SNPs within the DNA sample are detected.

In some embodiments, criterion (c) is at least 60% to 100% of expected SNPs within the DNA sample are detected. In some embodiments, criterion (c) is at least 70% to 100% of expected SNPs within the DNA sample are detected. In some embodiments, criterion (c) is at least 80% to 100% of expected SNPs within the DNA sample are detected. In some embodiments, criterion (c) is at least 90% to 100% of expected SNPs within the DNA sample are detected. In some embodiments, criterion (c) is at least 95% to 100% of expected SNPs within the DNA sample are detected. In some embodiments, criterion (c) is at least 96% to 100% of expected SNPs within the DNA sample are detected. In some embodiments, criterion (c) is at least 97% to 100% of expected SNPs within the DNA sample are detected. In some embodiments, criterion (c) is at least 98% to 100% of expected SNPs within the DNA sample are detected. In some embodiments, criterion (c) is at least about 99% to 100% of expected SNPs within the DNA sample are detected. In some embodiments, criterion (c) is 100% of expected SNPs within the DNA sample are detected.

In some embodiments of the kits of the disclosure, the pre-determined acceptance criteria for the probe QC process comprise a criterion (a) and a criterion (b). In some embodiments of the kits of the disclosure, the pre-determined acceptance criteria for the probe QC process comprise a criterion (a) and a criterion (c). In some embodiments of the kits of the disclosure, the pre-determined acceptance criteria for the probe QC process comprise a criterion (b) and a criterion (c). In some embodiments of the kits of the disclosure, the pre-determined acceptance criteria for the probe QC process comprise a criterion (a), a criterion (b), and a criterion (c).

In some embodiments of the kits of the disclosure, the DNA sample used in the probe QC process comprises human wild type DNA. In some embodiments of the kits of the disclosure, the DNA sample used in the probe QC process comprises a plurality of single nucleotide polymorphisms (SNPs). In some embodiments of the kits of the disclosure, the DNA sample used in the probe QC process comprises any suitable DNA.

In some embodiments of the kits of the disclosure, the kit comprises a DNA sample used as a positive control sample specific to the capture probe modules of the same kit. In some embodiments, the positive control sample comprises human wild type DNA. In some embodiments, the positive control sample comprises a plurality of SNPs. In some embodiments, the positive control sample comprises the DNA sample used in the probe QC process of the capture probe modules of the kit. In some embodiments, the positive control sample comprises any suitable DNA.

In some embodiments of the kits of the disclosure, the adaptor QC process is performed according to any one of the methods of performing an adaptor QC process of the disclosure.

In some embodiments of the kits of the disclosure, the probe QC process is performed according to any one of the methods of performing a probe QC process of the disclosure.

In some embodiments of the kits of the disclosure, the kit comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; and (c) a first primer pair comprising a first F primer and a first R primer, wherein the first F primer comprises an amplification region binding region and a sequencing primer binding region, wherein the first R primer comprises a tail sequence binding region and a sequencing primer binding region.

In some embodiments of the kits of the disclosure, the kit comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; and (c) a first primer pair comprising a first F primer and a first R primer, wherein the first F primer comprises an amplification region binding region and a sequencing primer binding region, wherein the first R primer comprises a tail sequence binding region and a sequencing primer binding region; wherein the capture probe modules have passed a probe QC process.

In some embodiments of the kits of the disclosure, the kit comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; and (c) a first primer pair comprising a first F primer and a first R primer, wherein the first F primer comprises an amplification region binding region and a sequencing primer binding region, wherein the first R primer comprises a tail sequence binding region and a sequencing primer binding region; wherein the set of adaptors has passed an adaptor QC process.

In some embodiments of the kits of the disclosure, the kit comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; and (c) a first primer pair comprising a first F primer and a first R primer, wherein the first F primer comprises an amplification region binding region and a sequencing primer binding region, wherein the first R primer comprises a tail sequence binding region and a sequencing primer binding region; wherein the set of adaptors has passed an adaptor QC process and the capture probe modules have passed a probe QC process.

In some embodiments of the kits of the disclosure, the kit comprises (a) a set of adaptors, wherein each adaptor comprises an adaptor module comprising an amplification region; and (b) a second primer pair comprising a second F primer and a second R primer, wherein each of the second F primer and the second R primer comprises an amplification region binding region and a sequencing primer binding region.

In some embodiments of the kits of the disclosure, the kit comprises (a) a set of adaptors, wherein each adaptor comprises an adaptor module comprising an amplification region; and (b) a second primer pair comprising a second F primer and a second R primer, wherein each of the second F primer and the second R primer comprises an amplification region binding region and a sequencing primer binding region; wherein the set of adaptors has passed an adaptor QC process.

In some embodiments of the kits of the disclosure, the kit comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a tail sequence binding region and a sequencing primer binding region; and (d) a second primer pair comprising a second F primer and a second R primer, wherein each of the second F primer and the second R primer comprises an amplification region binding region and a sequencing primer binding region.

In some embodiments of the kits of the disclosure, the kit comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a tail sequence binding region and a sequencing primer binding region; and (d) a second primer pair comprising a second F primer and a second R primer, wherein each of the second F primer and the second R primer comprises an amplification region binding region and a sequencing primer binding region; wherein the capture probe modules have passed a probe QC process.

In some embodiments of the kits of the disclosure, the kit comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a tail sequence binding region and a sequencing primer binding region; and (d) a second primer pair comprising a second F primer and a second R primer, wherein each of the second F primer and the second R primer comprises an amplification region binding region and a sequencing primer binding region; wherein the set of adaptors has passed an adaptor QC process.

In some embodiments of the kits of the disclosure, the kit comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a tail sequence binding region and a sequencing primer binding region; and (d) a second primer pair comprising a second F primer and a second R primer, wherein each of the second F primer and the second R primer comprises an amplification region binding region and a sequencing primer binding region; wherein the set of adaptors has passed an adaptor QC process and the capture probe modules have passed a probe QC process.

In some embodiments of the kits of the disclosure, the kit comprises (a) one or more capture probe modules, wherein each capture probe module comprises a tail sequence and a capture probe sequence capable of hybridizing to a target sequence in a test sample, wherein the tail sequence of each capture probe module comprises a Library Tag; and (b) a set of adaptors, wherein each adaptor comprises an adaptor module comprising an amplification region.

In some embodiments of the kits of the disclosure, the kit comprises (a) one or more capture probe modules, wherein each capture probe module comprises a tail sequence and a capture probe sequence capable of hybridizing to a target sequence in a test sample, wherein the tail sequence of each capture probe module comprises a Library Tag; and (b) a set of adaptors, wherein each adaptor comprises an adaptor module comprising an amplification region; wherein the set of adaptors has passed an adaptor QC process.

In some embodiments of the kits of the disclosure, the kit comprises (a) one or more capture probe modules, wherein each capture probe module comprises a tail sequence and a capture probe sequence capable of hybridizing to a target sequence in a test sample, wherein the tail sequence of each capture probe module comprises a Library Tag; and (b) a set of adaptors, wherein each adaptor comprises an adaptor module comprising an amplification region; wherein the one or more capture probe modules have passed a probe QC process.

In some embodiments of the kits of the disclosure, the kit comprises (a) one or more capture probe modules, wherein each capture probe module comprises a tail sequence and a capture probe sequence capable of hybridizing to a target sequence in a test sample, wherein the tail sequence of each capture probe module comprises a Library Tag; and (b) a set of adaptors, wherein each adaptor comprises an adaptor module comprising an amplification region; wherein the one or more capture probe modules have passed a probe QC process and the set of adaptors has passed an adaptor QC process.

In some embodiments of the kits of the disclosure, the kit comprises (a) one or more capture probe modules, wherein each capture probe module comprises a tail sequence and a capture probe sequence capable of hybridizing to a target sequence in a test sample, wherein the tail sequence of each capture probe module comprises a Library Tag; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a Library Tag binding region and a sequencing primer binding region; and (d) a second primer pair comprising a second F primer and a second R primer, wherein each of the second F primer and the second R primer comprises an amplification region binding region and a sequencing primer binding region, and wherein none of the primers of the second primer pair bind to the Library Tag.

In some embodiments of the kits of the disclosure, the kit comprises (a) one or more capture probe modules, wherein each capture probe module comprises a tail sequence and a capture probe sequence capable of hybridizing to a target sequence in a test sample, wherein the tail sequence of each capture probe module comprises a Library Tag; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a Library Tag binding region and a sequencing primer binding region; and (d) a second primer pair comprising a second F primer and a second R primer, wherein each of the second F primer and the second R primer comprises an amplification region binding region and a sequencing primer binding region, and wherein none of the primers of the second primer pair bind to the Library Tag; wherein the one or more capture probe modules have passed a probe QC process.

In some embodiments of the kits of the disclosure, the kit comprises (a) one or more capture probe modules, wherein each capture probe module comprises a tail sequence and a capture probe sequence capable of hybridizing to a target sequence in a test sample, wherein the tail sequence of each capture probe module comprises a Library Tag; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a Library Tag binding region and a sequencing primer binding region; and (d) a second primer pair comprising a second F primer and a second R primer, wherein each of the second F primer and the second R primer comprises an amplification region binding region and a sequencing primer binding region, and wherein none of the primers of the second primer pair bind to the Library Tag; wherein the set of adaptors has passed an adaptor QC process.

In some embodiments of the kits of the disclosure, the kit comprises (a) one or more capture probe modules, wherein each capture probe module comprises a tail sequence and a capture probe sequence capable of hybridizing to a target sequence in a test sample, wherein the tail sequence of each capture probe module comprises a Library Tag; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a Library Tag binding region and a sequencing primer binding region; and (d) a second primer pair comprising a second F primer and a second R primer, wherein each of the second F primer and the second R primer comprises an amplification region binding region and a sequencing primer binding region, and wherein none of the primers of the second primer pair bind to the Library Tag; wherein the one or more capture probe modules have passed a probe QC process and the set of adaptors has passed an adaptor QC process.

In some embodiments of the kits of the disclosure, the first primer pair is used to generate a first modified library.

In some embodiments of the kits of the disclosure, the second primer pair is used to generate a second modified library.

In some embodiments of the kits of the disclosure, the first primer pair is used to generate a first modified library and the second primer pair is used to generate a second modified library. In some embodiments, the first modified library and the second modified library are configured to be combined into a Sequence-Ready Library (SRL).

In some embodiments of the kits of the disclosure, the tail sequence of each capture probe module comprises a Library Tag.

In some embodiments of the kits of the disclosure, the Library Tag comprises a nucleic acid sequence or an amino acid sequence. In some embodiments, the Library Tag comprises one or more of a DNA, an RNA, a PNA, or a non-naturally occurring nucleic acid, a synthetic nucleic acid, a modified nucleic acid, a non-naturally occurring amino acid, a synthetic amino acid, and a modified amino acid.

In some embodiments of the kits of the disclosure, the Library Tag comprises a detectable label. In some embodiments, the detectable label comprises one or more of a fluorescent moiety, a magnetic or paramagnetic moiety, an enzymatic moiety, a binding moiety, an epitope, and a radioactive moiety.

In some embodiments of the kits of the disclosure, the Library Tag selectively or specifically binds to a detectable moiety. In some embodiments, the detectable moiety comprises one or more of a fluorescent moiety, a magnetic or paramagnetic moiety, an enzymatic moiety, a binding moiety, an epitope, and a radioactive moiety.

In some embodiments of the kits of the disclosure, the Library Tag comprises a unique polynucleotide sequence. In some embodiments, the unique polynucleotide sequence has no more than 70% sequence identity to any DNA fragment of the test sample.

In some embodiments of the kits of the disclosure, the kit comprises (a) a set of adaptors, wherein each adaptor comprises an adaptor module comprising an amplification region; (b) one or more capture probe modules, wherein each capture probe module comprises a tail sequence and a capture probe sequence capable of hybridizing to a target sequence in a test sample, wherein the tail sequence of each capture probe module comprises a Library Tag; (c) a first primer pair comprising a first F primer and a first R primer, wherein the first F primer comprises an amplification region binding region and a sequencing primer binding region; wherein the first R primer comprises a Library Tag binding region and a sequencing primer binding region; and (d) a second primer pair comprising a second F primer and a second R primer, wherein each of the second F primer and the second R primer comprises an amplification region binding region and a sequencing primer binding region, wherein none of the primers of the second primer pair bind to the Library Tag.

In some embodiments of the kits of the disclosure, the kit is used for generating a first modified library and a second modified library, wherein the kit comprises (a) a set of adaptors, wherein each adaptor comprises an adaptor module; and (b) one or more capture probe modules, wherein each capture probe module comprises a tail sequence and a capture probe sequence capable of hybridizing to a target sequence in a test sample, wherein the tail sequence of each capture probe module comprises a Library Tag. In some embodiments, the first modified library comprises a DNA fragment comprising an adaptor, a capture probe module, and the capture probe module's target sequence. In some embodiments, the second modified library comprises a DNA fragment comprising an adaptor and at least a portion of a DNA sequence of the test sample. In some embodiments, both the first modified library and the second modified library are generated from the same test sample.

In some embodiments of the kits of the disclosure, the first primer pair is used to generate a first modified library and the second primer pair is used to generate a second modified library.

In some embodiments of the kits of the disclosure, the first modified library comprises a first DNA fragment comprising an adaptor, a capture probe module, and the capture probe module's target sequence.

In some embodiments of the kits of the disclosure, the second modified library comprises a second DNA fragment comprising an adaptor and a at least a portion of a DNA sequence of the test sample.

In some embodiments of the kits of the disclosure, the first modified library and the second modified library are configured to be combined into a Sequence-Ready Library (SRL). In some embodiments, the SRL is also referred to as the combined library.

In some embodiments of the kits of the disclosure, each library fragment of the first modified library is an adaptor-tagged DNA fragment comprising an adaptor, a capture probe module, and at least a portion of a DNA sequence of the test sample.

In some embodiments of the kits of the disclosure, each library fragment of the second modified library is an adaptor-tagged DNA fragment comprising an adaptor and at least a portion of a DNA sequence of the test sample.

In some embodiments of the kits of the disclosure, none of the adaptor-tagged DNA fragments of the second modified library comprises a capture probe module.

In some embodiments of the kits of the disclosure, the first modified library is a Target Capture Library (TCL) or amplified Target Capture Library (TCLA).

In some embodiments of the kits of the disclosure, the second modified library is a Whole-Genome Library (WGL) or amplified Whole-Genome Library (WGLA).

In some embodiments of the kits of the disclosure, the first modified library or the second modified library comprises the Library Tag, wherein the Library Tag is configured to distinguish the first modified library from the second modified library.

In some embodiments of the kits of the disclosure, the first modified library comprises a first Library Tag and the second modified library comprises a second Library Tag, wherein the first Library Tag and the second Library Tag are not identical.

In some embodiments of the kits of the disclosure, each library fragment of the first modified library comprises, from 5' to 3': a 5' oligonucleotide (A1), a first 5' adaptor module, at least a portion of a DNA sequence of the test sample, and a 3' oligonucleotide (A2), or a 5' oligonucleotide (A1), at least a portion of a DNA sequence of the test sample, a first 3' adaptor module, and a 3' oligonucleotide (A2); wherein each library fragment of the second modified library comprises, from 5' to 3': a 5' oligonucleotide (B1), a second 5' adaptor module, at least a portion of a DNA sequence of the test sample, a second 3' adaptor module, and a 3' oligonucleotide (B2). In some embodiments, at least one of A1, A2, B1, and B2 contains at least one Library Tag. In some embodiments, the Library Tag comprises a unique polynucleotide sequence, wherein the unique polynucleotide sequence has 70% identity, or less, to a sequence selected from the list consisting of A1, B1, A2, B2, the first 5' adaptor module, the target sequence, the first 3' adaptor module, the second 5' adaptor module, any DNA fragment of the test sample, and the second 3' adaptor module.

In some embodiments of the kits of the disclosure, each library fragment of the first modified library comprises, from 5' to 3': a 5' oligonucleotide (A1), a first 5' adaptor module, at least a portion of a DNA sequence of the test sample, and a 3' oligonucleotide (A2), or a 5' oligonucleotide (A1), at least a portion of a DNA sequence of the test sample, a first 3' adaptor module, and a 3' oligonucleotide (A2); wherein each library fragment of the second modified library comprises, from 5' to 3': a 5' oligonucleotide (B1), a second 5' adaptor module, at least a portion of a DNA sequence of the test sample, a second 3' adaptor module, and a 3' oligonucleotide (B2); wherein at least one of A1, A2, B1, and B2 comprises the Library Tag; wherein the Library Tag comprises a unique polynucleotide sequence, wherein the unique polynucleotide sequence has no more than 70% sequence identity to a sequence selected from the list consisting of A1, B1, A2, B2, the first 5' adaptor module, the target sequence, the first 3' adaptor module, the second 5' adaptor module, any DNA fragment of the test sample, and the second 3' adaptor module.

In some embodiments of the kits of the disclosure, each adaptor of the set of adaptors comprises a ligation strand oligonucleotide and a non-ligation strand oligonucleotide.

In some embodiments of the kits of the disclosure, the non-ligation strand oligonucleotide is capable of hybridizing to a region at the 3' end of the ligation strand oligonucleotide and forming a duplex therewith.

In some embodiments of the kits of the disclosure, the ligation strand oligonucleotide comprises an adaptor module.

In some embodiments of the kits of the disclosure, the ligation strand oligonucleotide comprises a dT, dA, dC, or dG overhang at the 3' terminus.

In some embodiments of the kits of the disclosure, the non-ligation strand oligonucleotide comprises a modification at its 3' terminus that prevents ligation to the 5' end of a dsDNA fragment and/or adaptor dimer formation, wherein the non-ligation strand is configured to be displaced from the duplex.

In some embodiments of the kits of the disclosure, each adaptor of the set of adaptors comprises an ID region selected from a pool of unique ID regions, wherein the pool is selected from a plurality of pools, and wherein the selected pool is unique to the test sample.

In some embodiments of the kits of the disclosure, the adaptor module comprises (a) an amplification region comprising a primer binding site; (b) an ID region; and (c) an anchor region.

In some embodiments of the kits of the disclosure, the amplification region comprises a primer binding site, wherein the primer binding site allows for amplification using PCR (polymerase chain reaction), LAMP (loop-mediated isothermal amplification), NASBA (nucleic acid sequence-based amplification), SDA (standard displacement amplification), RCA (rolling circle replication), or LCR (ligase chain reaction).

In some embodiments of the kits of the disclosure, the amplification region comprises or consists of between 10 and 50 nucleotides. In some embodiments, the amplification region comprises or consists of between 20 and 30 nucleotides. In some embodiments, the amplification region comprises or consists of 25 nucleotides.

In some embodiments of the kits of the disclosure, the amplification region comprises or consists of about 10 to about 50 nucleotides. In some embodiments, the amplification region comprises or consists of about 20 to about 30 nucleotides. In some embodiments, the amplification region comprises or consists of about 25 nucleotides.

In some embodiments of the kits of the disclosure, the anchor region comprises an overhang at the 3' terminus.

In some embodiments of the kits of the disclosure, the anchor region comprises or consists of between 1 and 50 nucleotides. In some embodiments, the anchor region comprises or consists of between 5 and 25 nucleotides. In some embodiments, the anchor region comprises or consists of 10 nucleotides.

In some embodiments of the kits of the disclosure, the anchor region comprises or consists of about 1 to about 50 nucleotides. In some embodiments, the anchor region comprises or consists of about 5 to about 25 nucleotides. In some embodiments, the anchor region comprises or consists of about 10 nucleotides.

In some embodiments of the kits of the disclosure, the ID region comprises or consists of between 3 and 50 nucleotides. In some embodiments, the ID region comprises or consists of between 3 and 15 nucleotides. In some embodiments, the ID region comprises or consists of 8 nucleotides.

In some embodiments of the kits of the disclosure, the ID region comprises or consists of about 3 to about 50 nucleotides. In some embodiments, the ID region comprises or consists of about 3 to about 15 nucleotides. In some embodiments, the ID region comprises or consists of about 8 nucleotides.

In some embodiments of the kits of the disclosure, the adaptor module further comprises a unique molecule identifier (UMI) multiplier.

In some embodiments of the kits of the disclosure, the UMI multiplier is adjacent to or contained within the ID region.

In some embodiments of the kits of the disclosure, the UMI multiplier comprises or consists of between 1 and 5 nucleotides.

In some embodiments of the kits of the disclosure, the UMI multiplier comprises or consists of about 1 to about 5 nucleotides.

In some embodiments of the kits of the disclosure, the UMI multiplier is 3 nucleotides in length, and comprises a nucleic acid sequence selected from a group of 64 unique nucleotide sequences.

In some embodiments of the kits of the disclosure, the pool of ID regions comprises between 2 and 10,000 unique ID region sequences. In some embodiments, the pool of ID regions comprises between 10 and 500 unique ID region sequences. In some embodiments, the pool of ID regions comprises between 50 and 300 unique ID region sequences. In some embodiments, the pool of ID regions comprises 60 unique ID region sequences.

In some embodiments of the kits of the disclosure, the pool of ID regions comprises about 2 to about 10,000 unique ID region sequences. In some embodiments, the pool of ID regions comprises about 10 to about 500 unique ID region sequences. In some embodiments, the pool of ID regions comprises about 50 to about 300 unique ID region sequences. In some embodiments, the pool of ID regions comprises about 60 unique ID region sequences.

In some embodiments of the kits of the disclosure, each ID region of the pool of ID regions is 8 nucleotides in length.

In some embodiments of the kits of the disclosure, each ID region of the pool of ID regions is about 8 nucleotides in length.

In some embodiments of the kits of the disclosure, each ID region sequence is discrete from any other ID region sequence by Hamming distance of at least two.

In some embodiments of the kits of the disclosure, each ID region is configured to identify the DNA fragment attached thereto.

In some embodiments of the kits of the disclosure, each adaptor module of the set of adaptor modules is selected from a group consisting of between 64 and 2,560,000 unique nucleotide sequences.

In some embodiments of the kits of the disclosure, each adaptor module of the set of adaptor modules is selected from a group consisting of about 64 to about 2,560,000 unique nucleotide sequences.

In some embodiments of the kits of the disclosure, each adaptor module of the set of adaptor modules comprises a unique nucleotide sequence selected from 3840 unique nucleotide sequences, wherein each sequence of the 3840 unique nucleotide sequences is discrete from any other sequence by Hamming distance of at least two.

In some embodiments of the kits of the disclosure, the anchor region of each adaptor of the set of adaptors comprises one of four nucleotide sequences, and wherein each ID region of a given sequence is paired to only one of the four anchor regions of a given sequence.

In some embodiments of the kits of the disclosure, the amplification region of each adaptor of the set of adaptors comprises an identical primer binding site.

In some embodiments of the kits of the disclosure, each ID region of the pool of ID regions is 8 nucleotides in length, each ID region sequence is discrete from any other ID region sequence by Hamming distance of at least two; each adaptor of the set of adaptors comprises a UMI multiplier that is adjacent to or contained within the ID region, wherein the UMI multiplier of each adaptor of the set of adaptors is three nucleotides in length, and wherein the UMI multiplier of a given sequence is paired to one ID region of a given sequence; the anchor region of each adaptor of the set of adaptors comprises one of four nucleotide sequences, and wherein each ID region of a given sequence is paired to only one of the four anchor regions of a given sequence; and the amplification region of each adaptor of the set of adaptors comprises an identical primer binding site.

In some embodiments of the kits of the disclosure, the kit comprises one set of adaptors. In some embodiments, the kit comprises more than one set of adaptor modules.

In some embodiments, the kit comprises between 2-1000 sets of adaptor modules. In some embodiments, the kit comprises between 2-500 sets of adaptor modules. In some embodiments, the kit comprises between 2-100 sets of adaptor modules. In some embodiments, the kit comprises between 2-50 sets of adaptor modules. In some embodiments, the kit comprises between 50-100 sets of adaptor modules. In some embodiments, the kit comprises between 5-50 sets of adaptor modules. In some embodiments, the kit comprises between 10-50 sets of adaptor modules. In some embodiments, the kit comprises between 15-50 sets of adaptor modules. In some embodiments, the kit comprises between 20-50 sets of adaptor modules. In some embodiments, the kit comprises between 30-50 sets of adaptor modules. In some embodiments, the kit comprises between 40-50 sets of adaptor modules. In some embodiments, the kit comprises between 45-50 sets of adaptor modules. In some embodiments, the kit comprises 48 sets of adaptor modules.

In some embodiments, the kit comprises about 2 to about 1000 sets of adaptor modules. In some embodiments, the kit comprises about 2 to about 500 sets of adaptor modules. In some embodiments, the kit comprises about 2 to about 100 sets of adaptor modules. In some embodiments, the kit comprises about 2 to about 50 sets of adaptor modules. In some embodiments, the kit comprises about 50 to about 100 sets of adaptor modules. In some embodiments, the kit comprises about 5 to about 50 sets of adaptor modules. In some embodiments, the kit comprises about 10 to about 50 sets of adaptor modules. In some embodiments, the kit comprises about 15 to about 50 sets of adaptor modules. In some embodiments, the kit comprises about 20 to about 50 sets of adaptor modules. In some embodiments, the kit comprises about 30 to about 50 sets of adaptor modules. In some embodiments, the kit comprises about 40 to about 50 sets of adaptor modules. In some embodiments, the kit comprises about 45 to about 50 sets of adaptor modules. In some embodiments, the kit comprises about 48 sets of adaptor modules.

In some embodiments of the kits of the disclosure, each set of adaptor modules is unique to a given test sample.

In some embodiments of the kits of the disclosure, the kit comprises one or more reagents for ligating adaptors of the adaptor set to the DNA fragments of the test sample to generate an adaptor-tagged DNA library. In some embodiments, the one or more reagents for ligating adaptors comprises a DNA ligase. In some embodiments, the DNA ligase is T4 DNA ligase.

In some embodiments of the kits of the disclosure, the set of adaptors is configured to ligate to the DNA fragments of the test sample using a method comprising (a) ligating the set of adaptors with the DNA fragments to generate a plurality of adaptor/DNA fragment complexes; and (b) contacting the plurality of adaptor/DNA fragment complexes with one or more enzymes to form an adaptor-tagged DNA library comprising a plurality of adaptor-tagged DNA fragments. In some embodiments, each adaptor/DNA fragment complex comprises a ligation strand oligonucleotide ligated to each end of the DNA fragment. In some embodiments, the non-ligation strand oligonucleotide is displaced from the adaptor/DNA fragment complex in step (b).

In some embodiments of the kits of the disclosure, the kit comprises one or more reagents for amplifying the adaptor-tagged DNA library to create a Library Post-Amplification (LPA). In some embodiments, the one or more reagents for amplifying the adaptor-tagged DNA library comprises a DNA polymerase. In some embodiments, one or more reagents for amplifying the adaptor-tagged DNA library comprises one or more primers for PCR. In some embodiments, the one or more primers comprise a single primer sequence that is complementary to the primer binding site of the adaptor amplification region.

In some embodiments of the kits of the disclosure, the kit comprises one or more reagents for creating an amplified Whole-Genome Library (WGLA) from the LPA. In some embodiments, the one or more reagents for creating a WGLA comprises one or more primers that hybridize to the primer binding site of the adaptor amplification region. In some embodiments, the one or more primers comprise a sequencing adaptor capable of binding to a flow cell. In some embodiments, the sequencing adaptor comprises a sequencing primer binding site. In some embodiments, the one or more reagents for creating the WGLA comprises a DNA polymerase.

In some embodiments of the kits of the disclosure, the WGLA is the sequence ready.

In some embodiments of the kits of the disclosure, the tail sequence of the capture probe module comprises a primer binding site.

In some embodiments of the kits of the disclosure, the tail sequence comprises a sequencing primer binding site.

In some embodiments of the kits of the disclosure, the tail sequence is configured to hybridize to a partner oligonucleotide.

In some embodiments of the kits of the disclosure, each capture probe module is selected from a capture probe panel comprising a plurality of capture probe modules.

In some embodiments of the kits of the disclosure, at least one capture probe module is configured to hybridize downstream of a specific DNA target region and at least one capture probe module is configured to hybridize upstream of the specific DNA target region.

In some embodiments of the kits of the disclosure, each capture probe of the plurality of capture probe modules is configured to hybridize to its target sequence within about 200 bp of any other capture probe.

In some embodiments of the kits of the disclosure, each capture probe module of the capture probe panel hybridizes to an adaptor-tagged DNA fragment to form a plurality of adaptor-tagged DNA fragment-capture probe module complexes.

In some embodiments of the kits of the disclosure, the partner oligonucleotide comprises a specific member of a binding pair to enable isolation of the adaptor-tagged DNA fragment-capture probe module complexes.

In some embodiments of the kits of the disclosure, the partner oligonucleotide comprises a biotin molecule.

In some embodiments of the kits of the disclosure, each capture probe module comprises a specific member of a binding pair to enable isolation of the adaptor-tagged DNA fragment-capture probe module complex.

In some embodiments of the kits of the disclosure, the capture probe module comprises a biotin molecule.

In some embodiments of the kits of the disclosure, each capture probe module comprises a capture probe less than about 60 nucleotides in length.

In some embodiments of the kits of the disclosure, each capture probe module comprises a capture probe less than 60 nucleotides in length.

In some embodiments of the kits of the disclosure, each capture probe module comprises a capture probe that is about 40 nucleotides in length.

In some embodiments of the kits of the disclosure, each capture probe module comprises a capture probe that is 40 nucleotides in length.

In some embodiments of the kits of the disclosure, the kit comprises components for isolating the adaptor-tagged DNA fragment-capture probe module complexes. In some embodiments, the components for isolating the adaptor-tagged DNA fragment-capture probe module complexes comprises streptavidin.

In some embodiments of the kits of the disclosure, the kit comprises one of more enzymes for enzymatically processing the isolated adaptor-tagged DNA fragment-capture probe module complexes. In some embodiments, the one or more enzymes comprise a 5'-3' polymerase configured to extend each capture probe of the adaptor-tagged DNA fragment-capture probe module complexes using the adaptor-tagged DNA fragment as a template to create a plurality of Hybrid Molecules (Target Capture Library), wherein each Hybrid Molecule comprises a capture probe module and a complement of an adaptor-tagged DNA fragment.

In some embodiments of the kits of the disclosure, the kit comprises one or more reagents for amplifying the Hybrid Molecules to create a Target Capture Library Amplified (TCLA), wherein the first modified library is the TCLA. In some embodiments, the one or more reagents for amplifying the Hybrid Molecules comprise one or more primers comprising a sequencing adaptor capable of binding to a flow cell. In some embodiments, the sequencing adaptor comprises a sequencing primer binding site. In some embodiments, the one or more reagents for amplifying the Hybrid Molecules comprise a DNA polymerase.

In some embodiments of the kits of the disclosure, the SRL is configured to be sequenced on a single flow cell of a sequencing machine.

In some embodiments of the kits of the disclosure, the kit comprises components for performing quantitative genetic analysis on the SRL. In some embodiments, the components for performing quantitative genetic analysis comprises one or more sequencing primers. In some embodiments, the quantitative genetic analysis is used to detect a nucleotide transition or transversion, a nucleotide insertion or deletion, a genomic rearrangement, or a change in copy number in the test sample DNA fragments.

In some embodiments of the kits of the disclosure, the test sample is obtained from a tissue biopsy. In some embodiments, the tissue biopsy is obtained from a tumor or a tissue suspected of being a tumor. In some embodiments, the tissue biopsy is obtained from a malignant tumor or tumor suspected of being a malignant tumor.

In some embodiments of the kits of the disclosure, the test sample DNA fragments comprise cell free DNA (cfDNA), genomic DNA (gDNA), complementary DNA (cDNA), mitochondrial DNA, methylated DNA, demethylated DNA, or a combination thereof.

In some embodiments of the kits of the disclosure, the test sample DNA fragments comprise an epigenetic mark.

In some embodiments of the kits of the disclosure, the test sample DNA fragments are obtained from a library selected from the list consisting of a whole genome library, an amplicon library, a whole exome library, a cDNA library, or a methylated DNA library.

In some embodiments of the kits of the disclosure, the test sample is obtained from a biological sample selected from the group consisting of an amniotic fluid sample, a blood sample, a skin sample, a hair sample, a hair follicle sample, a saliva sample, a mucous sample, a sweat sample, a tear sample, an epithelial tissue sample, a urine sample, a semen sample, a seminal plasma sample, a serum sample, a prostatic fluid sample, a pre-ejaculatory fluid (Cowper's fluid) sample, an ocular fluid sample, an excreta sample, a biopsy sample, an ascites sample, a cerebrospinal fluid sample, a lymph sample, a tissue extract sample, a stool sample, and a formalin-fixed, paraffin embedded (FFPE) sample.

In some embodiments of the kits of the disclosure, the DNA fragments of the test sample have been end repaired prior to ligation to adaptors.

In some embodiments of the kits of the disclosure, the kit comprises one or more reagents for performing end repair. In some embodiments, the one or more reagents for performing end repair comprise one or more enzymes selected from a DNA polymerase, a kinase, and a Klenow fragment. In some embodiments, the one or more reagents for performing end repair comprise a DNA polymerase I, a T4 DNA polymerase, a T4 polynucleotide kinase, and a Klenow fragment. In some embodiments, the one or more reagents for performing end repair comprise an End Repair Buffer. In some embodiments, the End Repair Buffer comprises MgCl2, NaCl, Tris-HCL, DTT, KCL, and dNTPs.

Some embodiments of the disclosure provide methods for performing an adaptor Quality Control (QC) process on a set of adaptors, wherein each adaptor comprises an adaptor module.

In some embodiments of the methods for performing an adaptor QC process of the disclosure, the adaptor QC process comprises a test for adaptor ligation, wherein the test for adaptor ligation comprises (a) ligating the set of adaptors to a pre-determined amount of end-repaired DNA fragments to generate a library of adaptor-tagged DNA fragments (LIBS); and (b) amplifying the LIBS to generate a Library Post Amplification (LPA); wherein the set of adaptors is considered to have passed the test for adaptor ligation when the concentration of the LPA is higher than a pre-determined concentration.

In some embodiments of the methods for performing an adaptor QC process of the disclosure, the end-repaired DNA fragments of step (a) of the test for adaptor ligation is generated from a DNA sample. In some embodiments, said DNA sample is a blended DNA sample of at least two different cell lines. In some embodiments, said DNA sample is a blended DNA sample of at two different cell lines. In some embodiments, the two different cell lines are blended at a 50:50 ratio. In some embodiments, the two different cell lines are NA09596 and NA12878. In some embodiments, said DNA sample is the 50:50 blended sample described in Example 15.

In some embodiments of the methods for performing an adaptor QC process of the disclosure, the DNA sample used in the test for adaptor ligation is different from the DNA sample used in the test for adaptor distribution.

In some embodiments of the methods for performing an adaptor QC process of the disclosure, the pre-determined amount of end-repaired DNA fragments is about 5 ng. In some embodiments of the methods for performing an adaptor QC process of the disclosure, the pre-determined amount of end-repaired DNA fragments is about 10 ng. In some embodiments of the methods for performing an adaptor QC process of the disclosure, the pre-determined amount of end-repaired DNA fragments is about 15 ng. In some embodiments of the methods for performing an adaptor QC process of the disclosure, the pre-determined amount of end-repaired DNA fragments is about 20 ng. In some embodiments of the methods for performing an adaptor QC process of the disclosure, the pre-determined amount of end-repaired DNA fragments is about 25 ng. In some embodiments of the methods for performing an adaptor QC process of the disclosure, the pre-determined amount of end-repaired DNA fragments is about 30 ng. In some embodiments of the methods for performing an adaptor QC process of the disclosure, the pre-determined amount of end-repaired DNA fragments is about 35 ng. In some embodiments of the methods for performing an adaptor QC process of the disclosure, the pre-determined amount of end-repaired DNA fragments is about 40 ng. In some embodiments of the methods for performing an adaptor QC process of the disclosure, the pre-determined amount of end-repaired DNA fragments is about 50 ng.

In some embodiments of the methods for performing an adaptor QC process of the disclosure, the pre-determined amount of end-repaired DNA fragments is 5 ng. In some embodiments of the methods for performing an adaptor QC process of the disclosure, the pre-determined amount of end-repaired DNA fragments is 10 ng. In some embodiments of the methods for performing an adaptor QC process of the disclosure, the pre-determined amount of end-repaired DNA fragments is 15 ng. In some embodiments of the methods for performing an adaptor QC process of the disclosure, the pre-determined amount of end-repaired DNA fragments is 20 ng. In some embodiments of the methods for performing an adaptor QC process of the disclosure, the pre-determined amount of end-repaired DNA fragments is 25 ng. In some embodiments of the methods for performing an adaptor QC process of the disclosure, the pre-determined amount of end-repaired DNA fragments is 30 ng. In some embodiments of the methods for performing an adaptor QC process of the disclosure, the pre-determined amount of end-repaired DNA fragments is 35 ng. In some embodiments of the methods for performing an adaptor QC process of the disclosure, the pre-determined amount is 40 ng. In some embodiments of the methods for performing an adaptor QC process of the disclosure, the pre-determined amount of end-repaired DNA fragments is 50 ng.

In some embodiments of the methods for performing an adaptor QC process of the disclosure, the pre-determined amount of end-repaired DNA fragments is about 1 ng to about 100 ng. In some embodiments of the methods for performing an adaptor QC process of the disclosure, the pre-determined amount of end-repaired DNA fragments is about 1 ng to about 50 ng. In some embodiments of the methods for performing an adaptor QC process of the disclosure, the pre-determined amount of end-repaired DNA fragments is about 50 ng to about 100 ng. In some embodiments of the methods for performing an adaptor QC process of the disclosure, the pre-determined amount of end-repaired DNA fragments is about 5 ng to about 90 ng. In some embodiments of the methods for performing an adaptor QC process of the disclosure, the pre-determined amount of end-repaired DNA fragments is about 10 ng to about 80 ng. In some embodiments of the methods for performing an adaptor QC process of the disclosure, the pre-determined amount of end-repaired DNA fragments is about 20 ng to about 60 ng. In some embodiments of the methods for performing an adaptor QC process of the disclosure, the pre-determined amount of end-repaired DNA fragments is about 20 ng to about 50 ng. In some embodiments of the methods for performing an adaptor QC process of the disclosure, the pre-determined amount of end-repaired DNA fragments is about 20 ng to about 40 ng. In some embodiments of the methods for performing an adaptor QC process of the disclosure, the pre-determined amount of end-repaired DNA fragments is about 30 ng to about 50 ng. In some embodiments of the methods for performing an adaptor QC process of the disclosure, the pre-determined amount of end-repaired DNA fragments is about 40 ng to about 50 ng. In some embodiments of the methods for performing an adaptor QC process of the disclosure, the pre-determined amount of end-repaired DNA fragments is about 30 ng to about 40 ng. In some embodiments of the methods for performing an adaptor QC process of the disclosure, the pre-determined amount of end-repaired DNA fragments is about 20 ng to about 30 ng. In some embodiments of the methods for performing an adaptor QC process of the disclosure, the pre-determined amount of end-repaired DNA fragments is greater than about 100 ng.

In some embodiments of the methods for performing an adaptor QC process of the disclosure, the pre-determined amount of end-repaired DNA fragments is 1 ng to 100 ng. In some embodiments of the methods for performing an adaptor QC process of the disclosure, the pre-determined amount of end-repaired DNA fragments is 1 ng to 50 ng. In some embodiments of the methods for performing an adaptor QC process of the disclosure, the pre-determined amount of end-repaired DNA fragments is 50 ng to 100 ng. In some embodiments of the methods for performing an adaptor QC process of the disclosure, the pre-determined amount of end-repaired DNA fragments is 5 ng to 90 ng. In some embodiments of the methods for performing an adaptor QC process of the disclosure, the pre-determined amount of end-repaired DNA fragments is 10 ng to 80 ng. In some embodiments of the methods for performing an adaptor QC process of the disclosure, the pre-determined amount of end-repaired DNA fragments is 20 ng to 60 ng. In some embodiments of the methods for performing an adaptor QC process of the disclosure, the pre-determined amount of end-repaired DNA fragments is 20 ng to 50 ng. In some embodiments of the methods for performing an adaptor QC process of the disclosure, the pre-determined amount of end-repaired DNA fragments is 20 ng to 40 ng. In some embodiments of the methods for performing an adaptor QC process of the disclosure, the pre-determined amount of end-repaired DNA fragments is 30 ng to 50 ng. In some embodiments of the methods for performing an adaptor QC process of the disclosure, the pre-determined amount of end-repaired DNA fragments is 40 ng to 50 ng. In some embodiments of the methods for performing an adaptor QC process of the disclosure, the pre-determined amount of end-repaired DNA fragments is 30 ng to 40 ng. In some embodiments of the methods for performing an adaptor QC process of the disclosure, the pre-determined amount of end-repaired DNA fragments is 20 ng to 30 ng. In some embodiments of the methods for performing an adaptor QC process of the disclosure, the pre-determined amount of end-repaired DNA fragments is greater than 100 ng.

In some embodiments of the methods for performing an adaptor QC process of the disclosure, the pre-determined concentration of the LPA is about 1 ng/µL. In some embodiments of the methods for performing an adaptor QC process of the disclosure, the pre-determined concentration of the LPA is about 5 ng/µL. In some embodiments of the methods for performing an adaptor QC process of the disclosure, the pre-determined concentration of the LPA is about 10 ng/µL. In some embodiments of the methods for performing an adaptor QC process of the disclosure, the pre-determined concentration of the LPA is about 20 ng/µL. In some embodiments of the methods for performing an adaptor QC process of the disclosure, the pre-determined concentration of the LPA is about 30 ng/µL. In some embodiments of the methods for performing an adaptor QC process of the disclosure, the pre-determined concentration of the LPA is about 40 ng/µL. In some embodiments of the methods for performing an adaptor QC process of the disclosure, the pre-determined concentration of the LPA is about 50 ng/µL. In some embodiments of the methods for performing an adaptor QC process of the disclosure, the pre-determined concentration of the LPA is about 60 ng/µL. In some embodiments of the methods for performing an adaptor QC process of the disclosure, the pre-determined concentration of the LPA is about 70 ng/µL. In some embodiments of the methods for performing an adaptor QC process of the disclosure, the pre-determined concentration of the LPA is about 80 ng/µL. In some embodiments of the methods for performing an adaptor QC process of the disclosure, the pre-determined concentration of the LPA is about 90 ng/µL. In some embodiments of the methods for performing an adaptor QC process of the disclosure, the pre-determined concentration of the LPA is about 100 ng/µL. In some embodiments of the methods for performing an adaptor QC process of the disclosure, the pre-determined concentration of the LPA is about 150 ng/µL. In some embodiments of the methods for performing an adaptor QC process of the disclosure, the pre-determined concentration of the LPA is about 200 ng/µL.

In some embodiments of the methods for performing an adaptor QC process of the disclosure, the pre-determined concentration of the LPA is 1 ng/µL. In some embodiments of the methods for performing an adaptor QC process of the disclosure, the pre-determined concentration of the LPA is 5 ng/µL. In some embodiments of the methods for performing an adaptor QC process of the disclosure, the pre-determined concentration of the LPA is 10 ng/µL. In some embodiments of the methods for performing an adaptor QC process of the disclosure, the pre-determined concentration of the LPA is 20 ng/µL. In some embodiments of the methods for performing an adaptor QC process of the disclosure, the pre-determined concentration of the LPA is 30 ng/µL. In some embodiments of the methods for performing an adaptor QC process of the disclosure, the pre-determined concentration of the LPA is 40 ng/µL. In some embodiments of the methods for performing an adaptor QC process of the disclosure, the pre-determined concentration of the LPA is 50 ng/µL. In some embodiments of the methods for performing an adaptor QC process of the disclosure, the pre-determined concentration of the LPA is 60 ng/µL. In some embodiments of the methods for performing an adaptor QC process of the disclosure, the pre-determined concentration of the LPA is 70 ng/µL. In some embodiments of the methods for performing an adaptor QC process of the disclosure, the pre-determined concentration of the LPA is 80 ng/µL. In some embodiments of the methods for performing an adaptor QC process of the disclosure, the pre-determined concentration of the LPA is 90 ng/µL. In some embodiments of the methods for performing an adaptor QC process of the disclosure, the pre-determined concentration of the LPA is 100 ng/µL. In some embodiments of the methods for performing an adaptor QC process of the disclosure, the pre-determined concentration of the LPA is 150 ng/µL. In some embodiments of the methods for performing an adaptor QC process of the disclosure, the pre-determined concentration of the LPA is 200 ng/µL.

In some embodiments of the methods for performing an adaptor QC process of the disclosure, the pre-determined concentration of the LPA is about 1 ng/µL to about 200 ng/µL. In some embodiments of the methods for performing an adaptor QC process of the disclosure, the pre-determined concentration of the LPA is greater than about 200 ng/µL. In some embodiments of the methods for performing an adaptor QC process of the disclosure, the pre-determined concentration of the LPA is about 10 ng/µL to about 100 ng/µL. In some embodiments of the methods for performing an adaptor QC process of the disclosure, the pre-determined concentration of the LPA is about 10 ng/µL to about 90 ng/µL. In some embodiments of the methods for performing an adaptor QC process of the disclosure, the pre-determined concentration of the LPA is about 20 ng/µL to about 80 ng/µL. In some embodiments of the methods for performing an adaptor QC process of the disclosure, the pre-determined concentration of the LPA is about 10 ng/µL to about 80 ng/µL. In some embodiments of the methods for performing an adaptor QC process of the disclosure, the pre-determined concentration of the LPA is about 30 ng/µL to about 80 ng/µL. In some embodiments of the methods for performing an adaptor QC process of the disclosure, the pre-determined concentration of the LPA is about 40 ng/µL to about 80 ng/µL. In some embodiments of the methods for performing an adaptor QC process of the disclosure, the pre-determined concentration of the LPA is about 50 ng/µL to about 80 ng/µL. In some embodiments of the methods for performing an adaptor QC process of the disclosure, the pre-determined concentration of the LPA is about 50 ng/µL to about 70 ng/µL. In some embodiments of the methods for performing an adaptor QC process of the disclosure, the pre-determined concentration of the LPA is about 60 ng/µL to about 70 ng/µL. In some embodiments of the methods for performing an adaptor QC process of the disclosure, the pre-determined concentration of the LPA is about 50 ng/µL to about 60 ng/µL.

In some embodiments of the methods for performing an adaptor QC process of the disclosure, the pre-determined concentration of the LPA is 1 ng/µL to 200 ng/µL. In some embodiments of the methods for performing an adaptor QC process of the disclosure, the pre-determined concentration of the LPA is 10 ng/µL to 100 ng/µL. In some embodiments of the methods for performing an adaptor QC process of the disclosure, the pre-determined concentration of the LPA is 10 ng/µL to 90 ng/µL. In some embodiments of the methods for performing an adaptor QC process of the disclosure, the pre-determined concentration of the LPA is 20 ng/µL to 80 ng/µL. In some embodiments of the methods for performing an adaptor QC process of the disclosure, the pre-determined concentration of the LPA is 10 ng/µL to 80 ng/µL. In some embodiments of the methods for performing an adaptor QC process of the disclosure, the pre-determined concentration of the LPA is 30 ng/µL to 80 ng/µL. In some embodiments of the methods for performing an adaptor QC process of the disclosure, the pre-determined concentration of the LPA is 40 ng/µL to 80 ng/µL. In some embodiments of the methods for performing an adaptor QC process of the disclosure, the pre-determined concentration of the LPA is 50 ng/µL to 80 ng/µL. In some embodiments of the methods for performing an adaptor QC process of the disclosure, the pre-determined concentration of the LPA is 50 ng/µL to 70 ng/µL. In some embodiments of the methods for performing an adaptor QC process of the disclosure, the pre-determined concentration of the LPA is 60 ng/µL to 70 ng/µL. In some embodiments of the methods for performing an adaptor QC process of the disclosure, the pre-determined concentration of the LPA is 50 ng/µL to 60 ng/µL.

In some embodiments of the methods for performing an adaptor QC process of the disclosure, the pre-determined concentration of the LPA is greater than about 200 ng/µL. In some embodiments of the methods for performing an adaptor QC process of the disclosure, the pre-determined concentration of the LPA is greater than 200 ng/µL.

In some embodiments of the methods for performing an adaptor QC process of the disclosure, the set of adaptors is considered to have passed the adaptor QC process when it has passed the test for adaptor ligation.

In some embodiments of the methods for performing an adaptor QC process of the disclosure, the adaptor QC process comprises a test for adaptor distribution comprising (a) ligating the set of adaptors to a pre-determined amount of end-repaired DNA fragments to generate a library of adaptor-tagged DNA fragments (LIBS); (b) amplifying the LIBS using at least one primer comprising an index sequence to generate a Library Post Index Amplification (LPIA); and (c) performing a quantitative genetic analysis on the LPIA, wherein the set of adaptors is considered to have passed the test for adaptor distribution when one or more pre-determined acceptance criteria for the quantitative genetic analysis has been met.

In some embodiments of the methods for performing an adaptor QC process of the disclosure, the end-repaired DNA fragments of step (a) of the test for adaptor distribution are generated from a DNA sample. In some embodiments, said DNA sample comprises wild-type (wt) cell-free DNA (cfDNA). In some embodiments, said DNA sample consists of wt cfDNA. In some embodiments, said DNA sample comprises wt cfDNA obtained from a healthy human. In some embodiments, said DNA sample is the wt cfDNA sample described in Example 16.

In some embodiments of the methods for performing an adaptor QC process of the disclosure, the DNA sample used in the test for adaptor distribution is different from the DNA sample used in the test for adaptor ligation.

In some embodiments of the methods for performing an adaptor QC process of the disclosure, the pre-determined acceptance criteria for the adaptor QC process comprise a criterion (a). In some embodiments, criterion (a) is Barcode Crosstalk is present in no more than about 0.05% of reads. In some embodiments, criterion (a) is Barcode Crosstalk is present in no more than about 0.1% of reads. In some embodiments, criterion (a) is Barcode Crosstalk is present in no more than about 0.2% of reads. In some embodiments, criterion (a) is Barcode Crosstalk is present in no more than about 0.3% of reads. In some embodiments, criterion (a) is Barcode Crosstalk is present in no more than about 0.4% of reads. In some embodiments, criterion (a) is Barcode Crosstalk is present in no more than about 0.5% of reads. In some embodiments, criterion (a) is Barcode Crosstalk is present in no more than about 0.6% of reads. In some embodiments, criterion (a) is Barcode Crosstalk is present in no more than about 0.7% of reads. In some embodiments, criterion (a) is Barcode Crosstalk is present in no more than about 0.8% of reads. In some embodiments, criterion (a) is Barcode Crosstalk is present in no more than about 0.9% of reads. In some embodiments, criterion (a) is Barcode Crosstalk present in no more than about 1% of reads. In some embodiments, criterion (a) is Barcode Crosstalk present in no more than about 2% of reads. In some embodiments, criterion (a) is Barcode Crosstalk present in no more than about 3% of reads. In some embodiments, criterion (a) is Barcode Crosstalk present in no more than about 4% of reads. In some embodiments, criterion (a) is Barcode Crosstalk present in no more than about 5% of reads.

In some embodiments, criterion (a) is Barcode Crosstalk is present in no more than 0.05% of reads. In some embodiments, criterion (a) is Barcode Crosstalk is present in no more than 0.1% of reads. In some embodiments, criterion (a) is Barcode Crosstalk is present in no more than 0.2% of reads. In some embodiments, criterion (a) is Barcode Crosstalk present in no more than 0.3% of reads. In some embodiments, criterion (a) is Barcode Crosstalk present in no more than 0.4% of reads. In some embodiments, criterion (a) is Barcode Crosstalk is present in no more than 0.5% of reads. In some embodiments, criterion (a) is Barcode Crosstalk is present in no more than 0.6% of reads. In some embodiments, criterion (a) is Barcode Crosstalk is present in no more than 0.7% of reads. In some embodiments, criterion (a) is Barcode Crosstalk is present in no more than 0.8% of reads. In some embodiments, criterion (a) is Barcode Crosstalk present in no more than 0.9% of reads. In some embodiments, criterion (a) is Barcode Crosstalk present in no more than 1% of reads. In some embodiments, criterion (a) is Barcode Crosstalk present in no more than 2% of reads. In some embodiments, criterion (a) is Barcode Crosstalk present in no more than 3% of reads. In some embodiments, criterion (a) is Barcode Crosstalk present in no more than 4% of reads. In some embodiments, criterion (a) is Barcode Crosstalk present in no more than 5% of reads.

In some embodiments, criterion (a) is Barcode Crosstalk is present in no more than about 0.05% to about 10% of reads. In some embodiments, criterion (a) is Barcode Crosstalk is present in no more than about 0.05% to about 5% of reads. In some embodiments, criterion (a) is Barcode Crosstalk is present in no more than about 0.1% to about 5% of reads. In some embodiments, criterion (a) is Barcode Crosstalk is present in no more than about 0.05% to about 2% of reads. In some embodiments, criterion (a) is Barcode Crosstalk is present in no more than about 0.1% to about 1% of reads. In some embodiments, criterion (a) is Barcode Crosstalk is present in no more than about 0.1% to about 0.5% of reads. In some embodiments, criterion (a) is Barcode Crosstalk is present in no more than about 0.1% to about 0.4% of reads. In some embodiments, criterion (a) is Barcode Crosstalk is present in no more than about 0.1% to about 0.2% of reads.

In some embodiments, criterion (a) is Barcode Crosstalk is present in no more than 0.05% to 10% of reads. In some embodiments, criterion (a) is Barcode Crosstalk is present in no more than 0.05% to 5% of reads. In some embodiments, criterion (a) is Barcode Crosstalk is present in no more than 0.1% to 5% of reads. In some embodiments, criterion (a) is Barcode Crosstalk is present in no more than 0.05% to 2% of reads. In some embodiments, criterion (a) is Barcode Crosstalk is present in no more than 0.1% to 1% of reads. In some embodiments, criterion (a) is Barcode Crosstalk is present in no more than 0.1% to 0.5% of reads. In some embodiments, criterion (a) is Barcode Crosstalk is present in no more than 0.1% to 0.4% of reads. In some embodiments, criterion (a) is Barcode Crosstalk is present in no more than 0.1% to 0.2% of reads.

In some embodiments of the methods for performing an adaptor QC process of the disclosure, the pre-determined acceptance criteria for the adaptor QC process comprise a criterion (b). In some embodiments, criterion (b) is unknown adaptors are present in no more than about 1% of reads. In some embodiments, criterion (b) is unknown adaptors are present in no more than about 5% of reads. In some embodiments, criterion (b) is unknown adaptors are present in no more than about 10% of reads. In some embodiments, criterion (b) is unknown adaptors are present in no more than about 15% of reads. In some embodiments, criterion (b) is unknown adaptors are present in no more than about 20% of reads. In some embodiments, criterion (b) is unknown adaptors are present in no more than about 25% of reads. In some embodiments, criterion (b) is unknown adaptors are present in no more than about 30% of reads. In some embodiments, criterion (b) is unknown adaptors are present in no more than about 35% of reads. In some embodiments, criterion (b) is unknown adaptors are present in no more than about 40% of reads. In some embodiments, criterion (b) is unknown adaptors are present in no more than about 45% of reads. In some embodiments, criterion (b) is unknown adaptors are present in no more than about 50% of reads.

In some embodiments, criterion (b) is unknown adaptors are present in no more than 1% of reads. In some embodiments, criterion (b) is unknown adaptors are present in no more than 5% of reads. In some embodiments, criterion (b) is unknown adaptors are present in no more than 10% of reads. In some embodiments, criterion (b) is unknown adaptors are present in no more than 15% of reads. In some embodiments, criterion (b) is unknown adaptors are present in no more than 20% of reads. In some embodiments, criterion (b) is unknown adaptors are present in no more than 25% of reads. In some embodiments, criterion (b) is unknown adaptors are present in no more than 30% of reads. In some embodiments, criterion (b) is unknown adaptors are present in no more than 35% of reads. In some embodiments, criterion (b) is unknown adaptors are present in no more than 40% of reads. In some embodiments, criterion (b) is unknown adaptors are present in no more than 45% of reads. In some embodiments, criterion (b) is unknown adaptors are present in no more than 50% of reads.

In some embodiments, criterion (b) is unknown adaptors are present in no more than about 1% to about 50% of reads. In some embodiments, criterion (b) is unknown adaptors are present in no more than about 1% to about 20% of reads. In some embodiments, criterion (b) is unknown adaptors are present in no more than about 1% to about 15% of reads. In some embodiments, criterion (b) is unknown adaptors are present in no more than about 1% to about 10% of reads. In some embodiments, criterion (b) is unknown adaptors are present in no more than about 5% to about 20% of reads. In some embodiments, criterion (b) is unknown adaptors are present in no more than about 5% to about 15% of reads. In some embodiments, criterion (b) is unknown adaptors are present in no more than about 5% to about 10% of reads.

In some embodiments, criterion (b) is unknown adaptors are present in no more than 1% to 50% of reads. In some embodiments, criterion (b) is unknown adaptors are present in no more than 1% to 20% of reads. In some embodiments, criterion (b) is unknown adaptors are present in no more than 1% to 15% of reads. In some embodiments, criterion (b) is unknown adaptors are present in no more than 1% to 10% of reads. In some embodiments, criterion (b) is unknown adaptors are present in no more than 5% to 20% of reads. In some embodiments, criterion (b) is unknown adaptors are present in no more than 5% to 15% of reads. In some embodiments, criterion (b) is unknown adaptors are present in no more than 5% to 10% of reads.

In some embodiments of the methods for performing an adaptor QC process of the disclosure, the pre-determined acceptance criteria for the adaptor QC process comprise a criterion (c). In some embodiments, criterion (c) is no more than about 10% of unique adaptor sequences have a number of reads that is 0% to 50% of the average number of reads for all unique adaptor sequences. In some embodiments, criterion (c) is no more than about 20% of unique adaptor sequences have a number of reads that is 0% to 50% of the average number of reads for all unique adaptor sequences. In some embodiments, criterion (c) is no more than about 30% of unique adaptor sequences have a number of reads that is 0% to 50% of the average number of reads for all unique adaptor sequences. In some embodiments, criterion (c) is no more than about 40% of unique adaptor sequences have a number of reads that is 0% to 50% of the average number of reads for all unique adaptor sequences. In some embodiments, criterion (c) is no more than about 50% of unique adaptor sequences have a number of reads that is 0% to 50% of the average number of reads for all unique adaptor sequences. In some embodiments, criterion (c) is no more than about 60% of unique adaptor sequences have a number of reads that is 0% to 50% of the average number of reads for all unique adaptor sequences. In some embodiments, criterion (c) is no more than about 70% of unique adaptor sequences have a number of reads that is 0% to 50% of the average number of reads for all unique adaptor sequences. In some embodiments, criterion (c) is no more than about 80% of unique adaptor sequences have a number of reads that is 0% to 50% of the average number of reads for all unique adaptor sequences. In some embodiments, criterion (c) is no more than about 90% of unique adaptor sequences have a number of reads that is 0% to 50% of the average number of reads for all unique adaptor sequences.

In some embodiments, the pre-determined acceptance criteria for the adaptor QC process comprise a criterion (c). In some embodiments, criterion (c) is no more than 10% of unique adaptor sequences have a number of reads that is 0% to 50% of the average number of reads for all unique adaptor sequences. In some embodiments, criterion (c) is no more than 20% of unique adaptor sequences have a number of reads that is 0% to 50% of the average number of reads for all unique adaptor sequences. In some embodiments, criterion (c) is no more than 30% of unique adaptor sequences have a number of reads that is 0% to 50% of the average number of reads for all unique adaptor sequences. In some embodiments, criterion (c) is no more than 40% of unique adaptor sequences have a number of reads that is 0% to 50% of the average number of reads for all unique adaptor sequences. In some embodiments, criterion (c) is no more than 50% of unique adaptor sequences have a number of reads that is 0% to 50% of the average number of reads for all unique adaptor sequences. In some embodiments, criterion (c) is no more than 60% of unique adaptor sequences have a number of reads that is 0% to 50% of the average number of reads for all unique adaptor sequences. In some embodiments, criterion (c) is no more than 70% of unique adaptor sequences have a number of reads that is 0% to 50% of the average number of reads for all unique adaptor sequences. In some embodiments, criterion (c) is no more than 80% of unique adaptor sequences have a number of reads that is 0% to 50% of the average number of reads for all unique adaptor sequences. In some embodiments, criterion (c) is no more than 90% of unique adaptor sequences have a number of reads that is 0% to 50% of the average number of reads for all unique adaptor sequences.

In some embodiments, criterion (c) is no more than about 10% to about 90% of unique adaptor sequences have a number of reads that is 0% to 50% of the average number of reads for all unique adaptor sequences. In some embodiments, criterion (c) is no more than about 10% to about 80% of unique adaptor sequences have a number of reads that is 0% to 50% of the average number of reads for all unique adaptor sequences. In some embodiments, criterion (c) is no more than about 20% to about 80% of unique adaptor sequences have a number of reads that is 0% to 50% of the average number of reads for all unique adaptor sequences. In some embodiments, criterion (c) is no more than about 30% to about 70% of unique adaptor sequences have a number of reads that is 0% to 50% of the average number of reads for all unique adaptor sequences. In some embodiments, criterion (c) is no more than about 40% to about 60% of unique adaptor sequences have a number of reads that is 0% to 50% of the average number of reads for all unique adaptor sequences. In some embodiments, criterion (c) is no more than about 40% to about 50% of unique adaptor sequences have a number of reads that is 0% to 50% of the average number of reads for all unique adaptor sequences. In some embodiments, criterion (c) is no more than about 50% to about 60% of unique adaptor sequences have a number of reads that is 0% to 50% of the average number of reads for all unique adaptor sequences.

In some embodiments, criterion (c) is no more than 10% to 90% of unique adaptor sequences have a number of reads that is 0% to 50% of the average number of reads for all unique adaptor sequences. In some embodiments, criterion (c) is no more than 10% to 80% of unique adaptor sequences have a number of reads that is 0% to 50% of the average number of reads for all unique adaptor sequences. In some embodiments, criterion (c) is no more than 20% to 80% of unique adaptor sequences have a number of reads that is 0% to 50% of the average number of reads for all unique adaptor sequences. In some embodiments, criterion (c) is no more than 30% to 70% of unique adaptor sequences have a number of reads that is 0% to 50% of the average number of reads for all unique adaptor sequences. In some embodiments, criterion (c) is no more than 40% to 60% of unique adaptor sequences have a number of reads that is 0% to 50% of the average number of reads for all unique adaptor sequences. In some embodiments, criterion (c) is no more than 40% to 50% of unique adaptor sequences have a number of reads that is 0% to 50% of the average number of reads for all unique adaptor sequences. In some embodiments, criterion (c) is no more than 50% to 60% of unique adaptor sequences have a number of reads that is 0% to 50% of the average number of reads for all unique adaptor sequences.

In some embodiments of the methods for performing an adaptor QC process of the disclosure, the pre-determined acceptance criteria for the adaptor QC process comprise a criterion (d). In some embodiments, criterion (d) is at least about 60% of all unique adaptor sequences are present. In some embodiments, criterion (d) is at least about 65% of all unique adaptor sequences are present. In some embodiments, criterion (d) is at least about 70% of all unique adaptor sequences are present. In some embodiments, criterion (d) is at least about 75% of all unique adaptor sequences are present. In some embodiments, criterion (d) is at least about 80% of all unique adaptor sequences are present. In some embodiments, criterion (d) is at least about 85% of all unique adaptor sequences are present. In some embodiments, criterion (d) is at least about 90% of all unique adaptor sequences are present. In some embodiments, criterion (d) is at least about 95% of all unique adaptor sequences are present. In some embodiments, criterion (d) is at least about 96% of all unique adaptor sequences are present. In some embodiments, criterion (d) is at least about 97% of all unique adaptor sequences are present. In some embodiments, criterion (d) is at least about 98% of all unique adaptor sequences are present. In some embodiments, criterion (d) is at least about 99% of all unique adaptor sequences are present. In some embodiments, criterion (d) is at least about 99.5% of all unique adaptor sequences are present. In some embodiments, criterion (d) is at least about 99.9% of all unique adaptor sequences are present. In some embodiments, criterion (d) is about 100% of all unique adaptor sequences are present.

In some embodiments, criterion (d) is at least 60% of all unique adaptor sequences are present. In some embodiments, criterion (d) is at least 65% of all unique adaptor sequences are present. In some embodiments, criterion (d) is at least 70% of all unique adaptor sequences are present. In some embodiments, criterion (d) is at least 75% of all unique adaptor sequences are present. In some embodiments, criterion (d) is at least 80% of all unique adaptor sequences are present. In some embodiments, criterion (d) is at least 85% of all unique adaptor sequences are present. In some embodiments, criterion (d) is at least 90% of all unique adaptor sequences are present. In some embodiments, criterion (d) is at least 95% of all unique adaptor sequences are present. In some embodiments, criterion (d) is at least 96% of all unique adaptor sequences are present. In some embodiments, criterion (d) is at least 97% of all unique adaptor sequences are present. In some embodiments, criterion (d) is at least 98% of all unique adaptor sequences are present. In some embodiments, criterion (d) is at least 99% of all unique adaptor sequences are present. In some embodiments, criterion (d) is at least 99.5% of all unique adaptor sequences are present. In some embodiments, criterion (d) is at least 99.9% of all unique adaptor sequences are present. In some embodiments, criterion (d) is 100% of all unique adaptor sequences are present.

In some embodiments, criterion (d) is at least about 60% to about 99.9% of all unique adaptor sequences are present. In some embodiments, criterion (d) is at least about 70% to about 99.9% of all unique adaptor sequences are present. In some embodiments, criterion (d) is at least about 80% to about 99.9% of all unique adaptor sequences are present. In some embodiments, criterion (d) is at least about 90% to about 99.9% of all unique adaptor sequences are present. In some embodiments, criterion (d) is at least about 95% to about 99.9% of all unique adaptor sequences are present. In some embodiments, criterion (d) is at least about 96% to about 99.9% of all unique adaptor sequences are present. In some embodiments, criterion (d) is at least about 97% to about 99.9% of all unique adaptor sequences are present. In some embodiments, criterion (d) is at least about 98% to about 99.9% of all unique adaptor sequences are present. In some embodiments, criterion (d) is at least about 99% to about 99.9% of all unique adaptor sequences are present.

In some embodiments, criterion (d) is at least 60% to 99.9% of all unique adaptor sequences are present. In some embodiments, criterion (d) is at least 70% to 99.9% of all unique adaptor sequences are present. In some embodiments, criterion (d) is at least 80% to 99.9% of all unique adaptor sequences are present. In some embodiments, criterion (d) is at least 90% to 99.9% of all unique adaptor sequences are present. In some embodiments, criterion (d) is at least 95% to 99.9% of all unique adaptor sequences are present. In some embodiments, criterion (d) is at least 96% to 99.9% of all unique adaptor sequences are present. In some embodiments, criterion (d) is at least 97% to 99.9% of all unique adaptor sequences are present. In some embodiments, criterion (d) is at least 98% to 99.9% of all unique adaptor sequences are present. In some embodiments, criterion (d) is at least 99% to 99.9% of all unique adaptor sequences are present.

In some embodiments, criterion (d) is at least about 60% to about 100% of all unique adaptor sequences are present. In some embodiments, criterion (d) is at least about 70% to about 100% of all unique adaptor sequences are present. In some embodiments, criterion (d) is at least about 80% to about 100% of all unique adaptor sequences are present. In some embodiments, criterion (d) is at least about 90% to about 100% of all unique adaptor sequences are present. In some embodiments, criterion (d) is at least about 95% to about 100% of all unique adaptor sequences are present. In some embodiments, criterion (d) is at least about 96% to about 100% of all unique adaptor sequences are present. In some embodiments, criterion (d) is at least about 97% to about 100% of all unique adaptor sequences are present. In some embodiments, criterion (d) is at least about 98% to about 100% of all unique adaptor sequences are present. In some embodiments, criterion (d) is at least about 99% to about 100% of all unique adaptor sequences are present.

In some embodiments, criterion (d) is at least 60% to 100% of all unique adaptor sequences are present. In some embodiments, criterion (d) is at least 70% to 100% of all unique adaptor sequences are present. In some embodiments, criterion (d) is at least 80% to 100% of all unique adaptor sequences are present. In some embodiments, criterion (d) is at least 90% to 100% of all unique adaptor sequences are present. In some embodiments, criterion (d) is at least 95% to 100% of all unique adaptor sequences are present. In some embodiments, criterion (d) is at least 96% to 100% of all unique adaptor sequences are present. In some embodiments, criterion (d) is at least 97% to 100% of all unique adaptor sequences are present. In some embodiments, criterion (d) is at least 98% to 100% of all unique adaptor sequences are present. In some embodiments, criterion (d) is at least 99% to 100% of all unique adaptor sequences are present.

In some embodiments of the methods for performing an adaptor QC process of the disclosure, the pre-determined acceptance criteria for the adaptor QC process comprise a criterion (e). In some embodiments, criterion (e) is no more than about 5% of unique adaptor sequences have reads greater than twice the average number of reads for all unique adaptor sequences. In some embodiments, criterion (e) is no more than about 10% of unique adaptor sequences have reads greater than twice the average number of reads for all unique adaptor sequences. In some embodiments, criterion (e) is no more than about 15% of unique adaptor sequences have reads greater than twice the average number of reads for all unique adaptor sequences. In some embodiments, criterion (e) is no more than about 20% of unique adaptor sequences have reads greater than twice the average number of reads for all unique adaptor sequences. In some embodiments, criterion (e) is no more than about 25% of unique adaptor sequences have reads greater than twice the average number of reads for all unique adaptor sequences. In some embodiments, criterion (e) is no more than about 30% of unique adaptor sequences have reads greater than twice the average number of reads for all unique adaptor sequences. In some embodiments, criterion (e) is no more than about 35% of unique adaptor sequences have reads greater than twice the average number of reads for all unique adaptor sequences. In some embodiments, criterion (e) is no more than about 40% of unique adaptor sequences have reads greater than twice the average number of reads for all unique adaptor sequences. In some embodiments, criterion (e) is no more than about 45% of unique adaptor sequences have reads greater than twice the average number of reads for all unique adaptor sequences. In some embodiments, criterion (e) is no more than about 50% of unique adaptor sequences have reads greater than twice the average number of reads for all unique adaptor sequences.

In some embodiments, criterion (e) is no more than 5% of unique adaptor sequences have reads greater than twice the average number of reads for all unique adaptor sequences. In some embodiments, criterion (e) is no more than 10% of unique adaptor sequences have reads greater than twice the average number of reads for all unique adaptor sequences. In some embodiments, criterion (e) is no more than 15% of unique adaptor sequences have reads greater than twice the average number of reads for all unique adaptor sequences. In some embodiments, criterion (e) is no more than 20% of unique adaptor sequences have reads greater than twice the average number of reads for all unique adaptor sequences. In some embodiments, criterion (e) is no more than 25% of unique adaptor sequences have reads greater than twice the average number of reads for all unique adaptor sequences. In some embodiments, criterion (e) is no more than 30% of unique adaptor sequences have reads greater than twice the average number of reads for all unique adaptor sequences. In some embodiments, criterion (e) is no more than 35% of unique adaptor sequences have reads greater than twice the average number of reads for all unique adaptor sequences. In some embodiments, criterion (e) is no more than 40% of unique adaptor sequences have reads greater than twice the average number of reads for all unique adaptor sequences. In some embodiments, criterion (e) is no more than 45% of unique adaptor sequences have reads greater than twice the average number of reads for all unique adaptor sequences. In some embodiments, criterion (e) is no more than 50% of unique adaptor sequences have reads greater than twice the average number of reads for all unique adaptor sequences.

In some embodiments, criterion (e) is no more than about 1% to about 50% of unique adaptor sequences have reads greater than twice the average number of reads for all unique adaptor sequences. In some embodiments, criterion (e) is no more than about 5% to about 50% of unique adaptor sequences have reads greater than twice the average number of reads for all unique adaptor sequences. In some embodiments, criterion (e) is no more than about 5% to about 40% of unique adaptor sequences have reads greater than twice the average number of reads for all unique adaptor sequences. In some embodiments, criterion (e) is no more than about 10% to about 30% of unique adaptor sequences have reads greater than twice the average number of reads for all unique adaptor sequences. In some embodiments, criterion (e) is no more than about 10% to about 20% of unique adaptor sequences have reads greater than twice the average number of reads for all unique adaptor sequences. In some embodiments, criterion (e) is no more than about 20% to about 40% of unique adaptor sequences have reads greater than twice the average number of reads for all unique adaptor sequences. In some embodiments, criterion (e) is no more than about 20% to about 30% of unique adaptor sequences have reads greater than twice the average number of reads for all unique adaptor sequences. In some embodiments, criterion (e) is no more than about 15% to about 25% of unique adaptor sequences have reads greater than twice the average number of reads for all unique adaptor sequences. In some embodiments, criterion (e) is no more than about 15% to about 20% of unique adaptor sequences have reads greater than twice the average number of reads for all unique adaptor sequences.

**In** some embodiments, criterion (e) is no more than 1% to 50% of unique adaptor sequences have reads greater than twice the average number of reads for all unique adaptor sequences. In some embodiments, criterion (e) is no more than 5% to 50% of unique adaptor sequences have reads greater than twice the average number of reads for all unique adaptor sequences. In some embodiments, criterion (e) is no more than 5% to 40% of unique adaptor sequences have reads greater than twice the average number of reads for all unique adaptor sequences. In some embodiments, criterion (e) is no more than 10% to 30% of unique adaptor sequences have reads greater than twice the average number of reads for all unique adaptor sequences. In some embodiments, criterion (e) is no more than 10% to 20% of unique adaptor sequences have reads greater than twice the average number of reads for all unique adaptor sequences. In some embodiments, criterion (e) is no more than 20% to 40% of unique adaptor sequences have reads greater than twice the average number of reads for all unique adaptor sequences. In some embodiments, criterion (e) is no more than 20% to 30% of unique adaptor sequences have reads greater than twice the average number of reads for all unique adaptor sequences. In some embodiments, criterion (e) is no more than 15% to 25% of unique adaptor sequences have reads greater than twice the average number of reads for all unique adaptor sequences. In some embodiments, criterion (e) is no more than 15% to 20% of unique adaptor sequences have reads greater than twice the average number of reads for all unique adaptor sequences.

**In** some embodiments of the methods for performing an adaptor QC process of the disclosure, the pre-determined acceptance criteria for the adaptor QC process comprise a criterion (a) and a criterion (b). In some embodiments, the pre-determined acceptance criteria comprise a criterion (c), a criterion (d), and a criterion (e). In some embodiments, the pre-determined acceptance criteria comprise a criterion (a), a criterion (b), and a criterion (c). In some embodiments, the pre-determined acceptance criteria comprise a criterion (a), a criterion (b), and a criterion (d). In some embodiments, the pre-determined acceptance criteria comprise a criterion (a), a criterion (b), and a criterion (e). In some embodiments, the pre-determined acceptance criteria comprise a criterion (a), a criterion (b), a criterion (c), and a criterion (d). In some embodiments, the pre-determined acceptance criteria comprise a criterion (a), a criterion (b), a criterion (c), and a criterion (e). In some embodiments, the pre-determined acceptance criteria comprise a criterion (a), a criterion (b), a criterion (d), and a criterion (e). In some embodiments, the pre-determined acceptance criteria comprise a criterion (a), a criterion (b), a criterion (c), a criterion (d), and a criterion (e). In some embodiments, the pre-determined acceptance criteria comprise at least one criterion selected from criteria (a) (b) (c) (d) and (e).

In some embodiments of the methods for performing an adaptor QC process of the disclosure, the set of adaptors is considered to have passed the adaptor QC process when it has passed the test for adaptor distribution.

In some embodiments of the methods for performing an adaptor QC process of the disclosure, the set of adaptors is considered to have passed the adaptor QC process when it has passed both the test for adaptor ligation and the test for adaptor distribution.

In some embodiments of the methods for performing an adaptor QC process of the disclosure, each adaptor of the set of adaptors comprises a ligation strand oligonucleotide and a non-ligation strand oligonucleotide.

In some embodiments of the methods for performing an adaptor QC process of the disclosure, the non-ligation strand oligonucleotide is capable of hybridizing to a region at the 3' end of the ligation strand oligonucleotide and forming a duplex therewith.

In some embodiments of the methods for performing an adaptor QC process of the disclosure, the ligation strand oligonucleotide comprises an adaptor module.

In some embodiments of the methods for performing an adaptor QC process of the disclosure, the ligation strand oligonucleotide comprises a dT, dA, dC, or dG overhang at the 3' terminus.

In some embodiments of the methods for performing an adaptor QC process of the disclosure, the non-ligation strand oligonucleotide comprises a modification at its 3' terminus that prevents ligation to the 5' end of a dsDNA fragment and/or adaptor dimer formation, wherein the non-ligation strand is configured to be displaced from the duplex.

In some embodiments of the methods for performing an adaptor QC process of the disclosure, each adaptor of the set of adaptors comprises an ID region selected from a pool of unique ID regions, wherein the pool is selected from a plurality of pools, and wherein the selected pool is unique to the test sample.

In some embodiments of the methods for performing an adaptor QC process of the disclosure, the adaptor module comprises (a) an amplification region comprising a primer binding site; (b) an ID region; and (c) an anchor region.

In some embodiments of the methods for performing an adaptor QC process of the disclosure, the amplification region comprises a primer binding site, wherein the primer binding site allows for amplification using PCR (polymerase chain reaction), LAMP (loop-mediated isothermal amplification), NASBA (nucleic acid sequence-based amplification), SDA (standard displacement amplification), RCA (rolling circle replication), or LCR (ligase chain reaction).

In some embodiments of the methods for performing an adaptor QC process of the disclosure, the amplification region comprises or consists of between 10 and 50 nucleotides. In some embodiments, the amplification region comprises or consists of between 20 and 30 nucleotides. In some embodiments, the amplification region comprises or consists of 25 nucleotides.

In some embodiments of the methods for performing an adaptor QC process of the disclosure, the amplification region comprises or consists of about 10 to about 50 nucleotides. In some embodiments, the amplification region comprises or consists of about 20 to about 30 nucleotides. In some embodiments, the amplification region comprises or consists of about 25 nucleotides.

In some embodiments of the methods for performing an adaptor QC process of the disclosure, the anchor region comprises an overhang at the 3' terminus.

In some embodiments of the methods for performing an adaptor QC process of the disclosure, the anchor region comprises or consists of between 1 and 50 nucleotides. In some embodiments, the anchor region comprises or consists of between 5 and 25 nucleotides. In some embodiments, the anchor region comprises or consists of 10 nucleotides.

In some embodiments of the methods for performing an adaptor QC process of the disclosure, the anchor region comprises or consists of about 1 to about 50 nucleotides. In some embodiments, the anchor region comprises or consists of about 5 to about 25 nucleotides. In some embodiments, the anchor region comprises or consists of about 10 nucleotides.

In some embodiments of the methods for performing an adaptor QC process of the disclosure, the ID region comprises or consists of between 3 and 50 nucleotides. In some embodiments, the ID region comprises or consists of between 3 and 15 nucleotides. In some embodiments, the ID region comprises or consists of 8 nucleotides.

In some embodiments of the methods for performing an adaptor QC process of the disclosure, the ID region comprises or consists of about 3 to about 50 nucleotides. In some embodiments, the ID region comprises or consists of about 3 to about 15 nucleotides. In some embodiments, the ID region comprises or consists of about 8 nucleotides.

In some embodiments of the methods for performing an adaptor QC process of the disclosure, the adaptor module further comprises a unique molecule identifier (UMI) multiplier.

In some embodiments of the methods for performing an adaptor QC process of the disclosure, the UMI multiplier is adjacent to or contained within the ID region.

In some embodiments of the methods for performing an adaptor QC process of the disclosure, the UMI multiplier comprises or consists of between 1 and 5 nucleotides.

In some embodiments of the methods for performing an adaptor QC process of the disclosure, the UMI multiplier comprises or consists of about 1 to about 5 nucleotides.

In some embodiments of the methods for performing an adaptor QC process of the disclosure, the UMI multiplier is 3 nucleotides in length, and comprises a nucleic acid sequence selected from a group of 64 unique nucleotide sequences.

In some embodiments of the methods for performing an adaptor QC process of the disclosure, the pool of ID regions comprises between 2 and 10,000 unique ID region sequences. In some embodiments, the pool of ID regions comprises between 10 and 500 unique ID region sequences. In some embodiments, the pool of ID regions comprises between 50 and 300 unique ID region sequences. In some embodiments, the pool of ID regions comprises 60 unique ID region sequences.

In some embodiments of the methods for performing an adaptor QC process of the disclosure, the pool of ID regions comprises about 2 to about 10,000 unique ID region sequences. In some embodiments, the pool of ID regions comprises about 10 to about 500 unique ID region sequences. In some embodiments, the pool of ID regions comprises about 50 to about 300 unique ID region sequences. In some embodiments, the pool of ID regions comprises about 60 unique ID region sequences.

In some embodiments of the methods for performing an adaptor QC process of the disclosure, each ID region of the pool of ID regions is 8 nucleotides in length.

In some embodiments of the methods for performing an adaptor QC process of the disclosure, each ID region sequence is discrete from any other ID region sequence by Hamming distance of at least two.

In some embodiments of the methods for performing an adaptor QC process of the disclosure, each ID region is configured to identify the DNA fragment attached thereto.

In some embodiments of the methods for performing an adaptor QC process of the disclosure, each adaptor module of the set of adaptor modules is selected from a group consisting of between 64 and 2,560,000 unique nucleotide sequences.

In some embodiments of the methods for performing an adaptor QC process of the disclosure, each adaptor module of the set of adaptor modules is selected from a group consisting of about 64 to about 2,560,000 unique nucleotide sequences.

In some embodiments of the methods for performing an adaptor QC process of the disclosure, each adaptor module of the set of adaptor modules comprises a unique nucleotide sequence selected from 3840 unique nucleotide sequences, wherein each sequence of the 3840 unique nucleotide sequences is discrete from any other sequence by Hamming distance of at least two.

In some embodiments of the methods for performing an adaptor QC process of the disclosure, the anchor region of each adaptor of the set of adaptors comprises one of four nucleotide sequences, and wherein each ID region of a given sequence is paired to only one of the four anchor regions of a given sequence.

In some embodiments of the methods for performing an adaptor QC process of the disclosure, the amplification region of each adaptor of the set of adaptors comprises an identical primer binding site.

In some embodiments of the methods for performing an adaptor QC process of the disclosure, each ID region of the pool of ID regions is 8 nucleotides in length, each ID region sequence is discrete from any other ID region sequence by Hamming distance of at least two; each adaptor of the set of adaptors comprises a UMI multiplier that is adjacent to or contained within the ID region, wherein the UMI multiplier of each adaptor of the set of adaptors is three nucleotides in length, and wherein the UMI multiplier of a given sequence is paired to one ID region of a given sequence; the anchor region of each adaptor of the set of adaptors comprises one of four nucleotide sequences, and wherein each ID region of a given sequence is paired to only one of the four anchor regions of a given sequence; and the amplification region of each adaptor of the set of adaptors comprises an identical primer binding site.

In some embodiments of the methods for performing an adaptor QC process of the disclosure, the method comprises (a) ligating a set of adaptors to DNA fragments of a test sample to generate a plurality of adaptor/DNA fragment complexes; and (b) contacting the plurality of adaptor/DNA fragment complexes with one or more enzymes to form an adaptor-tagged DNA library comprising a plurality of adaptor-tagged DNA fragments. In some embodiments, each adaptor/DNA fragment complex comprises a ligation strand oligonucleotide ligated to each end of the DNA fragment. In some embodiments, the non-ligation strand oligonucleotide is displaced from the adaptor/DNA fragment complex in step (b). In some embodiments, the set of adaptors is any adaptor set of the disclosure.

Some embodiments of the disclosure provide methods for performing a probe Quality Control (QC) process on one or more capture probe modules.

In some embodiments of the methods for performing a probe QC process of the disclosure, the method comprises a test for capture probe modules comprising (a) ligating a set of adaptors to a DNA sample comprising end-repaired DNA fragments to generate a library of adaptor-tagged DNA fragments (LIBS); (b) amplifying the LIBS to generate a Library Post Amplification (LPA); (c) splitting or diluting the LPA to generate a Target Capture LPA (TC LPA) and a Whole-Genome LPA (WG LPA); (d) amplifying the WG LPA to generate a Whole-Genome Library Amplified (WGLA); (e) hybridizing the one or more capture probe modules to be tested to the TC LPA to form adaptor-tagged DNA fragment-capture probe module complexes; (f) isolating the adaptor-tagged DNA fragment-capture probe module complexes to form isolated adaptor-tagged DNA fragment-capture probe module complexes; (g) enzymatically processing the isolated adaptor-tagged DNA fragment-capture probe module complexes to generate Hybrid Molecules, wherein each Hybrid Molecule comprises the capture probe module and a complement of the adaptor-tagged DNA fragment; (h) amplifying the Hybrid Molecules to generate a Target Capture Library Amplified (TCLA); (i) combining the WGLA and the TCLA to form a Sequence-Ready Library (SRL); (j) performing a quantitative genetic analysis on the SRL.

In some embodiments of the methods for performing a probe QC process of the disclosure, the TC LPA is at least about 1 ng/µL. In some embodiments of the methods for performing a probe QC process of the disclosure, the TC LPA is at least about 5 ng/µL. In some embodiments of the methods for performing a probe QC process of the disclosure, the TC LPA is at least about 10 ng/µL. In some embodiments of the methods for performing a probe QC process of the disclosure, the TC LPA is at least about 15 ng/µL. In some embodiments of the methods for performing a probe QC process of the disclosure, the TC LPA is at least about 20 ng/µL. In some embodiments of the kits of the disclosure, the TC LPA is at least about 25 ng/µL. In some embodiments of the methods for performing a probe QC process of the disclosure, the TC LPA is at least about 30 ng/µL. In some embodiments of the methods for performing a probe QC process of the disclosure, the TC LPA is at least about 35 ng/µL. In some embodiments of the methods for performing a probe QC process of the disclosure, the TC LPA is at least about 40 ng/µL. In some embodiments of the methods for performing a probe QC process of the disclosure, the TC LPA is at least about 45 ng/µL. In some embodiments of the methods for performing a probe QC process of the disclosure, the TC LPA is at least about 50 ng/µL. In some embodiments of the methods for performing a probe QC process of the disclosure, the TC LPA is at least about 55 ng/µL. In some embodiments of the methods for performing a probe QC process of the disclosure, the TC LPA is at least about 60 ng/µL. In some embodiments of the methods for performing a probe QC process of the disclosure, the TC LPA is at least about 65 ng/µL. In some embodiments of the methods for performing a probe QC process of the disclosure, the TC LPA is at least about 70 ng/µL. In some embodiments of the methods for performing a probe QC process of the disclosure, the TC LPA is at least about 75 ng/µL. In some embodiments of the methods for performing a probe QC process of the disclosure, the TC LPA is at least about 80 ng/µL.

In some embodiments of the methods for performing a probe QC process of the disclosure, the TC LPA is at least 1 ng/µL. In some embodiments of the methods for performing a probe QC process of the disclosure, the TC LPA is at least 5 ng/µL. In some embodiments of the methods for performing a probe QC process of the disclosure, the TC LPA is at least 10 ng/µL. In some embodiments of the methods for performing a probe QC process of the disclosure, the TC LPA is at least 15 ng/µL. In some embodiments of the methods for performing a probe QC process of the disclosure, the TC LPA is at least 20 ng/µL. In some embodiments of the methods for performing a probe QC process of the disclosure, the TC LPA is at least 25 ng/µL. In some embodiments of the methods for performing a probe QC process of the disclosure, the TC LPA is at least 30 ng/µL. In some embodiments of the methods for performing a probe QC process of the disclosure, the TC LPA is at least 35 ng/µL. In some embodiments of the kits of the disclosure, the TC LPA is at least 40 ng/µL. In some embodiments of the methods for performing a probe QC process of the disclosure, the TC LPA is at least 45 ng/µL. In some embodiments of the methods for performing a probe QC process of the disclosure, the TC LPA is at least 50 ng/µL. In some embodiments of the methods for performing a probe QC process of the disclosure, the TC LPA is at least 55 ng/µL. In some embodiments of the methods for performing a probe QC process of the disclosure, the TC LPA is at least 60 ng/µL. In some embodiments of the methods for performing a probe QC process of the disclosure, the TC LPA is at least 65 ng/µL. In some embodiments of the methods for performing a probe QC process of the disclosure, the TC LPA is at least 70 ng/µL. In some embodiments of the methods for performing a probe QC process of the disclosure, the TC LPA is at least 75 ng/µL. In some embodiments of the methods for performing a probe QC process of the disclosure, the TC LPA is at least 80 ng/µL.

In some embodiments of the methods for performing a probe QC process of the disclosure, the TC LPA is at least about 1 ng/µL to about 100 ng/µL. In some embodiments of the methods for performing a probe QC process of the disclosure, the TC LPA is at least about 1 ng/µL to about 80 ng/µL. In some embodiments of the methods for performing a probe QC process of the disclosure, the TC LPA is at least about 10 ng/µL to about 80 ng/µL. In some embodiments of the methods for performing a probe QC process of the disclosure, the TC LPA is at least about 10 ng/µL to about 70 ng/µL. In some embodiments of the methods for performing a probe QC process of the disclosure, the TC LPA is at least about 10 ng/µL to about 60 ng/µL. In some embodiments of the methods for performing a probe QC process of the disclosure, the TC LPA is at least about 20 ng/µL to about 60 ng/µL. In some embodiments of the methods for performing a probe QC process of the disclosure, the TC LPA is at least about 20 ng/µL to about 50 ng/µL. In some embodiments of the methods for performing a probe QC process of the disclosure, the TC LPA is at least about 20 ng/µL to about 40 ng/µL. In some embodiments of the methods for performing a probe QC process of the disclosure, the TC LPA is at least about 30 ng/µL to about 50 ng/µL. In some embodiments of the methods for performing a probe QC process of the disclosure, the TC LPA is at least about 30 ng/µL to about 40 ng/µL. In some embodiments of the methods for performing a probe QC process of the disclosure, the TC LPA is at least about 20 ng/µL to about 30 ng/µL.

In some embodiments of the methods for performing a probe QC process of the disclosure, the TC LPA is at least 1 ng/µL to 100 ng/µL. In some embodiments of the methods for performing a probe QC process of the disclosure, the TC LPA is at least 1 ng/µL to 80 ng/µL. In some embodiments of the methods for performing a probe QC process of the disclosure, the TC LPA is at least 10 ng/µL to 80 ng/µL. In some embodiments of the methods for performing a probe QC process of the disclosure, the TC LPA is at least 10 ng/µL to 70 ng/µL. In some embodiments of the methods for performing a probe QC process of the disclosure, the TC LPA is at least 10 ng/µL to 60 ng/µL. In some embodiments of the methods for performing a probe QC process of the disclosure, the TC LPA is at least 20 ng/µL to 60 ng/µL. In some embodiments of the methods for performing a probe QC process of the disclosure, the TC LPA is at least 20 ng/µL to 50 ng/µL. In some embodiments of the methods for performing a probe QC process of the disclosure, the TC LPA is at least 20 ng/µL to 40 ng/µL. In some embodiments of the methods for performing a probe QC process of the disclosure, the TC LPA is at least 30 ng/µL to 50 ng/µL. In some embodiments of the methods for performing a probe QC process of the disclosure, the TC LPA is at least 30 ng/µL to 40 ng/µL. In some embodiments of the methods for performing a probe QC process of the disclosure, the TC LPA is at least 20 ng/µL to 30 ng/µL.

In some embodiments of the methods for performing a probe QC process of the disclosure, the WG LPA is at least about 0.1 ng/µL. In some embodiments of the methods for performing a probe QC process of the disclosure, the WG LPA is at least about 0.5 ng/µL. In some embodiments of the methods for performing a probe QC process of the disclosure, the WG LPA is at least about 1 ng/µL. In some embodiments of the methods for performing a probe QC process of the disclosure, the WG LPA is at least about 1.5 ng/µL. In some embodiments of the methods for performing a probe QC process of the disclosure, the WG LPA is at least about 2 ng/µL. In some embodiments of the methods for performing a probe QC process of the disclosure, the WG LPA is at least about 2.5 ng/µL. In some embodiments of the methods for performing a probe QC process of the disclosure, the WG LPA is at least about 3 ng/µL. In some embodiments of the methods for performing a probe QC process of the disclosure, the WG LPA is at least about 3.5 ng/µL. In some embodiments of the methods for performing a probe QC process of the disclosure, the WG LPA is at least about 4 ng/µL. In some embodiments of the methods for performing a probe QC process of the disclosure, the WG LPA is at least about 4.5 ng/µL. In some embodiments of the methods for performing a probe QC process of the disclosure, the WG LPA is at least about 5 ng/µL. In some embodiments of the methods for performing a probe QC process of the disclosure, the WG LPA is at least about 5.5 ng/µL. In some embodiments of the methods for performing a probe QC process of the disclosure, the WG LPA is at least about 6 ng/µL. In some embodiments of the methods for performing a probe QC process of the disclosure, the WG LPA is at least about 6.5 ng/µL. In some embodiments of the methods for performing a probe QC process of the disclosure, the WG LPA is at least about 7 ng/µL. In some embodiments of the methods for performing a probe QC process of the disclosure, the WG LPA is at least about 7.5 ng/µL. In some embodiments of the methods for performing a probe QC process of the disclosure, the WG LPA is at least about 8 ng/µL.

In some embodiments of the methods for performing a probe QC process of the disclosure, the WG LPA is at least 0.1 ng/µL. In some embodiments of the methods for performing a probe QC process of the disclosure, the WG LPA is at least 0.5 ng/µL. In some embodiments of the methods for performing a probe QC process of the disclosure, the WG LPA is at least 1 ng/µL. In some embodiments of the methods for performing a probe QC process of the disclosure, the WG LPA is at least 1.5 ng/µL. In some embodiments of the methods for performing a probe QC process of the disclosure, the WG LPA is at least 2 ng/µL. In some embodiments of the methods for performing a probe QC process of the disclosure, the WG LPA is at least 2.5 ng/µL. In some embodiments of the methods for performing a probe QC process of the disclosure, the WG LPA is at least 3 ng/µL. In some embodiments of the methods for performing a probe QC process of the disclosure, the WG LPA is at least 3.5 ng/µL. In some embodiments of the methods for performing a probe QC process of the disclosure, the WG LPA is at least 4 ng/µL. In some embodiments of the methods for performing a probe QC process of the disclosure, the WG LPA is at least 4.5 ng/µL. In some embodiments of the methods for performing a probe QC process of the disclosure, the WG LPA is at least 5 ng/µL. In some embodiments of the methods for performing a probe QC process of the disclosure, the WG LPA is at least 5.5 ng/µL. In some embodiments of the methods for performing a probe QC process of the disclosure, the WG LPA is at least 6 ng/µL. In some embodiments of the methods for performing a probe QC process of the disclosure, the WG LPA is at least 6.5 ng/µL. In some embodiments of the methods for performing a probe QC process of the disclosure, the WG LPA is at least 7 ng/µL. In some embodiments of the methods for performing a probe QC process of the disclosure, the WG LPA is at least 7.5 ng/µL. In some embodiments of the methods for performing a probe QC process of the disclosure, the WG LPA is at least 8 ng/µL.

In some embodiments of the methods for performing a probe QC process of the disclosure, the WG LPA is at least about 0.1 ng/µL to about 10 ng/µL. In some embodiments of the methods for performing a probe QC process of the disclosure, the WG LPA is at least about 0.1 ng/µL to about 8 ng/µL. In some embodiments of the methods for performing a probe QC process of the disclosure, the WG LPA is at least about 1 ng/µL to about 10 ng/µL. In some embodiments of the methods for performing a probe QC process of the disclosure, the WG LPA is at least about 1 ng/µL to about 5 ng/µL. In some embodiments of the methods for performing a probe QC process of the disclosure, the WG LPA is at least about 2 ng/µL to about 10 ng/µL. In some embodiments of the methods for performing a probe QC process of the disclosure, the WG LPA is at least about 2 ng/µL to about 6 ng/µL. In some embodiments of the methods for performing a probe QC process of the disclosure, the WG LPA is at least about 3 ng/µL to about 5 ng/µL. In some embodiments of the methods for performing a probe QC process of the disclosure, the WG LPA is at least about 3 ng/µL to about 4 ng/µL. In some embodiments of the methods for performing a probe QC process of the disclosure, the WG LPA is at least about 2 ng/µL to about 3 ng/µL.

In some embodiments of the methods for performing a probe QC process of the disclosure, the WG LPA is at least 0.1 ng/µL to 10 ng/µL. In some embodiments of the methods for performing a probe QC process of the disclosure, the WG LPA is at least 0.1 ng/µL to 8 ng/µL. In some embodiments of the methods for performing a probe QC process of the disclosure, the WG LPA is at least 1 ng/µL to 10 ng/µL. In some embodiments of the methods for performing a probe QC process of the disclosure, the WG LPA is at least 1 ng/µL to 5 ng/µL. In some embodiments of the methods for performing a probe QC process of the disclosure, the WG LPA is at least 2 ng/µL to 10 ng/µL. In some embodiments of the methods for performing a probe QC process of the disclosure, the WG LPA is at least 2 ng/µL to 6 ng/µL. In some embodiments of the methods for performing a probe QC process of the disclosure, the WG LPA is at least 3 ng/µL to 5 ng/µL. In some embodiments of the methods for performing a probe QC process of the disclosure, the WG LPA is at least 3 ng/µL to 4 ng/µL. In some embodiments of the methods for performing a probe QC process of the disclosure, the WG LPA is at least 2 ng/µL to 3 ng/µL.

In some embodiments of the methods for performing a probe QC process of the disclosure, the TCLA is at least about 1.0 ng/µL. In some embodiments of the methods for performing a probe QC process of the disclosure, the TCLA is at least about 1.5 ng/µL. In some embodiments of the methods for performing a probe QC process of the disclosure, the TCLA is at least about 2.0 ng/µL. In some embodiments of the methods for performing a probe QC process of the disclosure, the TCLA is at least about 2.5 ng/µL. In some embodiments of the methods for performing a probe QC process of the disclosure, the TCLA is at least about 3.0 ng/µL. In some embodiments of the methods for performing a probe QC process of the disclosure the TCLA is at least about 3.5 ng/µL. In some embodiments of the methods for performing a probe QC process of the disclosure, the TCLA is at least about 4.0 ng/µL. In some embodiments of the methods for performing a probe QC process of the disclosure, the TCLA is at least about at least 4.5 ng/µL. In some embodiments of the methods for performing a probe QC process of the disclosure, the TCLA is at least about 5.0 ng/µL. In some embodiments of the methods for performing a probe QC process of the disclosure, the TCLA is at least about 5.5 ng/µL. In some embodiments of the methods for performing a probe QC process of the disclosure, the TCLA is at least about 6.0 ng/µL. In some embodiments of the methods for performing a probe QC process of the disclosure, the TCLA is at least about 6.5 ng/µL. In some embodiments of the methods for performing a probe QC process of the disclosure, the TCLA is at least about 7.0 ng/µL. In some embodiments of the methods for performing a probe QC process of the disclosure, the TCLA is at least about 7.5 ng/µL. In some embodiments of the methods for performing a probe QC process of the disclosure, the TCLA is at least about 8.0 ng/µL. In some embodiments of the methods for performing a probe QC process of the disclosure, the TCLA is at least about 8.5 ng/µL. In some embodiments of the methods for performing a probe QC process of the disclosure, the TCLA is at least about 9.0 ng/µL. In some embodiments of the methods for performing a probe QC process of the disclosure, the TCLA is at least about 9.5 ng/µL. In some embodiments of the methods for performing a probe QC process of the disclosure, the TCLA is at least about 10 ng/µL. In some embodiments of the methods for performing a probe QC process of the disclosure, the TCLA is at least about 15 ng/µL. In some embodiments of the methods for performing a probe QC process of the disclosure, the TCLA is at least about 20 ng/µL. In some embodiments of the methods for performing a probe QC process of the disclosure, the TCLA is at least about 25 ng/µL. In some embodiments of the methods for performing a probe QC process of the disclosure, the TCLA is at least about 30 ng/µL. In some embodiments of the methods for performing a probe QC process of the disclosure, the TCLA is at least about 35 ng/µL. In some embodiments of the methods for performing a probe QC process of the disclosure, the TCLA is at least about 40 ng/µL. In some embodiments of the methods for performing a probe QC process of the disclosure, the TCLA is at least about 45 ng/µL. In some embodiments of the methods for performing a probe QC process of the disclosure, the TCLA is at least about 50 ng/µL. In some embodiments of the methods for performing a probe QC process of the disclosure, the TCLA is at least about 55 ng/µL. In some embodiments of the methods for performing a probe QC process of the disclosure, the TCLA is at least about 60 ng/µL. In some embodiments of the methods for performing a probe QC process of the disclosure, the TCLA is at least about 65 ng/µL. In some embodiments of the methods for performing a probe QC process of the disclosure, the TCLA is at least about 70 ng/µL. In some embodiments of the methods for performing a probe QC process of the disclosure, the TCLA is at least about 75 ng/µL. In some embodiments of the methods for performing a probe QC process of the disclosure, the TCLA is at least about 80 ng/µL.

In some embodiments of the methods for performing a probe QC process of the disclosure, the TCLA is at least 1.0 ng/µL. In some embodiments of the methods for performing a probe QC process of the disclosure, the TCLA is at least 1.5 ng/µL. In some embodiments of the methods for performing a probe QC process of the disclosure, the TCLA is at least 2.0 ng/µL. In some embodiments of the methods for performing a probe QC process of the disclosure, the TCLA is at least 2.5 ng/µL. In some embodiments of the methods for performing a probe QC process of the disclosure, the TCLA is at least 3.0 ng/µL. In some embodiments of the methods for performing a probe QC process of the disclosure the TCLA is at least 3.5 ng/µL. In some embodiments of the methods for performing a probe QC process of the disclosure, the TCLA is at least 4.0 ng/µL. In some embodiments of the methods for performing a probe QC process of the disclosure, the TCLA is at least about at least 4.5 ng/µL. In some embodiments of the methods for performing a probe QC process of the disclosure, the TCLA is at least 5.0 ng/µL. In some embodiments of the methods for performing a probe QC process of the disclosure, the TCLA is at least 5.5 ng/µL. In some embodiments of the methods for performing a probe QC process of the disclosure, the TCLA is at least 6.0 ng/µL. In some embodiments of the methods for performing a probe QC process of the disclosure, the TCLA is at least 6.5 ng/µL. In some embodiments of the methods for performing a probe QC process of the disclosure, the TCLA is at least 7.0 ng/µL. In some embodiments of the methods for performing a probe QC process of the disclosure, the TCLA is at least 7.5 ng/µL. In some embodiments of the methods for performing a probe QC process of the disclosure, the TCLA is at least 8.0 ng/µL. In some embodiments of the methods for performing a probe QC process of the disclosure, the TCLA is at least 8.5 ng/µL. In some embodiments of the methods for performing a probe QC process of the disclosure, the TCLA is at least 9.0 ng/µL. In some embodiments of the methods for performing a probe QC process of the disclosure, the TCLA is at least 9.5 ng/µL. In some embodiments of the methods for performing a probe QC process of the disclosure, the TCLA is at least 10 ng/µL. In some embodiments of the methods for performing a probe QC process of the disclosure, the TCLA is at least 15 ng/µL. In some embodiments of the methods for performing a probe QC process of the disclosure, the TCLA is at least 20 ng/µL. In some embodiments of the methods for performing a probe QC process of the disclosure, the TCLA is at least 25 ng/µL. In some embodiments of the methods for performing a probe QC process of the disclosure, the TCLA is at least 30 ng/µL. In some embodiments of the methods for performing a probe QC process of the disclosure, the TCLA is at least 35 ng/µL. In some embodiments of the methods for performing a probe QC process of the disclosure, the TCLA is at least 40 ng/µL. In some embodiments of the methods for performing a probe QC process of the disclosure, the TCLA is at least 45 ng/µL. In some embodiments of the methods for performing a probe QC process of the disclosure, the TCLA is at least 50 ng/µL. In some embodiments of the methods for performing a probe QC process of the disclosure, the TCLA is at least 55 ng/µL. In some embodiments of the methods for performing a probe QC process of the disclosure, the TCLA is at least 60 ng/µL. In some embodiments of the methods for performing a probe QC process of the disclosure, the TCLA is at least 65 ng/µL. In some embodiments of the methods for performing a probe QC process of the disclosure, the TCLA is at least 70 ng/µL. In some embodiments of the methods for performing a probe QC process of the disclosure, the TCLA is at least 75 ng/µL. In some embodiments of the methods for performing a probe QC process of the disclosure, the TCLA is at least 80 ng/µL.

In some embodiments of the methods for performing a probe QC process of the disclosure, the TCLA is at least about 1 ng/µL to about 200 ng/µL. In some embodiments of the methods for performing a probe QC process of the disclosure, the TCLA is at least about 100 ng/µL to about 200 ng/µL. In some embodiments of the methods for performing a probe QC process of the disclosure, the TCLA is at least about 1 ng/µL to about 100 ng/µL. In some embodiments of the kits of the disclosure, the TCLA is at least about 1 ng/µL to about 80 ng/µL. In some embodiments of the methods for performing a probe QC process of the disclosure, the TCLA is at least about 1 ng/µL to about 50 ng/µL. In some embodiments of the methods for performing a probe QC process of the disclosure, the TCLA is at least about 1 ng/µL to about 40 ng/µL. In some embodiments of the methods for performing a probe QC process of the disclosure, the TCLA is at least about 1 ng/µL to about 30 ng/µL. In some embodiments of the methods for performing a probe QC process of the disclosure, the TCLA is at least about 1 ng/µL to about 20 ng/µL. In some embodiments of the methods for performing a probe QC process of the disclosure, the TCLA is at least about 1 ng/µL to about 10 ng/µL. In some embodiments of the methods for performing a probe QC process of the disclosure, the TCLA is at least about 5 ng/µL to about 10 ng/µL.

In some embodiments of the methods for performing a probe QC process of the disclosure, the TCLA is at least 1 ng/µL to 200 ng/µL. In some embodiments of the methods for performing a probe QC process of the disclosure, the TCLA is at least 100 ng/µL to 200 ng/µL. In some embodiments of the methods for performing a probe QC process of the disclosure, the TCLA is at least 1 ng/µL to 100 ng/µL. In some embodiments of the kits of the disclosure, the TCLA is at least 1 ng/µL to 80 ng/µL. In some embodiments of the methods for performing a probe QC process of the disclosure, the TCLA is at least 1 ng/µL to 50 ng/µL. In some embodiments of the methods for performing a probe QC process of the disclosure, the TCLA is at least 1 ng/µL to 40 ng/µL. In some embodiments of the methods for performing a probe QC process of the disclosure, the TCLA is at least 1 ng/µL to 30 ng/µL. In some embodiments of the methods for performing a probe QC process of the disclosure, the TCLA is at least 1 ng/µL to 20 ng/µL. In some embodiments of the methods for performing a probe QC process of the disclosure, the TCLA is at least 1 ng/µL to 10 ng/µL. In some embodiments of the methods for performing a probe QC process of the disclosure, the TCLA is at least 5 ng/µL to 10 ng/µL.

In some embodiments of the methods for performing a probe QC process of the disclosure, the WGLA is at least about 1.0 ng/µL. In some embodiments of the methods for performing a probe QC process of the disclosure, the WGLA is at least about 1.5 ng/µL. In some embodiments of the methods for performing a probe QC process of the disclosure, the WGLA is at least about 2.0 ng/µL. In some embodiments of the methods for performing a probe QC process of the disclosure, the WGLA is at least about 2.5 ng/µL. In some embodiments of the methods for performing a probe QC process of the disclosure, the WGLA is at least about 3.0 ng/µL. In some embodiments of the methods for performing a probe QC process of the disclosure the WGLA is at least about 3.5 ng/µL. In some embodiments of the methods for performing a probe QC process of the disclosure, the WGLA is at least about 4.0 ng/µL. In some embodiments of the methods for performing a probe QC process of the disclosure, the WGLA is at least about at least 4.5 ng/µL. In some embodiments of the methods for performing a probe QC process of the disclosure, the WGLA is at least about 5.0 ng/µL. In some embodiments of the methods for performing a probe QC process of the disclosure, the WGLA is at least about 5.5 ng/µL. In some embodiments of the methods for performing a probe QC process of the disclosure, the WGLA is at least about 6.0 ng/µL. In some embodiments of the methods for performing a probe QC process of the disclosure, the WGLA is at least about 6.5 ng/µL. In some embodiments of the methods for performing a probe QC process of the disclosure, the WGLA is at least about 7.0 ng/µL. In some embodiments of the methods for performing a probe QC process of the disclosure, the WGLA is at least about 7.5 ng/µL. In some embodiments of the methods for performing a probe QC process of the disclosure, the WGLA is at least about 8.0 ng/µL. In some embodiments of the methods for performing a probe QC process of the disclosure, the WGLA is at least about 8.5 ng/µL. In some embodiments of the methods for performing a probe QC process of the disclosure, the WGLA is at least about 9.0 ng/µL. In some embodiments of the methods for performing a probe QC process of the disclosure, the WGLA is at least about 9.5 ng/µL. In some embodiments of the methods for performing a probe QC process of the disclosure, the WGLA is at least about 10 ng/µL. In some embodiments of the methods for performing a probe QC process of the disclosure, the WGLA is at least about 15 ng/µL. In some embodiments of the methods for performing a probe QC process of the disclosure, the WGLA is at least about 20 ng/µL. In some embodiments of the methods for performing a probe QC process of the disclosure, the WGLA is at least about 25 ng/µL. In some embodiments of the methods for performing a probe QC process of the disclosure, the WGLA is at least about 30 ng/µL. In some embodiments of the methods for performing a probe QC process of the disclosure, the WGLA is at least about 35 ng/µL. In some embodiments of the methods for performing a probe QC process of the disclosure, the WGLA is at least about 40 ng/µL. In some embodiments of the methods for performing a probe QC process of the disclosure, the WGLA is at least about 45 ng/µL. In some embodiments of the methods for performing a probe QC process of the disclosure, the WGLA is at least about 50 ng/µL. In some embodiments of the methods for performing a probe QC process of the disclosure, the WGLA is at least about 55 ng/µL. In some embodiments of the methods for performing a probe QC process of the disclosure, the WGLA is at least about 60 ng/µL. In some embodiments of the methods for performing a probe QC process of the disclosure, the WGLA is at least about 65 ng/µL. In some embodiments of the methods for performing a probe QC process of the disclosure, the WGLA is at least about 70 ng/µL. In some embodiments of the methods for performing a probe QC process of the disclosure, the WGLA is at least about 75 ng/µL. In some embodiments of the methods for performing a probe QC process of the disclosure, the WGLA is at least about 80 ng/µL.

In some embodiments of the methods for performing a probe QC process of the disclosure, the WGLA is at least 1.0 ng/µL. In some embodiments of the methods for performing a probe QC process of the disclosure, the WGLA is at least 1.5 ng/µL. In some embodiments of the methods for performing a probe QC process of the disclosure, the WGLA is at least 2.0 ng/µL. In some embodiments of the methods for performing a probe QC process of the disclosure, the WGLA is at least 2.5 ng/µL. In some embodiments of the methods for performing a probe QC process of the disclosure, the WGLA is at least 3.0 ng/µL. In some embodiments of the methods for performing a probe QC process of the disclosure the WGLA is at least 3.5 ng/µL. In some embodiments of the methods for performing a probe QC process of the disclosure, the WGLA is at least 4.0 ng/µL. In some embodiments of the methods for performing a probe QC process of the disclosure, the WGLA is at least about at least 4.5 ng/µL. In some embodiments of the methods for performing a probe QC process of the disclosure, the WGLA is at least 5.0 ng/µL. In some embodiments of the methods for performing a probe QC process of the disclosure, the WGLA is at least 5.5 ng/µL. In some embodiments of the methods for performing a probe QC process of the disclosure, the WGLA is at least 6.0 ng/µL. In some embodiments of the methods for performing a probe QC process of the disclosure, the WGLA is at least 6.5 ng/µL. In some embodiments of the methods for performing a probe QC process of the disclosure, the WGLA is at least 7.0 ng/µL. In some embodiments of the methods for performing a probe QC process of the disclosure, the WGLA is at least 7.5 ng/µL. In some embodiments of the methods for performing a probe QC process of the disclosure, the WGLA is at least 8.0 ng/µL. In some embodiments of the methods for performing a probe QC process of the disclosure, the WGLA is at least 8.5 ng/µL. In some embodiments of the methods for performing a probe QC process of the disclosure, the WGLA is at least 9.0 ng/µL. In some embodiments of the methods for performing a probe QC process of the disclosure, the WGLA is at least 9.5 ng/µL. In some embodiments of the methods for performing a probe QC process of the disclosure, the WGLA is at least 10 ng/µL. In some embodiments of the methods for performing a probe QC process of the disclosure, the WGLA is at least 15 ng/µL. In some embodiments of the methods for performing a probe QC process of the disclosure, the WGLA is at least 20 ng/µL. In some embodiments of the methods for performing a probe QC process of the disclosure, the WGLA is at least 25 ng/µL. In some embodiments of the methods for performing a probe QC process of the disclosure, the WGLA is at least 30 ng/µL. In some embodiments of the methods for performing a probe QC process of the disclosure, the WGLA is at least 35 ng/µL. In some embodiments of the methods for performing a probe QC process of the disclosure, the WGLA is at least 40 ng/µL. In some embodiments of the methods for performing a probe QC process of the disclosure, the WGLA is at least 45 ng/µL. In some embodiments of the methods for performing a probe QC process of the disclosure, the WGLA is at least 50 ng/µL. In some embodiments of the methods for performing a probe QC process of the disclosure, the WGLA is at least 55 ng/µL. In some embodiments of the methods for performing a probe QC process of the disclosure, the WGLA is at least 60 ng/µL. In some embodiments of the methods for performing a probe QC process of the disclosure, the WGLA is at least 65 ng/µL. In some embodiments of the methods for performing a probe QC process of the disclosure, the WGLA is at least 70 ng/µL. In some embodiments of the methods for performing a probe QC process of the disclosure, the WGLA is at least 75 ng/µL. In some embodiments of the methods for performing a probe QC process of the disclosure, the WGLA is at least 80 ng/µL.

In some embodiments of the methods for performing a probe QC process of the disclosure, the WGLA is at least about 1 ng/µL to about 200 ng/µL. In some embodiments of the methods for performing a probe QC process of the disclosure, the WGLA is at least about 100 ng/µL to about 200 ng/µL. In some embodiments of the methods for performing a probe QC process of the disclosure, the WGLA is at least about 1 ng/µL to about 100 ng/µL. In some embodiments of the kits of the disclosure, the WGLA is at least about 1 ng/µL to about 80 ng/µL. In some embodiments of the methods for performing a probe QC process of the disclosure, the WGLA is at least about 1 ng/µL to about 50 ng/µL. In some embodiments of the methods for performing a probe QC process of the disclosure, the WGLA is at least about 1 ng/µL to about 40 ng/µL. In some embodiments of the methods for performing a probe QC process of the disclosure, the WGLA is at least about 1 ng/µL to about 30 ng/µL. In some embodiments of the methods for performing a probe QC process of the disclosure, the WGLA is at least about 1 ng/µL to about 20 ng/µL. In some embodiments of the methods for performing a probe QC process of the disclosure, the WGLA is at least about 1 ng/µL to about 10 ng/µL. In some embodiments of the methods for performing a probe QC process of the disclosure, the WGLA is at least about 5 ng/µL to about 10 ng/µL.

In some embodiments of the methods for performing a probe QC process of the disclosure, the WGLA is at least 1 ng/µL to 200 ng/µL. In some embodiments of the methods for performing a probe QC process of the disclosure, the WGLA is at least 100 ng/µL to 200 ng/µL. In some embodiments of the methods for performing a probe QC process of the disclosure, the WGLA is at least 1 ng/µL to 100 ng/µL. In some embodiments of the kits of the disclosure, the WGLA is at least 1 ng/µL to 80 ng/µL. In some embodiments of the methods for performing a probe QC process of the disclosure, the WGLA is at least 1 ng/µL to 50 ng/µL. In some embodiments of the methods for performing a probe QC process of the disclosure, the WGLA is at least 1 ng/µL to 40 ng/µL. In some embodiments of the methods for performing a probe QC process of the disclosure, the WGLA is at least 1 ng/µL to 30 ng/µL. In some embodiments of the methods for performing a probe QC process of the disclosure, the WGLA is at least 1 ng/µL to 20 ng/µL. In some embodiments of the methods for performing a probe QC process of the disclosure, the WGLA is at least 1 ng/µL to 10 ng/µL. In some embodiments of the methods for performing a probe QC process of the disclosure, the WGLA is at least 5 ng/µL to 10 ng/µL.

In some embodiments of the methods for performing a probe QC process of the disclosure, the one or more capture probe modules are considered to have passed the probe QC process if one or more pre-determined acceptance criteria for the quantitative genetic analysis have been met.

In some embodiments of the methods for performing a probe QC process of the disclosure, the pre-determined acceptance criteria for the probe QC process comprise a criterion (a). In some embodiments, criterion (a) is about 100% of capture probes have at least 1 total reads. In some embodiments, criterion (a) is at least about 99.9% of capture probes have at least 1 total reads. In some embodiments, criterion (a) is at least about 99.8% of capture probes have at least 1 total reads. In some embodiments, criterion (a) is at least about 99.7% of capture probes have at least 1 total reads. In some embodiments, criterion (a) is at least about 99.6% of capture probes have at least 1 total reads. In some embodiments, criterion (a) is at least about 99.5% of capture probes have at least 1 total reads. In some embodiments, criterion (a) is at least about 99.4% of capture probes have at least 1 total reads. In some embodiments, criterion (a) is at least about 99.3% of capture probes have at least 1 total reads. In some embodiments, criterion (a) is at least about 99.2% of capture probes have at least 1 total reads. In some embodiments, criterion (a) is at least about 99.1% of capture probes have at least 1 total reads. In some embodiments, criterion (a) is at least about 99% of capture probes have at least 1 total reads. In some embodiments, criterion (a) is at least about 98.9% of capture probes have at least 1 total reads. In some embodiments, criterion (a) is at least about 98.8% of capture probes have at least 1 total reads. In some embodiments, criterion (a) is at least about 98.7% of capture probes have at least 1 total reads. In some embodiments, criterion (a) is at least about 98.6% of capture probes have at least 1 total reads. In some embodiments, criterion (a) is at least about 98.5% of capture probes have at least 1 total reads. In some embodiments, criterion (a) is at least about 98.4% of capture probes have at least 1 total reads. In some embodiments, criterion (a) is at least about 98.3% of capture probes have at least 1 total reads. In some embodiments, criterion (a) is at least about 98.2% of capture probes have at least 1 total reads. In some embodiments, criterion (a) is at least about 98.1% of capture probes have at least 1 total reads. In some embodiments, criterion (a) is at least about 98% of capture probes have at least 1 total reads. In some embodiments, criterion (a) is at least about 97% of capture probes have at least 1 total reads. In some embodiments, criterion (a) is at least about 96% of capture probes have at least 1 total reads. In some embodiments of the kits of the disclosure, criterion (a) is at least about 95% of capture probes have at least 1 total reads. In some embodiments of the kits of the disclosure, criterion (a) is at least about 90% of capture probes have at least 1 total reads. In some embodiments of the kits of the disclosure, criterion (a) is at least about 85% of capture probes have at least 1 total reads. In some embodiments of the kits of the disclosure, criterion (a) is at least about 80% of capture probes have at least 1 total reads. In some embodiments of the kits of the disclosure, criterion (a) is at least about 75% of capture probes have at least 1 total reads. In some embodiments of the kits of the disclosure, criterion (a) is at least about 70% of capture probes have at least 1 total reads. In some embodiments of the kits of the disclosure, criterion (a) is at least about 65% of capture probes have at least 1 total reads. In some embodiments of the kits of the disclosure, criterion (a) is at least about 60% of capture probes have at least 1 total reads.

In some embodiments, criterion (a) is 100% of capture probes have at least 1 total reads. In some embodiments, criterion (a) is at least 99.9% of capture probes have at least 1 total reads. In some embodiments, criterion (a) is at least 99.8% of capture probes have at least 1 total reads. In some embodiments, criterion (a) is at least 99.7% of capture probes have at least 1 total reads. In some embodiments, criterion (a) is at least 99.6% of capture probes have at least 1 total reads. In some embodiments, criterion (a) is at least 99.5% of capture probes have at least 1 total reads. In some embodiments, criterion (a) is at least 99.4% of capture probes have at least 1 total reads. In some embodiments, criterion (a) is at least 99.3% of capture probes have at least 1 total reads. In some embodiments, criterion (a) is at least 99.2% of capture probes have at least 1 total reads. In some embodiments, criterion (a) is at least 99.1% of capture probes have at least 1 total reads. In some embodiments, criterion (a) is at least 99% of capture probes have at least 1 total reads. In some embodiments, criterion (a) is at least 98.9% of capture probes have at least 1 total reads. In some embodiments, criterion (a) is at least 98.8% of capture probes have at least 1 total reads. In some embodiments, criterion (a) is at least 98.7% of capture probes have at least 1 total reads. In some embodiments, criterion (a) is at least 98.6% of capture probes have at least 1 total reads. In some embodiments, criterion (a) is at least 98.5% of capture probes have at least 1 total reads. In some embodiments, criterion (a) is at least 98.4% of capture probes have at least 1 total reads. In some embodiments, criterion (a) is at least 98.3% of capture probes have at least 1 total reads. In some embodiments, criterion (a) is at least 98.2% of capture probes have at least 1 total reads. In some embodiments, criterion (a) is at least 98.1% of capture probes have at least 1 total reads. In some embodiments, criterion (a) is at least 98% of capture probes have at least 1 total reads. In some embodiments, criterion (a) is at least 97% of capture probes have at least 1 total reads. In some embodiments, criterion (a) is at least 96% of capture probes have at least 1 total reads. In some embodiments of the kits of the disclosure, criterion (a) is at least 95% of capture probes have at least 1 total reads. In some embodiments of the kits of the disclosure, criterion (a) is at least 90% of capture probes have at least 1 total reads. In some embodiments of the kits of the disclosure, criterion (a) is at least 85% of capture probes have at least 1 total reads. In some embodiments of the kits of the disclosure, criterion (a) is at least 80% of capture probes have at least 1 total reads. In some embodiments of the kits of the disclosure, criterion (a) is at least 75% of capture probes have at least 1 total reads. In some embodiments of the kits of the disclosure, criterion (a) is at least 70% of capture probes have at least 1 total reads. In some embodiments of the kits of the disclosure, criterion (a) is at least 65% of capture probes have at least 1 total reads. In some embodiments of the kits of the disclosure, criterion (a) is at least 60% of capture probes have at least 1 total reads.

In some embodiments, criterion (a) is at least about 60% to about 99.9% of capture probes have at least 1 total reads. In some embodiments, criterion (a) is at least about 70% to about 99.9% of capture probes have at least 1 total reads. In some embodiments, criterion (a) is at least about 80% to about 99.9% of capture probes have at least 1 total reads. In some embodiments, criterion (a) is at least about 90% to about 99.9% of capture probes have at least 1 total reads. In some embodiments, criterion (a) is at least about 95% to about 99.9% of capture probes have at least 1 total reads. In some embodiments, criterion (a) is at least about 96% to about 99.9% of capture probes have at least 1 total reads. In some embodiments, criterion (a) is at least about 97% to about 99.9% of capture probes have at least 1 total reads. In some embodiments, criterion (a) is at least about 98% to about 99.9% of capture probes have at least 1 total reads. In some embodiments, criterion (a) is at least about 99% to about 99.9% of capture probes have at least 1 total reads. In some embodiments, criterion (a) is at least about 99% to about 100% of capture probes have at least 1 total reads.

In some embodiments, criterion (a) is at least 60% to 99.9% of capture probes have at least 1 total reads. In some embodiments, criterion (a) is at least 70% to 99.9% of capture probes have at least 1 total reads. In some embodiments, criterion (a) is at least 80% to 99.9% of capture probes have at least 1 total reads. In some embodiments, criterion (a) is at least 90% to 99.9% of capture probes have at least 1 total reads. In some embodiments, criterion (a) is at least 95% to 99.9% of capture probes have at least 1 total reads. In some embodiments, criterion (a) is at least 96% to 99.9% of capture probes have at least 1 total reads. In some embodiments, criterion (a) is at least 97% to 99.9% of capture probes have at least 1 total reads. In some embodiments, criterion (a) is at least 98% to 99.9% of capture probes have at least 1 total reads. In some embodiments, criterion (a) is at least 99% to 99.9% of capture probes have at least 1 total reads. In some embodiments, criterion (a) is at least 99% to 100% of capture probes have at least 1 total reads.

In some embodiments, criterion (a) is at least about 60% to about 100% of capture probes have at least 1 total reads. In some embodiments, criterion (a) is at least about 70% to about 100% of capture probes have at least 1 total reads. In some embodiments, criterion (a) is at least about 80% to about 100% of capture probes have at least 1 total reads. In some embodiments, criterion (a) is at least about 90% to about 100% of capture probes have at least 1 total reads. In some embodiments, criterion (a) is at least about 95% to about 100% of capture probes have at least 1 total reads. In some embodiments, criterion (a) is at least about 96% to about 100% of capture probes have at least 1 total reads. In some embodiments, criterion (a) is at least about 97% to about 100% of capture probes have at least 1 total reads. In some embodiments, criterion (a) is at least about 98% to about 100% of capture probes have at least 1 total reads. In some embodiments, criterion (a) is at least about 99% to about 100% of capture probes have at least 1 total reads. In some embodiments, criterion (a) is at least about 99.5% to about 100% of capture probes have at least 1 total reads. In some embodiments, criterion (a) is at least about 99.9% to about 100% of capture probes have at least 1 total reads.

In some embodiments, criterion (a) is at least 60% to 100% of capture probes have at least 1 total reads. In some embodiments, criterion (a) is at least 70% to 100% of capture probes have at least 1 total reads. In some embodiments, criterion (a) is at least 80% to 100% of capture probes have at least 1 total reads. In some embodiments, criterion (a) is at least 90% to 100% of capture probes have at least 1 total reads. In some embodiments, criterion (a) is at least 95% to 100% of capture probes have at least 1 total reads. In some embodiments, criterion (a) is at least 96% to 100% of capture probes have at least 1 total reads. In some embodiments, criterion (a) is at least 97% to 100% of capture probes have at least 1 total reads. In some embodiments, criterion (a) is at least 98% to 100% of capture probes have at least 1 total reads. In some embodiments, criterion (a) is at least 99% to 100% of capture probes have at least 1 total reads. In some embodiments, criterion (a) is at least 99.5% to 100% of capture probes have at least 1 total reads. In some embodiments, criterion (a) is at least 99.9% to 100% of capture probes have at least 1 total reads.

In some embodiments of the methods for performing a probe QC process of the disclosure, the pre-determined acceptance criteria for the probe QC process comprise a criterion (b). In some embodiments, criterion (b) is about 100% of capture probes have at least 10-200 on-target total reads. In some embodiments, criterion (b) is at least about 99.9% of capture probes have at least 10-200 on-target total reads. In some embodiments, criterion (b) is at least about 99.5% of capture probes have at least 10-200 on-target total reads. In some embodiments, criterion (b) is at least about 99% of capture probes have at least 10-200 on-target total reads. In some embodiments, criterion (b) is at least about 98.5% of capture probes have at least 10-200 on-target total reads. In some embodiments, criterion (b) is at least about 98% of capture probes have at least 10-200 on-target total reads. In some embodiments, criterion (b) is at least about 97.5% of capture probes have at least 10-200 on-target total reads. In some embodiments, criterion (b) is at least about 97% of capture probes have at least 10-200 on-target total reads. In some embodiments, criterion (b) is at least about 96.5% of capture probes have at least 10-200 on-target total reads. In some embodiments, criterion (b) is at least about 96% of capture probes have at least 10-200 on-target total reads. In some embodiments, criterion (b) is at least about 95.5% of capture probes have at least 10-200 on-target total reads. In some embodiments, criterion (b) is at least about 95% of capture probes have at least 10-200 on-target total reads. In some embodiments, the pre-determined acceptance criteria for the probe QC process comprise criterion (b) at least about 90% of capture probes have at least 10-200 on-target total reads. In some embodiments, criterion (b) is at least about 85% of capture probes have at least 10-200 on-target total reads. In some embodiments, criterion (b) is at least about 80% of capture probes have at least 10-200 on-target total reads. In some embodiments, criterion (b) is at least about 75% of capture probes have at least 10-200 on-target total reads. In some embodiments, criterion (b) is at least about 70% of capture probes have at least 10-200 on-target total reads. In some embodiments, criterion (b) is at least about 65% of capture probes have at least 10-200 on-target total reads. In some embodiments, criterion (b) is at least about 60% of capture probes have at least 10-200 on-target total reads.

In some embodiments, criterion (b) is 100% of capture probes have at least 10-200 on-target total reads. In some embodiments, criterion (b) is at least 99.9% of capture probes have at least 10-200 on-target total reads. In some embodiments, criterion (b) is at least 99.5% of capture probes have at least 10-200 on-target total reads. In some embodiments, criterion (b) is at least 99% of capture probes have at least 10-200 on-target total reads. In some embodiments, criterion (b) is at least 98.5% of capture probes have at least 10-200 on-target total reads. In some embodiments, criterion (b) is at least 98% of capture probes have at least 10-200 on-target total reads. In some embodiments, criterion (b) is at least 97.5% of capture probes have at least 10-200 on-target total reads. In some embodiments, criterion (b) is at least 97% of capture probes have at least 10-200 on-target total reads. In some embodiments, criterion (b) is at least 96.5% of capture probes have at least 10-200 on-target total reads. In some embodiments, criterion (b) is at least 96% of capture probes have at least 10-200 on-target total reads. In some embodiments, criterion (b) is at least 95.5% of capture probes have at least 10-200 on-target total reads. In some embodiments, criterion (b) is at least 95% of capture probes have at least 10-200 on-target total reads. In some embodiments of the kits of the disclosure, the pre-determined acceptance criteria for the probe QC process comprise criterion (b) at least 90% of capture probes have at least 10-200 on-target total reads. In some embodiments, criterion (b) is at least 85% of capture probes have at least 10-200 on-target total reads. In some embodiments, criterion (b) is at least 80% of capture probes have at least 10-200 on-target total reads. In some embodiments, criterion (b) is at least 75% of capture probes have at least 10-200 on-target total reads. In some embodiments, criterion (b) is at least 70% of capture probes have at least 10-200 on-target total reads. In some embodiments, criterion (b) is at least 65% of capture probes have at least 10-200 on-target total reads. In some embodiments, criterion (b) is at least 60% of capture probes have at least 10-200 on-target total reads.

In some embodiments, criterion (b) is about 100% of capture probes have at least 50 on-target total reads. In some embodiments, criterion (b) is at least about 99.9% of capture probes have at least 50 on-target total reads. In some embodiments, criterion (b) is at least about 99.5% of capture probes have at least 50 on-target total reads. In some embodiments, criterion (b) is at least about 99% of capture probes have at least 50 on-target total reads. In some embodiments, criterion (b) is at least about 98.5% of capture probes have at least 50 on-target total reads. In some embodiments, criterion (b) is at least about 98% of capture probes have at least 50 on-target total reads. In some embodiments, criterion (b) is at least about 97.5% of capture probes have at least 50 on-target total reads. In some embodiments, criterion (b) is at least about 97% of capture probes have at least 50 on-target total reads. In some embodiments, criterion (b) is at least about 96.5% of capture probes have at least 50 on-target total reads. In some embodiments, criterion (b) is at least about 96% of capture probes have at least 50 on-target total reads. In some embodiments, criterion (b) is at least about 95.5% of capture probes have at least 50 on-target total reads. In some embodiments, criterion (b) is at least about 95% of capture probes have at least 50 on-target total reads. In some embodiments, the pre-determined acceptance criteria for the probe QC process comprise criterion (b) at least about 90% of capture probes have at least 50 on-target total reads. In some embodiments, criterion (b) is at least about 85% of capture probes have at least 50 on-target total reads. In some embodiments, criterion (b) is at least about 80% of capture probes have at least 50 on-target total reads. In some embodiments, criterion (b) is at least about 75% of capture probes have at least 50 on-target total reads. In some embodiments, criterion (b) is at least about 70% of capture probes have at least 50 on-target total reads. In some embodiments, criterion (b) is at least about 65% of capture probes have at least 50 on-target total reads. In some embodiments, criterion (b) is at least about 60% of capture probes have at least 50 on-target total reads.

In some embodiments, criterion (b) is 100% of capture probes have at least 50 on-target total reads. In some embodiments, criterion (b) is at least 99.9% of capture probes have at least 50 on-target total reads. In some embodiments, criterion (b) is at least 99.5% of capture probes have at least 50 on-target total reads. In some embodiments, criterion (b) is at least 99% of capture probes have at least 50 on-target total reads. In some embodiments, criterion (b) is at least 98.5% of capture probes have at least 50 on-target total reads. In some embodiments, criterion (b) is at least 98% of capture probes have at least 50 on-target total reads. In some embodiments, criterion (b) is at least 97.5% of capture probes have at least 50 on-target total reads. In some embodiments, criterion (b) is at least 97% of capture probes have at least 50 on-target total reads. In some embodiments, criterion (b) is at least 96.5% of capture probes have at least 50 on-target total reads. In some embodiments, criterion (b) is at least 96% of capture probes have at least 50 on-target total reads. In some embodiments, criterion (b) is at least 95.5% of capture probes have at least 50 on-target total reads. In some embodiments, criterion (b) is at least 95% of capture probes have at least 50 on-target total reads. In some embodiments of the kits of the disclosure, the pre-determined acceptance criteria for the probe QC process comprise criterion (b) at least 90% of capture probes have at least 50 on-target total reads. In some embodiments, criterion (b) is at least 85% of capture probes have at least 50 on-target total reads. In some embodiments, criterion (b) is at least 80% of capture probes have at least 50 on-target total reads. In some embodiments, criterion (b) is at least 75% of capture probes have at least 50 on-target total reads. In some embodiments, criterion (b) is at least 70% of capture probes have at least 50 on-target total reads. In some embodiments, criterion (b) is at least 65% of capture probes have at least 50 on-target total reads. In some embodiments, criterion (b) is at least 60% of capture probes have at least 50 on-target total reads.

In some embodiments, criterion (b) is about 100% of capture probes have at least 60 on-target total reads. In some embodiments, criterion (b) is at least about 99.9% of capture probes have at least 60 on-target total reads. In some embodiments, criterion (b) is at least about 99.5% of capture probes have at least 60 on-target total reads. In some embodiments, criterion (b) is at least about 99% of capture probes have at least 60 on-target total reads. In some embodiments, criterion (b) is at least about 98.5% of capture probes have at least 60 on-target total reads. In some embodiments, criterion (b) is at least about 98% of capture probes have at least 60 on-target total reads. In some embodiments, criterion (b) is at least about 97.5% of capture probes have at least 60 on-target total reads. In some embodiments, criterion (b) is at least about 97% of capture probes have at least 60 on-target total reads. In some embodiments, criterion (b) is at least about 96.5% of capture probes have at least 60 on-target total reads. In some embodiments, criterion (b) is at least about 96% of capture probes have at least 60 on-target total reads. In some embodiments, criterion (b) is at least about 95.5% of capture probes have at least 60 on-target total reads. In some embodiments, criterion (b) is at least about 95% of capture probes have at least 60 on-target total reads. In some embodiments, the pre-determined acceptance criteria for the probe QC process comprise criterion (b) at least about 90% of capture probes have at least 60 on-target total reads. In some embodiments, criterion (b) is at least about 85% of capture probes have at least 60 on-target total reads. In some embodiments, criterion (b) is at least about 80% of capture probes have at least 60 on-target total reads. In some embodiments, criterion (b) is at least about 75% of capture probes have at least 60 on-target total reads. In some embodiments, criterion (b) is at least about 70% of capture probes have at least 60 on-target total reads. In some embodiments, criterion (b) is at least about 65% of capture probes have at least 60 on-target total reads. In some embodiments, criterion (b) is at least about 60% of capture probes have at least 60 on-target total reads.

In some embodiments, criterion (b) is 100% of capture probes have at least 60 on-target total reads. In some embodiments, criterion (b) is at least 99.9% of capture probes have at least 60 on-target total reads. In some embodiments, criterion (b) is at least 99.5% of capture probes have at least 60 on-target total reads. In some embodiments, criterion (b) is at least 99% of capture probes have at least 60 on-target total reads. In some embodiments, criterion (b) is at least 98.5% of capture probes have at least 60 on-target total reads. In some embodiments, criterion (b) is at least 98% of capture probes have at least 60 on-target total reads. In some embodiments, criterion (b) is at least 97.5% of capture probes have at least 60 on-target total reads. In some embodiments, criterion (b) is at least 97% of capture probes have at least 60 on-target total reads. In some embodiments, criterion (b) is at least 96.5% of capture probes have at least 60 on-target total reads. In some embodiments, criterion (b) is at least 96% of capture probes have at least 60 on-target total reads. In some embodiments, criterion (b) is at least 95.5% of capture probes have at least 60 on-target total reads. In some embodiments, criterion (b) is at least 95% of capture probes have at least 60 on-target total reads. In some embodiments of the kits of the disclosure, the pre-determined acceptance criteria for the probe QC process comprise criterion (b) at least 90% of capture probes have at least 60 on-target total reads. In some embodiments, criterion (b) is at least 85% of capture probes have at least 60 on-target total reads. In some embodiments, criterion (b) is at least 80% of capture probes have at least 60 on-target total reads. In some embodiments, criterion (b) is at least 75% of capture probes have at least 60 on-target total reads. In some embodiments, criterion (b) is at least 70% of capture probes have at least 60 on-target total reads. In some embodiments, criterion (b) is at least 65% of capture probes have at least 60 on-target total reads. In some embodiments, criterion (b) is at least 60% of capture probes have at least 60 on-target total reads.

In some embodiments, criterion (b) is about 100% of capture probes have at least 70 on-target total reads. In some embodiments, criterion (b) is at least about 99.9% of capture probes have at least 70 on-target total reads. In some embodiments, criterion (b) is at least about 99.5% of capture probes have at least 70 on-target total reads. In some embodiments, criterion (b) is at least about 99% of capture probes have at least 70 on-target total reads. In some embodiments, criterion (b) is at least about 98.5% of capture probes have at least 70 on-target total reads. In some embodiments, criterion (b) is at least about 98% of capture probes have at least 70 on-target total reads. In some embodiments, criterion (b) is at least about 97.5% of capture probes have at least 70 on-target total reads. In some embodiments, criterion (b) is at least about 97% of capture probes have at least 70 on-target total reads. In some embodiments, criterion (b) is at least about 96.5% of capture probes have at least 70 on-target total reads. In some embodiments, criterion (b) is at least about 96% of capture probes have at least 70 on-target total reads. In some embodiments, criterion (b) is at least about 95.5% of capture probes have at least 70 on-target total reads. In some embodiments, criterion (b) is at least about 95% of capture probes have at least 70 on-target total reads. In some embodiments, the pre-determined acceptance criteria for the probe QC process comprise criterion (b) at least about 90% of capture probes have at least 70 on-target total reads. In some embodiments, criterion (b) is at least about 85% of capture probes have at least 70 on-target total reads. In some embodiments, criterion (b) is at least about 80% of capture probes have at least 70 on-target total reads. In some embodiments, criterion (b) is at least about 75% of capture probes have at least 70 on-target total reads. In some embodiments, criterion (b) is at least about 70% of capture probes have at least 70 on-target total reads. In some embodiments, criterion (b) is at least about 65% of capture probes have at least 70 on-target total reads. In some embodiments, criterion (b) is at least about 60% of capture probes have at least 70 on-target total reads.

In some embodiments, criterion (b) is 100% of capture probes have at least 70 on-target total reads. In some embodiments, criterion (b) is at least 99.9% of capture probes have at least 70 on-target total reads. In some embodiments, criterion (b) is at least 99.5% of capture probes have at least 70 on-target total reads. In some embodiments, criterion (b) is at least 99% of capture probes have at least 70 on-target total reads. In some embodiments, criterion (b) is at least 98.5% of capture probes have at least 70 on-target total reads. In some embodiments, criterion (b) is at least 98% of capture probes have at least 70 on-target total reads. In some embodiments, criterion (b) is at least 97.5% of capture probes have at least 70 on-target total reads. In some embodiments, criterion (b) is at least 97% of capture probes have at least 70 on-target total reads. In some embodiments, criterion (b) is at least 96.5% of capture probes have at least 70 on-target total reads. In some embodiments, criterion (b) is at least 96% of capture probes have at least 70 on-target total reads. In some embodiments, criterion (b) is at least 95.5% of capture probes have at least 70 on-target total reads. In some embodiments, criterion (b) is at least 95% of capture probes have at least 70 on-target total reads. In some embodiments of the kits of the disclosure, the pre-determined acceptance criteria for the probe QC process comprise criterion (b) at least 90% of capture probes have at least 70 on-target total reads. In some embodiments, criterion (b) is at least 85% of capture probes have at least 70 on-target total reads. In some embodiments, criterion (b) is at least 80% of capture probes have at least 70 on-target total reads. In some embodiments, criterion (b) is at least 75% of capture probes have at least 70 on-target total reads. In some embodiments, criterion (b) is at least 70% of capture probes have at least 70 on-target total reads. In some embodiments, criterion (b) is at least 65% of capture probes have at least 70 on-target total reads. In some embodiments, criterion (b) is at least 60% of capture probes have at least 70 on-target total reads.

In some embodiments, criterion (b) is about 100% of capture probes have at least 80 on-target total reads. In some embodiments, criterion (b) is at least about 99.9% of capture probes have at least 80 on-target total reads. In some embodiments, criterion (b) is at least about 99.5% of capture probes have at least 80 on-target total reads. In some embodiments, criterion (b) is at least about 99% of capture probes have at least 80 on-target total reads. In some embodiments, criterion (b) is at least about 98.5% of capture probes have at least 80 on-target total reads. In some embodiments, criterion (b) is at least about 98% of capture probes have at least 80 on-target total reads. In some embodiments, criterion (b) is at least about 97.5% of capture probes have at least 80 on-target total reads. In some embodiments, criterion (b) is at least about 97% of capture probes have at least 80 on-target total reads. In some embodiments, criterion (b) is at least about 96.5% of capture probes have at least 80 on-target total reads. In some embodiments, criterion (b) is at least about 96% of capture probes have at least 80 on-target total reads. In some embodiments, criterion (b) is at least about 95.5% of capture probes have at least 80 on-target total reads. In some embodiments, criterion (b) is at least about 95% of capture probes have at least 80 on-target total reads. In some embodiments, the pre-determined acceptance criteria for the probe QC process comprise criterion (b) at least about 90% of capture probes have at least 80 on-target total reads. In some embodiments, criterion (b) is at least about 85% of capture probes have at least 80 on-target total reads. In some embodiments, criterion (b) is at least about 80% of capture probes have at least 80 on-target total reads. In some embodiments, criterion (b) is at least about 75% of capture probes have at least 80 on-target total reads. In some embodiments, criterion (b) is at least about 70% of capture probes have at least 80 on-target total reads. In some embodiments, criterion (b) is at least about 65% of capture probes have at least 80 on-target total reads. In some embodiments, criterion (b) is at least about 60% of capture probes have at least 80 on-target total reads.

In some embodiments, criterion (b) is 100% of capture probes have at least 80 on-target total reads. In some embodiments, criterion (b) is at least 99.9% of capture probes have at least 80 on-target total reads. In some embodiments, criterion (b) is at least 99.5% of capture probes have at least 80 on-target total reads. In some embodiments, criterion (b) is at least 99% of capture probes have at least 80 on-target total reads. In some embodiments, criterion (b) is at least 98.5% of capture probes have at least 80 on-target total reads. In some embodiments, criterion (b) is at least 98% of capture probes have at least 80 on-target total reads. In some embodiments, criterion (b) is at least 97.5% of capture probes have at least 80 on-target total reads. In some embodiments, criterion (b) is at least 97% of capture probes have at least 80 on-target total reads. In some embodiments, criterion (b) is at least 96.5% of capture probes have at least 80 on-target total reads. In some embodiments, criterion (b) is at least 96% of capture probes have at least 80 on-target total reads. In some embodiments, criterion (b) is at least 95.5% of capture probes have at least 80 on-target total reads. In some embodiments, criterion (b) is at least 95% of capture probes have at least 80 on-target total reads. In some embodiments of the kits of the disclosure, the pre-determined acceptance criteria for the probe QC process comprise criterion (b) at least 90% of capture probes have at least 80 on-target total reads. In some embodiments, criterion (b) is at least 85% of capture probes have at least 80 on-target total reads. In some embodiments, criterion (b) is at least 80% of capture probes have at least 80 on-target total reads. In some embodiments, criterion (b) is at least 75% of capture probes have at least 80 on-target total reads. In some embodiments, criterion (b) is at least 70% of capture probes have at least 80 on-target total reads. In some embodiments, criterion (b) is at least 65% of capture probes have at least 80 on-target total reads. In some embodiments, criterion (b) is at least 60% of capture probes have at least 80 on-target total reads.

In some embodiments, criterion (b) is at least about 60% to about 99.9% of capture probes have at least 10-200 on-target total reads. In some embodiments, criterion (b) is at least about 70% to about 99.9% of capture probes have at least 10-200 on-target total reads. In some embodiments, criterion (b) is at least about 80% to about 99.9% of capture probes have at least 10-200 on-target total reads. In some embodiments, criterion (b) is at least about 90% to about 99.9% of capture probes have at least 10-200 on-target total reads. In some embodiments, criterion (b) is at least about 95% to about 99.9% of capture probes have at least 10-200 on-target total reads. In some embodiments, criterion (b) is at least about 99% to about 99.9% of capture probes have at least 10-200 on-target total reads.

In some embodiments, criterion (b) is at least 60% to 99.9% of capture probes have at least 10-200 on-target total reads. In some embodiments, criterion (b) is at least 70% to 99.9% of capture probes have at least 10-200 on-target total reads. In some embodiments, criterion (b) is at least 80% to 99.9% of capture probes have at least 10-200 on-target total reads. In some embodiments, criterion (b) is at least 90% to 99.9% of capture probes have at least 10-200 on-target total reads. In some embodiments, criterion (b) is at least 95% to 99.9% of capture probes have at least 10-200 on-target total reads. In some embodiments, criterion (b) is at least 99% to 99.9% of capture probes have at least 10-200 on-target total reads.

In some embodiments, criterion (b) is at least about 60% to about 100% of capture probes have at least 10-200 on-target total reads. In some embodiments, criterion (b) is at least about 70% to about 100% of capture probes have at least 10-200 on-target total reads. In some embodiments, criterion (b) is at least about 80% to about 100% of capture probes have at least 10-200 on-target total reads. In some embodiments, criterion (b) is at least about 90% to about 100% of capture probes have at least 10-200 on-target total reads. In some embodiments, criterion (b) is at least about 95% to about 100% of capture probes have at least 10-200 on-target total reads. In some embodiments, criterion (b) is at least about 99% to about 100% of capture probes have at least 10-200 on-target total reads.

In some embodiments, criterion (b) is at least 60% to 100% of capture probes have at least 10-200 on-target total reads. In some embodiments, criterion (b) is at least 70% to 100% of capture probes have at least 10-200 on-target total reads. In some embodiments, criterion (b) is at least 80% to 100% of capture probes have at least 10-200 on-target total reads. In some embodiments, criterion (b) is at least 90% to 100% of capture probes have at least 10-200 on-target total reads. In some embodiments, criterion (b) is at least 95% to 100% of capture probes have at least 10-200 on-target total reads. In some embodiments, criterion (b) is at least 99% to 100% of capture probes have at least 10-200 on-target total reads.

In some embodiments, criterion (b) is at least about 60% to about 100% of capture probes have at least 50 on-target total reads. In some embodiments, criterion (b) is at least about 70% to about 100% of capture probes have at least 50 on-target total reads. In some embodiments, criterion (b) is at least about 80% to about 100% of capture probes have at least 50 on-target total reads. In some embodiments, criterion (b) is at least about 90% to about 100% of capture probes have at least 50 on-target total reads. In some embodiments, criterion (b) is at least about 95% to about 100% of capture probes have at least 50 on-target total reads. In some embodiments, criterion (b) is at least about 99% to about 100% of capture probes have at least 50 on-target total reads.

In some embodiments, criterion (b) is at least 60% to 100% of capture probes have at least 50 on-target total reads. In some embodiments, criterion (b) is at least 70% to 100% of capture probes have at least 50 on-target total reads. In some embodiments, criterion (b) is at least 80% to 100% of capture probes have at least 50 on-target total reads. In some embodiments, criterion (b) is at least 90% to 100% of capture probes have at least 50 on-target total reads. In some embodiments, criterion (b) is at least 95% to 100% of capture probes have at least 50 on-target total reads. In some embodiments, criterion (b) is at least 99% to 100% of capture probes have at least 50 on-target total reads.

In some embodiments, criterion (b) is at least about 60% to about 100% of capture probes have at least 60 on-target total reads. In some embodiments, criterion (b) is at least about 70% to about 100% of capture probes have at least 60 on-target total reads. In some embodiments, criterion (b) is at least about 80% to about 100% of capture probes have at least 60 on-target total reads. In some embodiments, criterion (b) is at least about 90% to about 100% of capture probes have at least 60 on-target total reads. In some embodiments, criterion (b) is at least about 95% to about 100% of capture probes have at least 60 on-target total reads. In some embodiments, criterion (b) is at least about 99% to about 100% of capture probes have at least 60 on-target total reads.

In some embodiments, criterion (b) is at least 60% to 100% of capture probes have at least 60 on-target total reads. In some embodiments, criterion (b) is at least 70% to 100% of capture probes have at least 60 on-target total reads. In some embodiments, criterion (b) is at least 80% to 100% of capture probes have at least 60 on-target total reads. In some embodiments, criterion (b) is at least 90% to 100% of capture probes have at least 60 on-target total reads. In some embodiments, criterion (b) is at least 95% to 100% of capture probes have at least 60 on-target total reads. In some embodiments, criterion (b) is at least 99% to 100% of capture probes have at least 60 on-target total reads.

In some embodiments, criterion (b) is at least about 60% to about 100% of capture probes have at least 70 on-target total reads. In some embodiments, criterion (b) is at least about 70% to about 100% of capture probes have at least 70 on-target total reads. In some embodiments, criterion (b) is at least about 80% to about 100% of capture probes have at least 70 on-target total reads. In some embodiments, criterion (b) is at least about 90% to about 100% of capture probes have at least 70 on-target total reads. In some embodiments, criterion (b) is at least about 95% to about 100% of capture probes have at least 70 on-target total reads. In some embodiments, criterion (b) is at least about 99% to about 100% of capture probes have at least 70 on-target total reads.

In some embodiments, criterion (b) is at least 60% to 100% of capture probes have at least 70 on-target total reads. In some embodiments, criterion (b) is at least 70% to 100% of capture probes have at least 70 on-target total reads. In some embodiments, criterion (b) is at least 80% to 100% of capture probes have at least 70 on-target total reads. In some embodiments, criterion (b) is at least 90% to 100% of capture probes have at least 70 on-target total reads. In some embodiments, criterion (b) is at least 95% to 100% of capture probes have at least 70 on-target total reads. In some embodiments, criterion (b) is at least 99% to 100% of capture probes have at least 70 on-target total reads.

In some embodiments, criterion (b) is at least about 60% to about 100% of capture probes have at least 80 on-target total reads. In some embodiments, criterion (b) is at least about 70% to about 100% of capture probes have at least 80 on-target total reads. In some embodiments, criterion (b) is at least about 80% to about 100% of capture probes have at least 80 on-target total reads. In some embodiments, criterion (b) is at least about 90% to about 100% of capture probes have at least 80 on-target total reads. In some embodiments, criterion (b) is at least about 95% to about 100% of capture probes have at least 80 on-target total reads. In some embodiments, criterion (b) is at least about 99% to about 100% of capture probes have at least 80 on-target total reads.

In some embodiments, criterion (b) is at least 60% to 100% of capture probes have at least 80 on-target total reads. In some embodiments, criterion (b) is at least 70% to 100% of capture probes have at least 80 on-target total reads. In some embodiments, criterion (b) is at least 80% to 100% of capture probes have at least 80 on-target total reads. In some embodiments, criterion (b) is at least 90% to 100% of capture probes have at least 80 on-target total reads. In some embodiments, criterion (b) is at least 95% to 100% of capture probes have at least 80 on-target total reads. In some embodiments, criterion (b) is at least 99% to 100% of capture probes have at least 80 on-target total reads.

In some embodiments of the methods for performing a probe QC process of the disclosure, the pre-determined acceptance criteria for the probe QC process comprise a criterion (c). In some embodiments, criterion (c) is at least about 99.9% of expected SNPs within the DNA sample are detected. In some embodiments, criterion (c) is at least about 99.5% of expected SNPs within the DNA sample are detected. In some embodiments, criterion (c) is at least about 99% of expected SNPs within the DNA sample are detected. In some embodiments, criterion (c) is at least about 98% of expected SNPs within the DNA sample are detected. In some embodiments, criterion (c) is at least about 97% of expected SNPs within the DNA sample are detected. In some embodiments, criterion (c) is at least about 96% of expected SNPs within the DNA sample are detected. In some embodiments, criterion (c) is at least about 95% of expected SNPs within the DNA sample are detected. In some embodiments, criterion (c) is at least about 90% of expected SNPs within the DNA sample are detected. In some embodiments, criterion (c) is at least about 85% of expected SNPs within the DNA sample are detected. In some embodiments, criterion (c) is at least about 80% of expected SNPs within the DNA sample are detected. In some embodiments, criterion (c) is at least about 75% of expected SNPs within the DNA sample are detected. In some embodiments, criterion (c) is at least about 70% of expected SNPs within the DNA sample are detected. In some embodiments, criterion (c) is at least about 65% of expected SNPs within the DNA sample are detected. In some embodiments, criterion (c) is at least about 60% of expected SNPs within the DNA sample are detected.

In some embodiments, criterion (c) is at least 99.9% of expected SNPs within the DNA sample are detected. In some embodiments, criterion (c) is at least 99.5% of expected SNPs within the DNA sample are detected. In some embodiments, criterion (c) is at least 99% of expected SNPs within the DNA sample are detected. In some embodiments, criterion (c) is at least 98% of expected SNPs within the DNA sample are detected. In some embodiments, criterion (c) is at least 97% of expected SNPs within the DNA sample are detected. In some embodiments, criterion (c) is at least 96% of expected SNPs within the DNA sample are detected. In some embodiments, criterion (c) is at least 95% of expected SNPs within the DNA sample are detected. In some embodiments, criterion (c) is at least 90% of expected SNPs within the DNA sample are detected. In some embodiments, criterion (c) is at least 85% of expected SNPs within the DNA sample are detected. In some embodiments, criterion (c) is at least 80% of expected SNPs within the DNA sample are detected. In some embodiments, criterion (c) is at least 75% of expected SNPs within the DNA sample are detected. In some embodiments, criterion (c) is at least 70% of expected SNPs within the DNA sample are detected. In some embodiments, criterion (c) is at least 65% of expected SNPs within the DNA sample are detected. In some embodiments, criterion (c) is at least 60% of expected SNPs within the DNA sample are detected.

In some embodiments, criterion (c) is at least about 60% to about 99.9% of expected SNPs within the DNA sample are detected. In some embodiments, criterion (c) is at least about 70% to about 99.9% of expected SNPs within the DNA sample are detected. In some embodiments, criterion (c) is at least about 80% to about 99.9% of expected SNPs within the DNA sample are detected. In some embodiments, criterion (c) is at least about 90% to about 99.9% of expected SNPs within the DNA sample are detected. In some embodiments, criterion (c) is at least about 95% to about 99.9% of expected SNPs within the DNA sample are detected. In some embodiments, criterion (c) is at least about 96% to about 99.9% of expected SNPs within the DNA sample are detected. In some embodiments, criterion (c) is at least about 97% to about 99.9% of expected SNPs within the DNA sample are detected. In some embodiments, criterion (c) is at least about 98% to about 99.9% of expected SNPs within the DNA sample are detected. In some embodiments, criterion (c) is at least about 99% to about 99.9% of expected SNPs within the DNA sample are detected. In some embodiments, criterion (c) is about 100% of expected SNPs within the DNA sample are detected.

In some embodiments, criterion (c) is at least 60% to 99.9% of expected SNPs within the DNA sample are detected. In some embodiments, criterion (c) is at least 70% to 99.9% of expected SNPs within the DNA sample are detected. In some embodiments, criterion (c) is at least 80% to 99.9% of expected SNPs within the DNA sample are detected. In some embodiments, criterion (c) is at least 90% to 99.9% of expected SNPs within the DNA sample are detected. In some embodiments, criterion (c) is at least 95% to 99.9% of expected SNPs within the DNA sample are detected. In some embodiments, criterion (c) is at least 96% to 99.9% of expected SNPs within the DNA sample are detected. In some embodiments, criterion (c) is at least 97% to 99.9% of expected SNPs within the DNA sample are detected. In some embodiments, criterion (c) is at least 98% to 99.9% of expected SNPs within the DNA sample are detected. In some embodiments, criterion (c) is at least about 99% to 99.9% of expected SNPs within the DNA sample are detected. In some embodiments, criterion (c) is 100% of expected SNPs within the DNA sample are detected.

In some embodiments, criterion (c) is at least about 60% to about 100% of expected SNPs within the DNA sample are detected. In some embodiments, criterion (c) is at least about 70% to about 100% of expected SNPs within the DNA sample are detected. In some embodiments, criterion (c) is at least about 80% to about 100% of expected SNPs within the DNA sample are detected. In some embodiments, criterion (c) is at least about 90% to about 100% of expected SNPs within the DNA sample are detected. In some embodiments, criterion (c) is at least about 95% to about 100% of expected SNPs within the DNA sample are detected. In some embodiments, criterion (c) is at least about 96% to about 100% of expected SNPs within the DNA sample are detected. In some embodiments, criterion (c) is at least about 97% to about 100% of expected SNPs within the DNA sample are detected. In some embodiments, criterion (c) is at least about 98% to about 100% of expected SNPs within the DNA sample are detected. In some embodiments, criterion (c) is at least about 99% to about 100% of expected SNPs within the DNA sample are detected. In some embodiments, criterion (c) is about 100% of expected SNPs within the DNA sample are detected.

In some embodiments, criterion (c) is at least 60% to 100% of expected SNPs within the DNA sample are detected. In some embodiments, criterion (c) is at least 70% to 100% of expected SNPs within the DNA sample are detected. In some embodiments, criterion (c) is at least 80% to 100% of expected SNPs within the DNA sample are detected. In some embodiments, criterion (c) is at least 90% to 100% of expected SNPs within the DNA sample are detected. In some embodiments, criterion (c) is at least 95% to 100% of expected SNPs within the DNA sample are detected. In some embodiments, criterion (c) is at least 96% to 100% of expected SNPs within the DNA sample are detected. In some embodiments, criterion (c) is at least 97% to 100% of expected SNPs within the DNA sample are detected. In some embodiments, criterion (c) is at least 98% to 100% of expected SNPs within the DNA sample are detected. In some embodiments, criterion (c) is at least about 99% to 100% of expected SNPs within the DNA sample are detected. In some embodiments, criterion (c) is 100% of expected SNPs within the DNA sample are detected.

In some embodiments of the methods for performing a probe QC process of the disclosure, the pre-determined acceptance criteria for the probe QC process comprise a criterion (a) and a criterion (b). In some embodiments of the methods for performing a probe QC process of the disclosure, the pre-determined acceptance criteria for the probe QC process comprise a criterion (a) and a criterion (c). In some embodiments of the methods for performing a probe QC process of the disclosure, the pre-determined acceptance criteria for the probe QC process comprise a criterion (b) and a criterion (c). In some embodiments of the methods for performing a probe QC process of the disclosure, the pre-determined acceptance criteria for the probe QC process comprise a criterion (a), a criterion (b), and a criterion (c).

In some embodiments of the methods for performing a probe QC process of the disclosure, the DNA sample used in the probe QC process comprises human wild type DNA.

In some embodiments of the methods for performing a probe QC process of the disclosure, the DNA sample used in the probe QC process comprises a plurality of single nucleotide polymorphisms (SNPs).

In some embodiments of the methods for performing a probe QC process of the disclosure, the DNA sample used in the probe QC process comprises any suitable DNA.

In some embodiments of the methods for performing a probe QC process of the disclosure, the tail sequence of the capture probe module comprises a primer binding site.

In some embodiments of the methods for performing a probe QC process of the disclosure, the tail sequence comprises a sequencing primer binding site.

In some embodiments of the methods for performing a probe QC process of the disclosure, the tail sequence is configured to hybridize to a partner oligonucleotide.

In some embodiments of the methods for performing a probe QC process of the disclosure, each capture probe module is selected from a capture probe panel comprising a plurality of capture probe modules.

In some embodiments of the methods for performing a probe QC process of the disclosure, at least one capture probe module is configured to hybridize downstream of a specific DNA target region and at least one capture probe module is configured to hybridize upstream of the specific DNA target region.

In some embodiments of the methods for performing a probe QC process of the disclosure, each capture probe of the plurality of capture probe modules is configured to hybridize to its target sequence within about 200 bp of any other capture probe.

In some embodiments of the methods for performing a probe QC process of the disclosure, each capture probe module of the capture probe panel hybridizes to an adaptor-tagged DNA fragment to form a plurality of adaptor-tagged DNA fragment-capture probe module complexes.

In some embodiments of the methods for performing a probe QC process of the disclosure, the partner oligonucleotide comprises a specific member of a binding pair to enable isolation of the adaptor-tagged DNA fragment-capture probe module complexes.

In some embodiments of the methods for performing a probe QC process of the disclosure, the partner oligonucleotide comprises a biotin molecule.

In some embodiments of the methods for performing a probe QC process of the disclosure, each capture probe module comprises a specific member of a binding pair to enable isolation of the adaptor-tagged DNA fragment-capture probe module complex.

In some embodiments of the methods for performing a probe QC process of the disclosure, the capture probe module comprises a biotin molecule.

In some embodiments of the methods for performing a probe QC process of the disclosure, each capture probe module comprises a capture probe less than about 60 nucleotides in length.

In some embodiments of the methods for performing a probe QC process of the disclosure, each capture probe module comprises a capture probe less than 60 nucleotides in length.

In some embodiments of the methods for performing a probe QC process of the disclosure, each capture probe module comprises a capture probe that is about 40 nucleotides in length.

In some embodiments of the methods for performing a probe QC process of the disclosure, each capture probe module comprises a capture probe that is 40 nucleotides in length.

In some embodiments of the methods for performing a probe QC process of the disclosure, streptavidin is used in isolating the adaptor-tagged DNA fragment-capture probe module complexes.

In some embodiments of the methods for performing a probe QC process of the disclosure, the step of enzymatically processing the isolated adaptor-tagged DNA fragment-capture probe module complexes to generate Hybrid Molecules comprises performing 5'-3' polymerase extension on each capture probe of the adaptor-tagged DNA fragment-capture probe module complexes using the adaptor-tagged DNA fragment as a template to create a plurality of Hybrid Molecules (Target Capture Library), wherein each Hybrid Molecule comprises a capture probe module and a complement of an adaptor-tagged DNA fragment.

In some embodiments of the methods for performing a probe QC process of the disclosure, the SRL is sequenced on a single flow cell of a sequencing machine.

In some embodiments of the methods for performing a probe QC process of the disclosure, quantitative genetic analysis on the SRL comprises use of one or more sequencing primers. In some embodiments, the quantitative genetic analysis is used to detect a nucleotide transition or transversion, a nucleotide insertion or deletion, a genomic rearrangement, or a change in copy number in the test sample DNA fragments.

Some embodiments of the disclosure provide methods for simultaneous DNA analysis, comprising: (a) to a first adaptor-tagged library comprising a first genetic locus, performing a first process to generate a first modified library; (b) to a second adaptor-tagged library comprising a second genetic locus, performing a second process to generate a second modified library; wherein the first process and the second process are not identical; and (c) contacting the first modified library and the second modified library to generate a combined library; wherein the first adaptor-tagged library and the second adaptor-tagged library are from the same adaptor-tagged parent library.

In some embodiments of the methods for simultaneous DNA analysis of the disclosure, the method comprises an initial step of splitting the adaptor-tagged parent library into at least two partitions, wherein a first partition is the first adaptor-tagged library and a second partition is the second adaptor-tagged library.

In some embodiments of the methods for simultaneous DNA analysis of the disclosure, the first modified library comprises: a first DNA molecule comprising, from 5' to 3', a 5' oligonucleotide (A1), a first 5' adaptor module, a sequence of the first genetic locus, and a 3' oligonucleotide (A2); or a first DNA molecule comprising, from 5' to 3', a 5' oligonucleotide (A1), a sequence of the first genetic locus, a first 3' adaptor module, and a 3' oligonucleotide (A2); or a first DNA molecule comprising, from 5' to 3', a 5' oligonucleotide (A1), a first 5' adaptor module, a sequence of the first genetic locus, a first 3' adaptor module, and a 3' oligonucleotide (A2); wherein the first genetic locus comprises a DNA region; wherein a first test sample comprises a DNA molecule having a sequence identical to a sequence of the first genetic locus or a complementary sequence thereof; and wherein the first 5' adaptor module, the first 3' adaptor module, or both, are coupled to the first genetic locus in the first adaptor-tagged library; and the second modified library comprises: a second DNA molecule comprising, from 5' to 3', a 5' oligonucleotide (B1), a second 5' adaptor module, a sequence of the second genetic locus, and a 3' oligonucleotide (B2); or a second DNA molecule comprising, from 5' to 3', a 5' oligonucleotide (B1), a sequence of the second genetic locus, a second 3' adaptor module and a 3' oligonucleotide (B2); or a second DNA molecule comprising, from 5' to 3', a 5' oligonucleotide (B1), a second 5' adaptor module, a sequence of the second genetic locus, a second 3' adaptor module and a 3' oligonucleotide (B2); wherein the second genetic locus comprises a DNA region; wherein a second test sample comprises a DNA molecule having a sequence identical to a sequence of the second genetic locus or a complementary sequence thereof; and wherein the second 5' adaptor module, the second 3' adaptor module, or both, are coupled to the second genetic locus in the second adaptor-tagged library; wherein the first modified library comprises a Library Tag.

In some embodiments of the methods for simultaneous DNA analysis of the disclosure, the first modified library comprises: a first DNA molecule comprising, from 5' to 3', a 5' oligonucleotide (A1), a first 5' adaptor module, a sequence of the first genetic locus, and a 3' oligonucleotide (A2); or a first DNA molecule comprising, from 5' to 3', a 5' oligonucleotide (A1), a sequence of the first genetic locus, a first 3' adaptor module, and a 3' oligonucleotide (A2); or a first DNA molecule comprising, from 5' to 3', a 5' oligonucleotide (A1), a first 5' adaptor module, a sequence of the first genetic locus, a first 3' adaptor module, and a 3' oligonucleotide (A2); wherein the first genetic locus comprises a DNA region; a first test sample comprises a DNA molecule having a sequence identical to a sequence of the first genetic locus or a complementary sequence thereof; and wherein the first 5' adaptor module, the first 3' adaptor module, or both, are coupled to the first genetic locus in the first adaptor-tagged library; and the second modified library comprises: a second DNA molecule comprising, from 5' to 3', a 5' oligonucleotide (B1), a second 5' adaptor module, a sequence of the second genetic locus, and a 3' oligonucleotide (B2); or a second DNA molecule comprising, from 5' to 3', a 5' oligonucleotide (B1), a sequence of the second genetic locus, a second 3' adaptor module and a 3' oligonucleotide (B2); or a second DNA molecule comprising, from 5' to 3', a 5' oligonucleotide (B1), a second 5' adaptor module, a sequence of the second genetic locus, a second 3' adaptor module and a 3' oligonucleotide (B2); wherein the second genetic locus comprises a DNA region; wherein a second test sample comprises a DNA molecule having a sequence identical to a sequence of the second genetic locus or a complementary sequence thereof; and wherein the second 5' adaptor module, the second 3' adaptor module, or both, are coupled to the second genetic locus in the second adaptor-tagged library; wherein the second modified library comprises a Library Tag.

In some embodiments of the methods for simultaneous DNA analysis of the disclosure, the first modified library comprises: a first DNA molecule comprising, from 5' to 3', a 5' oligonucleotide (A1), a first 5' adaptor module, a sequence of the first genetic locus, and a 3' oligonucleotide (A2); or a first DNA molecule comprising, from 5' to 3', a 5' oligonucleotide (A1), a sequence of the first genetic locus, a first 3' adaptor module, and a 3' oligonucleotide (A2); or a first DNA molecule comprising, from 5' to 3', a 5' oligonucleotide (A1), a first 5' adaptor module, a sequence of the first genetic locus, a first 3' adaptor module, and a 3' oligonucleotide (A2); wherein the first genetic locus comprises a DNA region; wherein a first test sample comprises a DNA molecule having a sequence identical to a sequence of the first genetic locus or a complementary sequence thereof; and wherein the first 5' adaptor module, the first 3' adaptor module, or both, are coupled to the first genetic locus in the first adaptor-tagged library; and the second modified library comprises: a second DNA molecule comprising, from 5' to 3', a 5' oligonucleotide (B1), a second 5' adaptor module, a sequence of the second genetic locus, and a 3' oligonucleotide (B2); or a second DNA molecule comprising, from 5' to 3', a 5' oligonucleotide (B1), a sequence of the second genetic locus, a second 3' adaptor module and a 3' oligonucleotide (B2); or a second DNA molecule comprising, from 5' to 3', a 5' oligonucleotide (B1), a second 5' adaptor module, a sequence of the second genetic locus, a second 3' adaptor module and a 3' oligonucleotide (B2); wherein the second genetic locus comprises a DNA region; wherein a second test sample comprises a DNA molecule having a sequence identical to a sequence of the second genetic locus or a complementary sequence thereof; and wherein the second 5' adaptor module, the second 3' adaptor module, or both, are coupled to the second genetic locus in the second adaptor-tagged library; wherein the first modified library comprises a first Library Tag and the second modified library comprises a second Library Tag, wherein the first and second Library Tags are not identical.

In some embodiments of the methods for simultaneous DNA analysis of the disclosure, the first test sample and the second test sample are the same sample.

In some embodiments of the methods for simultaneous DNA analysis of the disclosure, the first test sample and the second test sample are not the same sample.

In some embodiments of the methods for simultaneous DNA analysis of the disclosure, the first genetic locus and the second genetic locus are identical.

In some embodiments of the methods for simultaneous DNA analysis of the disclosure, the first genetic locus and the second genetic locus are not identical.

In some embodiments of the methods for simultaneous DNA analysis of the disclosure, the Library Tag distinguishes the first DNA sequence of the first modified library from the second DNA sequence of the second modified library.

In some embodiments of the methods for simultaneous DNA analysis of the disclosure, at least one of A1, A2, B1, and B2 comprises at least one Library Tag.

In some embodiments of the methods for simultaneous DNA analysis of the disclosure, the Library Tag comprises a nucleic acid sequence or an amino acid sequence.

In some embodiments of the methods for simultaneous DNA analysis of the disclosure, the Library Tag comprises one or more of a DNA, an RNA, a PNA, or a non-naturally occurring nucleic acid, a synthetic nucleic acid, a modified nucleic acid, a non-naturally occurring amino acid, a synthetic amino acid, and a modified amino acid.

In some embodiments of the methods for simultaneous DNA analysis of the disclosure, the Library Tag comprises a detectable label.

In some embodiments of the methods for simultaneous DNA analysis of the disclosure, the detectable label comprises one or more of a fluorescent moiety, a magnetic or paramagnetic moiety, an enzymatic moiety, a binding moiety, an epitope, and a radioactive moiety.

In some embodiments of the methods for simultaneous DNA analysis of the disclosure, the Library Tag selectively or specifically binds to a detectable moiety.

In some embodiments of the methods for simultaneous DNA analysis of the disclosure, the detectable moiety comprises one or more of a fluorescent moiety, a magnetic or paramagnetic moiety, an enzymatic moiety, a binding moiety, an epitope, and a radioactive moiety.

In some embodiments of the methods for simultaneous DNA analysis of the disclosure, the Library Tag comprises a unique polynucleotide sequence.

In some embodiments of the methods for simultaneous DNA analysis of the disclosure, the unique polynucleotide sequence comprises a sequence having 70% identity, or less, to a sequence selected from the list consisting of A1, B1, A2, B2, the first 5' adaptor module, the first genetic locus, the first 3' adaptor module, the second 5' adaptor module, the second genetic locus, and the second 3' adaptor module.

In some embodiments of the methods for simultaneous DNA analysis of the disclosure, the first test sample and the second test sample are not the same sample; A1, A2, B1, or B2 contains a Library Tag; the Library Tag is capable of distinguishing the first DNA sequence of the first modified library from the second DNA sequence of the second modified library; the Library Tag comprises a unique polynucleotide sequence; and the unique polynucleotide sequence comprises a sequence having 70% identity, or less, to a sequence selected from the list consisting of A1, B1, A2, B2, the first 5' adaptor module, the first genetic locus, the first 3' adaptor module, the second 5' adaptor module, the second genetic locus, and the second 3' adaptor module.

In some embodiments of the methods for simultaneous DNA analysis of the disclosure, the first adaptor-tagged library and the second adaptor-tagged library each comprises an adaptor module.

In some embodiments of the methods for simultaneous DNA analysis of the disclosure, each adaptor comprises a ligation strand oligonucleotide and a non-ligation strand oligonucleotide.

In some embodiments of the methods for simultaneous DNA analysis of the disclosure, the non-ligation strand oligonucleotide is capable of hybridizing to a region at the 3' end of the ligation strand oligonucleotide and forming a duplex therewith.

In some embodiments of the methods for simultaneous DNA analysis of the disclosure, the ligation strand oligonucleotide comprises an adaptor module.

In some embodiments of the methods for simultaneous DNA analysis of the disclosure, the ligation strand oligonucleotide comprises a dT, dA, dC, or dG overhang at the 3' terminus.

In some embodiments of the methods for simultaneous DNA analysis of the disclosure, the non-ligation strand oligonucleotide comprises a modification at its 3' terminus that prevents ligation to the 5' end of a dsDNA fragment and/or adaptor dimer formation, wherein the non-ligation strand is configured to be displaced from the duplex.

In some embodiments of the methods for simultaneous DNA analysis of the disclosure, each adaptor is selected from a set of adaptors, wherein each adaptor of the set of adaptors comprises an ID region selected from a pool of unique ID regions, wherein the pool is selected from a plurality of pools, and wherein the selected pool is unique to the test sample.

In some embodiments of the methods for simultaneous DNA analysis of the disclosure, the adaptor module comprises (a) an amplification region comprising a primer binding site; (b) an ID region; and (c) an anchor region.

In some embodiments of the methods for simultaneous DNA analysis of the disclosure, the adaptor module comprises (a) an amplification region comprising a polynucleotide comprising a primer binding site; (b) an ID region; and (c) an anchor region.

In some embodiments of the methods for simultaneous DNA analysis of the disclosure, the amplification region comprises a primer binding site, wherein the primer binding site allows for amplification using PCR (polymerase chain reaction), LAMP (loop-mediated isothermal amplification), NASBA (nucleic acid sequence-based amplification), SDA (standard displacement amplification), RCA (rolling circle replication), or LCR (ligase chain reaction).

In some embodiments of the methods for simultaneous DNA analysis of the disclosure, the amplification region comprises a polynucleotide sequence comprising a primer binding site, wherein the primer binding site allows for amplification of the nucleic acids of the first and/or second adaptor tagged library using PCR (polymerase chain reaction), LAMP (loop-mediated isothermal amplification), NASBA (nucleic acid sequence-based amplification), SDA (standard displacement amplification), RCA (rolling circle replication), or LCR (ligase chain reaction).

In some embodiments of the methods for simultaneous DNA analysis of the disclosure, the amplification region comprises or consists of between 10 and 50 nucleotides. In some embodiments, the amplification region comprises or consists of between 20 and 30 nucleotides. In some embodiments, the amplification region comprises or consists of 25 nucleotides.

In some embodiments of the methods for simultaneous DNA analysis of the disclosure, the amplification region comprises or consists of about 10 to about 50 nucleotides. In some embodiments, the amplification region comprises or consists of about 20 to about 30 nucleotides. In some embodiments, the amplification region comprises or consists of about 25 nucleotides.

In some embodiments of the methods for simultaneous DNA analysis of the disclosure, the anchor region comprises an overhang at the 3' terminus.

In some embodiments of the methods for simultaneous DNA analysis of the disclosure, the anchor region comprises or consists of between 1 and 50 nucleotides. In some embodiments, the anchor region comprises or consists of between 5 and 25 nucleotides. In some embodiments, the anchor region comprises or consists of 10 nucleotides.

In some embodiments of the methods for simultaneous DNA analysis of the disclosure, the anchor region comprises or consists of about 1 to about 50 nucleotides. In some embodiments, the anchor region comprises or consists of about 5 to about 25 nucleotides. In some embodiments, the anchor region comprises or consists of about 10 nucleotides.

In some embodiments of the methods for simultaneous DNA analysis of the disclosure, the ID region comprises or consists of between 3 and 50 nucleotides. In some embodiments, the ID region comprises or consists of between 3 and 15 nucleotides. In some embodiments, the ID region comprises or consists of 8 nucleotides.

In some embodiments of the methods for simultaneous DNA analysis of the disclosure, the ID region comprises or consists of about 3 to about 50 nucleotides. In some embodiments, the ID region comprises or consists of about 3 to about 15 nucleotides. In some embodiments, the ID region comprises or consists of about 8 nucleotides.

In some embodiments of the methods for simultaneous DNA analysis of the disclosure, the adaptor module further comprises a unique molecule identifier (UMI) multiplier.

In some embodiments of the methods for simultaneous DNA analysis of the disclosure, the UMI multiplier is adjacent to or contained within the ID region.

In some embodiments of the methods for simultaneous DNA analysis of the disclosure, the UMI multiplier comprises or consists of between 1 and 5 nucleotides.

In some embodiments of the methods for simultaneous DNA analysis of the disclosure, the UMI multiplier comprises or consists of about 1 to about 5 nucleotides.

In some embodiments of the methods for simultaneous DNA analysis of the disclosure, the UMI multiplier is 3 nucleotides in length, and comprises a nucleic acid sequence selected from a group of 64 unique nucleotide sequences.

In some embodiments of the methods for simultaneous DNA analysis of the disclosure, a plurality of adaptor modules comprises the adaptor molecule.

In some embodiments of the methods for simultaneous DNA analysis of the disclosure, each adaptor module of the plurality of adaptor modules is unique.

In some embodiments of the methods for simultaneous DNA analysis of the disclosure, the amplification region of each adaptor module of the plurality of adaptor modules comprises a custom primer binding site.

In some embodiments of the methods for simultaneous DNA analysis of the disclosure, the custom primer binding site comprises a sequence having 100% sequence identity to a sequence of the custom primer binding site of each adaptor module of the plurality of adaptor modules.

In some embodiments of the methods for simultaneous DNA analysis of the disclosure, the plurality of adaptor modules comprises a first subdivision and a second subdivision.

In some embodiments of the methods for simultaneous DNA analysis of the disclosure, each adaptor module comprises an ID region comprising a polynucleotide sequence that is distinct from the polynucleotide sequence of the ID region of any other adaptor module.

In some embodiments of the methods for simultaneous DNA analysis of the disclosure, the pool of ID regions comprises between 2 and 10,000 unique ID region sequences. In some embodiments, the pool of ID regions comprises between 10 and 500 unique ID region sequences. In some embodiments, the pool of ID regions comprises between 50 and 300 unique ID region sequences. In some embodiments, the pool of ID regions comprises 60 unique ID region sequences.

In some embodiments of the methods for simultaneous DNA analysis of the disclosure, the pool of ID regions comprises about 2 to about 10,000 unique ID region sequences. In some embodiments, the pool of ID regions comprises about 10 to about 500 unique ID region sequences. In some embodiments, the pool of ID regions comprises about 50 to about 300 unique ID region sequences. In some embodiments, the pool of ID regions comprises about 60 unique ID region sequences.

In some embodiments of the methods for simultaneous DNA analysis of the disclosure, each ID region of the pool of ID regions is 8 nucleotides in length.

In some embodiments of the methods for simultaneous DNA analysis of the disclosure, each ID region sequence is discrete from any other ID region sequence by Hamming distance of at least two.

In some embodiments of the methods for simultaneous DNA analysis of the disclosure, each ID region is configured to identify the DNA fragment attached thereto.

In some embodiments of the methods for simultaneous DNA analysis of the disclosure, each adaptor module of the set of adaptor modules is selected from a group consisting of between 64 and 2,560,000 unique nucleotide sequences.

In some embodiments of the methods for simultaneous DNA analysis of the disclosure, each adaptor module of the set of adaptor modules is selected from a group consisting of about 64 to about 2,560,000 unique nucleotide sequences.

In some embodiments of the methods for simultaneous DNA analysis of the disclosure, each adaptor module of the set of adaptor modules comprises a unique nucleotide sequence selected from 3840 unique nucleotide sequences, wherein each sequence of the 3840 unique nucleotide sequences is discrete from any other sequence by Hamming distance of at least two.

In some embodiments of the methods for simultaneous DNA analysis of the disclosure, the anchor region of each adaptor of the set of adaptors comprises one of four nucleotide sequences, and wherein each ID region of a given sequence is paired to only one of the four anchor regions of a given sequence.

In some embodiments of the methods for simultaneous DNA analysis of the disclosure, the amplification region of each adaptor of the set of adaptors comprises an identical primer binding site.

In some embodiments of the methods for simultaneous DNA analysis of the disclosure, each ID region of the pool of ID regions is 8 nucleotides in length, each ID region sequence is discrete from any other ID region sequence by Hamming distance of at least two; each adaptor of the set of adaptors comprises a UMI multiplier that is adjacent to or contained within the ID region, wherein the UMI multiplier of each adaptor of the set of adaptors is three nucleotides in length, and wherein the UMI multiplier of a given sequence is paired to one ID region of a given sequence; the anchor region of each adaptor of the set of adaptors comprises one of four nucleotide sequences, and wherein each ID region of a given sequence is paired to only one of the four anchor regions of a given sequence; and the amplification region of each adaptor of the set of adaptors comprises an identical primer binding site.

In some embodiments of the methods for simultaneous DNA analysis of the disclosure, the first subdivision comprises a first set of unique ID regions and the second subdivision comprises a second set of unique ID regions.

In some embodiments of the methods for simultaneous DNA analysis of the disclosure, each adaptor module further comprises a sample tag comprising a polynucleotide sequence that is not identical to the polynucleotide sequence of the ID region.

In some embodiments of the methods for simultaneous DNA analysis of the disclosure, each adaptor module in the first subdivision comprises a sample tag comprising a first polynucleotide sequence; wherein each adaptor module in the second subdivision comprises a sample tag comprising a second polynucleotide sequence; wherein the first and second polynucleotide sequences are not identical to each other.

In some embodiments of the methods for simultaneous DNA analysis of the disclosure, the first subdivision of adaptor modules identifies each DNA molecule within the first test sample and the second subdivision of adaptor modules identifies each DNA molecule within the second test sample.

In some embodiments of the methods for simultaneous DNA analysis of the disclosure, the ID region of each adaptor module of the plurality of adaptor modules is selected from a group consisting of between 2 and 10,000 unique nucleotide sequences.

In some embodiments of the methods for simultaneous DNA analysis of the disclosure, the ID region of each adaptor module of the plurality of adaptor modules is selected from a group consisting of between 50 and 500 unique nucleotide sequences.

In some embodiments of the methods for simultaneous DNA analysis of the disclosure, the ID region of each adaptor module of the plurality of adaptor modules is selected from a group consisting of between 100 and 400 unique nucleotide sequences.

In some embodiments of the methods for simultaneous DNA analysis of the disclosure, the ID region of each adaptor module of the plurality of adaptor modules is selected from a group consisting of 60 unique nucleotide sequences.

In some embodiments of the methods for simultaneous DNA analysis of the disclosure, the ID region of each adaptor module of the plurality of adaptor modules is 8 nucleotides in length

In some embodiments of the methods for simultaneous DNA analysis of the disclosure, each adaptor module of the plurality of adaptor modules is selected from a group consisting of between 64 and 2,560,000 unique nucleotide sequences.

In some embodiments of the methods for simultaneous DNA analysis of the disclosure, each adaptor module of the plurality of adaptor modules comprises one of 3840 unique nucleotide sequences, and each nucleotide sequence is discrete from any other sequence of the 3840 unique nucleotide sequences by Hamming distance of at least two.

In some embodiments of the methods for simultaneous DNA analysis of the disclosure, the anchor region of each adaptor module of the plurality of adaptor modules is selected from a group consisting of four nucleotide sequences.

In some embodiments of the methods for simultaneous DNA analysis of the disclosure, the amplification regions of each adaptor module of the plurality of adaptor modules comprise a universal primer sequence; the ID region of each adaptor module of the plurality of adaptor modules comprises or consists of 8 nucleotides; the plurality of adaptor modules is divided into two or more subdivisions, wherein each subdivision of adaptor modules comprises a set of unique ID regions; the polynucleotide sequence of each ID region is discrete from the nucleotide sequence of any other ID regions of the plurality of adaptor modules by Hamming distance of at least two; and each adaptor module of the plurality of adaptor modules comprises a UMI multiplier that is adjacent to or contained within the ID region, wherein the UMI multiplier of each adaptor module of the plurality of adaptor modules comprises or consists of three nucleotides.

In some embodiments of the methods for simultaneous DNA analysis of the disclosure, the method comprises (a) ligating a set of adaptors to DNA fragments of a test sample to generate a plurality of adaptor/DNA fragment complexes; and (b) contacting the plurality of adaptor/DNA fragment complexes with one or more enzymes to form an adaptor-tagged DNA library comprising a plurality of adaptor-tagged DNA fragments. In some embodiments, each adaptor/DNA fragment complex comprises a ligation strand oligonucleotide ligated to each end of the DNA fragment. In some embodiments, the non-ligation strand oligonucleotide is displaced from the adaptor/DNA fragment complex in step (b). In some embodiments, the set of adaptors is any adaptor set of the disclosure.

In some embodiments of the methods for simultaneous DNA analysis of the disclosure, the first process comprises: (a) contacting the first adaptor-tagged library with one or more capture probes under conditions suitable for hybridization to form one or more capture probe/adaptor-tagged DNA complexes, wherein each capture probe comprises: a first region comprising a primer binding site; and a second region capable of hybridizing to a target region in the first genetic locus of the first adaptor-tagged library; (b) isolating the one or more capture probe/adaptor-tagged DNA complexes from step (a), wherein each isolated capture probe/adaptor-tagged DNA complex comprises a capture probe and an adaptor-tagged DNA molecule; and (c) enzymatically processing the one or more isolated capture probe/adaptor-tagged DNA complexes from step (b) to generate one or more adaptor-tagged hybrid nucleic acid molecules (Hybrid Molecules), wherein each Hybrid Molecule comprises: at least a portion of the capture probe or a complement thereof; and at least a portion of the adaptor-tagged DNA molecule or a complement thereof.

In some embodiments of the methods for simultaneous DNA analysis of the disclosure, the first region of the capture probe further comprises a sequence having complementarity to a partner oligonucleotide.

In some embodiments of the methods for simultaneous DNA analysis of the disclosure, the enzymatic processing of one or more isolated capture probe/adaptor-tagged DNA complexes to generate one or more adaptor-tagged Hybrid Molecules comprises performing 5'-3' DNA polymerase extension of the capture probe using the adaptor-tagged DNA molecule in the complex as a template.

In some embodiments of the methods for simultaneous DNA analysis of the disclosure, at least one capture probe hybridizes downstream of the specific region in the target region and at least one capture probe hybridizes upstream of the specific region in the target region.

In some embodiments of the methods for simultaneous DNA analysis of the disclosure, the capture probe comprises a sequencing primer binding site.

In some embodiments of the methods for simultaneous DNA analysis of the disclosure, the Hybrid Molecule comprises at least one Library Tag.

In some embodiments of the methods for simultaneous DNA analysis of the disclosure, the first region of the capture probe comprises a Library Tag.

In some embodiments of the methods for simultaneous DNA analysis of the disclosure, the Library Tag comprises a sequencing primer binding site.

In some embodiments of the methods for simultaneous DNA analysis of the disclosure, the method comprises (a) contacting the first adaptor-tagged library with one or more capture probes under conditions suitable for hybridization to form one or more capture probe/adaptor-tagged DNA complexes, wherein each capture probe comprises: a first region comprising a primer binding site; and a second region capable of hybridizing to a target region in the first genetic locus of the first adaptor-tagged library; (b) isolating the one or more capture probe/adaptor-tagged DNA complexes from step (a), wherein each isolated capture probe/adaptor-tagged DNA complex comprises a capture probe and an adaptor-tagged DNA molecule; (c) enzymatically processing the one or more isolated capture probe/adaptor-tagged DNA complexes from step (b) to generate one or more adaptor-tagged hybrid nucleic acid molecules (Hybrid Molecules), and (d) amplifying the one or more Hybrid Molecules; wherein each Hybrid Molecule comprises: at least a portion of the capture probe or a complement thereof; and at least a portion of the adaptor-tagged DNA molecule or a complement thereof.

In some embodiments of the methods for simultaneous DNA analysis of the disclosure, the amplifying comprises a first primer comprising a 5'oligonucleotide (A1) and a second primer comprising a 3' oligonucleotide (A2), and wherein each of the amplified Hybrid Molecule(s) comprise A1 and A2.

In some embodiments of the methods for simultaneous DNA analysis of the disclosure, the target region comprises a genetic lesion.

In some embodiments of the methods for simultaneous DNA analysis of the disclosure, the target region comprises an epigenetic mark.

In some embodiments of the methods for simultaneous DNA analysis of the disclosure, the capture probe binds to the epigenetic mark.

In some embodiments of the methods for simultaneous DNA analysis of the disclosure, the epigenetic mark comprises a methylation mark.

In some embodiments of the methods for simultaneous DNA analysis of the disclosure, the first process comprises: (a) contacting the first adaptor-tagged library with one or more capture probes under conditions suitable for hybridization to form one or more capture probe/adaptor-tagged DNA complexes, wherein each capture probe comprises: a first region comprising a Library Tag comprising a sequencing primer binding site, and a sequence having complementarity to a partner oligonucleotide; and a second region capable of hybridizing to a target region in the first genetic locus of the first adaptor-tagged library; (b) isolating the one or more capture probe/adaptor-tagged DNA complexes from step (a), wherein each isolated capture probe/adaptor-tagged DNA complex comprises a capture probe and an adaptor-tagged DNA molecule; (c) enzymatically processing the one or more isolated capture probe/adaptor-tagged DNA complexes from step (b) to generate one or more adaptor-tagged hybrid nucleic acid molecules (Hybrid Molecules), wherein the enzymatic processing comprises performing 5'-3' DNA polymerase extension of the capture probe using the adaptor-tagged DNA molecule in the complex as a template; wherein each Hybrid Molecule comprises the capture probe and a complement of the adaptor-tagged DNA molecule that is 3' from where the capture probe hybridized to the target region in the first adaptor-tagged library; and (d) amplifying the one or more Hybrid Molecules in step (c), wherein the amplifying comprises hybridizing a first primer comprising a 5'oligonucleotide (A1) and a second primer comprising a 3' oligonucleotide (A2) to a Hybrid Molecule, and wherein each of the amplified Hybrid Molecule(s) comprise A1 and A2.

In some embodiments of the methods for simultaneous DNA analysis of the disclosure, the second process comprises amplifying or extending the second genetic locus of the second adaptor-tagged library.

In some embodiments of the methods for simultaneous DNA analysis of the disclosure, the amplifying or extending comprises hybridizing a first primer comprising a 5'oligonucleotide (B1) and a second primer comprising a 3' oligonucleotide (B2) to an adaptor-tagged DNA molecule, and wherein the amplified or extended second genetic locus comprises B1 and B2.

In some embodiments of the methods for simultaneous DNA analysis of the disclosure, at least one of B1 and B2 comprises a Library Tag.

In some embodiments of the methods for simultaneous DNA analysis of the disclosure, the second process comprises amplifying or extending the second genetic locus of the second adaptor-tagged library, wherein the amplifying or extending comprises hybridizing a first primer comprising a 5'oligonucleotide (B1) and a second primer comprising a 3' oligonucleotide (B2) to an adaptor-tagged DNA molecule, wherein at least one of B1 and B2 comprises a Library Tag, and wherein the amplified or extended second genetic locus comprises B1 and B2.

In some embodiments of the methods for simultaneous DNA analysis of the disclosure, the method further comprises a step of genetic analysis.

In some embodiments of the methods for simultaneous DNA analysis of the disclosure, the genetic analysis comprises detecting the presence of at least one Library Tag in at least one modified library.

In some embodiments of the methods for simultaneous DNA analysis of the disclosure, the genetic analysis comprises sequencing of the combined library to generate a plurality of sequencing reads and performing bioinformatics analysis on the plurality of sequencing reads.

In some embodiments of the methods for simultaneous DNA analysis of the disclosure, the first modified library comprises a first Library Tag and the second modified library comprises a second Library Tag.

In some embodiments of the methods for simultaneous DNA analysis of the disclosure, the first Library Tag generates a first signal and the second Library Tag generates a second signal.

In some embodiments of the methods for simultaneous DNA analysis of the disclosure, both the first and the second signals are detected or detectable.

In some embodiments of the methods for simultaneous DNA analysis of the disclosure, the first signal and the second signal are not identical.

In some embodiments of the methods for simultaneous DNA analysis of the disclosure, the first signal is detected or detectable and the second signal is not detected or detectable.

In some embodiments of the methods for simultaneous DNA analysis of the disclosure, the second signal is detected or detectable and the first signal is not detected or detectable.

In some embodiments of the methods for simultaneous DNA analysis of the disclosure, only one of the first modified library and the second modified library comprises a Library Tag.

In some embodiments of the methods for simultaneous DNA analysis of the disclosure, the Library Tag generates a first signal and the lack of the Library Tag generates a second signal.

In some embodiments of the methods for simultaneous DNA analysis of the disclosure, both the first and the second signals are detected or detectable.

In some embodiments of the methods for simultaneous DNA analysis of the disclosure, the first signal and the second signal are not identical.

In some embodiments of the methods for simultaneous DNA analysis of the disclosure, the first signal is detected or detectable and the second signal is not detected or detectable.

In some embodiments of the methods for simultaneous DNA analysis of the disclosure, the second signal is detected or detectable and the first signal is not detected or detectable.

In some embodiments of the methods for simultaneous DNA analysis of the disclosure, the first signal is a sequencing read and the second signal is an empty sequencing read.

In some embodiments of the methods for simultaneous DNA analysis of the disclosure, the empty sequencing read (the second signal) is detected as a series of two or more G nucleotides.

In some embodiments of the methods for simultaneous DNA analysis of the disclosure, the detected first signal and the detected second signal can be differentiated from one another.

In some embodiments of the methods for simultaneous DNA analysis of the disclosure, the detected first signal and the non-detected second signal can be differentiated from one another.

In some embodiments of the methods for simultaneous DNA analysis of the disclosure, the non-detected first signal and the detected second signal can be differentiated from one another.

In some embodiments of the methods for simultaneous DNA analysis of the disclosure, the genetic analysis is used to identify one or more genetic lesions.

In some embodiments of the methods for simultaneous DNA analysis of the disclosure, the one or more genetic lesions comprise a nucleotide transition, a nucleotide transversion, a nucleotide insertion, a nucleotide deletion, a genomic rearrangement, or a change in copy number.

In some embodiments of the methods for simultaneous DNA analysis of the disclosure, the genetic analysis is used to detect one or more genetic lesions that cause or are associated with a genetic disease.

In some embodiments of the methods for simultaneous DNA analysis of the disclosure, the genetic disease is cancer.

In some embodiments of the methods for simultaneous DNA analysis of the disclosure, the one or more genetic lesions are chromosomal rearrangements.

In some embodiments of the methods for simultaneous DNA analysis of the disclosure, the first modified library and second modified library are demultiplexed after sequencing by identification of at least one Library Tag or a portion thereof.

Some embodiments of the disclosure provide a composition comprising a first modified library and a second modified library, wherein the first modified library comprises: a first DNA molecule comprising, from 5' to 3', a 5' oligonucleotide (A1), a first 5' adaptor module, a sequence of the first genetic locus, and a 3' oligonucleotide (A2); or a first DNA molecule comprising, from 5' to 3', a 5' oligonucleotide (A1), a sequence of the first genetic locus, a first 3' adaptor module, and a 3' oligonucleotide (A2); or a first DNA molecule comprising, from 5' to 3', a 5' oligonucleotide (A1), a first 5' adaptor module, a sequence of the first genetic locus, a first 3' adaptor module, and a 3' oligonucleotide (A2); wherein the first genetic locus comprises a DNA region; wherein a first test sample comprises a DNA molecule having a sequence identical to a sequence of the first genetic locus or a complementary sequence thereof; and wherein the first 5' adaptor module, the first 3' adaptor module, or both, are coupled to the first genetic locus in the first adaptor-tagged library; wherein the second modified library comprises: a second DNA molecule comprising, from 5' to 3', a 5' oligonucleotide (B1), a second 5' adaptor module, a sequence of the second genetic locus, and a 3' oligonucleotide (B2); or a second DNA molecule comprising, from 5' to 3', a 5' oligonucleotide (B1), a sequence of the second genetic locus, a second 3' adaptor module and a 3' oligonucleotide (B2); or a second DNA molecule comprising, from 5' to 3', a 5' oligonucleotide (B1), a second 5' adaptor module, a sequence of the second genetic locus, a second 3' adaptor module and a 3' oligonucleotide (B2); wherein the second genetic locus comprises a DNA region; wherein a second test sample comprises a DNA molecule having a sequence identical to a sequence of the second genetic locus or a complementary sequence thereof; and wherein the second 5' adaptor module, the second 3' adaptor module, or both, are coupled to the second genetic locus in the second adaptor-tagged library; wherein either the first modified library or the second modified library comprises a Library Tag.

Some embodiments of the disclosure provide a composition comprising a first modified library and a second modified library, wherein the first modified library comprises: a first DNA molecule comprising, from 5' to 3', a 5' oligonucleotide (A1), a first 5' adaptor module, a sequence of the first genetic locus, and a 3' oligonucleotide (A2); or a first DNA molecule comprising, from 5' to 3', a 5' oligonucleotide (A1), a sequence of the first genetic locus, a first 3' adaptor module, and a 3' oligonucleotide (A2); or a first DNA molecule comprising, from 5' to 3', a 5' oligonucleotide (A1), a first 5' adaptor module, a sequence of the first genetic locus, a first 3' adaptor module, and a 3' oligonucleotide (A2); wherein the first genetic locus comprises a DNA region; wherein a first test sample comprises a DNA molecule having a sequence identical to a sequence of the first genetic locus or a complementary sequence thereof; and wherein the first 5' adaptor module, the first 3' adaptor module, or both, are coupled to the first genetic locus in the first adaptor-tagged library; wherein the second modified library comprises: a second DNA molecule comprising, from 5' to 3', a 5' oligonucleotide (B1), a second 5' adaptor module, a sequence of the second genetic locus, and a 3' oligonucleotide (B2); or a second DNA molecule comprising, from 5' to 3', a 5' oligonucleotide (B1), a sequence of the second genetic locus, a second 3' adaptor module and a 3' oligonucleotide (B2); or a second DNA molecule comprising, from 5' to 3', a 5' oligonucleotide (B1), a second 5' adaptor module, a sequence of the second genetic locus, a second 3' adaptor module and a 3' oligonucleotide (B2); wherein the second genetic locus comprises a DNA region; wherein a second test sample comprises a DNA molecule having a sequence identical to a sequence of the second genetic locus or a complementary sequence thereof; and wherein the second 5' adaptor module, the second 3' adaptor module, or both, are coupled to the second genetic locus in the second adaptor-tagged library; wherein the first modified library comprises a first Library Tag and the second modified library comprises a second Library Tag, wherein the first and second Library Tags are not identical.

In some embodiments of the compositions of the disclosure, the first test sample and the second test sample are the same sample.

In some embodiments of the compositions of the disclosure, the first test sample and the second test sample are not the same sample.

In some embodiments of the compositions of the disclosure, the first genetic locus and the second genetic locus are identical.

In some embodiments of the compositions of the disclosure, the first genetic locus and the second genetic locus are not identical.

In some embodiments of the compositions of the disclosure, the Library Tag distinguishes the first DNA sequence of the first modified library from the second DNA sequence of the second modified library.

In some embodiments of the compositions of the disclosure, at least one of A1, A2, B1, and B2 contains at least one Library Tag.

In some embodiments of the compositions of the disclosure, the Library Tag comprises a nucleic acid sequence or an amino acid sequence.

In some embodiments of the compositions of the disclosure, the Library Tag comprises one or more of a DNA, an RNA, a PNA, or a non-naturally occurring nucleic acid, a synthetic nucleic acid, a modified nucleic acid, a non-naturally occurring amino acid, a synthetic amino acid, and a modified amino acid.

In some embodiments of the compositions of the disclosure, the Library Tag comprises a detectable label.

In some embodiments of the compositions of the disclosure, the detectable label comprises one or more of a fluorescent moiety, a magnetic or paramagnetic moiety, an enzymatic moiety, a binding moiety, an epitope, and a radioactive moiety.

In some embodiments of the compositions of the disclosure, the Library Tag selectively or specifically binds to a detectable moiety.

In some embodiments of the compositions of the disclosure, the detectable moiety comprises one or more of a fluorescent moiety, a magnetic or paramagnetic moiety, an enzymatic moiety, a binding moiety, an epitope, and a radioactive moiety.

In some embodiments of the compositions of the disclosure, the Library Tag comprises a unique polynucleotide sequence.

In some embodiments of the compositions of the disclosure, the unique polynucleotide sequence comprises a sequence having 70% identity, or less, to a sequence selected from the list consisting of A1, B1, A2, B2, the first 5' adaptor module, the first genetic locus, the first 3' adaptor module, the second 5' adaptor module, the second genetic locus, and the second 3' adaptor module.

In some embodiments of the compositions of the disclosure, the first test sample and the second test sample are not the same sample; A1, A2, B1, or B2 contains a Library Tag; the Library Tag is capable of distinguishing the first DNA sequence of the first modified library from the second DNA sequence of the second modified library; the Library Tag comprises a unique polynucleotide sequence; and the unique polynucleotide sequence comprises a sequence having 70% identity, or less, to a sequence selected from the list consisting of A1, B1, A2, B2, the first 5' adaptor module, the first genetic locus, the first 3' adaptor module, the second 5' adaptor module, the second genetic locus, and the second 3' adaptor module.

In some embodiments of the compositions of the disclosure, each adaptor of the set of adaptors comprises a ligation strand oligonucleotide and a non-ligation strand oligonucleotide.

In some embodiments of the compositions of the disclosure, the non-ligation strand oligonucleotide is capable of hybridizing to a region at the 3' end of the ligation strand oligonucleotide and forming a duplex therewith.

In some embodiments of the compositions of the disclosure, the ligation strand oligonucleotide comprises an adaptor module.

In some embodiments of the compositions of the disclosure, the ligation strand oligonucleotide comprises a dT, dA, dC, or dG overhang at the 3' terminus.

In some embodiments of the compositions of the disclosure, the non-ligation strand oligonucleotide comprises a modification at its 3' terminus that prevents ligation to the 5' end of a dsDNA fragment and/or adaptor dimer formation, wherein the non-ligation strand is configured to be displaced from the duplex.

In some embodiments of the compositions of the disclosure, each adaptor of the set of adaptors comprises an ID region selected from a pool of unique ID regions, wherein the pool is selected from a plurality of pools, and wherein the selected pool is unique to the test sample.

In some embodiments of the compositions of the disclosure, the adaptor module comprises (a) an amplification region comprising a primer binding site; (b) an ID region; and (c) an anchor region.

In some embodiments of the compositions of the disclosure, the adaptor module comprises: (a) an amplification region comprising a polynucleotide sequence comprising a primer binding site; (b) an ID region; and (c) an anchor region.

In some embodiments of the compositions of the disclosure, the amplification region comprises a primer binding site, wherein the primer binding site allows for amplification using PCR (polymerase chain reaction), LAMP (loop-mediated isothermal amplification), NASBA (nucleic acid sequence-based amplification), SDA (standard displacement amplification), RCA (rolling circle replication), or LCR (ligase chain reaction).

In some embodiments of the compositions of the disclosure, the amplification region comprises a polynucleotide sequence comprising a primer binding site, wherein the primer binding site allows for amplification of the nucleic acids of the first and/or second adaptor-tagged library using for PCR (polymerase chain reaction), LAMP (loop-mediated isothermal amplification), NASBA (nucleic acid sequence-based amplification), SDA (standard displacement amplification), RCA (rolling circle replication), or LCR (ligase chain reaction).

In some embodiments of the compositions of the disclosure, the amplification region comprises or consists of between 10 and 50 nucleotides. In some embodiments, the amplification region comprises or consists of between 20 and 30 nucleotides. In some embodiments, the amplification region comprises or consists of 25 nucleotides.

In some embodiments of the compositions of the disclosure, the amplification region comprises or consists of about 10 to about 50 nucleotides. In some embodiments, the amplification region comprises or consists of about 20 to about 30 nucleotides. In some embodiments, the amplification region comprises or consists of about 25 nucleotides.

In some embodiments of the compositions of the disclosure, the anchor region comprises an overhang at the 3' terminus.

In some embodiments of the compositions of the disclosure, the anchor region comprises or consists of between 1 and 50 nucleotides. In some embodiments, the anchor region comprises or consists of between 5 and 25 nucleotides. In some embodiments, the anchor region comprises or consists of 10 nucleotides.

In some embodiments of the compositions of the disclosure, the anchor region comprises or consists of about 1 to about 50 nucleotides. In some embodiments, the anchor region comprises or consists of about 5 to about 25 nucleotides. In some embodiments, the anchor region comprises or consists of about 10 nucleotides.

In some embodiments of the compositions of the disclosure, the ID region comprises or consists of between 3 and 50 nucleotides. In some embodiments, the ID region comprises or consists of between 3 and 15 nucleotides. In some embodiments, the ID region comprises or consists of 8 nucleotides.

In some embodiments of the compositions of the disclosure, the ID region comprises or consists of about 3 to about 50 nucleotides. In some embodiments, the ID region comprises or consists of about 3 to about 15 nucleotides. In some embodiments, the ID region comprises or consists of about 8 nucleotides.

In some embodiments of the compositions of the disclosure, the adaptor module further comprises a unique molecule identifier (UMI) multiplier.

In some embodiments of the compositions of the disclosure, the UMI multiplier is adjacent to or contained within the ID region.

In some embodiments of the compositions of the disclosure, the UMI multiplier comprises or consists of between 1 and 5 nucleotides.

In some embodiments of the compositions of the disclosure, the UMI multiplier comprises or consists of about 1 to about 5 nucleotides.

In some embodiments of the compositions of the disclosure, the UMI multiplier is 3 nucleotides in length, and comprises a nucleic acid sequence selected from a group of 64 unique nucleotide sequences.

In some embodiments of the compositions of the disclosure, the pool of ID regions comprises between 2 and 10,000 unique ID region sequences. In some embodiments, the pool of ID regions comprises between 10 and 500 unique ID region sequences. In some embodiments, the pool of ID regions comprises between 50 and 300 unique ID region sequences. In some embodiments, the pool of ID regions comprises 60 unique ID region sequences.

In some embodiments of the compositions of the disclosure, the pool of ID regions comprises about 2 to about 10,000 unique ID region sequences. In some embodiments, the pool of ID regions comprises about 10 to about 500 unique ID region sequences. In some embodiments, the pool of ID regions comprises about 50 to about 300 unique ID region sequences. In some embodiments, the pool of ID regions comprises about 60 unique ID region sequences.

In some embodiments of the compositions of the disclosure, each ID region of the pool of ID regions is 8 nucleotides in length.

In some embodiments of the compositions of the disclosure, each ID region sequence is discrete from any other ID region sequence by Hamming distance of at least two.

In some embodiments of the compositions of the disclosure, each ID region is configured to identify the DNA fragment attached thereto.

In some embodiments of the compositions of the disclosure, each adaptor module of the set of adaptor modules is selected from a group consisting of between 64 and 2,560,000 unique nucleotide sequences.

In some embodiments of the compositions of the disclosure, each adaptor module of the set of adaptor modules is selected from a group consisting of about 64 to about 2,560,000 unique nucleotide sequences.

In some embodiments of the compositions of the disclosure, each adaptor module of the set of adaptor modules comprises a unique nucleotide sequence selected from 3840 unique nucleotide sequences, wherein each sequence of the 3840 unique nucleotide sequences is discrete from any other sequence by Hamming distance of at least two.

In some embodiments of the compositions of the disclosure, the anchor region of each adaptor of the set of adaptors comprises one of four nucleotide sequences, and wherein each ID region of a given sequence is paired to only one of the four anchor regions of a given sequence.

In some embodiments of the compositions of the disclosure, the amplification region of each adaptor of the set of adaptors comprises an identical primer binding site.

In some embodiments of the compositions of the disclosure, each ID region of the pool of ID regions is 8 nucleotides in length, each ID region sequence is discrete from any other ID region sequence by Hamming distance of at least two; each adaptor of the set of adaptors comprises a UMI multiplier that is adjacent to or contained within the ID region, wherein the UMI multiplier of each adaptor of the set of adaptors is three nucleotides in length, and wherein the UMI multiplier of a given sequence is paired to one ID region of a given sequence; the anchor region of each adaptor of the set of adaptors comprises one of four nucleotide sequences, and wherein each ID region of a given sequence is paired to only one of the four anchor regions of a given sequence; and the amplification region of each adaptor of the set of adaptors comprises an identical primer binding site.

In some embodiments of the compositions of the disclosure, a plurality of adaptor modules comprises the adaptor module.

In some embodiments of the compositions of the disclosure, each adaptor module of the plurality of adaptor modules is unique.

In some embodiments of the compositions of the disclosure, wherein the amplification region of each adaptor module of the plurality of adaptor modules comprises a custom primer binding site.

In some embodiments of the compositions of the disclosure, the custom primer binding site comprises a sequence having 100% sequence identity to a sequence of the custom primer binding site of each adaptor module of the plurality of adaptor modules.

In some embodiments of the compositions of the disclosure, the plurality of adaptor modules comprises a first subdivision and a second subdivision.

In some embodiments of the compositions of the disclosure, each adaptor module comprises an ID region comprising a polynucleotide sequence that is distinct from the polynucleotide sequence of the ID region of any other adaptor module.

In some embodiments of the compositions of the disclosure, the first subdivision comprises a first set of unique ID regions and the second subdivision comprises a second set of unique ID regions.

In some embodiments of the compositions of the disclosure, each adaptor module further comprises a sample tag comprising a polynucleotide sequence that is not identical to the polynucleotide sequence of the ID region.

In some embodiments of the compositions of the disclosure, each adaptor module in the first subdivision comprises a sample tag comprising a first polynucleotide sequence and each adaptor module in the second subdivision comprises a sample tag comprising a second polynucleotide sequence; wherein the first and second polynucleotide sequences are not identical to each other.

In some embodiments of the compositions of the disclosure, the first subdivision of adaptor modules identifies each DNA molecule within the first test sample and the second subdivision of adaptor modules identifies each DNA molecule within the second test sample.

In some embodiments of the compositions of the disclosure, the first subdivision of adaptor modules identifies the first test sample and the second subdivision of adaptor modules identifies the second test sample.

In some embodiments of the compositions of the disclosure, the ID region of each adaptor module of the plurality of adaptor modules is selected from a group consisting of between 2 and 10,000 unique nucleotide sequences.

In some embodiments of the compositions of the disclosure, the ID region of each adaptor module of the plurality of adaptor modules is selected from a group consisting of between 50 and 500 unique nucleotide sequences.

In some embodiments of the compositions of the disclosure, the ID region of each adaptor module of the plurality of adaptor modules is selected from a group consisting of between 100 and 400 unique nucleotide sequences.

In some embodiments of the compositions of the disclosure, the ID region of each adaptor module of the plurality of adaptor modules is selected from a group consisting of 60 unique nucleotide sequences.

In some embodiments of the compositions of the disclosure, the ID region of each adaptor module of the plurality of adaptor modules comprises or consists of 8 nucleotides.

In some embodiments of the compositions of the disclosure, each adaptor module of the plurality of adaptor modules is selected from a group consisting of between 64 and 2,560,000 unique nucleotide sequences.

In some embodiments of the compositions of the disclosure, each adaptor module of the plurality of adaptor modules comprises a unique nucleotide sequence selected from among 3840 unique nucleotide sequences, and each nucleotide sequence is discrete from any other sequence of the 3840 unique nucleotide sequences by Hamming distance of at least two.

In some embodiments of the compositions of the disclosure, the anchor region of each adaptor module of the plurality of adaptor modules is selected from a group consisting of four nucleotide sequences.

In some embodiments of the compositions of the disclosure, the amplification regions of each adaptor module of the plurality of adaptor modules comprise a universal primer sequence; the ID region of each adaptor module of the plurality of adaptor modules comprises or consists of 8 nucleotides; the plurality of adaptor modules is divided into two or more subdivisions, wherein each subdivision of adaptor modules comprises a set of unique ID regions, wherein the polynucleotide sequence of each ID region is discrete from the nucleotide sequence of any other ID regions of the plurality of adaptor modules by Hamming distance of at least two; and each adaptor module of the plurality of adaptor modules comprises a UMI multiplier that is adjacent to or contained within the ID region, wherein the UMI multiplier of each adaptor module of the plurality of adaptor modules comprises or consists of three nucleotides.

In some embodiments of the compositions of the disclosure, the test sample is a tissue biopsy.

In some embodiments of the compositions of the disclosure, the tissue biopsy is obtained from a tumor or a tissue suspected of being a tumor.

In some embodiments of the compositions of the disclosure, the tissue biopsy is obtained from a malignant tumor or tumor suspected of being a malignant tumor.

In some embodiments of the compositions of the disclosure, the DNA molecule is cell free DNA (cfDNA), genomic DNA (gDNA), complementary DNA (cDNA), mitochondrial DNA, methylated DNA, or demethylated DNA.

In some embodiments of the compositions of the disclosure, the DNA molecule comprises an epigenetic mark.

In some embodiments of the compositions of the disclosure, the DNA molecule is obtained from a library selected from the list consisting of a whole genome library, an amplicon library, a whole exome library, a cDNA library, or a methylated DNA library.

In some embodiments of the compositions of the disclosure, the DNA molecule is isolated or generated from the test sample.

In some embodiments of the compositions of the disclosure, the test sample comprises a biological sample selected from the group consisting of an amniotic fluid sample, a blood sample, a skin sample, a hair sample, a hair follicle sample, a saliva sample, a mucous sample, a sweat sample, a tear sample, an epithelial tissue sample, a urine sample, a semen sample, a seminal plasma sample, a serum sample, a prostatic fluid sample, a pre-ejaculatory fluid (Cowper's fluid) sample, an ocular fluid sample, an excreta sample, a biopsy sample, an ascites sample, a cerebrospinal fluid sample, a lymph sample, a tissue extract sample, a stool sample, and a formalin-fixed, paraffin embedded (FFPE) sample.

In some embodiments of the compositions of the disclosure, the DNA molecule is obtained by the steps comprising (a) isolating cellular DNA from the test sample; or (b) fragmenting the cellular DNA to obtain the genomic DNA fragment.

In some embodiments of the compositions of the disclosure, step (b) fragmenting the cellular DNA is performed by contacting the cellular DNA with at least one digestion enzyme.

In some embodiments of the compositions of the disclosure, step (b) fragmenting the cellular DNA is performed by applying mechanical stress to the cellular DNA.

In some embodiments of the compositions of the disclosure, step (b) fragmenting the cellular DNA is performed by contacting the cellular DNA with one or more compounds to chemically disrupt one or more bonds of the cellular DNA.

### BRIEF DESCRIPTION OF THE DRAWINGS

Various objects and advantages and a more complete understanding of the disclosure are apparent and more readily appreciated by reference to the following Detailed Description and to the appended claims when taken in conjunction with the accompanying Drawings, wherein:
FIGURE 1 is a schematic diagram depicting 3 different types of adaptor modules.
FIGURE 2 is a schematic diagram depicting an illustrative process for matching anchor regions to ID regions of adaptors (adaptor elements are illustrative and length is not to-scale), as well as the generation of adaptor sets assigned to different samples. In this embodiment, black boxes represent identical amplification regions, and patterned boxes represent distinct ID regions, with each pattern representing a different ID region. Grey boxes represent anchor regions, with each type of anchor region sequence represented by a different shade of grey.
FIGURE 3 is a schematic diagram depicting one embodiment of the methods of the disclosure. Patterns indicate compositions of a library, wherein the same pattern indicates an identical composition (e.g., the 1^{st} adaptor-tagged library and the 2^{nd} adaptor-tagged library), and two patterns indicate a mixture of different compositions (e.g., the Combined Library).
FIGURE 4 is a schematic diagram depicting an illustrative workflow of some embodiments of the methods of the disclosure. In this embodiment, an amplified Target Capture Library (TCLA) and an LPWG library are separately generated then combined for sequencing.

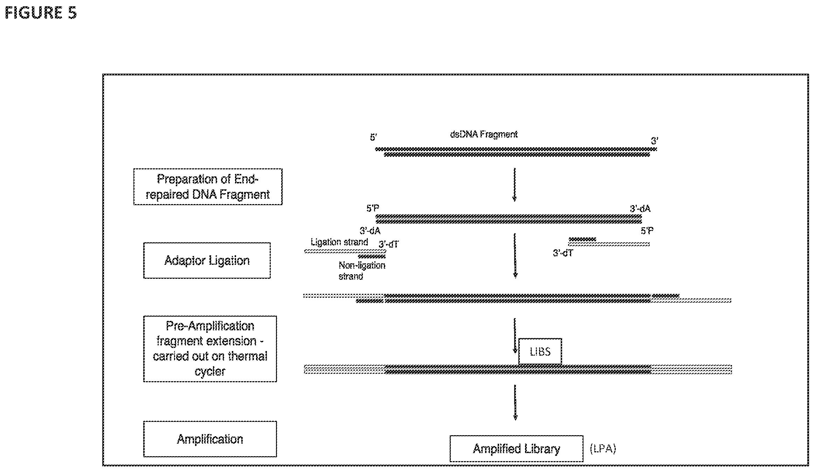

FIGURE 5 is a schematic diagram depicting an illustrative process for generating an adaptor-tagged Parent Library. In some embodiments, the Parent Library is generating in the following order: in a first step, dsDNA fragments are end-repaired, providing fragments with 5' phosphate groups and 3'-dA(deoxyribonucleic acid adenine) tails at both strand ends; in a second step, the ligation strand of adaptors are ligated to the 5' ends of the fragments, tagging the fragments with ID regions and PCR primer binding sites; in a third step, the tagged DNA is filled-in (*i.e.*, extended) and the non-ligation strand is dissociated from the adaptor, generating an adaptor-tagged Parent Library comprising adaptor-tagged DNA fragments. In some embodiments, the adaptor-tagged Parent Library and/or the collection of adaptor-tagged DNA fragments is referred as the LIB or LIBS. In some embodiments, in a fourth step, the extended adaptor-tagged DNA fragments are amplified by PCR to generate an amplified library. In some embodiments, the amplified library is referred to as the Library Post-Amplification (LPA). In some embodiments, the third and fourth step may be performed continuously (*i.e.*, without adding additional reagents) in a thermal cycler without adding additional reagents to the reaction mixture.

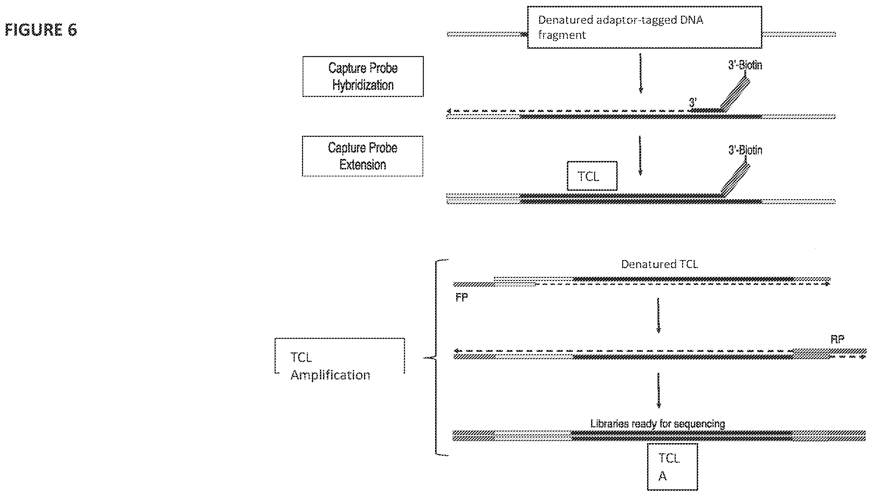

FIGURE 6 is a schematic diagram depicting an illustrative process for generating a Target Capture Library (TCL). In some embodiments, in a first step, a capture probe module is hybridized to a denatured adaptor-tagged DNA fragment (*e.g*., a LIBS fragment or an LPA fragment). The capture probe module comprises a tail sequence (green) and a capture probe sequence (dark blue). In some embodiments, the tail sequence is hybridized to a partner oligonucleotide (grey) comprising a biotin label at the 3' end of the partner oligonucleotide. In some embodiments, in a second step, the capture probe module is extended at its 3' end using the adaptor-tagged DNA fragment as template, generating a Target Capture Library (TCL). In some embodiments, in a third step, the TCL is amplified using primers FP and RP, where FP binds to at least a portion of the amplification region of the adaptor and RP binds to at least a portion of the tail sequence of the capture probe module. In some embodiments, FP comprises a binding site (red) for a first sequencing primer and RP comprises a binding site (green) for a second sequencing primer. In some embodiments, a Library Tag is embedded in the tail region of the capture probe (green). The resulting amplified library is referred to as the amplified Target Capture Library (TCLA) and is ready for sequencing.

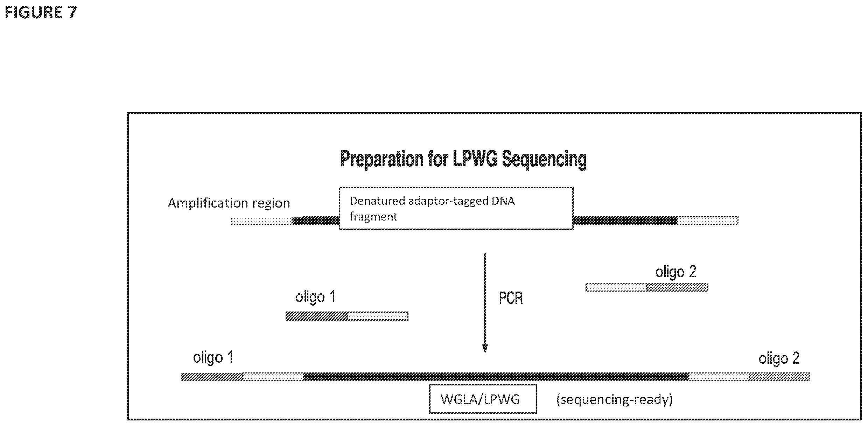

FIGURE 7 is a schematic diagram depicting an illustrative embodiment of a method for the generation of a Low-pass Whole Genome (LPWG) library. In some embodiments, the LPWG is referred to as the Amplified Whole Genome Library or Whole Genome Library Amplified (WGLA). In some embodiments, adaptor-tagged DNA fragments of the adaptor-tagged Parent Library, the amplified adaptor-tagged Parent Library, the LIBS, or the LPA are amplified with primers comprising sequencing adaptors (*e.g*., oligo 1 (red) and oligo 2 (blue)) and an amplification region binding region (yellow). In some embodiments, the amplification region binding regions is referred to as the ACA sequence. In some embodiments, oligo 1 and oligo 2 may be Illumina^{®} P5 and P7 sequences; amplification with these primers produces a library of DNA fragments with oligo 1 and oligo 2 sequences at both ends, ready for sequencing. In some embodiments, the LPWG library (WGLA) does not possess a Read 2 sequencing primer binding domain. In some embodiments, the WGLA does not posses a Library Tag.
FIGURE 8 is a graph showing library concentrations after 8, 12, or 16 cycles of LPWG amplification with either LIB or LPA input.
FIGURE 9 is a graph showing concentrations of LPWG libraries measured by qPCR or by a standard assay used to determine concentration of DNA (Assay), amplified for 8, 12, or 16 cycles with annealing temperature of either 58°C or 69°C.
FIGURE 10 is a graph showing library concentrations measured by Assay, by qPCR with oligo 1 and/or oligo 2 primers, or by qPCR with Primer Mix 1S ("ACA") after 4, 6, 8, 12, 14, or 16 cycles. Error bars represent standard deviation.
FIGURE 11 is a graph showing the ratio of measured concentration by Assay to measured concentration by qPCR with oligo 1/oligo 2 primers after 8, 12, 14, or 18 cycles of amplification.
FIGURE 12A is a graph showing the overlay of Bioanalzyer traces of LPWG libraries amplified for 4 (blue), 6 (green), or 8 (red) cycles.
FIGURE 12B is a graph showing the overlay of Bioanalzyer traces of LPWG libraries amplified for 8 (blue), 12 (green), 14 (turquoise), or 16 (red) cycles.
FIGURE 13 is a graph showing overlay of Bioanalzyer traces of LPWG libraries amplified from 145 bp size-selected fragment for 0 (blue), 6 (green), 8 (turquoise), 12 (pink), or 16 (red) cycles.
FIGURE 14 is a graph showing concentration of LPWG libraries amplified from 5, 10, 25, 50, 75, or 100 ng wild type LPA for 8 cycles, measured by either Assay or by qPCR.
FIGURE 15 is a graph showing concentration of LPWG libraries amplified from 10, 20, 50, 100, or 200 ng wild type LPA for 16 cycles, measured by either Assay or by qPCR.
FIGURE 16 is a graph showing the number of sequencing reads for LPWG libraries amplified from 10, 20, 50, 100, or 200 ng wild type LPA for 16 cycles, normalized before loading onto the flow cell. (Sequencing run number 16)
FIGURE 17 is a graph showing the average number of reads from 2 replicate samples with varying inputs into the LPWG PCR. Error bars denote standard deviation between the replicates.
FIGURE 18 is a graph showing the concentrations of LPWG libraries amplified for 16 cycles in either 0.5x (50 µL), 1x (100 µL) or 2x (200 µL) reaction volumes.
FIGURE 19 is a graph showing the overlay of Bioanalzyer traces of LPWG libraries amplified in 0.5x, 1x, or 2x reaction volumes.
FIGURE 20 is a graph showing concentrations of LPWG libraries amplified for 16 cycles in either 0.5x (0.5 µM), 1x (1 µM) or 2x (2 µM) primers.
FIGURE 21 is a graph showing the overlay of Bioanalzyer traces of LPWG libraries amplified with 0.5x, 1x, or 2x primer concentrations.
FIGURE 22 is a graph showing concentrations of LPWG libraries amplified for 8, 12, 14, or 16 cycles with Enzyme 1 or Enzyme 2.
FIGURE 23 is a graph showing the overlay of Bioanalzyer traces of LPWG libraries amplified with Enzyme 1.
FIGURE 24 is a graph showing the distribution of co-loaded LPWG and Target Capture Library Amplified (TCLA).
FIGURE 25 is a graph showing the average depth for two capture libraries co-loaded with LPWG libraries. Target coverage and number of LPWG libraries are described along the x-axis.
FIGURE 26 is a graph showing the coverage of 16 clinical and wild type samples.
FIGURE 27 is a graph showing the calculated coverage for wild type LPWG libraries, co-loaded with target capture libraries. The diagonal stripes boxplots show libraries loaded to a target of 0.5x coverage while the vertical stripes boxplot shows libraries targeted to 1x coverage.
FIGURE 28 is a schematic diagram depicting an illustrative embodiment of demultiplexing (*i.e.*, differentiating between) the TCLA and the WGLA library using the Read 2 sequence. In some embodiments, the Library Tag comprises or consists of the Read 2 sequence. The FC sequence represents a sequence capable of binding to a flow cell of a sequencing machine. In this figure, Read 1 and Read 2 sequences that bind to the DNA molecules to be sequenced represent sequencing primers; the barcode sequence represents at least a portion of the adaptor module comprising a unique sequence.
FIGURE 29 is a diagram showing variant calls to TCLA libraries with or without the addition of LPWG Libraries.

### DETAILED DESCRIPTION

Some embodiments of the disclosure provide compositions and methods that combine the benefits of different genetic analysis approaches by performing simultaneous genetic analysis on a single pool of different libraries, where each of the different libraries is specific to a different genetic analysis approach. Genetic analysis approaches that utilize next-generation sequencing (NGS) methods currently used in the art include, but are not limited to, targeted sequencing of target-enriched libraries, whole genome sequencing of whole-genome libraries, and epigenetic analysis of chemically transformed libraries. Genetic analysis approaches are generally chosen based on several factors, including the final application (*e.g*., clinical vs research), the required quality of results, technical efficiency, and cost, while also taking into consideration the benefits and disadvantages of each approach. For example, in some embodiments, a targeted genetic analysis approach focuses on pre-determined DNA target regions, therefore it may not identify additional DNA abnormalities in other regions (non-target regions) of the genome. Conversely, in some embodiments, LPWG sequencing provides coverage for the entire genome and may be performed at lower sequencing depth but may result in less sensitivity for specific DNA regions of interest.

Some embodiments of the disclosure provide compositions and methods that may be used in combining libraries specific to different genetic analysis approaches into a single Sequence-Ready Library (SRL), where the different libraries within the SRL may be analyzed simultaneously in a single sequencing analysis experiment (e.g., on a single flow cell of a sequencing machine). In some embodiments of the disclosure, this approach increases sequencing efficiency, expands sequencing coverage, and maintains necessary sensitivity. In some embodiments, targeted genetic analysis at specific loci may be performed by deep sequencing to detect low frequency genetic variants, and simultaneous whole-genome sequencing may provide more accurate assessment of copy number variations.

The compositions and methods of the disclosure provide numerous benefits, some of which are described below:

Reduced bias: In some embodiments, an adaptor-tagged Parent Library or a Library Post-Amplification (LPA) generated from a test sample is split into two parts-a first adaptor-tagged library and a second adaptor-tagged library-prior to each adaptor-tagged library undergoing a different modification process. Upon performing the respective modification processes, the first adaptor-tagged library may generate a first modified library, and the second adaptor-tagged library may generate a second modified library. By generating the first and second modified libraries from the same adaptor-tagged Parent Library or the same LPA, the first and second modified DNA libraries will have an identical starting point (*i.e.*, a common parent library) prior to their respective modification process. In contrast, two modified libraries generated in parallel without first generating a common parent library will not have an identical starting point, because each modified library is subject to bias introduced during adaptor-ligation prior to their respective modification process. Therefore, generating a first and a second modified library from a common parent library reduces bias that may otherwise have been introduced by generating these libraries in separate processes (*e.g*., from different samples) or parallel processes(*e.g*., from the same sample but different intermediate libraries). This reduction in bias may significantly reduce noise in the resultant data from downstream genetic analysis and increase sensitivity.

Comprehensive genetic profiling: In some embodiments of the compositions and methods described herein, the first modified library is a Target Capture Library (TCL) or amplified Target Capture Library (TCLA) and the second modified library is a Whole-Genome Library (WGL) or Whole-Genome Library Amplified (WGLA). WGLA and low-pass whole-genome (LPWG) library may be used interchangeably in the disclosure. Targeted genetic analysis on the TCL or TCLA may provide higher specificity and sensitivity for targeted regions, and it may be less robust in identifying larger genomic variations that may span a section of the chromosome. By combining the TCL or TCLA and the WGL or WGLA into a single combined library, both targeted genetic analysis and broad-coverage whole genome analysis may be performed on the results of one sequencing run.

Improved quantification of sample content: In some embodiments, the frequency of one or more genetic lesions associated with cancer is determined using methods and compositions of the disclosure. In such embodiments, somatic mutations that reside within the tumor and not within the germline of the individual may be used to quantify the amount of circulating DNA that is shed from the tumor. The combination of, for example, a TCLA and a WGLA library will promote the discovery of mutations originating from the tumor while retaining the ability to interrogate focused areas of interest. The discovery of new mutations, regardless of their biological significance, also improves quantification of tumor content in admixed cfDNA.

Reduced cost: In some embodiments, genetic analysis performed using compositions and methods of the disclosure may reduce cost. For example, genetic analysis may be done by sequencing, which may be expensive for each individual sequencing run. By combining multiple libraries (*e.g*., a Target Capture library and a LPWG library) into a single library and performing sequencing on this single library, costs may be lower due to the use of fewer reagents, lower facility costs, as well as reduced personnel time.

The present invention is described more fully hereinafter using illustrative, non-limiting embodiments, and references to the accompanying figures. This invention may, however, be embodied in many different forms and should not be construed as to be limited to the embodiments set forth below. Rather, these embodiments are provided so that this disclosure is thorough and conveys the scope of the invention to those skilled in the art.

### Compositions

Some embodiments of the disclosure provide kits that may be used to generate DNA libraries for genetic analysis, for example, target-captured libraries for use in targeted genetic analysis. Some embodiments of the disclosure provide kits that may be used to generate different libraries that may be combined into an SRL, for example, a combined library comprising a target-captured library and a whole-genome library. In some embodiments, an SRL generated by a kit of the disclosure may be used in a single sequencing analysis experiment. In some embodiments, a kit of the disclosure may be used in both targeted genetic analysis and whole-genome analysis in a single sequencing analysis experiment, where the targeted genetic analysis at specific loci detects low frequency genetic variants and the simultaneous whole-genome sequencing provides an assessment of copy number variations. In some embodiments, kits of the disclosure may increase sequencing efficiency, expand sequencing coverage, and/or maintain sensitivity. In some embodiments, kits of the disclosure may increase sequencing efficiency. In some embodiments, kits of the disclosure may expand sequencing coverage. In some embodiments, kits of the disclosure may maintain sensitivity.

In some embodiments, at least one component of a kit of the disclosure has passed a QC process of the disclosure. In some embodiments, at least one component of a kit of the disclosure has passed multiple QC processes of the disclosure. In some embodiments, multiple components of a kit of the disclosure have passed a QC process of the disclosure. In some embodiments, multiple components of a kit of the disclosure have passed multiple QC processes of the disclosure.

In some embodiments, the adaptors of a kit of the disclosure have passed an adaptor QC process of the disclosure. In some embodiments, the adaptor sets of a kit of the disclosure have passed an adaptor QC process of the disclosure. In some embodiments, the adaptors of a kit of the disclosure have passed an adaptor QC process as described in Example 16. In some embodiments, the adaptor sets of a kit of the disclosure have passed an adaptor QC process as described in Example 16.

In some embodiments, the capture probe modules of a kit of the disclosure have passed a probe QC process of the disclosure. In some embodiments, the capture probe modules of a kit of the disclosure have passed a probe QC process as described in Example 18.

In some embodiments, a kit of the disclosure comprises one or more reagents and/or primers selected from an End Repair Buffer, an End Repair Enzyme, a Ligation Mix, a Library PCR Mix, a Library Primer, a Post Amp PCR Mix, a Post Hyb Primer mix, a Genomic Mix, a FWD Seq Primer and a REV Seq Primer. Each of these reagents and primers may be the corresponding composition described elsewhere in the disclosure, or as described in Examples 11-19. In some embodiments, one or more reagents and/or primers of a kit of the disclosure have passed a QC process of the disclosure. In some embodiments, one or more reagents and/or primers of a kit of the disclosure have passed a QC process as described in Example 15.

In some embodiments, the positive control DNA sample of a kit of the disclosure has passed a QC process of the disclosure. In some embodiments, the positive control DNA sample of a kit of the disclosure has passed a QC process as described in Example 17.

### ADAPTORS

In some embodiments, the disclosure provides compositions and methods comprising an adaptor or adaptor module. In some embodiments, the disclosure provides a kit comprising a set of adaptors. In some embodiments, a set of adaptors refers to a collection of two or more adaptors, each comprising an adaptor module. In some embodiments, the term "adaptor module" refers to a polynucleotide sequence that is attached to a DNA fragment to form an adaptor-tagged DNA molecule.

In some embodiments, the term "adaptor module" refers to a polynucleotide that comprises at least three elements: (i) an amplification region comprising a primer binding site; (ii) an ID region; and (iii) an anchor region. The term "adaptor" may refer to a DNA molecule comprising the adaptor module or the complement thereof. In some embodiments, the term "adaptor" refers to a partially double-stranded DNA structure comprising an adaptor module. In some embodiments, the term "adaptor" refers to a single-stranded DNA molecule comprising an adaptor module, for example the ligation strand oligonucleotide.

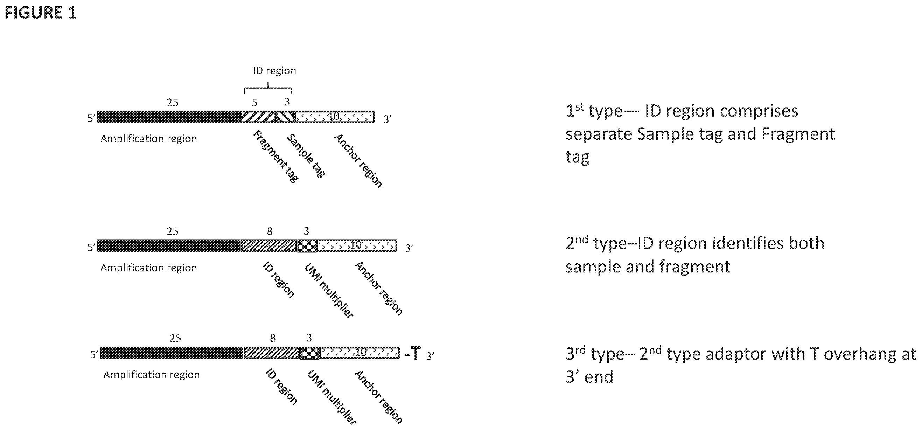

FIGURE 1 provides illustrative compositions of an adaptor module of the disclosure, including three different types of adaptors. As shown in FIGURE 1, a first type of adaptor module may include an ID region comprising distinct sections, wherein one section corresponds to a sample tag and another section corresponds to a fragment tag. A second type of adaptor module may include an ID region that does *not* comprise distinct sections as in the first type; in other words, for the second type adaptor, the sample tag consists of the entirety of the ID region and the fragment tag also consists of the entirety of the ID region. Additionally, the second type adaptor may also include a UMI multiplier. A third type of adaptor module may include an ID region, a UMI multiplier, and a T nucleotide overhang.

In some embodiments, the adaptor comprises one or more amplification regions, one or more ID regions, one or more UMI multipliers, and one or more anchor regions.

In some embodiments, the adaptor comprises, in order from 5' to 3', an amplification region, an ID region, a UMI multiplier, and an anchor region. In certain embodiments, the UMI is contained within the ID region, and the adaptor comprises, in order from 5' to 3', an amplification region, an integrated ID region /UMI multiplier region, and an anchor region.

In some embodiments, the adaptor comprises one or more amplification regions, one or more ID regions, one or more UMI multipliers, one or more anchor regions, and one or more nucleotides in the 3 overhang that are efficient ligation substrates. In additional embodiments, the adaptor module further comprises one or more sequencing primer binding sites.

In some embodiments, a partially double-stranded DNA adaptor structure is coupled to the DNA molecule prior to ligation to form an adaptor/DNA complex. Said partially double-stranded adaptor structure comprises a ligation strand oligonucleotide and a non-ligation strand oligonucleotide.

In some embodiments, the ligation strand oligonucleotide comprises an adaptor module, and the non-ligation strand oligonucleotide is capable of hybridizing to a region at the 3' end of the ligation strand oligonucleotide and forming a duplex therewith. In such embodiments, the non-ligation strand is complementary to at least a portion of the ligation strand in order to form said duplex.

In some embodiments, the adaptor further comprises a 3' terminal overhang, and the DNA molecule comprises a 3' terminal overhang that is complementary to the 3' terminal overhang of the adaptor. In some embodiments, the 3' terminal overhang (*e.g.,* a dT tail) aids in the ligation of the ligation strand to the 5' end of the DNA fragment, in order to drive the efficient ligation of the adapter to the DNA fragment having a complementary overhang (*e.g*., dA-overhang/tail).

The structure of illustrative adapters that may be used in the compositions and methods of the disclosure are provided in Table 9 and Table 10.

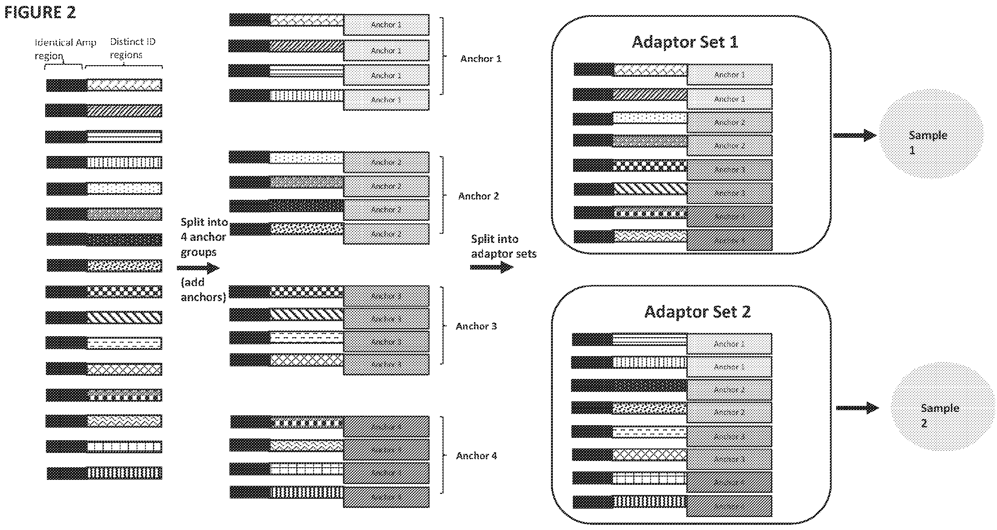

In some embodiments, one or more sets of adaptors are provided, where each set of adaptors is uniquely assigned to a given sample, and each adaptor is distinct from any other adaptor in any of the one or more adaptor sets. FIGURE 2 provides a schematic diagram of adaptor sets in one embodiment; a total of 16 different adaptors are used in this embodiment.

In some embodiments, compositions of the disclosure are assembled into a kit comprising one or more adaptor sets.

In some embodiments of the disclosure, a kit of the disclosure is used to generate a combined sequence-ready DNA library, where the combined library is any of the combined libraries described in the disclosure. In some embodiments, the kit is used to generate a TCL. In some embodiments, the kit is used to generate a TCLA. In some embodiments, the kit is used to generate adaptor-tagged DNA fragments or LIBS. In some embodiments, the kit is used to generate an LPA. In some embodiments, the kit is used to generate a LPWG. In some embodiments, the kit is used to generate a WGLA. In some embodiments, the kit is used to generate a LIBS and/or an LPA and a WGLA. In some embodiments, the kit is used to generate a LIBS and/or an LPA, a WGLA, and a TCL. In some embodiments, the kit is used to generate a LIBS and/or an LPA, a WGLA, and a TCLA. In some embodiments, the kit is used to generate a LIBS and/or an LPA and a TCL. In some embodiments, the kit is used to generate a LIBS and/or an LPA and a TCLA. In some embodiments, the TCL, TCLA, LIBS, LPA, LPWG and WGLA are any one of the DNA libraries of the disclosure. In some embodiments, a kit used to generate a LPWG comprises adaptors that have passed an adaptor QC process of the disclosure. In some embodiments, a kit used to generate a WGLA comprises adaptors that have passed an adaptor QC process of the disclosure. In some embodiments, a kit used to generate a TCLA comprises adaptors that have passed an adaptor QC process of the disclosure and capture probes that have passed a capture probe QC process of the disclosure. In some embodiments, a kit used to generate TCL comprises adaptors that have passed an adaptor QC process of the disclosure and capture probes that have passed a capture probe QC process of the disclosure.

In some embodiments, the kit comprises a set of adaptors. In some embodiments, adaptor sets of the kit may comprise any of the adaptor modules described in the disclosure. To assure high performance of the kit, each of adaptor sets may be subject to a Quality Control (QC) process prior to inclusion in the kit.

In some embodiments, the QC process for adaptor sets tests adaptor ligation efficiency. In some embodiments, the QC process for adaptor sets comprises a test for adaptor ligation. As described above, the efficiency of adaptor attachment refers to the conversion rate or percentage of input DNA fragments to adaptor-tagged DNA library molecules.

In some embodiments, the test for adaptor ligation is performed using a DNA sample. In some embodiments, said DNA sample is blended DNA sample of at least two different cell lines. In some embodiments, said DNA sample is a blended DNA sample of at two different cell lines. In some embodiments, the two different cell lines are blended at a 50:50 ratio. In some embodiments, the two different cell lines are NA09596 and NA12878. In some embodiments, the test for adaptor ligation is performed using the 50:50 blended sample described in Example 15.

In some embodiments, the DNA sample used in the test for adaptor ligation is different from the DNA sample used in the test for adaptor distribution.

In some embodiments, for each test sample in the QC process for adaptor ligation efficiency, a unique adaptor set is assigned. The QC process for adaptor ligation efficiency may be performed using any of the end repair, adaptor ligation, and library amplification methods of the disclosure to generate an LPA (Library Post Amplification). In some embodiments, the QC process for adaptor ligation efficiency is performed according to the methods of Example 11 to generate an LPA.

In some embodiments, one or more acceptance criteria is set for the QC process for adaptor attachment efficiency, where only the adaptor sets that pass such criteria will be included in a kit of the disclosure. An illustrative acceptance criterion for adaptor attachment efficiency is the LPA has a concentration of at least 1 ng/µL to at least 200 ng/µL with 5 ng-50 ng input DNA.

In some embodiments, the QC process for adaptor sets tests adaptor distribution. As used herein, adaptor distribution may refer to the number of sequencing reads assigned to a specific adaptor sequence in comparison to an average number of sequencing reads for all adaptor sequences with an adaptor set.

In general, other adaptor QC processes used in the art do not test adaptor distribution. Because a random index sequence is commonly used as an adaptor tag in many adaptor ligation processes in the art, randomness and/or distribution evenness of the adaptor tag is assumed but not tested during QC. In contrast, the adaptor QC processes of the disclosure are designed to measure and quantify the actual distribution of the adaptors of the disclosure, thereby ensuring high quality and performance of the adaptors of the disclosure. In some embodiments of the disclosure, the distribution of the adaptors is uniform. In some embodiments, adaptor distribution is considered to be uniform when the number of sequencing reads for a unique adaptor sequence is not significantly different from the average number of sequencing reads for all unique adaptor sequences. In some embodiments, adaptor distribution is measured according to the methods of Example 16. In some embodiments, adaptor distribution is considered to have passed the acceptance criteria no more than 10%-80% of unique adaptor sequences have a number of reads that is 0% to 50% of the average number of reads for all unique adaptor sequences. In some embodiments of the disclosure, the distribution of the adaptors is improved compared to the distribution of a method or kit that do not use adaptors of the disclosure.

In some embodiments, the QC process to test adaptor distribution may be performed using a DNA sample. In some embodiments, said DNA sample comprises WT cfDNA. In some embodiments, said DNA sample consists of WT cfDNA. In some embodiments, said DNA sample comprises WT cfDNA obtained from a healthy human. In some embodiments, said DNA sample is the WT cfDNA sample described in Example 16. An illustrative WT cfDNA sample has a concentration of at least about 0.2 ng/µL and is obtained from a healthy human or a number of healthy humans.

In some embodiments, the DNA sample used in the test for adaptor distribution is different from the DNA sample used in the test for adaptor ligation.

In some embodiments, the QC process for adaptor distribution comprises assigning each test sample to a unique adaptor set. The QC process for adaptor ligation efficiency may be performed using any of the end repair and adaptor ligation methods of the disclosure to generate a collection of adaptor-tagged DNA fragments (LIBS) for each test sample. In some embodiments, end repair and adaptor ligation is performed according to the methods of Example 11 to generate LIBS for each test sample.

In some embodiments, the QC process for adaptor distribution comprises amplification of the LIBS of each test sample using Index Primers, where one or more unique Index Primers are assigned to each LIBS of each test sample. In some embodiments, one primer pair comprising an Index Primer is assigned to each LIBS of each test sample. In some embodiments, the primer pair comprises an Index Primer and a Primer F. In some embodiments, the Primer F comprises the sequence of SEQ ID NO: 7. In some embodiments, two Index Primers are assigned to each LIBS of each test sample. In some embodiments, the Index Primers comprise Illumina^{®} P5 and P7 sequences. In some embodiments, the Index Primers comprise the sequences of SEQ ID NO: 13 and SEQ ID NO: 14.

In some embodiments, the QC process for adaptor distribution comprises a library amplification process according to any of the library amplification methods of the disclosure. In some embodiments, the QC process for adaptor distribution comprises a library amplification process according to the methods of Example 16.

In some embodiments, the QC process for adaptor distribution comprises isolating or purifying the amplified libraries. In some embodiments, the isolating or purifying is performed using DNA purification beads. In some embodiments, the isolating or purifying is performed according to the methods of Example 16. Libraries at this stage (after isolation or purification) may be referred to as Library Post Index Amplification (LPIA).

In some embodiments, the QC process for adaptor distribution comprises sequencing the LPIA of each test sample. In some embodiments, the QC process for adaptor distribution comprises sequencing a combined LPIA pool by combining a certain amount of LPIA from each test sample. In some embodiments, the combined LPIA pool may be sequenced according to the methods of Example 16. In some embodiments, the combined LPIA pool may be sequenced using sequencing primers below:
FWD Seq Primer:
   CAAGCAGAAGACGGCATACGAGATGTGACTGGCACGGGACCAGAGAATTCGAAT ACA (SEQ ID NO: 10);
REV Seq Primer:
   GTGACTGGCACGGGACCAGAGAATTCGAATACA (SEQ ID NO: 11);
and an Index Sequencing Primer comprising the sequence of an Index Primer used.

In some embodiments, the Index Sequencing Primer comprises:
TGTATTCGAATTCTCTGGTCCCGTGCCAGTCAC (SEQ ID NO: 83).

In some embodiments, one or more acceptance criteria is provided in the QC process to determine whether the adaptor sets meet the quality requirements to enable high performance of the kit. In some embodiments, cross-contamination of adaptor sets are analyzed. Cross-contamination of adaptor sets may occur when an adaptor that is not assigned to a given sample appears in the sequencing reads of this sample. In some embodiments, acceptance criteria for a QC process of adaptor set cross-contamination may include: a. for each sample, no more than 0.05%-2% of "Barcode Crosstalk"; and b. for each sample, no more than 1%-20% of reads are from unknown adaptors (*i.e.,* not included in the adaptor set). Barcode Crosstalk as used herein refers to the reads of one barcode (adaptor) being mislabeled as adaptor A when they are actually generated from adaptor B.

In some embodiments, one or more acceptance criteria for the QC process of adaptor distribution is selected from Table 13.

In some embodiments, only adaptors that have passed one or more of the QC processes of the disclosure is included in a kit of the disclosure.

### Overview of Adaptor Features

In some embodiments, the adaptor includes the following features: (i) one-step attachment; (ii) high efficiency attachment; (iii) uniform adaptor distribution; (iv) accommodation of sample multiplexing and sample identification; and/or (v) high number of unique molecule identifiers (UMIs). For example, some embodiments of the kits and methods of the disclosure provide the following:

One-step attachment: In some embodiments, the full-length adaptor may be attached to the DNA fragment in one step. A "full length" adaptor comprises at least 4 regions comprising: an amplification region comprising a primer binding site, an ID region comprising a unique molecule identifier (UMI), a UMI multiplier, and an anchor region. Attaching a full-length adapter eliminates the need for adapter ligation in a stepwise fashion where the anchor is attached first, then the remaining regions of the adaptor are attached (for example, see the stepwise manner of adaptor ligation in the kinase/ligase ligation method described below).

High efficiency attachment: In some embodiments, the adapters may be attached to the DNA molecules with high efficiency. For the purposes of the instant disclosure, the efficiency of adaptor attachment refers to the conversion rate or percentage of input DNA fragments to adaptor-tagged DNA library molecules. For example, a DNA fragment may be identified by the ID region of an attached adaptor, and a DNA fragment would not be identifiable using the ID region if it was not attached to an adaptor. Accordingly, a higher efficiency of adaptor attachment may lower the number of input DNA fragments lost in the library conversion process. This is particularly useful in situations where the quantity of available DNA is limited, or where the frequency of variant DNA is very low, for example in samples analyzed in connection with many oncology applications and other genetic diseases (*e.g*., multiple sclerosis, rheumatoid arthritis, Alzheimer's disease). In such situations the occurrence of DNA alterations (*e.g*., single nucleotide variants (SNVs), indels, copy number changes, DNA rearrangements, optionally related to tumors/cancers) are typically infrequent and thus can be difficult to detect. Highly efficient attachment of adaptors of the disclosure to these DNA molecules may facilitates capture of such infrequent variations. In some embodiments of the disclosure, at least 50% of input DNA molecules are converted into DNA library molecules by attachment of the adapters.

Uniform adaptor distribution: Bioinformatics analysis may analyze intra-sample adaptor performance and inter-sample adaptor performance. Performance fluctuation between adaptor sets across samples may negatively impact the sensitivity of the analysis. Uniform adaptor distribution in the adaptor-tagged DNA libraries as measured by sequence reads is desirable. In some embodiments, there is the possibility of bias in the distribution of adaptors in the adaptor-tagged DNA libraries, where some adaptors may be less efficient in ligating to the DNA molecules or less efficiently amplified compared to the others in the adapter pool. This may result in fewer amplicons, fewer reads of those less efficient adapters, and fewer reads of the DNA fragments ligated to those less efficient adaptors during sequencing. While such biased distribution of adaptors may be tolerated or compensated for by increasing the amount of the less-efficient adapters in the adapter pool to provide a more balanced representation of the adapters in the tagged DNA libraries and sequencing reads, the compositions and methods of the disclosure provide the option of eliminating such compensation. The compositions and methods of the disclosure can provide the benefit of achieving uniform adaptor distribution, wherein each adaptor is represented at roughly the same ratio in sequencing results. This uniform adaptor distribution provides increased sensitivity.

In some embodiments, the uniform adaptor distribution may be achieved by having multiple types of anchor regions that are all represented in each set of adapters.

In some embodiments, adaptor module sequences that have poor performance in ligation or amplification (for example sequences that include a string of G nucleotides) are not selected as a candidate adaptor module sequence to be used in the methods of the disclosure.

In some embodiments, the uniform adaptor distribution may be achieved by having unique ID regions (each ID region identifies both the sample and the DNA fragment attached thereto) randomly selected for each set of adaptors.

Accommodation of sample multiplexing and sample identification: To achieve sample multiplexing (*i.e.,* the ability to run different samples simultaneously), in some embodiments, one or more sets of adaptors are provided, where each adaptor set is uniquely assigned to one sample. Adaptor-tagged DNA fragments are constructed where a given adaptor within the same adaptor set is attached to a DNA molecule of the same test sample. From a sequence counting perspective, it is beneficial for each unique adaptor of the same set of adaptors to possess essentially identical behavior to all other adaptors in this set. In order to achieve this, in some embodiments, each ID region has a Hamming distance of at least 2 between any other ID region, thus reducing the chance for a read to be spuriously assigned to the wrong sample. The term "Hamming distance" as used in the disclosure refers to the number of nucleotides that are different between two nucleotide sequences. In some embodiments, each set of adaptors is split into further groups that are paired with specific anchor regions, allowing for further reduction in the possibility of an error in sample de-multiplexing. For example, in an 8mer sequence with Hamming distance of 2, the total number of possible sequences is 16,384. The term "paired" or "matched" when used with respect to two different polynucleotide sequences or regions of DNA comprising different polynucleotide sequences, means that the two different polynucleotide sequences or regions of DNA comprising different polynucleotide sequences are present on the same molecule. For example, if a particular ID region of DNA is said to be paired to a particular amplification region of DNA, it is meant that the ID region and the amplification tag are present on the same DNA polynucleotide molecule.

High number of Unique Molecule Identifiers (UMIs): While it is beneficial in general for adaptors to be functionally equivalent from a molecular biology perspective, it is also desirable that adaptors possess a very large number of unique molecule identifiers (UMIs) (≥ 10,000) that augment the identification of unique genomic fragments. In this context, by "augment", it is meant that the power of identifying a uniquely derived fragment is increased. In some embodiments, a uniquely derived fragment (unique genomic clone) refers to a library clone fragment derived from a single DNA fragment in the test sample. Each genomic clone fragment has a particular pair of fragmentation sites corresponding to the position in the genomic sequence where the double-strand DNA was cleaved. In some embodiments, this cleavage site may be used to differentiate unique genomic clones, because each clone is likely to possess a different cleavage site. However, in libraries that possess thousands of independent clones, uniquely derived fragments may often possess the exact same cleavage sites. Genomic clones sharing the same cleavage site may be classified as either unique or as redundant (*i.e.,* generated through amplification) with respect to other clone sequences derived from the same sample. By attaching adaptors that introduce a high diversity of sequence tags, unique genomic clones sharing the same cleavage site are more likely to be identified as unique. In some embodiments, the ID region is the UMI. In some embodiments, the UMI is created by the combination of the ID region with the anchor region. In some embodiments, the UMI is created by a combination of the ID region with the UMI multiplier. In some embodiments, the UMI is created by a combination of the ID region, the UMI multiplier, and the anchor region. In some embodiments, the combination of the UMI and the cleavage site create a unique molecular identifier element (UMIE), which facilitates the classification of sequence reads as redundant reads or unique reads. Some embodiments contemplate that the UMI multiplier could comprise longer or shorter sequences to increase or lower the overall UMI complexity.

### Ligation strand

The terms "ligation strand oligonucleotide" and "ligation strand" may be used interchangeably.

The disclosure provides, in some embodiments, a ligation strand oligonucleotide comprising (i) a 3' terminal overhang; (ii) an amplification region comprising a primer binding site; (iii) a unique ID region; (iv) a unique molecule identifier (UMI) multiplier; and (v) an anchor region comprising a polynucleotide sequence that is at least partially complementary to the non-ligation strand oligonucleotide.

In some embodiments, the ligation strand comprises (i) a 3' terminal overhang; (ii) an amplification region comprising a primer binding site; (iii) a unique ID region; and (iv) an anchor region comprising a polynucleotide sequence that is at least partially complementary to the non-ligation strand oligonucleotide.

In some embodiments, the ligation strand of an adaptor may comprise the following structure: AMP-ID Region/UMI Multiplier-ACGTATGCCA-3'dT (SEQ ID NO: 1). In some embodiments, the ligation strand of an adaptor may comprise the following structure: AMP-ID Region/UMI Multiplier-CTAGCGTTAC-3'dT (SEQ ID NO: 2). In some embodiments, the ligation strand of an adaptor may comprise the following structure: AMP-ID Region/UMI Multiplier-GATCGACATG-3'dT (SEQ ID NO: 3). In some embodiments, the ligation strand of an adaptor may comprise the following structure: AMP-ID Region/UMI Multiplier-TGCATCAGGT-3'dT (SEQ ID NO: 4).

In some embodiments, an adaptor may comprise a ligation strand with a 3' dT overhang. In some embodiments, the ligation strand with a 3' dT overhang may comprise any one of the sequences shown in Table 9. For example, the ligation strand with a 3' dT overhang may comprise a sequence of any one of SEQ ID NO: 22 to 81. The "NNN" within these ligation strand sequences represents a 3-nucleotide UMI multiplier wherein each N may be selected from any one of A, G, C, or T. In some embodiments, the ligation strand with a 3' dT overhang may comprise a sequence of any one of SEQ ID NO: 22 to 81 with 1, 2, 3, 4, 5, 6, 7, 8, 9, 10 or more nucleotide substitutions.

In some embodiments, the ligation strand oligonucleotide upon contacting with a DNA molecule is capable of ligating to the 5' end of each strand of the DNA molecule.

In some embodiments, the ligation strand oligonucleotide comprises a dT overhang at the 3' terminus and the DNA molecule comprises a dA overhang at the 3' terminus of each strand. The term "3' terminal overhang" refers to one or more nucleotide overhangs or tails at the 3' terminus of a polynucleotide. In some embodiments, the 3' terminal overhang (*e.g.,* a dT tail) aids in the ligation of the ligation strand to ligate to the 5' end of the DNA fragment, in order to drive the efficient ligation of the multifunctional adaptor to the DNA fragment having a complementary overhang (*e.g*. dA-overhang/tail).

In certain embodiments, the ligation strand oligonucleotide comprises a dA overhang at the 3' terminus and the DNA molecule comprises a dT overhang at the 3' terminus of each strand. In some embodiments, the ligation strand oligonucleotide comprises a dC overhang at the 3' terminus and the DNA molecule comprises a dG overhang at the 3' terminus of each strand. In certain embodiments, the ligation strand oligonucleotide comprises a dG overhang at the 3' terminus and the DNA molecule comprises a dC overhang at the 3' terminus of each strand.

In some embodiments, the ligation strand oligonucleotide is not phosphorylated at the 5' terminus.

In some embodiments, the ligation strand oligonucleotide comprises an adaptor module, wherein the anchor region of the ligation strand oligonucleotide comprises a polynucleotide sequence that is at least partially complementary to the non-ligation strand oligonucleotide. In such embodiments, the non-ligation strand oligonucleotide is capable of hybridizing to a region at the 5' end of the ligation strand oligonucleotide and forming a duplex therewith.

In some embodiments, the ligation strand oligonucleotide is between about 30 nucleotides and about 70 nucleotides in length. In some embodiments, the ligation strand oligonucleotide is between about 35 and about 65 nucleotides, between about 40 and about 60 nucleotides, or between about 40 and about 50 nucleotides in length. In some embodiments, the ligation strand oligonucleotide is about 47 nucleotides in length.

In some embodiments, the ligation strand oligonucleotide is between 30 nucleotides and 70 nucleotides in length. In some embodiments, the ligation strand oligonucleotide is between 35 and 65 nucleotides, between 40 and 60 nucleotides, or between 40 and 50 nucleotides in length. In some embodiments, the ligation strand oligonucleotide is 47 nucleotides in length.

### Non-ligation Strand

the terms "non-ligation strand oligonucleotide" and "non-ligation strand" may be used interchangeably.

In some embodiments, the non-ligation strand of an adaptor may comprise the sequence TGGCATACGT (SEQ ID NO: 18). In some embodiments, the non-ligation strand of an adaptor may comprise the sequence GTAACGCTAG (SEQ ID NO: 19). In some embodiments, the non-ligation strand of an adaptor may comprise the sequence CATGTCGATC (SEQ ID NO: 20). In some embodiments, the non-ligation strand of an adaptor may comprise the sequence ACCTGATGCA_(SEQ ID NO: 21).

In some embodiments, the non-ligation strand is not phosphorylated. Lack of phosphorylation of the non-ligation strand may prevent the non-ligation strand from attaching to the 3' end of the DNA fragment and may reduce the formation of adapter dimers.

In some embodiments, the non-ligation strand oligonucleotide comprises a modification at its 3' terminus that prevents ligation to the 5' end of the DNA molecule and/or adaptor dimer formation. In some embodiments, said modification is a chemical modification.

### Amplification Region

The term "amplification region" refers to an element of the adaptor molecule that comprises a primer recognition site or primer binding site. The primer binding site can be for any primer that is suitable for any amplification known in the art, such as methods disclosed in Fakruddin et al. "Nucleic acid amplification: Alternative methods of polymerase chain reaction" J Pharm Bioallied Sci. 2013 Oct-Dec; 5(4): 245-252. For example, such amplification methods may include PCR (polymerase chain reaction), LAMP (loop-mediated isothermal amplification), NASBA (nucleic acid sequence-based amplification), SDA (strand displacement amplification), RCA (rolling circle amplification), or LCR (ligase chain reaction).

In some embodiments, an adaptor comprises an amplification region that comprises one or more custom primer binding sites. In some embodiments, the custom primer binding site comprises a sequence having 100% sequence identity to a sequence of the custom primer binding site of each adaptor module of the plurality of adaptor modules. In some embodiments, the custom primer binding sites facilitate single-primer amplification of a DNA library. In some embodiments, the amplification region comprises one, two, three, four, five, six, seven, eight, nine, ten, or more primer binding sites for single-primer amplification of a DNA library. In some embodiments, the amplification region comprises a PCR primer binding site for Library Primer (SEQ ID NO: 5). The Library Primer may be a single amplification primer, also referred to herein as the AKA primer.

In some embodiments, sequence identity is determined by aligning two polynucleotide sequences. Various methods exist in the art to align sequences and determine sequence identity or sequence percent identity, including but not limited to, using the BLAST suite from the National Center for Biotechnology Information (NCBI). In some embodiments, the term "sequence identity" as used herein refers to sequence identity determined using BLAST version 2.12.0 under default parameters.

In some embodiments, the amplification region is between about 5 and about 50 nucleotides, between about 10 and about 45 nucleotides, between about 15 and about 40 nucleotides, or between about 20 and about 30 nucleotides in length. In some embodiments, the amplification region is about 10 nucleotides, about 11 nucleotides, about 12 nucleotides, about 13 nucleotides, about 14 nucleotides, about 15 nucleotides, about 16 nucleotides, about 17 nucleotides, about 18 nucleotides, about 19 nucleotides, about 20 nucleotides, about 21 nucleotides, about 22 nucleotides, about 23 nucleotides, about 24 nucleotides, about 25 nucleotides, about 26 nucleotides, about 27 nucleotides, about 28 nucleotides, about 29 nucleotides, about 30 nucleotides, about 31 nucleotides, about 32 nucleotides, about 33 nucleotides, about 34 nucleotides, about 35 nucleotides, about 36 nucleotides, about 37 nucleotides, about 38 nucleotides, about 39 nucleotides, or about 40 nucleotides or more in length. In some embodiments, the amplification region is about 25 nucleotides in length.

In some embodiments, the amplification region is between 5 and 50 nucleotides, between 10 and 45 nucleotides, between 15 and 40 nucleotides, or between 20 and 30 nucleotides in length. In some embodiments, the amplification region is 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides, 30 nucleotides, 31 nucleotides, 32 nucleotides, 33 nucleotides, 34 nucleotides, 35 nucleotides, 36 nucleotides, 37 nucleotides, 38 nucleotides, 39 nucleotides, or 40 nucleotides or more in length. In some embodiments, the amplification region is 25 nucleotides in length.

### ID Region

As used in the disclosure, the terms "multifunctional ID region" and "ID region" may be used interchangeably and refer to an element of the adaptor that comprises a polynucleotide sequence that uniquely identifies the particular DNA fragment and/or the sample from which it was derived. In some embodiments, the ID region uniquely identifies both the DNA fragment and the sample from which it was derived.

In some embodiments, the multifunctional ID region is between about 3 and about 50 nucleotides, between about 3 and about 25 nucleotides, or between about 5 and about 15 nucleotides in length. In some embodiments, the multifunctional ID region is about 3 nucleotides, 4 nucleotides, about 5 nucleotides, about 6 nucleotides, about 7 nucleotides, about 8 nucleotides, about 9 nucleotides, about 10 nucleotides, about 11 nucleotides, about 12 nucleotides, about 13 nucleotides, about 14 nucleotides, about 15 nucleotides, about 16 nucleotides, about 17 nucleotides, about 18 nucleotides, about 19 nucleotides, or about 20 nucleotides or more in length. In some embodiments, the multifunctional ID region is about 8 nucleotides in length.

In some embodiments, the multifunctional ID region is between 3 and 50 nucleotides, between 3 and 25 nucleotides, or between 5 and 15 nucleotides in length. In some embodiments, the multifunctional ID region is 3 nucleotides, 4 nucleotides, 5 nucleotides, 6 nucleotides, 7 nucleotides, 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, or 20 nucleotides or more in length. In some embodiments, the multifunctional ID region is 8 nucleotides in length.

In some embodiments, the ID region comprises one of between about 2 and about 10,000 unique nucleotide sequences, between about 50 and about 500 unique nucleotide sequences, or between about 100 and about 400 unique nucleotide sequences. In some embodiments, the ID region of each adaptor of the plurality of adaptors comprises one of about 60 unique nucleotide sequences.

In some embodiments, the ID region comprises one of between 2 and 10,000 unique nucleotide sequences, between 50 and 500 unique nucleotide sequences, or between 100 and 400 unique nucleotide sequences. In some embodiments, the ID region of each adaptor of the plurality of adaptors comprises one of 60 unique nucleotide sequences.

In some embodiments, the adaptor comprises one of between 64 and 2,560,000 unique nucleotide sequences.

In some embodiments, one or more sets of adaptors are provided, where each adaptor set is uniquely assigned to a given sample, and wherein each adaptor has a unique ID region that is distinct from the ID region of any other adaptor of the one or more adaptor sets.

In some embodiments, a pre-specified first adaptor set and a pre-specified second adaptor set are provided. Such pre-specified adaptor sets are each used to represent a single sample. In some embodiments, the first adaptor set comprises a first set of unique ID regions and the second adaptor set comprises a second set of unique ID regions. That is, each adapter module sequence in the first set is distinct from each adapter module sequence in the second set; additionally, each adaptor module sequence within a set is also distinct from every other adaptor module sequence within the same set. Thus, in some embodiments, the uniqueness of each adaptor module sequence allows identification of each uniquely derived library fragment.

One of skill in the art will recognize the number of distinct oligonucleotides in a given adaptor set that are possible for the adapter oligonucleotides will depend on the length of the ID region and/or the UMI multiplier.

In one embodiment, the ID region serves to identify both the DNA fragment attached thereto and the individual sample from which the DNA fragment was derived, for example, a genomic library source. In some embodiments, each sample is assigned an adaptor set comprising a plurality of between about 64 and about 2.5 million unique adapters. In some embodiments, each sample is assigned an adaptor set comprising a plurality of between 64 and 2.5 million unique adapters. In some embodiments, each sample is assigned an adaptor set comprising a plurality of about 3,840 unique adaptors. In some embodiments, each sample is assigned an adaptor set comprising a plurality of 3,840 unique adaptors.

In some embodiments, each sample is assigned an adaptor set comprising a plurality of between about 1 and about 100 unique ID regions. In some embodiments, each sample is assigned an adaptor set comprising between about 1 and about 60 unique ID regions. In some embodiments, each sample is assigned an adaptor set comprising between 1 and 60 unique ID regions. In some embodiments, each sample is assigned an adaptor set comprising 60 unique ID regions, wherein each set of 60 unique ID regions is further divided into 4 groups (each group comprising 15 unique adaptors), wherein each ID region of one group is paired to one of 4 anchor sequences. Therefore, in such embodiments, the sample can be identified by the combination of the ID region and the anchor region.

In some embodiments, the nucleotide sequence of each ID region is discrete from the nucleotide sequence of any other ID regions of the plurality of adaptor modules by a Hamming distance of at least two (meaning at least two base changes are required to change one ID region into another).

In certain embodiments, the ID region is 8 nucleotides in length and comprises one of 240 unique nucleotide sequences, and the UMI multiplier is 3 nucleotide sequences in length, therefore the total number of unique adaptor sequences would be 240 x 4³= 15,360. Thus, in this embodiment each sample may be assigned a set of adaptors ranging from 1~ 15,360 unique adaptors for DNA fragment identification. In some embodiments, each nucleotide sequence is discrete from any other sequence of the 15,360 unique nucleotide sequences by Hamming distance of at least two.

In some embodiments, the multifunctional ID region is 8 nucleotides in length and comprises one of 60 unique nucleotide sequences, the UMI multiplier is 3 nucleotides in length, therefore the total number of unique adaptor sequences would be 60 x 4³= 3840. Thus, in this embodiment each sample may be assigned a set of adaptors ranging from 1~ 3840 unique adaptors for DNA fragment identification. In some embodiments, each nucleotide sequence is discrete from any other sequence of the 3840 unique nucleotide sequences by Hamming distance of at least two.

In some embodiments, the ID region is capable of identifying both the sample and the DNA fragment. In some embodiments, the ID region is used to identify the individual DNA fragment attached thereto. Thus, in such embodiments, the ID region can also serve as a fragment tag that can enumerate clone diversity for sensitive variant detection. This is in stark contrast to other adaptors that are used in the art which use a randomly generated barcode to identify the sequence and a separate barcode or sequencer indexing to allow for sample identification (sample de-multiplexing). One embodiment of such an adaptor module comprising separate barcodes (tags) is depicted in FIGURE 1 (1^{st} type), wherein the ID region comprises a separate sample tag and fragment tag. In this context, by "separate", it is meant that the sample tag is an individual section capable of identifying the sample but incapable of identifying the DNA fragment attached thereto, and the fragment tag is an individual section capable of identifying the DNA fragment. In contrast, in some embodiments, for example the 2^{nd} type and 3^{rd} type adaptor modules of FIGURE 1, where the ID region in its entirety is used to identify the sample, and the ID region in its entirety is used to identify the DNA fragment.

In some embodiments, each adaptor module further comprises a sample tag comprising a polynucleotide sequence that is not identical to the polynucleotide sequence of the ID region. In some embodiments, each adaptor module in the a first adaptor set comprises a sample tag comprising a first polynucleotide sequence and each adaptor module in a second adaptor set comprises a sample tag comprising a second polynucleotide sequence; wherein the first and second polynucleotide sequences are not identical to each other.

### UMI Multiplier

In some embodiments, to further increase the diversity of possible sequence tags (UMIs) and augment the identification of unique genomic fragments, UMI multipliers are included in the adaptor modules. A UMI multiplier is a short sequence of bases which, when combined with a UMI, increases the diversity of and total number of adaptor sequences in an adaptor pool. In some embodiments, the adaptor module comprises a UMI multiplier, wherein the UMI multiplier is adjacent to or contained within the ID region. In some embodiments, the adaptor comprises an ID region that is eight nucleotides in length and a UMI multiplier that is three nucleotides in length. In some embodiments, the UMI multiplier comprises one of 64 possible sequences. In some embodiments, the UMI multiplier is adjacent to or contained within the ID region.

In some embodiments, each nucleotide position of the UMI multiplier can comprise any one of adenine, guanine, cytosine, or thymine. Thus, in some embodiments, a UMI multiplier comprising n number of nucleotides can comprise any of 4ⁿ possible nucleotide sequences. In some embodiments, the UMI multiplier is one nucleotide in length and comprises one of four possible sequences. In some embodiments, the UMI multiplier is two nucleotides in length and comprises one of sixteen possible sequences. In some embodiments, the UMI multiplier is three nucleotides in length and comprises one of 64 possible sequences. In some embodiments, the UMI multiplier is four nucleotides in length and comprises one of 256 possible sequences. In some embodiments, the UMI multiplier is five nucleotides in length and comprises one of 1,024 possible sequences. In some embodiments, the UMI multiplier is six nucleotides in length and comprises one of 4,096 possible sequences. In some embodiments, the UMI multiplier is seven nucleotides in length and comprises one of 16,384 possible sequences. In some embodiments, the UMI multiplier is eight nucleotides in length and comprises one of 65,536 possible sequences. In some embodiments, the UMI multiplier is nine nucleotides in length and comprises one of 262,144 possible sequences. In some embodiments, the UMI multiplier is ten or more nucleotides in length and comprises one of 1,048,576 or more possible sequences.

In some embodiments, the UMI multiplier is at least 1, at least 2, at least 3, at least 4, at least 5, at least 6, at least 7, at least 8, at least 9, or at least 10 nucleotides in length. In some embodiments, the UMI multiplier is between 1 and 5 nucleotides in length. In some embodiments, the UMI multiplier is 3 nucleotides in length.

### Anchor Region

As used in the disclosure, the terms "anchor region" and "anchor sequence" may be used interchangeably and refer to a polynucleotide sequence that is at least partially complementary to the non-ligation strand oligonucleotide. The anchor region may, in some embodiments, comprise one or more of the following properties:
(1) Each anchor sequence may be part of a set of two or more unique anchor types that collectively represent each of the four possible DNA bases at each site within extension; this feature, balanced base representation, is useful to calibrate proper base calling in sequencing reads in some embodiments. The number of total types of anchor sequences should match the total number of detection modes. For example, four colors are detected in Illumina^{®} sequencing, therefore four types of anchor sequences may be used. To achieve maximum sensitivity, each detection mode may be utilized. The methods and compositions of the disclosure may be used in any mode of detection known in the art, including but not limited to light-based detection, enzyme-based detection, and magnetic detection.
(2) Each anchor sequence may be composed of only two of four possible bases, which are specifically chosen to be either an equal number of A + C or an equal number of G + T; an anchor sequence formed from only two bases reduces the possibility that the anchor sequence will participate in secondary structure formation that would preclude proper adaptor function.
(3) Because each anchor sequence may be composed of equal numbers of A + C or G + T, each anchor sequence may share roughly the same melting temperature and duplex stability as every other anchor sequence in the set.
(4) Each type of anchor sequence (ending in either A/T/G/C) may be approximately equally distributed in the sequencing reads, for example in approximately equimolar amounts (*i.e.* about 25% of the set have adapter sequences ending in A, about 25% ending in T, about 25% ending in G, and about 25% ending in C).

In some embodiments, adapter modules are mixed with DNA fragments in equimolar amounts of adapters containing different anchor types (e.g. equimolar amounts of anchor 1, anchor 2, anchor 3, anchor 4) to provide a more even adapter distribution. Exemplary anchor sequences include but are not limited to: Anchor 1 ACGTATGCCA (SEQ ID NO: 1); Anchor 2 CTAGCGTTAC (SEQ ID NO: 2); Anchor 3 GATCGACATG (SEQ ID NO: 3); and Anchor 4 TGCATCAGGT (SEQ ID NO: 4).

In some embodiments, adapter sequences end with a T nucleotide at the 3' terminus (3' T overhang). In some embodiments, some adaptors have TT as the last 2 nucleotides of the 3' terminus, others may have AT, CT, or GT as the last 2 nucleotides of the 3' terminus. In some embodiments, the plurality of adapters in an adaptor set comprises a 3' dT overhang that does not hybridize to the non-ligation strand.

In general, an ideal distribution of anchor types would result in each anchor type having an identical distribution percentage (*i.e.,* 100% divided by the number of anchor types), resulting in a "uniform" distribution of different adaptors comprising different anchors among all DNA fragments. In some embodiments, distribution of anchor types refers to the distribution of different anchor sequences in a DNA library that is sequence-ready or has been sequenced. In some embodiments, an ideal distribution of four anchor types would result in about 25% distribution of each anchor type. In some embodiments, the anchor sequences of a given adaptor set have a distribution percentage of between about 5% to about 75% (*i.e.,* the distribution % of the most infrequent anchor type is about 5% and the distribution % of the most frequent anchor type is about 75%). In some embodiments, each anchor sequence of a given set has a distribution % of about 50%, about 34%, about 28%, about 27%, about 23%, about 14%, or about 9%.

In some embodiments, adapters having anchors of a specific sequence or type may be present in higher amounts. Such adapters may be 1X, 2X, 4X, 5X, 6X, 7X, 8X, 9X, or more than 10X the amount of other anchor types in the adaptor set, resulting in more even distribution of adaptors in sequencing reads.

In some embodiments, the adaptor set can comprise more than one anchor sequence. For example, a set of adaptors may contain 4 different anchor sequences are used simultaneously. These anchor sequences may also be used during sample de-multiplexing to lower errors. In some embodiments, sequencing reads need to pass inclusion filters for downstream consideration. In such embodiments, because the position of sequences within a sequencing read is fixed, the ID regions and anchors should have a fixed position within a sequencing read in order to pass the inclusion filters.

In some embodiments, the anchor region of each adaptor of a given set of adaptors comprises one of four sequences. In the embodiment depicted in FIGURE 2, a total of 16 distinct ID regions is split into four anchor groups (one group per each nucleotide A, C, T and G), with each anchor group comprising 4 ID regions and each ID region is matched with one of four anchor sequences. In some embodiments, a total of 240 ID regions may be split into four anchor groups (one group per each nucleotide A, C, T and G), with each anchor group comprising 60 ID regions and each ID region matched with one of four anchor sequences. In other words, the 240 ID regions are divided into four sets of 60 sequences, with each set paired to a specific anchor region. In some embodiments, each adaptor set has an equimolar amount of each anchor sequence. Therefore, in some embodiments, identification involves not only the sequence information from the ID region, but also the sequence information from the associated anchor region. This may result in each adaptor having a unique sequence and the plurality of adaptors in each adaptor set having a balanced anchor composition. In some embodiments, a total of 60 ID regions may be split into four sets, with each set comprising 15 ID regions and each ID region matched with one of four anchor sequences.

In some embodiments, the anchor region is between 1 and 50 nucleotides in length. In some embodiments, the anchor region is between 4 and 40 nucleotides in length. In certain embodiments, the anchor region is between 5 and 25 nucleotides in length. In some embodiments, the anchor region is at least 4 nucleotides, at least six nucleotides, at least 8 nucleotides, at least 10 nucleotides, at least 12 nucleotides, at least 14 nucleotides, or at least 16 nucleotides in length. In some embodiments, the anchor region is 10 nucleotides in length.

### CAPTURE PROBE MODULES

Some embodiments of the disclosure provides compositions and methods comprising one or more capture probe modules. In some embodiments, the disclosure provides a kit comprising one or more capture probe modules.

In various embodiments, the compositions and methods of the disclosure can be used for targeted genetic analysis of one or more target genetic loci of the DNA library clones. In some embodiments, targeted genetic analysis comprises one or more of the following steps: capturing and/or isolating DNA clones comprising a target genetic locus; amplification of the captured targeted genetic locus; sequencing of the amplified captured targeted genetic locus; and bioinformatic analysis of the resulting sequence reads. As used in the disclosure, the terms "DNA library clone" refer to a DNA library fragment wherein the combination of the adaptor and the genomic DNA fragment result in a unique DNA sequence (*e.g.,* a DNA sequence that can be distinguished from that of another DNA library clone).

In some embodiments, the disclosure provides, in part, a capture probe module designed to retain the efficiency and reliability of larger probes but that minimizes uninformative sequence generation in a genomic DNA library that comprises smaller DNA fragments, e.g., a cfDNA clone library.

In some embodiments, the capture probe module comprises a tail sequence and a capture probe sequence. In some embodiments, the capture probe module comprises a first region comprising the tail sequence and a second region capable of hybridizing to a target sequence in the tagged DNA library. In some embodiments, the tail sequence comprises a PCR primer binding site. In some embodiments, the first region is referred to as the tail region or tail sequence.

As used in the disclosure, the term "tail sequence" refers to a polynucleotide at the 5' end of the capture probe module, which in some embodiments can serve as a primer binding site. In some embodiments, the tail sequence comprises a sequencing primer binding site.

In some embodiments, the tail sequence is capable of hybridizing to a partner oligonucleotide. In some embodiments, an exemplary partner oligonucleotide can be: GTGAAAACCAGGATCAACTCCCGTGCCAGTCACATCTCAGATGAGCT/3BioTEG/ (SEQ ID NO: 6). "3BioTEG" may be a Biotin-TEG label that binds to streptavidin coated magnetic beads. In some embodiments, the "3BioTEG" label may be any type of label known in the art capable of binding to a solid support.

In some embodiments, the tail sequence is about 5 to about 100 nucleotides, about 10 to about 100 nucleotides, about 5 to about 75 nucleotides, about 5 to about 50 nucleotides, about 5 to about 25 nucleotides, or about 5 to about 20 nucleotides. In some embodiments, the tail sequence is about 30 nucleotides, about 31 nucleotides, about 32 nucleotides, about 33 nucleotides, about 34 nucleotides, about 35 nucleotides, about 36 nucleotides, about 37 nucleotides, about 38 nucleotides, about 39 nucleotides, or about 40 nucleotides.

In some embodiments, the tail sequence is 5 to 100 nucleotides, 10 to 100 nucleotides, 5 to 75 nucleotides, 5 to 50 nucleotides, 5 to 25 nucleotides, or 5 to 20 nucleotides. In some embodiments, the tail sequence is 30 nucleotides, 31 nucleotides, 32 nucleotides, 33 nucleotides, 34 nucleotides, 35 nucleotides, 36 nucleotides, 37 nucleotides, 38 nucleotides, 39 nucleotides, or 40 nucleotides.

The contiguous adaptor-tagged DNA fragments (unamplified) and tagged DNA library (amplified) are each useful for a variety of sequencing-based genetic analyses including the preparation of libraries containing Hybrid Molecules enriched for one or more genetic loci of interest and may be unamplified or amplified libraries.

The unamplified adaptor-tagged DNA fragments and/or amplified tagged DNA libraries, prepared as described above, can be hybridized to capture probes modules to generate libraries targeted to specific genetic loci, *i.e.,* targeted libraries. The adaptor-tagged DNA fragments can be hybridized with one or more capture probes. Each capture probe can target the same genetic loci in the adapter-tagged DNA fragments or they may target different genetic loci in the adapter-tagged DNA fragments. In some embodiments, a plurality of genetic loci in the amplified tagged DNA library fragments are targeted.

In some embodiments, the capture probe modules are used with genomic DNA library constructed from cellular DNA. In some embodiments, the capture probe modules are used with genomic DNA library constructed from cfDNA. Because the average size of cfDNA is about 150 to about 170 bp and is highly fragmented, certain compositions and methods of the disclosure comprise the use of high density and relatively short capture probes to interrogate DNA target regions of interest. In some embodiments, the capture probes are capable of hybridizing to DNA target regions that are distributed across all chromosomal segments at a uniform density. A set of such capture probes may be referred to as "chromosomal stability probes." Chromosomal stability probes are used to interrogate copy number variations on a genome-wide scale in order to provide a genome-wide measurement of chromosomal copy number (*e.g*., chromosomal ploidy).

One particular concern with using high density capture probes is that generally capture probes are designed using specific "sequence rules." For example, in some embodiments, regions of redundant sequence or that exhibit extreme base composition biases may be excluded in designing capture probes. "Base composition", as used in the disclosure, refers to the relative amounts of various purines and pyrimidines (*e.g*., A, G, C, T) in a DNA composition. However, the lack of flexibility in capture probe design rules may not substantially impact probe performance of capture probe modules of the disclosure. In some embodiments, capture probes of the disclosure chosen strictly by positional constraint, without considering the limitations of base composition, may provide on-target sequence information; exhibit very little off-target and unmappable read capture; and yield uniform, useful, on-target reads with only few exceptions. Moreover, the high redundancy at close probe spacing may compensate for potentially poor-performing capture probes.

In some embodiments, the capture probe modules of the disclosure are configured to be used in a high-density capture probe strategy wherein a plurality of capture probes hybridize to a target region or target genetic locus, wherein each of the plurality. In some embodiments, any two or more capture probes are designed to bind to the target region within about 5 nucleotides of each other, within about10 nucleotides of each other, within about 15 nucleotides of each other, within about 20 nucleotides of each other, within about 25 nucleotides of each other, within about 30 nucleotides of each other, within about 35 nucleotides of each other, within about 40 nucleotides of each other, within about 45 nucleotides of each other, within about 50 nucleotides of each other, within about 100 nucleotides of each other, within about 200 nucleotides or more of each other, as well as all intervening nucleotide lengths.

In some embodiments, the target region comprises a target sequence to which a capture probe hybridizes. In some embodiments, the target region comprises a plurality of target sequences to which a plurality of capture probes hybridizes.

In some embodiments, the capture probe is about 20 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides, 30 nucleotides, 31 nucleotides, 32 nucleotides, 33 nucleotides, 34 nucleotides, 35 nucleotides, 36 nucleotides, 37 nucleotides, 38 nucleotides, 39 nucleotides, 40 nucleotides, 41 nucleotides, 42 nucleotides, 43 nucleotides, 44 nucleotides, 45 nucleotides, 46 nucleotides, 47 nucleotides, 48 nucleotides, 49 nucleotides, 50 nucleotides, 51 nucleotides. 52 nucleotides, 53 nucleotides, 54 nucleotides, 55 nucleotides, 56 nucleotides, 57 nucleotides, 58 nucleotides, 59 nucleotides, or 60 nucleotides.

In some embodiments, the capture probe is about 60 nucleotides. In some embodiments, the capture probe is about 65 nucleotides. In some embodiments, the capture probe is about 70 nucleotides. In some embodiments, the capture probe is about 75 nucleotides. In some embodiments, the capture probe is about 80 nucleotides. In some embodiments, the capture probe is about 85 nucleotides. In some embodiments, the capture probe is about 90 nucleotides. In some embodiments, the capture probe is about 95 nucleotides. In some embodiments, the capture probe is about 100 nucleotides, about 200 nucleotides, about 300 nucleotides, about 400 nucleotides, or about 100 nucleotides. In some embodiments, the capture probe is from about 10 nucleotides to about 20 nucleotides, about 20 nucleotides to about 60 nucleotides, about 60 nucleotides to about 100 nucleotides, about 100 nucleotides to about 500 nucleotides, about 200 nucleotides to about 500 nucleotides, about 300 nucleotides to about 500 nucleotides, or about 400 nucleotides to about 500 nucleotides, or any intervening range thereof.

In some embodiments, the capture probe is 60 nucleotides, 65 nucleotides, 70 nucleotides, 75 nucleotides, 80 nucleotides, 85 nucleotides, 90 nucleotides, 95 nucleotides, 100 nucleotides, 200 nucleotides, 300 nucleotides, 400 nucleotides, or 100 nucleotides. In some embodiments, the capture probe is from 10 nucleotides to 20 nucleotides, 20 nucleotides to 60 nucleotides, 60 nucleotides to 100 nucleotides, 100 nucleotides to 500 nucleotides, 200 nucleotides to 500 nucleotides, 300 nucleotides to 500 nucleotides, or 400 nucleotides to 500 nucleotides, or any intervening range thereof.

In some embodiments, the capture probe is substantially smaller than 60 nucleotides but hybridizes comparably, as well as, or better than a 60-nucleotide capture probe targeting the same DNA target region. In some embodiments, the capture probe is 40 nucleotides.

In various embodiments, the capture probe module comprises a specific member of a binding pair to enable isolation and/or purification of one or more captured fragments of a tagged and or amplified genomic DNA library (*e.g*., a cellular or cfDNA library) that hybridizes to the capture probe. In some embodiments, the capture probe module comprises a biotin molecule. In some embodiments, the capture probe module is conjugated to biotin or another suitable hapten, *e.g*., dinitrophenol, digoxigenin.

In some embodiments, the partner oligonucleotide comprises a specific member of a binding pair to enable isolation and/or purification of one or more captured fragments of a tagged and or amplified genomic DNA library (*e.g.,* a cellular or cfDNA library) that hybridizes to the capture probe. In some embodiments, the partner oligonucleotide comprises a biotin molecule. In some embodiments, the partner oligonucleotide is conjugated to biotin or another suitable hapten, *e.g*., dinitrophenol, digoxigenin.

In various embodiments, the capture probe is hybridized to a tagged and optionally amplified DNA library to form a complex. In some embodiments, the capture probe substantially hybridizes to a specific genomic target region or target sequence in the DNA library.

Hybridization or hybridizing conditions can include any reaction conditions where two nucleotide sequences form a stable complex; for example, in some embodiments, the adaptor-tagged DNA library fragment and capture probe forms a stable adaptor-tagged DNA library fragment-capture probe complex. Such reaction conditions are well known in the art and those of skill in the art will appreciate that such conditions can be modified as appropriate, e.g., decreased annealing temperatures with shorter length capture probes, and within the scope of the disclosure. Substantial hybridization can occur when the second region of the capture probe complex exhibits 100%, 99%, 98%, 97%, 96%, 95%, 94%, 93%, 92% 91%, 90%, 89%, 88%, 85%, 80%, 75%, or 70% sequence identity, homology or complementarity to a region of the tagged DNA library.

In some embodiments, the capture probe is about 40 nucleotides and has an optimal annealing temperature of about 44° C to about 47° C.

In some embodiments, the capture probe is 40 nucleotides and has an optimal annealing temperature of 44° C to 47° C.

In some embodiments, compositions of the disclosure are assembled into a kit comprising one or more capture probe modules. In some embodiments, kits of the disclosure may be used to generate a combined sequence-ready DNA library, where the combined library is any of the combined libraries described in the disclosure.

In some embodiments, a kit of the disclosure comprises a set of capture probe modules designed to target a specific collection of genes, *e.g.,* a set of lung cancer genes as described in Table 11. Such a set of capture probe modules may be referred to as a capture probe panel. These capture probe modules or capture probe panels may comprise any capture probe module of the disclosure. To assure high performance of the kit, each of the capture probe modules or capture probe panels may be subject to a Quality Control (QC) process prior to inclusion in the kit.

In some embodiments, for each test sample in the QC process for capture probe modules, a unique adaptor set is assigned. The QC process for capture probe modules may be performed using any of the end repair, adaptor ligation, and library amplification methods of the disclosure to generate an LPA. In some embodiments, the QC process for capture probe modules comprises performing end repair, adaptor ligation, and library amplification according to the methods of Example 11 to generate an LPA.

In some embodiments, the QC process for capture probe modules comprises creating a TC LPA and a WG LPA from an LPA. In some embodiments, the TC LPA and WG LPA are created according to the methods of Example 11. In some embodiments, a QC process on the TC LPA and WG LPA may be performed and libraries that passed QC are selected for downstream use. In some embodiments, for QC of the capture probe modules, a TC LPA of at least 1-80 ng/ µL and a WG LPA of at least 0.1-8 ng/ µL are used in downstream processes.

In some embodiments, the QC process for capture probe modules comprises using a TC LPA to create a TCL using a specific capture probe panel. In some embodiments, the QC process for capture probe modules comprises creating a TCL according to any of the methods of the disclosure. In some embodiments, the QC process for capture probe modules comprises creating a TCL according to the methods of Example 12. In some embodiments, the QC process for capture probe modules comprises creating a TCLA from a TCL according to any of the methods of the disclosure. In some embodiments, the QC process for capture probe modules comprises creating a TCLA from a TCL according to the methods of Example 12. In some embodiments, the capture probe panel tested in the QC process for capture probe modules comprises a set of Lung Probes. In some embodiments, each capture probe module within the set of Lung Probes binds to a specific target sequence in a gene associated with lung cancer. In some embodiments, each capture probe module within the set of Lung Probes binds to a specific target sequence in a gene listed in Table 11.

In some embodiments, the QC process for capture probe modules comprises using a WG LPA to create a WGLA according to any of the methods of the disclosure. In some embodiments, the QC process for capture probe modules comprises using a WG LPA to create a WGLA according to the methods of Example 13. In some embodiments, each TCLA and WGLA is required to have a concentration of at least 10 ng/µL to be used in downstream processes of the QC process for capture probe modules.

In some embodiments, the QC process for capture probe modules comprises combining the TCLA and WGLA to create a sequence-ready library. In some embodiments, the TCLA and WGLA are combined according to the methods of Example 14. In some embodiments, the QC process for capture probe modules comprises sequencing the combined TCLA and WGLA library according to any of the sequencing methods of the disclosure.

In some embodiments, one or more acceptance criteria is provided in the QC process to determine whether the capture probe modules meet the quality requirements to enable high performance of the kit.

In some embodiments, one or more acceptance criteria for the QC process for capture probe modules is selected from Table 15.

In some embodiments, only capture probe modules that have passed one or more of the QC processes of the disclosure is included in a kit of the disclosure.

In some embodiments, the QC process for capture probe modules may be performed using a positive control DNA sample. In some embodiments, the positive control DNA sample comprises a blended DNA sample of at least two different cell lines. In some embodiments, the positive control DNA sample comprises sheared genomic DNA from a blend of cell lines: 8% NA18511 in NA12878 (cell line gDNA is sourced from Coriell Institute). In some embodiments, the positive control DNA sample has passed the QC process described in Example 17. In some embodiments, a positive control DNA sample is included in a kit of the disclosure.

### LIBRARY TAG

Some embodiments of the disclosure provides Library Tags which may be used to distinguish a first modified library from a second modified library. In some embodiments, a library generated using a kit of the disclosure comprises a Library Tag. In some embodiments, the kits of the disclosure may be used to generate a combined library comprising a first modified library and a second modified library. In some embodiments, the combined library is a Sequence-Ready Library (SRL).

In some embodiments, a first modified library comprises a first Library Tag and a second modified library comprises a second Library Tag. In some embodiments, a first modified library comprises a Library Tag and a second modified library does not comprise a Library Tag. In some embodiments, a first modified library does not comprise a Library Tag and a second modified library comprises a Library Tag.

In some embodiments, the Library Tag generates a first signal and the lack of the Library Tag generates a second signal. In some embodiments, both the first and the second signals are detected or detectable. In some embodiments, the first signal and the second signal are not identical. In some embodiments, the first signal is detected or detectable and the second signal is not detected or detectable. In some embodiments, the second signal is detected or detectable and the first signal is not detected or detectable.

In some embodiments, a first Library Tag generates a first signal and a second Library Tag generates a second signal. In some embodiments, both the first and the second signals are detected or detectable. In some embodiments, the first signal and the second signal are not identical. In some embodiments, the first signal is detected or detectable and the second signal is not detected or detectable. In some embodiments, the second signal is detected or detectable and the first signal is not detected or detectable.

In some embodiments, the detected first signal and the detected second signal can be differentiated from one another. In some embodiments, the detected first signal and the non-detected second signal can be differentiated from one another. In some embodiments, the non-detected first signal and the detected second signal can be differentiated from one another.

In some embodiments, the Library Tag allows multiplexing of different libraries in the same sequencing run, as well as demultiplexing of the libraries during sequencing analysis.

In some embodiments, multiplexing of different libraries in the same sequencing run may be achieved by having the first modified library comprise a first Library Tag and the second modified library comprise a second Library Tag. In such embodiments, the first Library Tag generates a first signal and the second Library Tag generates a second signal.

In some embodiments, multiplexing of different libraries in the same sequencing run may be achieved by having only one of the first modified library and the second modified library comprise a Library Tag. In some embodiments, the Library Tag generates a first signal and the lack of the Library Tag generates a second signal.

In some embodiments, demultiplexing of libraries during sequencing analysis may be achieved by differentiating between the first signal and the second signal. In some embodiments, the detected first signal and the detected second signal can be differentiated from one another. In some embodiments, the detected first signal and the non-detected second signal can be differentiated from one another. In some embodiments, the non-detected first signal and the detected second signal can be differentiated from one another.

In some embodiments, the first modified library is generated by target capture of a DNA target using a capture probe module, wherein the capture probe module comprises the Library Tag. In some embodiments, the second modified library may be generated by amplifying or extending a DNA fragment of the second adaptor-tagged library.

In some embodiments, the Library Tag within a capture probe module may further comprise a binding site for a sequencing primer. In embodiments where a capture probe is only used to generate the first modified library and not used to generate the second modified library, the specific sequencing primer only anneals to its binding site within the first modified library and will not anneal to any target within the second modified library. In such embodiments, the lack of hybridization of the specific sequencing primer with the second modified library results in an "empty sequencing read" for this specific primer in the second modified library.

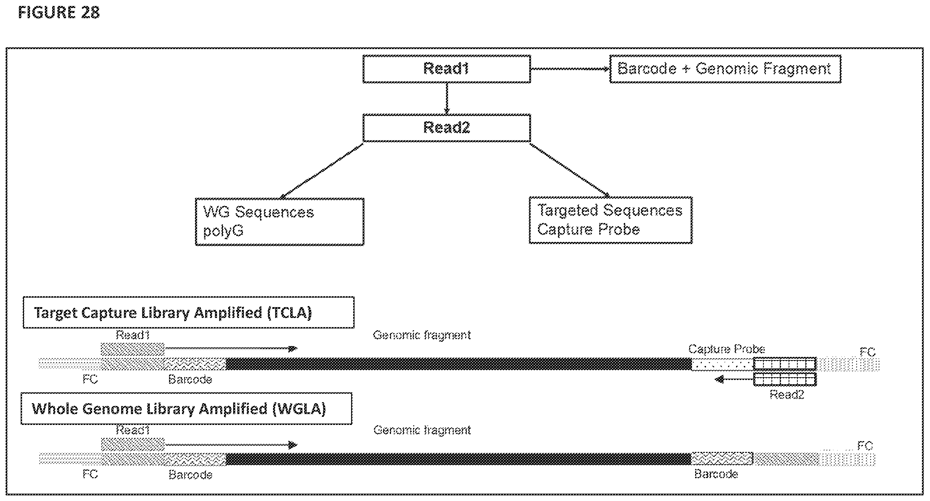

In some embodiments, the first signal is a sequencing read and the second signal is an "empty" sequencing read. In some embodiments, the first signal is detected or detectable and the second signal is not detected or detectable. In some embodiments of the methods of the disclosure, both the first signal and the second signal are detected or detectable. In some embodiments of the methods of the disclosure, the empty sequencing read (the second signal) is detected as a series of two or more G nucleotides. For example, in one embodiment as depicted in the "Target Capture Library" panel of FIGURE 28, when a DNA molecule comprising both a primer binding site for sequencing primer Read 1 and a primer binding site for sequencing primer Read 2 is sequenced using sequencing primers Read 1 and Read 2, two sequencing reads will be generated, wherein each sequencing read corresponds to at least a portion of the sequenced molecule. In one embodiment as depicted in the "Whole Genome Library" panel of FIGURE 28, when a DNA molecule comprising only a primer binding site for sequencing primer Read 1 sequence and no primer binding site for sequencing primer Read 2 is sequenced using sequencing primers Read 1 and Read 2, only sequencing primer Read 1 will generate a sequencing read corresponding to at least a portion of the sequenced molecule, while sequencing primer Read 2 will result in an "empty" sequencing read, which may be a series of two or more G nucleotides. An apparatus capable of detecting empty sequencing reads in this manner may include, for example, a sequencer using the Illumina NextSeq two-color chemistry.

In some embodiments, the first modified library is a TCL or amplified TCL and the second modified library is a WGL or WGLA. In some embodiments, the TCL or TCLA possesses a Library Tag. In some embodiments, the Library Tag is a binding domain for a custom-made "Read 2" sequencing primer. In some embodiments, the Library Tag is embedded in the tail region of the capture probe module. In some embodiments, the WGL or WGLA libraries of the disclosure do not possess a Read 2 sequencing primer binding domain, and therefore, produce no Read 2 sequencing reads. In some embodiments, while the Library Tag may designate capture probe genomic positions for a Target Capture library or a TCLA, the resulting Read 2 sequences for the LPWG libraries are a string of Gs (Guanines); this is a result of the Illumina 2-color sequencing scheme where clusters that exhibit no fluorescent signal are interpreted as G bases.

In some embodiments, the Library Tag comprises a detection label. Said detection label may comprise one or more of a fluorescent moiety, a magnetic or paramagnetic moiety, an enzymatic moiety, a binding moiety, an epitope, and a radioactive moiety. In some embodiments, the detection label is a fluorescent moiety comprising, for example, fluorescent dyes chemically attached to functional groups in biomolecules of the library. The fluorescent detection label may be visualized using fluorescence imaging methods known in the art. In some embodiments, the detection label is a magnetic or paramagnetic moiety comprising, for example, magnetic beads that are made of nanometric-sized iron oxide particles encapsulated or attached to polymers. Detection methods of said magnetic or paramagnetic moiety may include Magnetic Immunoassay (MIA) or any other method known in the art. In some embodiments, the detection label is an enzymatic moiety comprising, for example, horseradish peroxidase (HRP) and/or alkaline phosphatase (AP). Detection methods of said enzymatic moiety may include, for example, chromogenic, chemiluminescent, fluorescent methods, or any other method known in the art. In some embodiments, the detection label is a binding moiety comprising, for example, a chemosensor containing a signaling moiety and a recognition moiety, wherein the signaling moiety is capable of producing an output signal when the recognition moiety recognizes an input signal. Detection methods of said binding moiety may include, for example, optical methods based on electromagnetic radiation, electrochemical detection methods, or any other method known in the art. In some embodiments, the detection label is an epitope comprising, for example, a polypeptide that may be recognized by an antibody. Detection methods of said epitope may include, for example, ELISA methods, surface plasmon resonance (SPR) methods, or any other method known in the art. In some embodiments, the detection label is a radioactive moiety comprising, for example, a radionuclide capable of emitting gamma, beta, alpha, or multiple radiation emissions. Detection methods of said radioactive moiety may include, for example, using an ionization chamber, photographic film, or any other method known in the art.

In some embodiments, the Library Tag selectively or specifically binds to a detectable moiety. Said detection label may comprise one or more of a fluorescent moiety, a magnetic or paramagnetic moiety, an enzymatic moiety, a binding moiety, an epitope, and a radioactive moiety. In some embodiments, the detection label is a fluorescent moiety comprising, for example, fluorescent dyes chemically attached to functional groups in biomolecules of the library. The fluorescent detection label may be visualized using fluorescence imaging methods known in the art. In some embodiments, the detection label is a magnetic or paramagnetic moiety comprising, for example, magnetic beads that are made of nanometric-sized iron oxide particles encapsulated or attached to polymers. Detection methods of said magnetic or paramagnetic moiety may include Magnetic Immunoassay (MIA) or any other method known in the art. In some embodiments, the detection label is an enzymatic moiety comprising, for example, horseradish peroxidase (HRP) and/or alkaline phosphatase (AP). Detection methods of said enzymatic moiety may include, for example, chromogenic, chemiluminescent, fluorescent methods, or any other method known in the art. In some embodiments, the detection label is a binding moiety comprising, for example, a chemosensor containing a signaling moiety and a recognition moiety, wherein the signaling moiety is capable of producing an output signal when the recognition moiety recognizes an input signal. Detection methods of said binding moiety may include, for example, optical methods based on electromagnetic radiation, electrochemical detection methods, or any other method known in the art. In some embodiments, the detection label is an epitope comprising, for example, a polypeptide that may be recognized by an antibody. Detection methods of said epitope may include, for example, ELISA methods, surface plasmon resonance (SPR) methods, or any other method known in the art. In some embodiments, the detection label is a radioactive moiety comprising, for example, a radionuclide capable of emitting gamma, beta, alpha, or multiple radiation emissions. Detection methods of said radioactive moiety may include, for example, using an ionization chamber, photographic film, or any other method known in the art.

### TEST SAMPLE

The compositions and methods of the disclosure may be used on any suitable test sample comprising DNA. The kits of the disclosure may be used with any suitable test sample comprising DNA. As used in the disclosure, the term "DNA" refers to deoxyribonucleic acid. In various embodiments, the term DNA refers to any form of DNA, naturally occurring or non-naturally occurring, including, but not limited to, cfDNA, genomic DNA, recombinant DNA, synthetic DNA, cDNA, mitochondrial DNA, methylated DNA, or demethylated DNA. In some embodiments, DNA refers to genomic DNA or cfDNA (cell-free DNA). In some embodiments, DNA comprises an epigenetic mark. The epigenetic mark may be any epigenetic mark known in the art, including, but not limited to, DNA methylation.

In some embodiments, DNA comprises a DNA sequence. The DNA sequence may be any form of DNA, naturally occurring or non-naturally occurring, including, but not limited to, cfDNA, genomic DNA, recombinant DNA, synthetic DNA, cDNA, mitochondrial DNA, methylated DNA, or demethylated DNA. In some embodiments, the DNA sequence is genomic DNA or cfDNA. In some embodiments, the DNA sequence comprises an epigenetic mark. The epigenetic mark may be any epigenetic mark known in the art, including, but not limited to, DNA methylation.

In some embodiments, the DNA sequence comprises a DNA target. The DNA target may be any form of DNA, naturally occurring or non-naturally occurring, including, but not limited to, cfDNA, genomic DNA, recombinant DNA, synthetic DNA, cDNA, mitochondrial DNA, methylated DNA, or demethylated DNA. In some embodiments, the DNA target is genomic DNA or cfDNA. In some embodiments, the DNA target comprises an epigenetic mark. The epigenetic mark may be any epigenetic mark known in the art, including, but not limited to, DNA methylation.

In some embodiments, DNA libraries of the disclosure may include genomic DNA libraries, cDNA libraries constructed from RNA, *e.g.,* an RNA expression library, amplicon libraries, whole exome libraries, and methylated libraries. In various embodiments, the DNA libraries comprise one or more additional DNA sequences and/or tags.

As used in the disclosure, the terms "circulating DNA," "circulating cell-free DNA," and "cell-free DNA" may be used interchangeably and refer to DNA that is extracellular DNA, DNA that has been extruded from cells, or DNA that has been released from necrotic or apoptotic cells. This term is often used in contrast to "cellular genomic DNA" or "cellular DNA," which are used interchangeably in the disclosure and refer to genomic DNA that is contained within the cell (*i.e.,* the nuclease) and is only accessible to molecular biological techniques such as those described in the disclosure, by lysing or otherwise disrupting the integrity of the cell. In some embodiments, cfDNA is a double stranded extracellular molecule of DNA. In some embodiments, cfDNA may comprise small DNA fragments with lengths between about 70 bp and about 200 bp.

In some embodiments, the term "DNA fragment" may refer to a fragmented piece of a DNA molecule. In some embodiments, the fragmentation may occur naturally, or through a mechanical process (such as sonication). In some embodiments, the DNA fragment may be an end-repaired DNA fragment. In some embodiments, the DNA fragment may be attached to adaptor modules. In some embodiments, a DNA molecule may be a DNA fragment.

As used herein, the term "test sample" refers to the source of DNA used for genetic analysis. In some embodiments, the test sample is a tissue biopsy. In certain embodiments, the test sample is a biopsy of a tumor or a tissue suspected of being a tumor. In some embodiments, the test sample is a biopsy obtained from a malignant tumor or tumor suspected of being malignant. In some embodiments, the test sample comprises cancer cells or cells suspected of being cancerous.

In some embodiments, a DNA molecule or DNA fragment is isolated or generated from the test sample.

In some embodiments, a library may be produced from any suitable amount of DNA. In some embodiments, the amount of DNA is between about 1 pg and about 500 ng, between about 1 ng and about 400 ng, between about 5 ng and about 300 ng, between about 10 ng and about 250 ng, or between about 20 ng and about 200 ng. In some embodiments the amount of DNA is between about 5 ng and about 50 ng. In some embodiments, the amount of DNA is between 1 pg and 500 ng, between 1 ng and 400 ng, between 5 ng and 300 ng, between 10 ng and 250 ng, or between 20 ng and 200 ng. In some embodiments the amount of DNA is between 5 ng and 50 ng.

In some embodiments, the methods and compositions contemplated in the disclosure use double-stranded DNA (dsDNA), such as dsDNA that is selected from cell free DNA (cfDNA), genomic DNA (gDNA), complementary DNA (cDNA), mitochondrial DNA, methylated DNA, or demethylated DNA.

In some embodiments, genomic DNA is obtained from a tissue sample or biopsy taken from a tissue, including but not limited to, bone marrow, esophagus, stomach, duodenum, rectum, colon, ileum, pancreases, lung, liver, prostate, brain, nerves, meningeal tissue, renal tissue, endometrial tissue, cervical tissue, breast, lymph node, muscle, or skin.

Methods for purifying genomic DNA from cells or from a biologic tissue comprised of cells are well known in the art, and the skilled artisan will recognize optimal procedures or commercial kits depending on the tissue and the conditions in which the tissue is obtained. Some embodiments contemplate that purifying cellular DNA from a tissue will require cell disruption or cell lysis to expose the cellular DNA within, for example by chemical and physical methods such as blending, grinding or sonicating the tissue sample; removing membrane lipids by adding a detergent or surfactants which also serves in cell lysis, optionally removing proteins, for example by adding a protease; removing RNA, for example by adding an RNase; and DNA purification, for example from detergents, proteins, salts and reagents used during cell lysis step. DNA purification may be performed by precipitation, for example with ethanol or isopropanol; or by phenol-chloroform extraction.

In some embodiments, cellular DNA obtained from tissues and/or cells is fragmented prior to and/or during the process of obtaining, generating, making, forming, and/or producing a genomic DNA library as described in the instant disclosure. One of skill in the art will understand that there are several suitable techniques for DNA fragmentation and is able to recognize and identify suitable techniques for fragmenting cellular DNA for the purposes of generating a genomic DNA library for DNA sequencing, including, but not limited to, next-generation sequencing. Certain embodiments contemplate that cellular DNA can be fragmented into fragments of appropriate and/or sufficient length for generating a library by methods including, but not limited to, physical fragmentation, enzymatic fragmentation, and chemical shearing.

Physical fragmentation can include, but is not limited to, acoustic shearing, sonication, and hydrodynamic shear. In some embodiments, cellular DNA is fragmented by physical fragmentation. In some embodiments, cellular DNA is fragmented by acoustic shearing or sonication. Some embodiments contemplate that acoustic shearing and sonication are common physical methods used to shear cellular DNA. The Covaris^{®} instrument (Woburn, MA) is an acoustic device for breaking DNA into 100-5kb bp. The Bioruptor^{®} (Denville, NJ) is a sonication device utilized for shearing chromatin, DNA and disrupting tissues. Small volumes of DNA can be sheared to 150bp-1kb in length. Hydroshear from Digilab (Marlborough, MA) utilizes hydrodynamic forces to shear DNA. Nebulizers (Life Tech, Grand Island, NY) can also be used to atomize liquid using compressed air, shearing DNA into 100bp-3kb fragments in seconds. Nebulization is low cost, but the process can cause a loss of about 30% of the cellular DNA from the original sample. In certain embodiments, cellular DNA is fragmented by sonication.

Enzymatic fragmentation can include, but is not limited to, treatment with a restriction endonuclease, *e.g*. DNase I, or treatment with a nonspecific nuclease. In some embodiments, cellular DNA is fragmented by enzymatic fragmentation. In some embodiments, the cellular DNA is fragmented by treatment with a restriction endonuclease. In some embodiments, the cellular DNA is fragmented by treatment with a nonspecific nuclease. In certain embodiments, the cellular DNA is fragmented by treatment with a transposase. Certain embodiments contemplate that enzymatic methods to shear cellular DNA into small pieces include DNAse I, a combination of maltose binding protein (MBP)-T7 Endo I and a non-specific nuclease Vibrio vulnificus (Vvn) New England Biolabs's (Ipswich, MA) Fragmentase and Nextera tagmentation technology (Illumina, San Diego, CA). The combination of non-specific nuclease and T7 Endo synergistically work to produce non-specific nicks and counter nicks, generating fragments that disassociate 8 nucleotides or less from the nick site. Tagmentation uses a transposase to simultaneously fragment and insert adapters onto double stranded DNA.

Chemical fragmentation can include treatment with heat and divalent metal cation. In some embodiments, genomic DNA is fragmented by chemical fragmentation. Some embodiments contemplate that chemical shear is more commonly used for the breakup of long RNA fragments as opposed to genomic DNA. Chemical fragmentation is typically performed through the heat digestion of DNA with a divalent metal cation (magnesium or zinc). The length of DNA fragments can be adjusted by increasing or decreasing the time of incubation.

In some embodiments, genomic DNA may be fragmented by sonication using an ultra-sonicator (Covaris) on a suitable setting for generating 200 bp fragments.

In some embodiments, the generated fragments may be further purified and size-selected using "double-sided" bead purification with paramagnetic AMPure XP beads (Beckman).

In some embodiments, mixtures of sheared cell line DNA can be at various ratios as suitable for the purpose of the studies, and they can be blended with wildtype (WT) cfDNA from female and/or male subjects (to account for genes on X and/or Y chromosomes) to produce lab-generated samples with single nucleotide variants (SNVs) such as single gene polymorphisms (SNPs), insertions and/or deletions (Indels), gene arrangements such as translocations, fusions, inversions, duplications (copy number changes) and other variants at defined allele frequencies (AF).

In some embodiments, the methods and compositions contemplated in the disclosure use dsDNA that is obtained from a low pass whole genome library, an amplicon library, a whole exome library, a cDNA library, or a methylated DNA library.

In some embodiments, the methods and compositions contemplated in the disclosure use cell-free DNA (cfDNA) as an analyte. In some embodiments, the size distribution of cfDNA ranges from about 150 bp to about 180 bp fragments. In some embodiments, the size distribution of cfDNA ranges from 150 bp to 180 bp fragments. The small, fragmented nature of cfDNA may be the result of endonucleolytic and/or exonucleolytic activity and presents a formidable challenge to the accurate, reliable, and robust analysis of cfDNA. Another challenge for analyzing cfDNA is its short half-life in the blood stream, on the order of about 15 minutes. Without wishing to be bound to any particular theory, the disclosure contemplates, in part, that analysis of cfDNA is like a "liquid biopsy" and is a real-time snapshot of current biological processes.

Moreover, because cfDNA is not found within cells and may be obtained from a number of suitable sources including, but not limited to, biological fluids and stool samples, it is not subject to the existing limitations that plague next generation sequencing analysis of tumors, such as direct access to the tissues being analyzed.

In some embodiments, methods of genetic analysis of the disclosure comprise generating a cfDNA library comprising treating cfDNA with one or more end-repair enzymes to generate end-repaired cfDNA and ligating one or more adaptors to each end of the end-repaired cfDNA to generate the adaptor-tagged cfDNA library.

Illustrative examples of biological fluids that are suitable sources from which to isolate cfDNA in some embodiments include, but are not limited to amniotic fluid, blood, plasma, serum, semen, lymphatic fluid, cerebral spinal fluid, ocular fluid, urine, saliva, mucous, and sweat.

In some embodiments, the biological fluid is blood or blood plasma.

In certain embodiments, commercially available kits and other methods known to the skilled artisan can used to isolate cfDNA directly from the biological fluids of a subject or from a previously obtained and optionally stabilized biological sample, *e.g*., by freezing and/or addition of enzyme chelating agents including, but not limited to EDTA, EGTA, or other chelating agents specific for divalent cations.

In some embodiments, cell free DNA or genomic DNA (*e.g*. cfDNA or gDNA) isolated from immortalized cells harboring gene variants (Coriell Institute for Medical Research or SeraCare Life Sciences, Inc.) can be used for NGS library construction.

In some embodiments, cell-free DNA may be extracted from plasma samples using a QIAmp DSP Circulating NA kit (Qiagen).

In some embodiments, the kits of the disclosure comprise a Positive Control DNA sample. In some embodiments, the Positive Control sample comprises a blended DNA sample of at least two different cell lines. In some embodiments, the Positive Control sample comprises sheared genomic DNA from a blend of cell lines: 8% NA18511 in NA12878 (cell line gDNA is sourced from Coriell Institute). In some embodiments, the Positive Control DNA sample has passed the QC process described in Example 17. In some embodiments, the Positive Control DNA sample is configured to be used with the capture probe modules of the disclosure.

### Detailed Methods

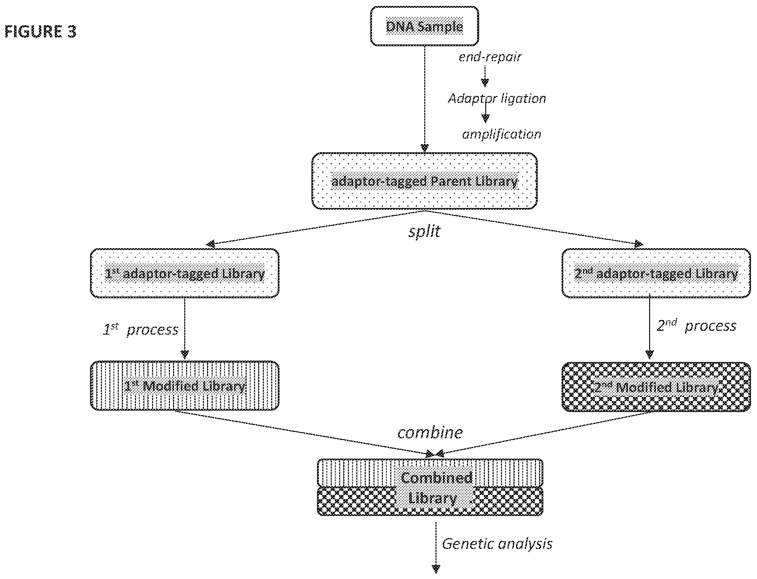

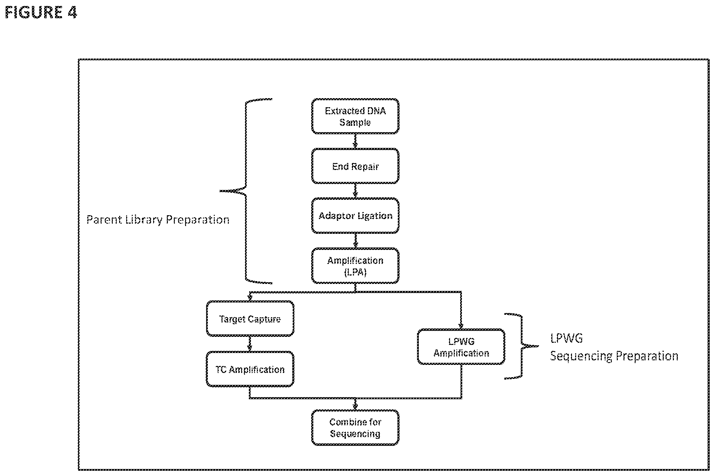

In some embodiments, the compositions, kits, and methods of the disclosure are used for targeted genetic analysis. An illustrative process of using any one of the compositions, kits, or methods of the disclosure is depicted in FIGURE 3. An illustrative workflow illustrating specific examples of using any one of the compositions, kits, or methods of is depicted in FIGURE 4 and discussed below. Some embodiments of the methods of the disclosure may also be used in the Quality Control (QC) process for generating kit components.

### DNA END-REPAIR

In some embodiments, input DNA molecules (*e.g*., cell-free DNA fragments) are converted to end-repaired DNA molecules such that the end-repaired DNA fragments possess 5' phosphate groups and 3' dA nucleotide overhangs in a single reaction mixture. A commercially available kit (*e.g.,* NEB Ultra II End Repair/dA tailing module E7546L) may be used to end repair the DNA fragments or one or more of the individual enzymes and buffers as disclosed may be combined for preparation of end-repaired DNA fragments that possess 5' phosphate groups and 3' dA nucleotide overhangs. In some embodiments, the end-repair reaction volume is lower than 50 µL.

While DNA fragments of the disclosure may be obtained in a processed form, the methods of the disclosure allow for the processing of biological samples to obtain DNA fragments that are amenable for ligation to adaptors of the disclosure. For example, in some embodiments, a processed form of a DNA fragment of the disclosure includes, but is not limited to, a DNA fragment comprising one or more of a blunted end, a blunted 3' end, a blunted 5' end, a deoxyribonucleic acid adenine (dA)-tail, a dA-tail at a 3' end, a dA-tail at a 5' end, a deoxyribonucleic acid cytosine (dC)-tail, a dC-tail at a 3' end, a dC-tail at a 5' end, a deoxyribonucleic acid guanine (dG)-tail, a dG-tail at a 3' end, a dG-tail at a 5' end, a deoxyribonucleic acid thymine (dT)-tail, a dT-tail at a 3' end, a dT-tail at a 5' end, a d(X)n tail wherein the number X is at least 2 and n may be selected from any one of A, C, G, and T, a phosphorylated nucleic acid, a phosphorylated nucleic acid at a 3' end, and a phosphorylated nucleic acid at a 5' end.

In certain embodiments, "end repair" may be performed to generate DNA fragments that are dephosphorylated, internally damage repaired, blunt ended, 5' phosphorylated, or to generate DNA fragments with 3' overhangs.

In some embodiments of the methods of the disclosure that include processing of DNA fragments, one or more of a DNA modification reaction may be performed simultaneously and/or within the same reaction vessel. In some embodiments, one or more of a DNA repair reaction to blunt an end (*e.g*., exonucleolytic cleavage or polymerase extension), an A-tailing reaction, and a phosphorylation reaction may be performed simultaneously in a single step.

In some embodiments, generating an adaptor-tagged DNA library comprises the end-repair of isolated cfDNA or fragmented cellular DNA. The fragmented cfDNA or cellular DNA is processed by end-repair enzymes to generate end-repaired cfDNA with blunt ends, 5'-overhangs, or 3'-overhangs. In some embodiments, the end-repaired cfDNA or cellular DNA contains blunt ends. In some embodiments, the end-repaired cellular DNA or cfDNA is processed to contain blunt ends. In some embodiments, the blunt ends of the end-repaired cfDNA or cellular DNA are further modified to contain a single base pair overhang. In some embodiments, end-repaired cfDNA or cellular DNA containing blunt ends can be further processed to contain adenine (A)/thymine (T) overhang. In some embodiments, end-repaired cfDNA or cellular DNA containing blunt ends can be further processed to contain adenine (A)/thymine (T) overhang as the single base pair overhang. In some embodiments, the end-repaired cfDNA or cellular DNA has non-templated 3' overhangs. In some embodiments, the end-repaired cfDNA or cellular DNA is processed to contain 3' overhangs. In some embodiments, the end-repaired cfDNA or cellular DNA is processed with terminal transferase (TdT) to contain 3' overhangs. In some embodiments, a G-tail can be added by TdT. In some embodiments, the end-repaired cfDNA or cellular DNA is processed to contain overhang ends using partial digestion with any known restriction enzymes (*e.g*., with the enzyme Sau3A, and the like).

In some embodiments, dephosphorylation of DNA fragment can be performed by thermolabile phosphatases such as alkaline phosphatases. Commercial examples include APex^{™} Heat Labile Alkaline phosphatase, NTPhos^{™} Thermolabile Phosphatase, KT^{™} Thermolabile Phosphatase and shrimp alkaline phosphatase (SAP).

In some embodiments, internal DNA damage may be repaired by one or more repair enzymes that may repair internal damage in the DNA fragments. Examples include Taq DNA ligase, Endonuclease IV, Bst DNA polymerase Fpg, Uracil-DNA Glycolase (UDG), T4 PDG, and endonuclease VIII. In some embodiments, all the foregoing enzymes may be used. A commercially available cocktail of the foregoing enzymes (*e.g*. the PreCR Enzyme kit) may be used or a cocktail may be prepared by addition of one or more the individual enzymes in any combination. In some embodiments, the DNA internal damage repair may not be performed.

In some embodiments, internal DNA damage repair, end-repair, and terminal transferase (TdT) for dA-tailing may be performed in a single step and single reaction mixture. In some embodiments, a commercially available kit such as the PreCR enzyme kit or Quick blunt kit from NEB can be used for said single step reaction.

In some embodiments, DNA end repairing may be done by use of one or more end-repair enzymes to create blunt ended DNA fragments. Said enzymes may include 3'- 5' exonuclease, 5' - 3' DNA polymerase (*e.g*. Klenow fragment), and 5' FLAP endonuclease.

In some embodiments, DNA end-repair, 5' phosphorylation, and terminal transferase (TdT) for dA-tailing may be performed in a single step and single reaction mixture to generate dsDNA fragments that are 5' phosphorylated with 3'-overhang ends, *e.g.,* 5' phosphorylated and 3'dA-tailed. In some embodiments, commercially available kits such as the Next Ultra II End repair/dA-tailing kit from NEB can be used for said single step reaction.

In some embodiments, the disclosure contemplates that appropriate amounts of fragmented DNA samples can be "single-step end-repaired", by combining into a single mixture enzymes and reagents for each of the following reactions: dephosphorylation, internal DNA damage repair , blunt end creation, 5' end phosphorylation, and 3' overhang creation. This single-step single-reaction process generates end-repaired double stranded DNA fragments having a 5' phosphorylated end and a 3' overhang. In some embodiments, the 3' overhang comprises a dA tail.

In some embodiments, the amount of DNA that can be end-repaired can be any suitable amount. In some embodiments, the amount of DNA to be end-repaired is between 1 ng and 500 ng, between 5 ng and 400 ng, between 10 ng and 300 ng, between 15 ng and 250 ng, or between 20 ng and 200 ng. In some embodiments, the amount of DNA to be end-repaired is between 20 ng and 50 ng.

In some embodiments, DNA end repair is performed according to the methods of Example 11.

### ADAPTOR LIGATION

In some embodiments, a ligation step comprises ligating an adaptor module to the end-repaired cfDNA to generate a "tagged" cfDNA library. In some embodiments, a plurality of adaptors is ligated to end-repaired dsDNA molecules from one or more samples (multiplexing), resulting in adaptor attachment to the 5' end of dsDNA molecules. In some embodiments, the ligation reaction volume is lower than 100 µL. In some embodiments, the adapter-tagged DNA fragments are isolated and washed in reaction volumes lower than 100 µL. FIGURE 5 provides an illustrative diagram of one embodiment of the adaptor ligation process.

In some embodiments, a single adaptor module is employed. In some embodiments, two, three, four or five adaptor modules are employed. In some embodiments, an adaptor module of identical sequence is ligated to each end of the fragmented end-repaired DNA.

In some embodiments, ligation of one or more adaptors of the disclosure may be carried out by methods known to those of ordinary skill in the art.

In some embodiments, one or more adaptors of the disclosure are ligated to end-repaired cfDNA that comprises blunt ends. In certain embodiments, one or more adaptors of the disclosure are ligated to end-repaired cfDNA that comprises complementary ends appropriate for the ligation method employed. In certain embodiments, one or more adaptors of the disclosure are ligated to end-repaired cfDNA that comprises a 3' overhang.

In some embodiments, the adapters and DNA fragments can be mixed with ligation buffer, reagents and ligation enzymes such as DNA ligases (*e.g.* T4 ligase or Taq ligase) and/or RNA ligases. Such ligases can be used for ligating the ligation strand as described above to the 5' end of the DNA fragment.

In some embodiments, the ligation strand of the adapter ligates to the 5' end of the dsDNA fragment in a single step via the complementation of the 3' terminal overhang of the ligation strand and the 3' overhang of the DNA fragment, while the non-ligation strand remains unattached to the 3' end of the DNA fragment.

In some embodiments, a ligation step comprises ligating an adaptor to a dsDNA fragment to generate an adaptor/dsDNA fragment complex. In some embodiments, a single adaptor is employed. In some embodiments, two, three, four or five adaptors are employed. In some embodiments, an adaptor module of identical sequence is attached to each end of the fragmented end-repaired DNA.

In some embodiments, the same adaptor is attached to both ends of the DNA fragment. In some embodiments, a different adaptor is attached to each end of the dsDNA fragment.

In some embodiments, a ligation step comprises:
(a) ligating a plurality of adaptors with a plurality of dsDNA fragments to generate a plurality of adaptor/dsDNA complexes, wherein the adaptor is any one of the adaptors in the disclosure;
(b) contacting the adaptor/DNA complexes from step (a) with one or more enzymes to form an adaptor-tagged DNA library comprising a plurality of contiguous adaptor-tagged DNA fragments.

In some embodiments, the adaptor/DNA complexes in step (b) is made into contiguous double stranded adaptor-tagged DNA fragments by DNA polymerase extension using the ligation strand as template.

In some embodiments, the unattached non-ligation strand is disassociated from the ligation strand by 5'-3' polymerase extension of the DNA fragment using the ligation strand as template. In some embodiments, the non-ligation strand may optionally comprise a modification at its 3' terminus that prevents ligation to the 5' end of the dsDNA fragment and/or adaptor dimer formation.

In some embodiments, the non-ligation strand is ligated to the 3' end of the DNA fragment by DNA polymerase nick-repair (nick translation), using the ligation strand as template and a DNA ligase for nick closure.

In some embodiments, the dsDNA fragment is cell free DNA (cfDNA), genomic DNA (gDNA), complementary DNA (cDNA), mitochondrial DNA, or methylated DNA, or demethylated DNA.

In some embodiments, the plurality of dsDNA fragments is end-repaired prior to ligating with a plurality of adaptors.

In some embodiments, the plurality of dsDNA fragments is obtained from a library selected from the list consisting of a low pass whole genome library, an amplicon library, a whole exome library, a cDNA library, or a methylated DNA library.

In some embodiments, the plurality of dsDNA fragments comprises cell free DNA (cfDNA), genomic DNA (gDNA), complementary DNA (cDNA), mitochondrial DNA, methylated DNA, or demethylated DNA.

In some embodiments, the plurality of dsDNA fragments is isolated or generated from the test sample; and wherein the test sample comprises a biological sample selected from the group consisting of an amniotic fluid sample, a blood sample, a skin sample, a hair sample, a hair follicle sample, a saliva sample, a mucous sample, a sweat sample, a tear sample, an epithelial tissue sample, a urine sample, a semen sample, a seminal plasma sample, a serum sample, a prostatic fluid sample, a pre-ejaculatory fluid (Cowper's fluid) sample, an ocular fluid sample, an excreta sample, a biopsy sample, an ascites sample, a cerebrospinal fluid sample, a lymph sample, a tissue extract sample, a stool sample, and a formalin-fixed, paraffin embedded (FFPE) sample.

In some embodiments, the plurality of dsDNA fragments is obtained from a library selected from the list consisting of a whole genome library, an amplicon library, a whole exome library, a cDNA library, or a methylated DNA library.

In some embodiments, the non-ligation strand oligonucleotide comprises a modification at its 3' terminus that prevents ligation to the 5' end of the dsDNA fragment and/or adaptor dimer formation

In some embodiments, the plurality of dsDNA fragments are damage-repaired prior to step (c) in the kinase/ligase ligation method.

In some embodiments, the DNA damage is a deaminated cytosine (Uracil), an abasic site, methylation of guanine to O6MeG, one or more DNA nicks, one or more DNA gaps, or a thymine dimer.

In some embodiments, the non-ligation strand oligonucleotide is shorter than the ligation strand oligonucleotide and comprises an anchor region that is at least partially complementary to the anchor region of the ligation strand oligonucleotide. In some embodiments, the non-ligation strand oligonucleotide is shorter than 10 bp. In some embodiments, the ligation strand oligonucleotide is 8 bp.

In some embodiments, the adaptor ligation period can be any period suitable for ligation. In some embodiments, the period is at least about 5 minutes. In some embodiments, the period is between about 5 minutes and about 72 hours. In some embodiments, the period is between about 5 minutes and about 2 hours. In some embodiments, the ligation period is less than about 1 hour, less than about 30 minutes, less than about 15 minutes, or less than about 10 minutes.

In some embodiments, the adaptor ligation period can be any period suitable for ligation. In some embodiments, the period is at least 5 minutes. In some embodiments, the period is between 5 minutes and 72 hours. In some embodiments, the period is between 5 minutes and 2 hours. In some embodiments, the ligation period is less than 1 hour, less than 30 minutes, less than 15 minutes, or less than 10 minutes.

In some embodiments, the adaptor ligation volume is one that is suitable for automation and for sample handling robotics. In some embodiments, the reaction volume is between about 1 µL and about 1000 µL, between about 1 µL and about 350 µL, between about 1 µL and about 200µL, between about 1 µL and about 100 µL, between about 1 µL and about 50 µL, is between about 5 µL and about 25 µL, between about 10 µL and about 40 µL, between about 20 µL and about 40 µL. In some embodiments, the reaction volume is about 100 µL. In one embodiment, the volume is about 30 µL.

In some embodiments, the adaptor ligation volume is one that is suitable for automation and for sample handling robotics. In some embodiments, the reaction volume is between 1 µL and 1000 µL, between 1 µL and 350 µL, between 1 µL and 200µL, between 1 µL and 100 µL, between 1 µL and 50 µL, is between 5 µL and 25 µL, between 10 µL and 40 µL, between 20 µL and 40 µL. In some embodiments, the reaction volume is 100 µL. In one embodiment, the volume is 30 µL.

In some embodiments, the, adaptor ligation can be done in strips of tubes or in microtiter plate wells or any other format suitable to allow for automated and/or high throughput processing.

In some embodiments, the adaptor amount can be any suitable concentration. In some embodiments, the concentration of adapters is at least 0.01 µM. In some embodiments, the concentration of adapters is between about 0.01 µM and about 200 µM, between about 0.01 µM and about 50 µM, between about 0.1 µM and about 50 µM, between about 0.2 µM and about 20 µM, between about 0.2 µM and about 10 µM, between about 1 µM and about 10 µM, or between about 2 µM and about 8 µM. In some embodiments, the adaptor concentration is at least about 2 µM. In some embodiments, the adaptor concentration is about 5 µM.

In some embodiments, the concentration of adapters is between 0.01 µM and 200 µM, between 0.01 µM and 50 µM, between 0.1 µM and 50 µM, between 0.2 µM and 20 µM, between 0.2 µM and 10 µM, between 1 µM and 10 µM, or between 2 µM and 8 µM. In some embodiments, the adaptor concentration is at least 2 µM. In some embodiments, the adaptor concentration is 5 µM.

In some embodiments, the ligation reaction mixture can be incubated at temperatures between about 10 °C and about 30 °C. In some embodiments, the ligation reaction mixture can be incubated at about 20°C.

In some embodiments, the ligation reaction mixture can be incubated at temperatures between 10 °C and 30 °C. In some embodiments, the ligation reaction mixture can be incubated at 20°C.

In some embodiments, following ligation, adapter-tagged DNA molecules can be isolated and washed. This can be done using DNA purification beads such as Ampure XP (Beckman) or Spectra mix such that Adapter-DNA molecules remain attached to the beads and contaminating materials are washed away. The eluted clarified supernatant contains an isolated library comprising a plurality of adaptor-tagged DNA fragments. The supernatant containing the library can be transferred to a fresh PCR tube or microliter plate well for amplification.

In some embodiments, adaptors of the disclosure is ligated according to the methods of Example 11.

In some embodiments, the adaptors may be ligated to dsDNA fragments using a kinase/ligase ligation method. Said kinase/ligase ligation method comprises the steps of: (a) treating dsDNA fragments with one or more end-repair enzymes to generate end-repaired dsDNA fragments; (b) ligating the ligation strand of a double-stranded DNA pre-adaptor to the 3' end of each strand of the end-repaired DNA fragments to form pre-adaptor/end-repaired DNA complexes; (c) displacing the non-ligation strand oligonucleotide from the pre-adaptor/end-repaired DNA complexes with a repair oligonucleotide; (d) ligating the repair oligonucleotide to the 5' end of the end-repaired DNA fragments using a kinase/ligase strategy to form adaptor-tagged DNA complexes; (e) treating the adaptor/end-repaired DNA complexes with one or more enzymes to form an adaptor-tagged library comprising one or more contiguous adaptor-tagged dsDNA fragments, wherein each contiguous adaptor-tagged dsDNA fragment comprises an adaptor ligated to each end of the dsDNA fragment; and, optionally, (f) amplifying the adaptor-tagged library of step (e) to generate an amplified adaptor-tagged library comprising one or more amplified contiguous adaptor-tagged dsDNA fragments.

In some embodiments where the kinase/ligase strategy is used, a partially double-stranded DNA pre-adaptor structure is coupled to the DNA molecule prior to ligation to form a pre-adaptor/DNA complex. Said dsDNA pre-adaptor structure comprises a ligation strand oligonucleotide and a non-ligation strand oligonucleotide.

In some embodiments, the non-ligation strand oligonucleotide in the pre-adaptor structure disassociates from the pre-adaptor/DNA complex and is replaced by a repair oligonucleotide. In some embodiments, the repair oligonucleotide comprises an anchor region that is at least partially complementary to the anchor region of the ligation strand oligonucleotide. In certain embodiments, the repair oligonucleotide further comprises an adaptor module and is at least partially complementary to the 5' region of the ligation strand oligonucleotide.

In some embodiments, each of the ligation strand oligonucleotide and the repair oligonucleotide further comprises a PCR primer binding site, wherein the PCR primer binding site of the ligation strand oligonucleotide is not complementary to the PCR primer binding site of the repair oligonucleotide. In certain embodiments, a primer that binds the PCR primer binding site of the ligation strand oligonucleotide does not substantially bind the PCR primer binding site of the repair oligonucleotide.

In some embodiments, attaching the genomic DNA fragments to a plurality of adaptors includes the steps of attaching the end repaired cfDNA or cellular DNA fragments to an oligonucleotide containing at least a portion of an anchor region. In some embodiments, the oligonucleotide contains the whole anchor region. In some embodiments, the oligonucleotide is a DNA duplex comprising a 5' phosphorylated ligation strand hybridized to a non-ligation strand, wherein the non-ligation strand is blocked from attachment by chemical modification at its 3' end, and wherein the ligation strand is attached to the genomic DNA fragment. In certain embodiments, the DNA fragments attached with at least a portion of the anchor region are then annealed with DNA oligonucleotides encoding the full-length adaptor sequences. In some embodiments, one or more polynucleotide kinases, one or more DNA ligases, and/or one or more DNA polymerases are added to the genomic DNA fragments and the DNA oligonucleotides encoding the full-length adaptor sequence. In some embodiments, the polynucleotide kinase is T4 polynucleotide kinase. In some embodiments, the DNA ligase is Taq DNA ligase. In certain embodiments, the DNA polymerase is Taq polymerase. In some embodiments, the DNA polymerase is full length Bst polymerase.

In some embodiments, treating dsDNA fragments with one or more end-repair enzymes comprises removing the terminal phosphate residues from the dsDNA fragments.

In some embodiments, the dsDNA pre-adaptor is selected from a plurality of dsDNA pre-adaptors.

In some embodiments, the repair oligonucleotide comprises an anchor region that is at least partially complementary to the anchor region of the ligation strand oligonucleotide. In some embodiments, the repair oligonucleotide further comprises an adaptor module and is at least partially complementary to the 5' region of the ligation strand oligonucleotide. In some embodiments, the repair oligonucleotide is longer than the ligation strand oligonucleotide and comprises an anchor region that is at least partially complementary to the anchor region of the ligation strand oligonucleotide, an ID region , and a PCR primer binding site.

In some embodiments, the ligation strand oligonucleotide is shorter than the repair oligonucleotide and comprises an anchor region that is at least partially complementary to the anchor region of the repair strand oligonucleotide. In some embodiments, the ligation strand oligonucleotide is shorter than 15 bp. In some embodiments, the ligation strand oligonucleotide is 10 bp.

In some embodiments, each of the ligation strand oligonucleotide and the repair oligonucleotide further comprises a PCR primer binding site, and wherein the PCR primer binding site of the ligation strand oligonucleotide is not complementary to the PCR primer binding site of the repair oligonucleotide. In some embodiments, a primer that binds the PCR primer binding site of the ligation strand oligonucleotide does not substantially bind the PCR primer binding site of the repair oligonucleotide.

In some embodiments, the kinase/ligase strategy of step (e) comprises adding a phosphate group to the 5' terminal nucleotide of each strand of the end-repaired DNA fragments and ligating the repair oligonucleotide to the phosphorylated 5' end of each strand of the end-repaired DNA fragments. In some embodiments, the kinase/ligase strategy does not comprise removal of the 5' terminal nucleotide of the end-repaired DNA fragment.

In some embodiments, step (c) of the kinase/ligase ligation method is performed at a first temperature and step (d) is performed at a second temperature, wherein the second temperature is higher than the first temperature and results in the displacement of the non-ligation strand oligonucleotide from the ligation strand oligonucleotide. In some embodiments, the first temperature is 22° C or lower and the second temperature is 37° C or higher.

In some embodiments, one or more primers are used for amplification in step (g) of the kinase ligation method. In some embodiments, the one or more primers comprise a universal primer binding sequence that hybridizes to the primer recognition site of the adaptor module.

In some embodiments, each dsDNA pre-adaptor comprises: (1) a ligation strand oligonucleotide comprising an adaptor module, and (2) a non-ligation strand oligonucleotide that is capable of hybridizing to a region at the 5' end of the ligation strand oligonucleotide and forming a duplex therewith, wherein the anchor region of the ligation strand oligonucleotide comprises a polynucleotide sequence that is at least partially complementary to the non-ligation strand oligonucleotide.

### DNA LIBRARY AMPLIFICATION

In some embodiments, an Adaptor-tagged Parent Library is generated. FIGURE 5 depicts one embodiment of the process of generating the Adaptor-tagged Parent Library.

In some embodiments, 3' dA-tailed DNA molecules are extended from the 3' end of the DNA fragment using the ligation strand that is attached to the 5' end of the DNA fragment as template to make contiguous adapter-tagged dsDNA fragments that are suitable for amplification, and the non-ligation strand is dissociated from the ligation strand. In some embodiments, said extension of the 3' end of the DNA fragment may occur simultaneously with PCR amplification. The collection of "contiguous adapter-tagged dsDNA fragments" is the adaptor-tagged DNA library. In some embodiments, the extension reaction volume is lower than 100 µL.

In some embodiments, the Adaptor-tagged Parent Library is the adaptor-tagged DNA library.

In some embodiments, the adapter-tagged DNA library is PCR amplified with a primer which recognizes the primer binding site in the amplification region of the adapter, resulting in an amplified adaptor-tagged DNA library. The amplified adaptor-tagged DNA library may also be referred to as Library Post-Amplification (LPA) or amplified library (LIB AMP). In the disclosure, "amplified adaptor-tagged-DNA library", "amplified tagged DNA library" and "LIB AMP" may be used interchangeably.

In some embodiments, the amplification reaction volume is lower than 100 µL. In some embodiments, the amplified tagged DNA library is further isolated and washed in volumes lower than 100 µL. In some embodiments, the isolated or purified LIB AMP is referred to as Target Capture Library Post-Amplification (TC LPA).

In some embodiments, the Adaptor-tagged Parent Library is the amplified adaptor-tagged DNA library. In some embodiments, the Adaptor-tagged Parent Library is the TC LPA.

In some embodiments of the methods disclosed, the 5'-3' extension of the dsDNA fragments is first performed to generate contiguous adapter-tagged dsDNA fragments, then said contiguous adaptor-tagged dsDNA fragments are amplified to generate an adapter-tagged DNA library comprising a plurality of contiguous adaptor-tagged dsDNA fragments.

In some embodiments, the first step of 5'-3'extension of the dsDNA fragments forming contiguous adapter-tagged dsDNA fragments and the second step of amplifying said contiguous adapter-tagged dsDNA fragments is combined, in order to generate the adaptor-tagged DNA library in a single step. The adapter-tagged dsDNA fragments in the DNA library may be flanked by adapters on both ends comprising the same amplification regions, wherein the sequences in the amplification region can function as amplification primer binding sites recognizable by a single amplification primer, such as a PCR amplification primer.

In some embodiments, the unattached non-ligation strand disassociates from the ligation strand by 5'-3'polymerase extension of the DNA fragment using the ligation strand as template. In some embodiments, the non-ligation strand may optionally comprise a modification at its 3' terminus that prevents ligation to the 5' end of the dsDNA fragment and/or adaptor dimer formation.

In certain embodiments, the non-ligation strand is ligated to the 3' end of the DNA fragment by DNA polymerase nick-repair, using the ligation strand as template and a DNA ligase for nick closure.

In some embodiments, methods of the disclosure comprise amplification of a DNA library to generate a DNA clone library or a library of DNA clones. Each molecule of the DNA library may comprise an adaptor ligated to each end of an end-repaired DNA, and each adaptor comprises one or more PCR primer binding sites. In one embodiment, different adaptors are ligated to different ends of the end-repaired DNA.

In one embodiment, the same adaptor is ligated to both ends of the DNA. Ligation of the same adaptor to both ends of end-repaired DNA allows for PCR amplification with a single primer sequence. In some embodiments, a portion of the adaptor-ligated DNA library can be amplified using standard PCR techniques with a single primer sequence driving amplification. In one embodiment, the single primer sequence is about 25 nucleotides, optionally with a projected Tm of ≥ 55° C under standard ionic strength conditions.

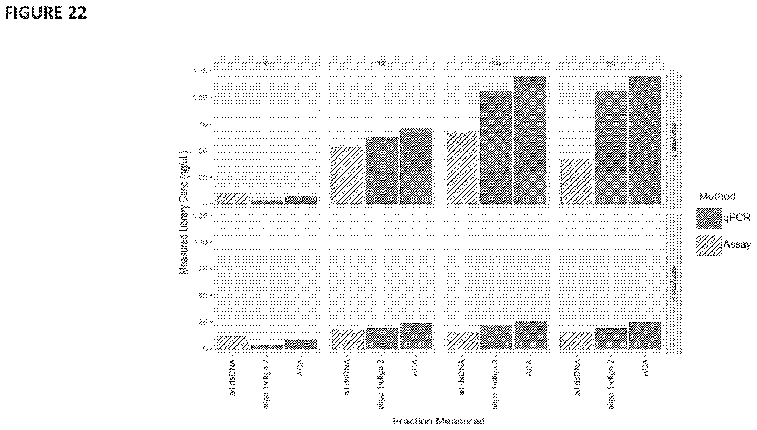

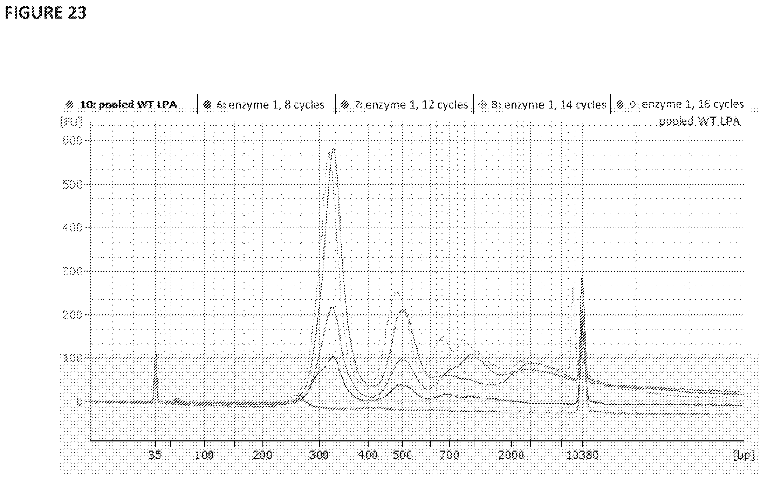

In some embodiments, a DNA polymerase is used. The DNA polymerase may be thermophilic for PCR or thermostatic/isothermal amplification. In some embodiments, a master mix (MM) containing reagents and the enzymes for the 5'-3' extension and enzymes for the subsequent amplification are combined. Commercially available enzymes and reagent kits for such extension and amplification include, for example, NEB Ultra II 2X PCR Amplification kit (New England Biolabs), Hi-Fidelity Q5 enzyme PCR (NEB), KAPA (Roche), KAPA 2X (Roche), TruSeq Nano (thermofisher), and AmpliTaq (Thermofisher). In FIGURE 22 and FIGURE 23, Enzyme 1 may refer to a KAPA enzyme, and Enzyme 2 may refer to a Hi-Fidelity Q5 enzyme.

In some embodiments, a portion of the adaptor-tagged DNA library will be amplified using standard PCR techniques with a single primer sequence driving amplification. In one embodiment, the single primer sequence is about 25 nucleotides, optionally with a projected Tm of ≥ 55° C under standard ionic strength conditions. In some embodiments, the single amplification primer is complementary to a sequence within the amplification region of the adapter module. In one embodiment, the single amplification primer comprises a sequence of TGCAGGACCAGAGAATTCGAATACA (SEQ ID NO: 5).

In some embodiments, amplification can be performed by any amplification known in the art, such as for PCR (polymerase chain reaction), LAMP (loop-mediated isothermal amplification), NASBA (nucleic acid sequence-based amplification), SDA (strand displacement amplification), RCA (rolling circle amplification), LCR (ligase chain reaction).

In some embodiments, during amplification some amplicons will form a stem-loop structures due to adaptors being ligated to both ends of the fragments. This strategy is efficient in preventing very short products (*e.g*., primer dimers) from being amplified and biasing the resulting library.

In some embodiments, an initial 3 min incubation cycle is performed to form a plurality of contiguous adapter-tagged dsDNA fragments by ligation strand templated extension.

In some embodiments, PCR amplification is performed on the plurality of contiguous adapter-tagged dsDNA fragments to form an amplified tagged DNA library.

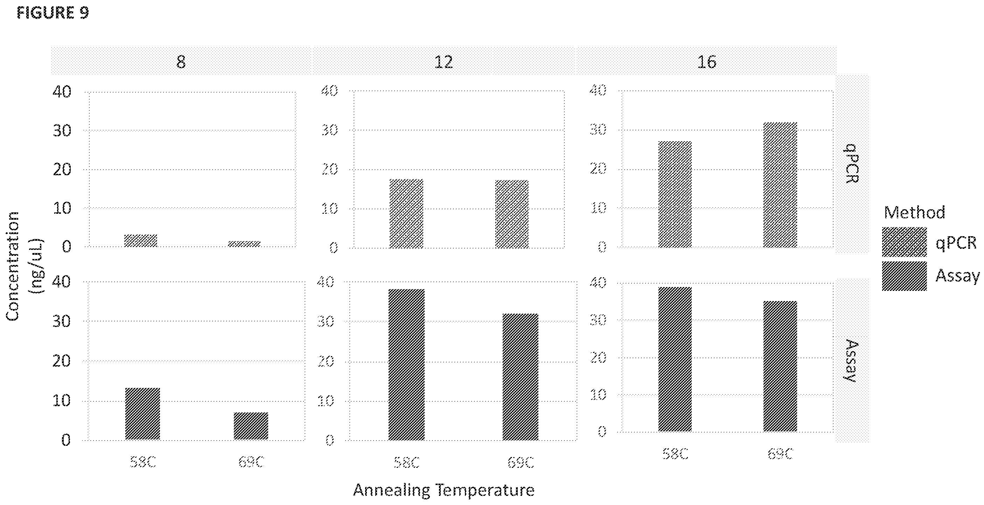

In some embodiments, picograms of the plurality of contiguous adapter-tagged dsDNA fragments is amplified into micrograms of DNA clones (adaptor-tagged DNA library), implying a 10,000-fold amplification. The amount of amplified product can be measured using methods known in the art, e.g., quantification on a Qubit 2.0 or Nanodrop instrument. In some embodiments, the amount of the amplified library can be measured by a standard assay used to determine concentration of DNA (Assay), such as an assay using Qubit 2.0. The "Assay" in FIGURES 9, 10, 11, 14, and 15 may refer to such a standard assay. In some embodiments, a commercially available kit is used for quantification, e.g., the Qubit^{™} dsDNA HS Assay Kit (Invitrogen^{™}).

In some embodiments, the adaptor-tagged library is amplified according to the methods of Example 11.

In some embodiments, the amplified adaptor-tagged DNA library can be isolated by use of DNA purification beads and washed with wash buffers, e.g., Tris-EDTA buffer (TEZ) pH 8.0. Clarified supernatant can be transferred to a fresh PCR tube or microtiter plate well or any other format suitable for automation and/or high throughput.

In general, it is preferable to use as few PCR cycles as possible to amplify libraries. In addition to reducing workflow time, this also limits the risk of introducing bias during PCR. In some embodiments, the methods of the disclosure may increase efficiency in end-repair and adaptor ligation, such that fewer PCR cycles are required to achieve the library yields necessary for sequencing or other intermediate downstream workflows. The workflow and processes disclosed may provide advantages such as: reduced turnaround time, reduced number of reagents, fewer instruments/machines used, and reduced expenses.

These above processes, adapters, and tagged DNA libraries can be used for making capture probe libraries that are enriched for genetic loci of interest present in any test sample.

In some embodiments, DNA library amplification is performed according to the methods of Example 11.

### GENERATING A 1^{ST} MODIFIED LIBRARY AND A 2^{ND} MODIFIED LIBRARY

In some embodiments, the Adaptor-tagged Parent Library is split into a first adaptor-tagged library and a second adaptor-tagged library. In some embodiments, the Adaptor-tagged Parent Library is split into more than two adaptor-tagged Libraries. In some embodiments, the first adaptor-tagged library is the TC LPA (Target Capture Library Post-Amplification). In some embodiments, the TC LPA is used to generate the second adaptor-tagged library. In some embodiments, the second adaptor-tagged library is generated by creating a 10-fold dilution of the TC LPA. In some embodiments, the second adaptor-tagged library is referred to as the Whole Genome Library Post-Amplification (WG LPA).

In some embodiments, a capture probe comprises a first region hybridized at least partially to a biotinylated partner oligonucleotide and a second region comprising a sequence having complementarity to a specific region in the 1^{st} adaptor-tagged library. The second region of the capture probe may be hybridized to a specific target region in the 1^{st} Adaptor-tagged library to form one or more capture probe/adaptor-tagged DNA complexes. In some embodiments, the target region is a genomic region of a DNA molecule in the adaptor-tagged library and does not include a region within the adaptors attached to said DNA molecule. In some embodiments, the capture probe's first region comprises a Library Tag.

In some embodiments, the capture probe/adaptor-tagged DNA complexes are separated from un-hybridized fragments using magnetic streptavidin-beads.

In some embodiments, the capture probe/adaptor-tagged DNA complexes are attached to beads. In some embodiments, bead-supported capture probes in the complex are extended from the 3' end using the tagged DNA fragments as templates, creating adaptor-tagged Hybrid Molecules (Hybrid Molecules), wherein each Hybrid Molecule comprises the capture probe and a complement of the adaptor-tagged DNA fragment that is 3' from where the capture probe hybridized to the targeted DNA sequence.

In some embodiments, the capture probes in the separated capture probe/ adaptor-tagged DNA complexes are extended from the 3' end using the tagged DNA fragments as templates, creating adaptor-tagged Hybrid Molecules (Hybrid Molecules), wherein each Hybrid Molecule comprises the capture probe and a complement of the adaptor-tagged DNA fragment that is 3' from where the capture probe hybridized to the targeted DNA sequence.

In some embodiments, Hybrid Molecules are released from beads using any suitable method known in the art. In some embodiments, thermal denaturation releases the Hybrid Molecules from the magnetic beads into the solution. In some embodiments, an elution buffer is added to release the Hybrid Molecules from the magnetic beads into the solution. The collection of released Hybrid Molecules may be referred to as the Target Capture Library (TCL).

In some embodiments, the 1^{st} modified library is the TCL or TCLA.

In some embodiments, a Forward Primer (FP) comprising a 5' oligonucleotide (A1) hybridizes to the primer binding site in the amplification region of the adapter module within the Hybrid Molecules and extends 5' -> 3' the capture probe using the Hybrid Molecule as template to make a contiguous double stranded Hybrid Molecule.

In some embodiments, the FP-extended strand in the contiguous double stranded Hybrid Molecule is denatured and a Reverse Primer (RP) comprising a 3' oligonucleotide (A2) hybridizes to the denatured FP-extended molecule/strand at the incorporated capture probe first region in the Hybrid Molecule. In some embodiments, the Library Tag comprises the primer binding site for the RP.

In some embodiments, the RP extends 5' -> 3' using the Hybrid Molecule as template to make an amplified double stranded Hybrid Molecule that is ready for Illumina sequencing or sequencing by any other known methods in the art.

In some embodiments, each end of the amplified Hybrid Molecule includes a sequencing adaptor, such as a P5 or P7 sequence, or any other sequencing adaptor. In some embodiments, the sequencing adaptor comprises a sequencing primer binding site.

In some embodiments, the collection of amplified Hybrid Molecules is referred to as the amplified Target Capture Library or Target Capture Library Amplified (TCLA).

In some embodiments, the amplification reaction volume is lower than 100 µL. In some embodiments, the amplified Hybrid Molecules are isolated and washed.

In some embodiments, the 1^{st} modified library is the amplified Target Capture Library.

FIGURE 6 depicts a schematic process of one embodiment of generating the Target Capture Library.

In certain embodiments, the methods of the disclosure comprise isolating a tagged cfDNA library-capture probe complex. In some embodiments, methods for isolating DNA complexes are well known to those skilled in the art and any methods deemed appropriate by one of skill in the art can be employed with the methods of the disclosure (Ausubel et al., Current Protocols in Molecular Biology, 2007-2012). In some embodiments, the complexes are isolated using biotin-streptavidin isolation techniques.

In some embodiments, the methods of the disclosure comprise (a) contacting the adaptor-tagged library with one or more capture probes under conditions suitable for hybridization to form one or more capture probe/adaptor-tagged DNA complexes, wherein each capture probe comprises: a first region comprising a primer binding site; and a second region capable of hybridizing to a target region in a genetic locus of the adaptor-tagged library; (b) isolating the one or more capture probe/adaptor-tagged DNA complexes from step (a), wherein each isolated capture probe/adaptor-tagged DNA complex comprises a capture probe and an adaptor-tagged DNA molecule; and (c) enzymatically processing the one or more isolated capture probe/adaptor-tagged DNA complexes from step b) to generate one or more adaptor-tagged Hybrid Molecules (Hybrid Molecules), wherein each Hybrid Molecule comprises at least a portion of the capture probe or a complement thereof and at least a portion of the adaptor-tagged DNA molecule or a complement thereof.

In some embodiments, the enzymatic processing of step c) comprises performing 3'-5' exonuclease enzymatic processing on the one or more capture probe-adaptor-tagged DNA complexes from b) using an enzyme with 3'-5' exonuclease activity to remove the single stranded 3' ends.

A variety of enzymes can be employed for the 3'-5' exonuclease enzymatic processing of the isolated tagged DNA library-capture probe complex. Illustrative examples of suitable enzymes, which exhibit 3'-5' exonuclease enzymatic activity, that can be employed in some embodiments include, but are not limited to: T4 or Exonucleases I, III, V (*See also,* Shevelev IV, Hübscher U., Nat Rev Mol Cell Biol. 3(5):364-76 (2002)). In some embodiments, the enzyme comprising 3'-5' exonuclease activity is T4 polymerase. In some embodiments, an enzyme which exhibits 3'-5' exonuclease enzymatic activity and is capable of primer template extension can be employed, including, for example, T4 or Exonucleases I, III, V. *Id.*

In some embodiments, removal of the single stranded 3'-ends from the isolated capture probe/adaptor-tagged DNA complexes is contemplated. In certain embodiments, the methods comprise 3'-5' exonuclease enzymatic processing of the isolated tagged DNA library-capture probe complex to remove the single stranded 3' ends.

In some embodiments, the methods of the disclosure comprise performing sequencing and/or PCR on the 3'-5' exonuclease enzymatically processed complex discussed *supra* and elsewhere in the disclosure. In some embodiments, a tail portion of a capture probe molecule is copied in order to generate a Hybrid Molecule. In one embodiment, the Hybrid Molecule generated comprises the target region capable of hybridizing to the capture probe and the complement of the capture probe tail sequence.

In certain embodiments, the enzymatic processing step c) comprises performing 5'-3' DNA polymerase extension of the capture probe utilizing the adaptor-tagged DNA molecule as template.

Enzymes that are suitable for the 5'-3' DNA polymerase extension process can be any thermophilic, thermostable DNA polymerase. Examples of commercially available DNA polymerases include, but are not limited to, high fidelity Q5 DNA polymerase (NEB), NEBNext Ultra PCR, NEBNext Ultra II PCR (NEB), and KAPA 2X (Roche).

In one embodiment, the enzymatic processing of step c) comprises creating one or more hybrid capture probe- adaptor-tagged DNA molecules through the concerted action of a 5' FLAP endonuclease, DNA polymerization and nick closure by a DNA ligase.

In certain embodiments, the methods comprise creating a hybrid capture probe-adaptor-tagged DNA target molecule, *e.g.,* a tagged cfDNA target molecule or a tagged cellular DNA target molecule, through the concerted action of a 5' FLAP endonuclease, DNA polymerization and nick closure by a DNA ligase.

In a particular embodiment, genetic analysis comprises a) hybridizing one or more capture probes to one or more target genetic loci in a plurality of adaptor-tagged DNA molecules to form one or more capture probe- adaptor-tagged DNA complexes; b) isolating the one or more capture probe- adaptor-tagged DNA complexes from a); c) enzymatically processing the one or more isolated capture probe- adaptor-tagged DNA complexes from step b); d) performing PCR on the enzymatically processed complex from c) to generate amplified Hybrid Molecules, wherein each Hybrid Molecule comprises at least a portion of the capture probe or a complement thereof and at least a portion of the adaptor-tagged DNA molecule or a complement thereof; and e) performing quantitative genetic analysis on the amplified Hybrid Molecules from d).

In some embodiments, PCR can be performed using any standard PCR reaction conditions well known to those of skill in the art. In certain embodiments, the PCR reaction in d) employs two PCR primers. In some embodiments, the PCR reaction in d) employs a first PCR primer that hybridizes to a region within the adaptor sequence of the Hybrid Molecule. In some embodiments, the PCR reaction in d) employs a second PCR primer that hybridizes to at least a portion of the tail sequence. In certain embodiments, the PCR reaction in d) employs a first PCR primer that hybridizes to a region within the adaptor sequence of the Hybrid Molecule and a second PCR primer hybridizes to at least a portion of the tail sequence. In some embodiments, the second primer hybridizes to the target genetic locus/tail junction such that at least one or more nucleotides of the primer hybridize to the target genetic locus and at least one or more nucleotides of the primer hybridize to the tail sequence.

In some embodiments, amplification can be isothermal such as by Loop mediated isothermal amplification (LAMP), whole genome amplification (WGA), Strand displacement amplification (SDA), helicase-dependent amplification (HDA), Recombinase polymerase amplification (RPA), Nucleic acid sequence-based amplification (NASBA), Nicking Enzyme Amplification Reaction (NEAR), and Ligase Chain Reaction (LCR).

In some embodiments, DNA polymerases for isothermal amplification include DNA polymerases such as Klenow Fragment, Bst large fragment, and phi29 for moderate temperature reactions (25-40°C) and the large fragment of Bst DNA polymerase for higher temperature (50-65°C) reactions. Enzymes suitable for LCR include a thermostable Taq ligase and a thermostable DNA polymerase such as Taq Polymerase.

In certain embodiments, the amplified Hybrid Molecules obtained from step d) are sequenced and the sequences aligned horizontally, *i.e.,* aligned to one another but not aligned to a reference sequence. In some embodiments, steps a) through d) are repeated one or more times with one or more capture probes. The capture probes can be the same or different and designed to target either cfDNA strand of a target genetic locus. In some embodiments, when the capture probes are different, they hybridize at overlapping or adjacent target sequences within a target genetic locus in the adaptor-tagged cfDNA library. In one embodiment, a high-density capture probe strategy is used wherein a plurality of capture probes hybridize to a target genetic locus, and wherein each of the plurality of capture probes hybridizes to the target genetic locus within about 5, 10, 15, 20, 25, 30, 35, 40, 45, 50, 100, 200 bp or more of any other capture probe that hybridizes to the target genetic locus in an adaptor-tagged DNA library, including all intervening distances. In one embodiment, a high-density capture probe strategy is used wherein a plurality of capture probes hybridize to a target genetic locus, and wherein each of the plurality of capture probes hybridizes to the target genetic locus within 5, 10, 15, 20, 25, 30, 35, 40, 45, 50, 100, 200 bp or more of any other capture probe that hybridizes to the target genetic locus in an adaptor-tagged DNA library, including all intervening distances.

In some embodiments, the method can be performed using two capture probes per target genetic locus, wherein one hybridizes to the "Watson" strand (non-coding or template strand) upstream of the target region and one hybridizes to the "Crick" strand (coding or non-template strand) downstream of the target region.

In some embodiments, the methods of the disclosure can further be performed multiple times with any number of capture probes, for example, 2, 3, 4, 5, 6, 7, 8, 9, or 10 or more capture probes per target genetic locus any number of which hybridize to the Watson or Crick strand in any combination. In some embodiments, the sequences obtained can be aligned to one another in order to identify any of a number of differences.

In certain embodiments, a plurality of target genetic loci are interrogated, e.g., 100, 200, 300, 400, 500, 600, 700, 800, 900, 1000, 1500, 2000, 2500, 3000, 3500, 4000, 4500, 5000, 10000, 50000, 100000, 500000 or more in a single reaction, using one or more capture probes.

In some embodiments, the enzymatic end-repair step is not performed. In some embodiments, the isolated capture probe/adaptor-tagged DNA complexes are directly amplified wherein the DNA polymerase performs 5' -> 3' extension to form a library of Hybrid Molecules. In some embodiments, the library containing the Hybrid Molecules is further amplified using forward and reverse primers that contain sequencing adaptors (such as P5 and P7 of Illumina NextSeq NGS technology) to generate the targeted library of sequencing ready amplified Hybrid Molecules.

By eliminating the enzymatic end-repair step, the targeted library is thus generated faster using the methods disclosed. Such faster methods lead to improved and faster performance of genetic analysis of the genetic loci of interest that are present in the DNA fragments in the tagged DNA libraries and in targeted libraries. One of skill in the art will recognize that the genetic loci can be analyzed for DNA alterations e.g., SNV, Indels, gene reorganizations, and copy number changes.

In some embodiments, the 1^{st} modified library is a Target Capture Library (TCL) and is generated according to the method of Example 12. In some embodiments, the 1^{st} modified library is a Target Capture Library (TCLA) and is generated according to the method of Example 12.

In various embodiments, a method for genetic analysis of genomic DNA, e.g., genomic cellular DNA or cfDNA, comprises genetic analysis of multiple genetic loci of the adaptor-tagged DNA library. In some embodiments, genetic analysis comprises amplification of the adaptor-tagged DNA molecules to create an amplified library; sequencing of the amplified library; and bioinformatic analysis of the resulting sequence reads.

In some embodiments, a Forward Primer (FP) hybridizes to the primer binding site in the amplification region of the 5' adapter attached to a DNA molecule, a Reverse Primer (RP) hybridizes to the primer binding site in the amplification region of the 3' adapter attached to the DNA molecule, and the DNA molecule is amplified or extended by PCR using the primers.

In some embodiments, the FP and RP each have an ACA binding site. The ACA binding site of the FP and RP may hybridize to an "ACA sequence" in the amplification region of the adaptor, located at the 5' end of the adaptor-tagged DNA molecule. The "ACA sequence" in the adaptor may hybridize to a sequence comprising the nucleotides ACA. In some embodiments, a primer having an ACA binding site is referred to as a Library Primer or ACA primer. In some embodiments, the Library Primer comprises the sequence SEQ ID NO: 5.

In some embodiments, each end of the amplified or extended second DNA targets preferentially includes a sequencing adaptor capable of binding to a flow cell, such as a P5 or P7 sequence, or any other Illumina^{®} sequencing adaptor. In some embodiments, the sequencing adaptor includes a sequencing primer binding site.

In some embodiments, the collection of the amplified or extended second adaptor-tagged library is referred to as the Low Pass Whole Genome (LPWG) Library or the Whole Genome Library Amplified (WGLA).

In some embodiments, the LPWG library does not comprise a Library Tag.

In some embodiments, the 2nd modified library is a WGLA library. In some embodiments, the 2nd modified library is a WGLA library generated according to the methods of Example 13.

### COMBINING THE MODIFIED LIBRARIES

In some embodiments, the 1^{st} and 2^{nd} Modified Libraries are combined to create a combined library. In some embodiments, the combined library is also referred to as the Sequence-Ready Library (SRL). In some embodiments, the Target Capture Library and the LPWG library are contacted to generate a combined library. In some embodiments, the TCLA and the WGLA are contacted to generate a combined library.

In some embodiments, the first modified library and second modified library are generated by the same entity. In some embodiments, the first and second Modified Libraries are also combined by said same entity.

In some embodiments, the first modified library is generated by a first entity, and the second modified library is generated by a second entity, wherein the first and second entities are not the same. In some embodiments, the first and second Modified Libraries are combined by the first entity. In certain embodiments, the first and second Modified Libraries are combined by the second entity. In some embodiments, the first and second Modified Libraries are combined by a third entity, wherein the third entity is different from the first and second entities.

In some embodiments, at least one modified library is generated using a kit comprising the adaptors of the disclosure.

In some embodiments, the first and second modified libraries are combined according to the methods of Example 14.

### DETERMINING THE NUMBER OF GENOME EQUIVALENTS

In various embodiments, a method for genetic analysis of DNA comprises determining the number of genome equivalents in the DNA clone library. As used in the disclosure, the term "genome equivalent" refers to the number of genome copies in each library. An important challenge met by the compositions and methods of the disclosure is achieving sufficient assay sensitivity to detect and analyze rare genetic mutations or differences in genetic sequence. To determine assay sensitivity value on a sample-by-sample basis, the numbers of different and distinct sequences that are present in each sample are measured, by measuring the number of genome equivalents that are present in a sequencing library. To establish sensitivity, the number of genome equivalents should be measured for each sample library.

The number of genome equivalents can be determined by qPCR assay or by using bioinformatics-based counting after sequencing is performed. In the process flow of clinical samples, qPCR measurement of genome equivalents is used as a QC step for DNA libraries. It establishes an expectation for assay sensitivity prior to sequence analysis and allows a sample to be excluded from analysis if its corresponding DNA clone library lacks the required depth of genome equivalents. Ultimately, the bioinformatics-based counting of genome equivalents is also used to identify the genome equivalents - and hence the assay sensitivity and false negative estimates - for each given DNA clone library.

The empirical qPCR assay and statistical counting assays should be well correlated. In cases where sequencing fails to reveal the sequence depth in a DNA clone library, reprocessing of the DNA clone library and/or additional sequencing may be required.

In one embodiment, the genome equivalents in a cellular DNA or cfDNA clone library are determined using a quantitative PCR (qPCR) assay. In a particular embodiment, a standard library of known concentration is used to construct a standard curve and the measurements from the qPCR assay are fit to the resulting standard curve and a value for genome equivalents is derived from the fit. The present inventors have discovered that a qPCR "repeat-based" assay comprising one primer that specifically hybridizes to a common sequence in the genome, *e.g.,* a repeat sequence, and another primer that binds to the primer binding site in the adaptor, measured an 8-fold increase in genome equivalents compared to methods using just the adaptor specific primer (present on both ends of the DNA clone). The number of genome equivalents measured by the repeat-based assays provides a more consistent library-to-library performance and a better alignment between qPCR estimates of genome equivalents and bioinformatically counted tag equivalents in sequencing runs.

Illustrative examples of repeats suitable for use in the repeat-based genome equivalent assays of the disclosure include, but are not limited to: short interspersed nuclear elements (SINEs), *e.g.,* Alu repeats; long interspersed nuclear elements (LINEs), *e.g.,* LINE1, LINE2, LINE3; microsatellite repeat elements, e.g., short tandem repeats (STRs), simple sequence repeats (SSRs); and mammalian-wide interspersed repeats (MIRs).

In one embodiment, the repeat is an Alu repeat.

### SEQUENCING

In some embodiments, next generation Sequencing (NGS) of the combined library is performed using an Illumina NextSeq 550 sequencer.

In some embodiments, Sequencing Read 1 (151 nt in length) and Sequencing Read 2 (17 nt in length) is conducted using custom-made forward and reverse sequencing primers.

In some embodiments, the quantitative genetic analysis comprises sequencing a plurality of Hybrid Molecules, as discussed elsewhere in the disclosure, *supra,* to generate sufficient sequencing depths to obtain a plurality of unique sequencing reads. As used in the disclosure, the terms "unique reads" or "unique genomic sequences" (UGS) may be used interchangeably in the disclosure and are identified by grouping individual redundant reads together into a "family." Redundant reads are sequence reads that share an identical UMIE *(e.g.,* share the same read code and the same DNA sequence start position within genomic sequence) and are derived from a single attachment event and are therefore amplification-derived "siblings" of one another. A single consensus representative of a family of redundant reads is carried forward as a unique read or UGS. Each unique read or UGS is considered a unique attachment event. The sum of unique reads corresponding to a particular capture probe is referred to as the "raw genomic depth" (RGD) for that particular capture probe. Each capture probe yields a set of unique reads that are computationally distilled from total reads by grouping into families. In some embodiments, the entire capture probe region in the hybrid molecule is sequenced. In some embodiments, a portion of the capture probe region in the hybrid molecule is sequenced.

The unique reads for a given sample (*e.g*., raw genomic depth for a sample) are then computed as the average of all the unique reads observed on a probe-by-probe basis. Unique reads are important because each unique read should be derived from a unique genomic DNA clone. Each unique read represents the input and analysis of a haploid equivalent of genomic DNA. The sum of unique reads is the sum of haploid genomes analyzed. The number of genomes analyzed, in turn, defines the sensitivity of the sequencing assay. By way of a non-limiting example, if the average unique read count is 100 genome equivalents, then that particular assay has a sensitivity of being able to detect one mutant read in 100, or 1%. Any observation less than this is not defensible.

Cases where there is an obvious copy number change (*e.g*., instances of noisy probes) are excluded from the data set used to compute the sample average. In the disclosure, a "noisy probe" refers to a probe that captures a highly variable number of unique reads among a large set identical samples (*e.g.,* a highly variable number of unique reads among 12 - 16 sample replicates). In some embodiments, the number of unique reads associated with a noisy probe is increased compared to the average number of unique reads for the sample by 50% or more. In some embodiments, the number of unique reads associated with a noisy probe is decreased compared to the average number of unique reads for the sample by 50% or more. In some embodiments, about 2% to about 4% of probes used in a particular analysis are identified as noisy probes and are excluded from calculations to determine the average number of unique reads for a given sample. In some embodiments, 2% to 4% of probes used in a particular analysis are identified as noisy probes and are excluded from calculations to determine the average number of unique reads for a given sample.

In some embodiments, sequencing reads are identified as either "on-target reads" or "off-target reads." On-target reads possess a genomic DNA sequence that maps within the vicinity of a capture probe used to create the genomic library. In some embodiments, where each genomic sequence is physically linked to a specific capture probe and where the sequence of the genomic segment and capture probe are both determined as a unified piece of information, an on-target read is defined as any genomic sequence whose starting coordinate maps within 400 bp, and more generally within 200 bp of the 3' end of the corresponding capture probe. Off-target reads are defined as having genomic sequence that aligns to the reference genome at a location ≥ 500 base pairs (and more often mapping to entirely different chromosomes) relative to the capture probe.

In some embodiments, the quantitative genetic analysis comprises multiplex sequencing of Hybrid Molecules derived from a plurality of samples.

In various embodiments, the quantitative genetic analysis comprises obtaining one or more or a plurality of adaptor-tagged DNA molecules, each clone comprising a first DNA sequence and a second DNA sequence, wherein the first DNA sequence comprises a sequence in a targeted genetic locus and the second DNA sequence comprises a capture probe sequence; performing a paired end sequencing reaction on the one or more clones and obtaining one or more sequencing reads or performing a sequencing reaction on the one or more clones in which a single long sequencing read of greater than about 100, 200, 300, 400, 500 or more nucleotides is obtained, wherein the read is sufficient to identify both the first DNA sequence and the second DNA sequence; and ordering or clustering the sequencing reads of the one or more clones according to the probe sequences of the sequencing reads.

### BIOINFORMATICS ANALYSIS

In various embodiments, the quantitative genetic analysis further comprises bioinformatic analysis of the sequencing reads. Bioinformatic analysis excludes any purely mental analysis performed in the absence of a composition or method for sequencing. In certain embodiments, bioinformatics analysis includes, but is not limited to: sequence alignments; genome equivalents analysis; single nucleotide variant (SNV) analysis; gene copy number variation (CNV) analysis; measurement of chromosomal copy number; detection of genetic lesions; and detection of epigenetic marks. In some embodiments, bioinformatics analysis is useful to quantify the number of genome equivalents analyzed in the cfDNA clone library; to detect the genetic state of a target genetic locus; to detect genetic lesions in a target genetic locus; and to measure copy number fluctuations within a target genetic locus.

In some embodiments, the term "genetic analysis" may refer to any method that comprises analyzing a nucleotide sequence and/or an epigenetic mark.

In some embodiments, the molecules generated according to methods of the disclosure that can be subject to sequencing are subjected to genetic analysis.

In some embodiments, sequence reads 1 and 2 are used for genetic analysis.

In some embodiments, the Library Tag in the Target Capture Library comprises a binding site for a custom sequencing primer and is used to de-multiplex sequencing reads of the Target Capture Library from sequencing read of the LPWG library.

In some embodiments, bioinformatics analysis is performed to identify genetic variants, such as copy numbers, SNVs, Indels, gene and chromosome rearrangements.

Sequence alignments may be performed between the sequence reads and one or more human reference DNA sequences. In some embodiments, sequencing alignments can be used to detect genetic lesions in a target genetic locus including, but not limited to detection of a nucleotide transition or transversion, a nucleotide insertion or deletion, a genomic rearrangement, a change in copy number, or a gene fusion. Detection of genetic lesions that are causal or prognostic indicators may be useful in the diagnosis, prognosis, treatment, and/or monitoring of a particular genetic condition or disease.

The term "target genetic locus" used in the disclosure may refer to a region of interest within a DNA sequence. In various embodiments, targeted genetic analyses are performed on the target genetic locus. In some embodiments, the DNA target region is a region of a gene that is associated with a particular genetic state, genetic condition, genetic diseases; fetal testing; genetic mosaicism, paternity testing; predicting response to drug treatment; diagnosing or monitoring a medical condition; microbiome profiling; pathogen screening; or organ transplant monitoring. In further embodiments, the DNA target region is a DNA sequence that is associated with a particular human chromosome, such as a particular autosomal or X-linked chromosome, or region thereof (*e.g.,* a unique chromosome region).

Also in the disclosure are methods for sequence alignment analysis that can be performed without the need for alignment to a reference sequence, referred to in the disclosure as horizontal sequence analysis. Such analysis can be performed on any sequences generated by the methods of the disclosure or any other methods. In some embodiments, the sequence analysis comprises performing sequence alignments on the reads obtained by the methods of the disclosure.

In one embodiment, the genome equivalents in a cfDNA clone library are determined using bioinformatics-based counting after sequencing is performed. Each sequencing read is associated with a particular capture probe, and the collection of reads assigned to each capture probe is parsed into groups. Within a group, sets of individual reads share the same read code and the same DNA sequence start position within genomic sequence. These individual reads are grouped into a "family" and a single consensus representative of this family is carried forward as a "unique read." All of the individual reads that constituted a family are derived from a single attachment event and thus, they are amplification-derived "siblings" of one another. Each unique read is considered a unique attachment event and the sum of unique reads is considered equivalent to the number of genome equivalents analyzed.

As the number of unique clones approaches the total number of possible sequence combinations, probability dictates that the same code and start site combinations will be created by independent events and that these independent events will be inappropriately grouped within single families. The net result will be an underestimate of genome equivalents analyzed, and rare mutant reads may be discarded as sequencing errors because they overlap with wild-type reads bearing the same identifiers.

In some embodiments, to provide an accurate analysis for cfDNA clone libraries, the number of genome equivalents analyzed is about 1/10, about 1/12, about 1/14, about 1/16, about 1/18, about 1/20, about 1/25 or less the number of possible unique clones. In some embodiments, to provide an accurate analysis for cfDNA clone libraries, the number of genome equivalents analyzed is 1/10, 1/12, 1/14, 1/16, 1/18, 1/20, 1/25 or less the number of possible unique clones. It should be understood that the procedure outlined above is merely illustrative and not limiting.

In some embodiments, the number of genome equivalents to be analyzed may need to be increased. To expand the depth of genome equivalents, at least two solutions are contemplated. The first solution is to use more than one adaptor set per sample. By combining adaptors, it is possible to multiplicatively expand the total number of possible clones and therefore, expand the comfortable limits of genomic input. The second solution is to expand the read code by 1, 2, 3, 4, or 5, or more bases. The number of possible read codes that differ by at least 2 bases from every other read code scales as 4⁽ⁿ⁻¹⁾ where n is the number of bases within a read code. Thus, in a non-limiting example, if a read code is 5 nucleotides and 4⁽⁵⁻¹⁾ = 256; therefore, the inclusion of additional bases expands the available repertoire by a factor of four for each additional base.

In one embodiment, quantitative genetic analysis comprises bioinformatic analysis of sequencing reads to identify rare single nucleotide variants (SNV).

Next-generation sequencing has an inherent error rate of roughly 0.2%-0.5%, meaning that anywhere from 1/200 to 1/500 base calls are incorrect. To detect variants and other mutations that occur at frequencies lower than this, for example, at frequencies of 1 per 1000 sequences, it is necessary to invoke molecular annotation strategies. By way of a non-limiting example, analysis of 5000 unique molecules using targeted sequence capture technology would generate - at sufficient sequencing depths of >50,000 reads - a collection of 5000 unique reads, with each unique read belonging to a "family" of reads that all possess the same read code. A SNV that occurs within a family is a candidate for being a rare variant. When this same variant is observed in more than one family, it becomes a very strong candidate for being a rare variant that exists within the starting sample. In contrast, variants that occur sporadically within families are likely to be sequencing errors and variants that occur within one and only one family are either rare or the result of a base alteration that occurred *ex vivo* (*e.g.,* oxidation of a DNA base or PCR-introduced errors).

In one embodiment, the methods of detecting SNVs comprise introducing 10-fold more genomic input (genomes or genome equivalents) as the desired target sensitivity of the assay. In one non-limiting example, if the desired sensitivity is 2% (2 in 100), then the experimental target is an input of 2000 genomes.

In some embodiments, bioinformatics analysis of sequencing data is used to detect or identify SNV associated with a genetic state, condition or disease, genetic mosaicism, fetal testing, paternity testing, predicting response to drug treatment, diagnosing or monitoring a medical condition, microbiome profiling, pathogen screening, and monitoring organ transplants.

### COPY NUMBER ANALYSIS

The disclosure includes, *inter alia,* compositions and methods that are useful for the detection of a mutational change, SNP, translocation, inversion, deletion, change in copy number or other genetic variation within a sample of cellular genomic DNA (*e.g.* from a tissue biopsy sample) or cfDNA (*e.g*. from a blood sample). The compositions and methods of the disclosure are particularly useful in detecting incredibly hard to detect copy number variations in cfDNA from a biological sample (*e.g*. blood) with exquisite resolution. In particular, some embodiments of the disclosure are drawn to a method for detecting copy number of a DNA target region from a test sample by generating a genomic DNA library made up of genomic DNA fragments attached to an adaptor, capturing DNA target regions with a plurality of capture probes, isolating the DNA library fragments comprising the DNA target region, and performing a quantitative genetic analysis of the DNA target region to thereby determine the copy number of the DNA target region. Additionally, some embodiments of the disclosure are drawn to a method for detecting copy number of numerous genetic loci across a genome by generating, for example, LPWG library according to methods of the disclosure and performing a quantitative genetic analysis of the genetic loci in the LPWG library. By analyzing a broader scope of the genome, the quality of statistical data for copy number analysis of each genetic locus improves. The adaptors described in the disclosure allow for the identification of the individual DNA fragment that is being sequenced, as well as the identity of the sample or source of the genomic DNA.

The disclosure includes, in part, compositions and methods for detection of target-specific copy number changes that are applicable to several sample types, including but not limited to direct tissue biopsies and peripheral blood. In the context of cancer genomics, and in particular cell free DNA (cfDNA) assays for the analysis of solid tumors, the amount of tumor DNA is often a very small fraction of the overall DNA. Further, copy number loss is difficult to detect in genomic DNA assays, and in particular, genomic DNA assays where copy number change may only be present in a portion of the total genomic DNA from a sample, e.g., cfDNA assays. For example, in some embodiments, most of the cell-free DNA extracted from a cancer patient will be derived from normal sources and have a diploid copy number (except for X-linked genes in male subjects). In a cancer patient, the fraction of DNA derived from tumors often has a low minor allele frequency, such as for example, a patient in which 2% of the circulating DNA extracted from plasma is derived from the tumor. The loss of one copy of a tumor suppressor gene (*e.g.* BRCA1 in breast cancer) means that the minor allele frequency for the absence of detectable genomic fragments is 1%. In this scenario, a copy number loss assay engineered should be able to discriminate between 100 copies (normal) and 99 copies (heterozygous gene loss). Thus, some embodiments contemplate that the methods and compositions of the disclosure allow for the detection of copy number change with sufficient resolution to detect changes in copy number at minor allele frequencies even in the context of cfDNA.

In various embodiments, a method for copy number analysis of a DNA target region DNA is provided. In certain embodiments, copy number analysis is performed by generating a genomic DNA library of DNA library fragments that each contain genomic DNA fragment and an adaptor, isolating the DNA library fragments containing the DNA target regions, and performing a quantitative genetic analysis of the DNA target region. By "quantitative genetic analysis" it is meant an analysis performed by any molecular biological technique that is able to quantify changes in a DNA *(e.g.,* a gene, genetic locus, target region of interest, etc.) including but not limited to DNA mutations, SNPs, translocations, deletions, and copy number variations (CNVs). In certain embodiments, the quantitative genetic analysis is performed by sequencing, for example, next generation sequencing.

In various embodiments, a method for copy number determination analysis is provided comprising obtaining one or more or a plurality of clones, each clone comprising a first DNA sequence and a second DNA sequence, wherein the first DNA sequence comprises a sequence in a targeted genetic locus and the second DNA sequence comprises a capture probe sequence. In related embodiments, a paired end sequencing reaction on the one or more clones is performed and one or more sequencing reads are obtained. In another embodiment, a sequencing reaction on the one or more clones is performed in which a single long sequencing read of greater than about 100 nucleotides is obtained, wherein the read is sufficient to identify both the first DNA sequence and the second DNA sequence. The sequencing reads of the one or more clones can be ordered or clustered according to the probe sequence of the sequencing reads.

Copy number analyses include, but are not limited to, analyses that examine the number of copies of a particular gene or mutation that occurs in a given genomic DNA sample and can further include quantitative determination of the number of copies of a given gene or sequence differences in a given sample. In some embodiments, copy number analysis is used to detect or identify gene amplification associated with genetic states, conditions, or diseases, fetal testing, genetic mosaicism, paternity testing, predicting response to drug treatment, diagnosing or monitoring a medical condition, microbiome profiling, pathogen screening, and monitoring organ transplants.

In some embodiments, copy number analysis is used to measure chromosomal instability. In such embodiments, sets of capture probes that comprise chromosomal stability probes are used to determine copy number variations at a uniform density across all sets of chromosomes. Copy number analyses are performed for each chromosomal stability probe and the chromosomal stability probes are then ordered according to their chromosomal target. This allows for visualization of copy number losses or gains across the genome and can serve as a measure of chromosomal stability.

In some embodiments, bioinformatics analysis of sequencing data is used to detect or identify one or more sequences or genetic lesions in a target locus including, but not limited to detection of a nucleotide transition or transversion, a nucleotide insertion or deletion, a genomic rearrangement, a change in copy number, or a gene fusion. Detection of genetic lesions that are causal or prognostic indicators may be useful in the diagnosis, prognosis, treatment, and/or monitoring of a particular genetic condition or disease. In one embodiment, genetic lesions are associated with genetic states, conditions, or diseases, fetal testing, genetic mosaicism, paternity testing, predicting response to drug treatment, diagnosing or monitoring a medical condition, microbiome profiling, pathogen screening, and monitoring organ transplants.

In some embodiments, the number of copies of the DNA target region present in the sample is determined by the quantitative genetic analysis. In some embodiments, the copy number of the DNA target region is determined by comparing the amount of copies of DNA target regions present in the sample and comparing it to amounts of DNA target regions present in one or more samples with known copy number.

Some embodiments contemplate that the compositions and methods described in the disclosure are particularly useful for detecting changes in copy number in a sample of genomic DNA, where only a portion of the total genomic DNA in the sample has a change in copy number. For example, in some embodiments, a significant tumor mutation may be present in a sample, *e.g.* a sample of cell free DNA, that is present in a minor allele frequency that is significantly less than 50% ( *e.g.,* in the range of 0.1% to >20%), in contrast to conventional SNP genotyping where allele frequencies are generally ~100%, 50% or 0%. One of skill of the art will recognize that the compositions and methods of the disclosure are also useful in detecting other types of mutation including single nucleotide variants (SNVs), short *(e.g.,* less than 40 base pairs (bp)) insertions, and deletions (indels), and genomic rearrangements including oncogenic gene fusions.

In certain embodiments, the compositions and/or methods of the disclosure are useful for, capable of, suited for, and/or able to detect, identify, observe, and/or reveal a change in copy number of one or more DNA target regions present in less than about 20%, less than about 19%, less than about 18%, less than about 17%, less than about 16%, less than about 15%, less than about 14%, less than about 13%, less than about 12%, less than about 11%, less than about 10%, less than about 9%, less than about 8%, less than about 7%, less than about 6%, less than about 5%, less than about 4%, less than about 3%, less than about 2%, less than about 1%, less than about 0.5%, less than about 0.2%, or less than about 0.1% of the total genomic DNA from the sample. In some embodiments, the methods of the disclosure are useful for, capable of, suited for, and/or able to detect, identify, observe, and/or reveal a change in copy number of one or more DNA target regions present in between about 0.01% to about 100%, about 0.01% to about 50%, or about 0.1% to about 20% of the total genomic DNA from the sample.

In certain embodiments, the compositions and/or methods of the disclosure are useful for, capable of, suited for, and/or able to detect, identify, observe, and/or reveal a change in copy number of one or more DNA target regions present in less than 20%, less than 19%, less than 18%, less than 17%, less than 16%, less than 15%, less than 14%, less than 13%, less than 12%, less than 11%, less than 10%, less than 9%, less than 8%, less than 7%, less than 6%, less than 5%, less than 4%, less than 3%, less than 2%, less than 1%, less than 0.5%, less than 0.2%, or less than 0.1% of the total genomic DNA from the sample. In some embodiments, the methods of the disclosure are useful for, capable of, suited for, and/or able to detect, identify, observe, and/or reveal a change in copy number of one or more DNA target regions present in between 0.01% to 100%, 0.01% to 50%, or 0.1% to 20% of the total genomic DNA from the sample.

In some embodiments, a method for genetic analysis of cfDNA comprises: generating and amplifying a cfDNA library, determining the number of genome equivalents in the cfDNA library; and performing a quantitative genetic analysis of one or more genomic target loci.

Some embodiments contemplate that the any of the methods and compositions described in the disclosure are effective for use to efficiently analyze, detect, diagnose, and/or monitor genetic states, genetic conditions, genetic diseases, genetic mosaicism, fetal diagnostics, paternity testing, microbiome profiling, pathogen screening, and organ transplant monitoring using genomic DNA, *e.g.,* cellular or cfDNA, where all or where only a portion of the total genomic DNA in the sample has a feature of interest, *e.g*. a genetic lesion, mutation, single nucleotide variant (SNV). In some embodiments, a feature of interest is a genetic feature associated with a disease or condition. For example, in some embodiments, a significant tumor mutation may be present in a sample, *e.g.* a sample of cfDNA, that is present in a minor allele frequency that is significantly less than 50% (*e.g.* in the range of 0.1% to >20%), in contrast to conventional SNP genotyping where allele frequencies are generally ~100%, 50% or 0%.

### CLINICAL APPLICATIONS

In various embodiments, the disclosure contemplates a method of detecting, identifying, predicting, diagnosing, or monitoring a condition or disease in a subject by detecting a mutational change, SNP, translocation, inversion, deletion, change in copy number or other genetic variation in a region of interest.

In various embodiments, the disclosure contemplates a method of detecting, identifying, predicting, diagnosing, or monitoring a condition or disease in a subject.

In some embodiments, a method of detecting, identifying, predicting, diagnosing, or monitoring a genetic state, condition or disease in a subject comprises performing a quantitative genetic analysis of one or more target genetic loci in a DNA clone library to detect or identify a change in the sequence at the one or more target genetic loci. In some embodiments, the change is a change in copy number.

In one embodiment, a method of detecting, identifying, predicting, diagnosing, or monitoring a genetic state, condition or disease comprises isolating or obtaining cellular DNA or cfDNA from a biological sample of a subject; treating the cellular DNA or cfDNA with one or more end-repair enzymes to generate end-repaired DNA; attaching one or more adaptors to each end of the end-repaired DNA to generate a genomic DNA library; amplifying the DNA library to generate a DNA clone library; determining the number of genome equivalents in the DNA clone library; and performing a quantitative genetic analysis of one or more target genetic loci in a DNA clone library to detect or identify a change in the sequence, e.g., an SNP, a translocation, an inversion, a deletion, or a change in copy number at of the one or more target genetic loci.

In some embodiments, the disclosure provides a method of detecting, identifying, predicting, diagnosing, or monitoring a genetic state, or genetic condition or disease selected from the group consisting of: genetic diseases; genetic mosaicism; fetal testing; paternity testing; paternity testing; predicting and/or monitoring response to drug treatment; diagnosing or monitoring a medical condition; microbiome profiling; pathogen screening; and organ transplant monitoring comprising isolating or obtaining genomic DNA from a biological sample of a subject; treating the DNA with one or more end-repair enzymes to generate end-repaired DNA; attaching one or more adaptors to each end of the end-repaired DNA to generate a genomic DNA library; amplifying the genomic DNA library to generate a DNA clone library; determining the number of genome equivalents in the DNA clone library; and performing a quantitative genetic analysis of one or more target genetic loci in a DNA clone library to detect or identify a nucleotide transition or transversion, a nucleotide insertion or deletion, a genomic rearrangement, a change in copy number, or a gene fusion in the sequence at the one or more target genetic loci.

In some embodiments, the disclosure provides a method of determining the efficacy of a targeted therapy in a subject having cancer, comprising: detecting a first cell-free tumor load (cfTL) in a biological sample isolated from the subject at a first time point; detecting a second cfTL in a biological sample obtained from the subject at a second time point, wherein the subject has received at least one dose of the targeted therapy between the first time point and the second time point; and identifying the targeted therapy as being effective in the subject when the subject exhibits a second cfTL that is reduced as compared to the first cfTL. For example, in some embodiments, the second dfTL may be reduced as compared to the first cfTL by at least about 1%, at least about 2%, at least about 5%, at least about 10%, at least about 20%, at least about 30%, at least about 40%, at least about 50%, at least about 60%, at least about 70%, at least about 80%, at least about 90%, or more than about 90%. As used in the disclosure, cfTL may refer to circulating tumor DNA having one or more genetic alterations and/or aneuploidy.

Illustrative examples of genetic diseases that can be detected, identified, predicted, diagnosed, or monitored with the compositions and methods of the disclosure include, but are not limited to cancer, Alzheimer's disease (APOE1), Charcot-Marie-Tooth disease, Leber hereditary optic neuropathy (LHON), Angelman syndrome (UBE3A, ubiquitin-protein ligase E3A), Prader-Willi syndrome (region in chromosome 15), β-Thalassaemia (HBB, β-Globin), Gaucher disease (type I) (GBA, Glucocerebrosidase), Cystic fibrosis (CFTR Epithelial chloride channel), Sickle cell disease (HBB, β-Globin), Tay-Sachs disease (HEXA, Hexosaminidase A), Phenylketonuria (PAH, Phenylalanine hydrolyase), Familial hypercholesterolaemia (LDLR, Low density lipoprotein receptor), Adult polycystic kidney disease (PKD1, Polycystin), Huntington disease (HDD, Huntingtin), Neurofibromatosis type I (NF1, NF1 tumour suppressor gene), Myotonic dystrophy (DM, Myotonin), Tuberous sclerosis (TSC1, Tuberin), Achondroplasia (FGFR3, Fibroblast growth factor receptor), Fragile X syndrome (FMR1, RNA-binding protein), Duchenne muscular dystrophy (DMD, Dystrophin), Haemophilia A (F8C, Blood coagulation factor VIII), Lesch-Nyhan syndrome (HPRT1, Hypoxanthine guanine ribosyltransferase 1), and Adrenoleukodystrophy (ABCD1).

Illustrative examples of cancers that can be detected, identified, predicted, diagnosed, or monitored with the compositions and methods of the disclosure include, but are not limited to: B cell cancer, *e.g*., multiple myeloma, melanomas, breast cancer, lung cancer (such as non-small cell lung carcinoma or NSCLC), bronchus cancer, colorectal cancer, prostate cancer, pancreatic cancer, stomach cancer, ovarian cancer, urinary bladder cancer, brain or central nervous system cancer, peripheral nervous system cancer, esophageal cancer, cervical cancer, uterine or endometrial cancer, cancer of the oral cavity or pharynx, liver cancer, kidney cancer, testicular cancer, biliary tract cancer, small bowel or appendix cancer, salivary gland cancer, thyroid gland cancer, adrenal gland cancer, osteosarcoma, chondrosarcoma, cancer of hematological tissues, adenocarcinomas, inflammatory myofibroblastic tumors, gastrointestinal stromal tumor (GIST), colon cancer, multiple myeloma (MM), myelodysplastic syndrome (MDS), myeloproliferative disorder (MPD), acute lymphocytic leukemia (ALL), acute myelocytic leukemia (AML), chronic myelocytic leukemia (CML), chronic lymphocytic leukemia (CLL), polycythemia Vera, Hodgkin lymphoma, non-Hodgkin lymphoma (NHL), soft-tissue sarcoma, fibrosarcoma, myxosarcoma, liposarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, seminoma, embryonal carcinoma, Wilms' tumor, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, retinoblastoma, follicular lymphoma, diffuse large B-cell lymphoma, mantle cell lymphoma, hepatocellular carcinoma, thyroid cancer, gastric cancer, head and neck cancer, small cell cancers, essential thrombocythemia, agnogenic myeloid metaplasia, hypereosinophilic syndrome, systemic mastocytosis, familiar hypereosinophilia, chronic eosinophilic leukemia, neuroendocrine cancers, carcinoid tumors, and the like.

In one embodiment, the genetic lesion is a lesion annotated in the Cosmic database (the lesions and sequence data are available online and can be downloaded from the Cancer Gene Census section of the Cosmic website) or a lesion annotated in the Cancer Genome Atlas (the lesions and sequence data are available online and can be downloaded from The Cancer Genome Atlas website).

Illustrative examples of genes that harbor one or more genetic lesions associated with cancer that can be detected, identified, predicted, diagnosed, or monitored with the compositions and methods of the disclosure include, but are not limited to ABCB1, ABCC2, ABCC4, ABCG2, ABL1, ABL2, AKT1, AKT2, AKT3, ALDH4A1, ALK, APC, AR, ARAF, ARFRP1, ARID1A, ATM, ATR, AURKA, AURKB, BCL2, BCL2A1, BCL2L1, BCL2L2, BCL6, BRAF, BRCA1, BRCA2, Clorf144, CARD11, CBL, CCND1, CCND2, CCND3, CCNE1, CDH1, CDH2, CDH20, CDH5, CDK4, CDK6, CDK8, CDKN2A, CDKN2B, CDKN2C, CEBPA, CHEK1, CHEK2, CRKL, CRLF2, CTNNB1, CYP1B1, CYP2C19, CYP2C8, CYP2D6, CYP3A4, CYP3A5, DNMT3A, DOT1L, DPYD, EGFR, EPHA3, EPHA5, EPHA6, EPHA7, EPHB1, EPHB4, EPHB6, EPHX1, ERBB2, ERBB3, ERBB4, ERCC2, ERG, ESR1, ESR2, ETV1, ETV4, ETV5, ETV6, EWSR1, EZH2, FANCA, FBXW7, FCGR3A, FGFR1, FGFR2, FGFR3, FGFR4, FLT1, FLT3, FLT4, FOXP4, GATA1, GNA11, GNAQ, GNAS, GPR124, GSTP1, GUCY1A2, HOXA3, HRAS, HSP90AA1, IDH1, IDH2, IGF1R, IGF2R, IKBKE, IKZF1, INHBA, IRS2, ITPA, JAK1, JAK2, JAK3, JUN, KDR, KIT, KRAS, LRP1B, LRP2, LTK, MAN1B1, MAP2K1, MAP2K2, MAP2K4, MCL1, MDM2, MDM4, MEN1, MET, MITF, MLH1, MLL, MPL, MRE11A, MSH2, MSH6, MTHFR, MTOR, MUTYH, MYC, MYCL1, MYCN, NF1, NF2, NKX2-1, NOTCH1, NPM1, NQO1, NRAS, NRP2, NTRK1, NTRK3, PAK3, PAX5, PDGFRA, PDGFRB, PIK3CA, PIK3R1, PKHD1, PLCG1, PRKDC, PTCH1, PTEN, PTPN11, PTPRD, RAF1, RARA, RB1, RET, RICTOR, RPTOR, RUNX1, SLC19A1, SLC22A2, SLCO1B3, SMAD2, SMAD3, SMAD4, SMARCA4, SMARCB1, SMO, SOD2, SOX10, SOX2, SRC, STK11, SULT1A1, TBX22, TET2, TGFBR2, TMPRSS2, TNFRSF14, TOP1, TP53, TPMT, TSC1, TSC2, TYMS, UGT1A1, UMPS, USP9X, VHL, and WT1.

In some embodiments, the genetic lesion comprises a nucleotide transition or transversion, a nucleotide insertion or deletion, a genomic rearrangement, a change in copy number, or a gene fusion.

In one embodiment, the genetic lesion is a gene fusion that fuses the 3' coding region of the ALK gene to another gene.

In one embodiment, the genetic lesion is a gene fusion that fuses the 3' coding region of the ALK gene to the EML4 gene.

Illustrative examples of conditions suitable for fetal testing that can be detected, identified, predicted, diagnosed, or monitored with the compositions and methods of the disclosure include but are not limited to: Down Syndrome (Trisomy 21), Edwards Syndrome (Trisomy 18), Patau Syndrome (Trisomy 13), Klinefelter's Syndrome (XXY), Triple X syndrome, XYY syndrome, Trisomy 8, Trisomy 16, Turner Syndrome (XO), Robertsonian translocation, DiGeorge Syndrome and Wolf-Hirschhorn Syndrome.

Illustrative examples of alleles suitable for paternity testing that can be detected, identified, predicted, diagnosed, or monitored with the compositions and methods of the disclosure include but are not limited to 16 or more of: D20S1082, D6S474, D12ATA63, D22S1045, D10S1248, D1S1677, D11S4463, D4S2364, D9S1122, D2S1776, D10S1425, D3S3053, D5S2500, D1S1627, D3S4529, D2S441, D17S974, D6S1017, D4S2408, D9S2157, Amelogenin, D17S1301, D1GATA113, D18S853, D20S482, and D14S1434.

Illustrative examples of genes suitable for predicting the response to drug treatment that can be detected, identified, predicted, diagnosed, or monitored with the compositions and methods of the disclosure include, but are not limited to, one or more of the following genes: ABCB1 (ATP-binding cassette, sub-family B (MDR/TAP), member 1), ACE (angiotensin I converting enzyme), ADH1A (alcohol dehydrogenase 1A (class I), alpha polypeptide), ADH1B (alcohol dehydrogenase IB (class I), beta polypeptide), ADH1C (alcohol dehydrogenase 1C (class I), gamma polypeptide), ADRB1 (adrenergic, beta-1-, receptor), ADRB2 (adrenergic, beta-2-, receptor, surface), AHR (aryl hydrocarbon receptor), ALDH1A1 (aldehyde dehydrogenase 1 family, member A1), ALOX5 (arachidonate 5-lipoxygenase), BRCA1 (breast cancer 1, early onset), COMT (catechol-O-methyltransferase), CYP2A6 (cytochrome P450, family 2, subfamily A, polypeptide 6), CYP2B6 (cytochrome P450, family 2, subfamily B, polypeptide 6), CYP2C9 (cytochrome P450, family 2, subfamily C, polypeptide 9), CYP2C19 (cytochrome P450, family 2, subfamily C, polypeptide 19), CYP2D6 (cytochrome P450, family 2, subfamily D, polypeptide 6), CYP2J2 (cytochrome P450, family 2, subfamily J, polypeptide 2), CYP3A4 (cytochrome P450, family 3, subfamily A, polypeptide 4), CYP3A5 (cytochrome P450, family 3, subfamily A, polypeptide 5), DPYD (dihydropyrimidine dehydrogenase), DRD2 (dopamine receptor D2), F5 (coagulation factor V), GSTP1 (glutathione S-transferase pi), HMGCR (3-hydroxy-3-methylglutaryl-Coenzyme A reductase), KCNH2 (potassium voltage-gated channel, subfamily H (eag-related), member 2), KCNJ11 (potassium inwardly-rectifying channel, subfamily J, member 11), MTHFR (5,10-methylenetetrahydrofolate reductase (NADPH)), NQO1 (NAD(P)H dehydrogenase, quinone 1), P2RY1 (purinergic receptor P2Y, G-protein coupled, 1), P2RY12 (purinergic receptor P2Y, G-protein coupled, 12), PTGIS (prostaglandin I2 (prostacyclin) synthase), SCN5A (sodium channel, voltage-gated, type V, alpha (long QT syndrome 3)), SLC19A1 (solute carrier family 19 (folate transporter), member 1), SLCO1B1 (solute carrier organic anion transporter family, member 1B1), SULT1A1 (sulfotransferase family, cytosolic, 1A, phenol-preferring, member 1), TPMT (thiopurine S-methyltransferase), TYMS (thymidylate synthetase), UGT1A1 (UDP glucuronosyltransferase 1 family, polypeptide A1), VDR (vitamin D (1,25- dihydroxyvitamin D3) receptor), VKORC1 (vitamin K epoxide reductase complex, subunit 1).

Illustrative examples of medical conditions that can be detected, identified, predicted, diagnosed, or monitored with the compositions and methods of the disclosure include, but are not limited to: stroke, transient ischemic attack, traumatic brain injury, heart disease, heart attack, angina, atherosclerosis, and high blood pressure.

Illustrative examples of pathogens that can be screened for with the compositions and methods of the disclosure include, but are not limited to: bacteria fungi, and viruses.

Illustrative examples of bacterial species that can be screened for with the compositions and methods of the disclosure include, but are not limited to: a Mycobacterium spp., a Pneumococcus spp., an Escherichia spp., a Campylobacter spp., a Corynebacterium spp., a Clostridium spp., a Streptococcus spp., a Staphylococcus spp., a Pseudomonas spp., a Shigella spp., a Treponema spp., or a Salmonella spp.

Illustrative examples of fungal species that can be screened for with the compositions and methods of the disclosure include, but are not limited to: an Aspergillis spp., a Blastomyces spp., a Candida spp., a Coccicioides spp., a Cryptococcus spp., dermatophytes, a Tinea spp., a Trichophyton spp., a Microsporum spp., a Fusarium spp., a Histoplasma spp., a Mucoromycotina spp., a Pneumocystis spp., a Sporothrix spp., an Exserophilum spp., or a Cladosporium spp.

Illustrative examples of viruses that can be screened for with the compositions and methods of the disclosure include, but are not limited to: Influenza A such as H1N1, H1N2, H3N2 and H5N1 (bird flu), Influenza B, Influenza C virus, Hepatitis A virus, Hepatitis B virus, Hepatitis C virus, Hepatitis D virus, Hepatitis E virus, Rotavirus, any virus of the Norwalk virus group, enteric adenoviruses, parvovirus, Dengue fever virus, Monkey pox, Mononegavirales, Lyssavirus such as rabies virus, Lagos bat virus, Mokola virus, Duvenhage virus, European bat virus 1 & 2 and Australian bat virus, Ephemerovirus, Vesiculovirus, Vesicular Stomatitis Virus (VSV), Herpesviruses such as Herpes simplex virus types 1 and 2, varicella zoster, cytomegalovirus, Epstein-Bar virus (EBV), human herpesviruses (HHV), human herpesvirus type 6 and 8, Moloney murine leukemia virus (M-MuLV), Moloney murine sarcoma virus (MoMSV), Harvey murine sarcoma virus (HaMuSV), murine mammary tumor virus (MuMTV), gibbon ape leukemia virus (GaLV), feline leukemia virus (FLV), spumavirus, Friend murine leukemia virus, Murine Stem Cell Virus (MSCV) and Rous Sarcoma Virus (RSV), HIV (human immunodeficiency virus; including HIV type 1, and HIV type 2), visna-maedi virus (VMV) virus, the caprine arthritis-encephalitis virus (CAEV), equine infectious anemia virus (EIAV), feline immunodeficiency virus (FIV), bovine immune deficiency virus (BIV), and simian immunodeficiency virus (SIV), papilloma virus, murine gammaherpesvirus, Arenaviruses such as Argentine hemorrhagic fever virus, Bolivian hemorrhagic fever virus, Sabia-associated hemorrhagic fever virus, Venezuelan hemorrhagic fever virus, Lassa fever virus, Machupo virus, Lymphocytic choriomeningitis virus (LCMV), Bunyaviridiae such as Crimean-Congo hemorrhagic fever virus, Hantavirus, hemorrhagic fever with renal syndrome causing virus, Rift Valley fever virus, Filoviridae (filovirus) including Ebola hemorrhagic fever and Marburg hemorrhagic fever, Flaviviridae including Kaysanur Forest disease virus, Omsk hemorrhagic fever virus, Tick-borne encephalitis causing virus and Paramyxoviridae such as Hendra virus and Nipah virus, variola major and variola minor (smallpox), alphaviruses such as Venezuelan equine encephalitis virus, eastern equine encephalitis virus, western equine encephalitis virus, SARS-associated coronavirus (SARS-CoV), severe acute respiratory syndrome coronavirus 2 (SARS-CoV-2), West Nile virus, and any encephaliltis causing virus.

Illustrative examples of genes suitable for monitoring an organ transplant in a transplant recipient that can be detected, identified, predicted, diagnosed, or monitored with the compositions and methods of the disclosure include, but are not limited to, one or more of the following genes: HLA-A, HLA-B, HLA-C, HLA-DR, HLA-DP, and HLA-DQ.

In some embodiments, a bioinformatic analysis is used to quantify the number of genome equivalents analyzed in the cfDNA clone library; detect genetic variants in a target genetic locus; detect mutations within a target genetic locus; detect genetic fusions within a target genetic locus; or measure copy number fluctuations within a target genetic locus.

In various embodiments, a companion diagnostic for a genetic disease is provided, comprising: isolating or obtaining genomic DNA from a biological sample of a subject; treating the DNA with one or more end-repair enzymes to generate end-repaired DNA; attaching one or more adaptors to each end of the end-repaired DNA to generate a DNA library; amplifying the DNA library to generate a DNA clone library; determining the number of genome equivalents in the DNA clone library; and performing a quantitative genetic analysis of one or more biomarkers associated with the genetic disease in the DNA clone library, wherein detection of, or failure to detect, at least one of the one or more biomarkers indicates whether the subject should be treated for the genetic disease. In some embodiments, the DNA is cfDNA. In some embodiments, the DNA is cellular DNA.

As used in the disclosure, the term "companion diagnostic" refers to a diagnostic test that is linked to a particular anti-cancer therapy. In a particular embodiment, the diagnostic methods comprise detection of genetic lesion in a biomarker associated with in a biological sample, thereby allowing for prompt identification of patients should or should not be treated with the anti-cancer therapy.

Anti-cancer therapy includes, but is not limited to surgery, radiation, chemotherapeutics, anti-cancer drugs, and immunomodulators.

Illustrative examples of anti-cancer drugs include, but are not limited to: alkylating agents such as thiotepa and cyclophosphamide (CYTOXAN^{™}); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine resume; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin and its pegylated formulations, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elformithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK^{®}; razoxane; sizofiran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxoids, e.g., paclitaxel (TAXOL^{®}, Bristol-Myers Squibb Oncology, Princeton, N.J.) and doxetaxel (TAXOTERE^{®}., Rhne-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; aminopterin; xeloda; ibandronate; CPT-11; topoisomerase inhibitor RFS 2000; difluoromethylomithine (DMFO); retinoic acid derivatives such as Targretin^{™} (bexarotene), Panretin^{™} (alitretinoin); ONTAK^{™} (denileukin diftitox) ; esperamicins; capecitabine; and pharmaceutically acceptable salts, acids or derivatives of any of the above. Also included in this definition are anti-hormonal agents that act to regulate or inhibit hormone action on cancers such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

Illustrative examples of immunomodulators include, but are not limited to: cyclosporine, tacrolimus, tresperimus, pimecrolimus, sirolimus, verolimus, laflunimus, laquinimod and imiquimod, as well as analogs, derivatives, salts, ions and complexes thereof.

In some embodiments, an anti-cancer drug may include a poly-ADP ribose polymerase (PARP) inhibitor. Illustrative examples of PARP inhibitors include, but are not limited to, olaparib (AZD-2281), rucaparib (AG014699 or PF-01367338, niraparib (MK-4827), talazoparib (BMN-673) veliparib (ABT-888), CEP 9722, E7016, BGB-290, 3-aminobenzamide.

All publications, patent applications, and issued patents cited in this specification are herein incorporated by reference as if each individual publication, patent application, or issued patent were specifically and individually indicated to be incorporated by reference. In particular, the entire contents of International PCT Publications No. WO 2014/093330, WO 2016/028316, WO 2017/083562, and WO 2018/039463 are specifically incorporated by reference.

Although the foregoing disclosure has been described in some detail by way of illustration and example for purposes of clarity of understanding, it will be readily apparent to one of ordinary skill in the art in light of the teachings of this disclosure that certain changes and modifications may be made thereto without departing from the spirit or scope of the appended claims. The following examples are provided by way of illustration only and not by way of limitation. Those of skill in the art will readily recognize a variety of noncritical parameters that could be changed or modified to yield essentially similar results.

The practice of some embodiments of the disclosure will employ, unless indicated specifically to the contrary, conventional methods of chemistry, biochemistry, organic chemistry, molecular biology, microbiology, recombinant DNA techniques, genetics, immunology, and cell biology that are within the skill of the art, many of which are described below for the purpose of illustration. Such techniques are explained fully in the literature. See, *e.g.,* Sambrook, et al., Molecular Cloning: A Laboratory Manual (3rd Edition, 2001); Sambrook, et al., Molecular Cloning: A Laboratory Manual (2nd Edition, 1989); Maniatis et al., Molecular Cloning: A Laboratory Manual (1982); Ausubel et al., Current Protocols in Molecular Biology (John Wiley and Sons, updated July 2008); Short Protocols in Molecular Biology: A Compendium of Methods from Current Protocols in Molecular Biology, Greene Pub. Associates and Wiley-Interscience; Glover, DNA Cloning: A Practical Approach, vol. I & II (IRL Press, Oxford, 1985); Anand, Techniques for the Analysis of Complex Genomes, (Academic Press, New York, 1992); Transcription and Translation (B. Hames & S. Higgins, Eds., 1984); Perbal, A Practical Guide to Molecular Cloning (1984); and Harlow and Lane, Antibodies, (Cold Spring Harbor Laboratory Press, Cold Spring Harbor, N.Y., 1998).

### Other Embodiments of the Disclosure

Notwithstanding the appended claims, the following numbered embodiments also form part of the instant disclosure.
1. A kit comprising one or more capture probe modules,
   wherein each capture probe module comprises a tail sequence and a capture probe sequence capable of hybridizing to a target sequence in a test sample;
   wherein the one or more capture probe modules have passed a probe Quality Control (QC) process.
2. A kit comprising a set of adaptors,
   wherein each adaptor comprises an adaptor module;
   wherein the set of adaptors have passed an adaptor Quality Control (QC) process.
3. A kit comprising:
   a. a set of adaptors, wherein each adaptor comprises an adaptor module;
   b. one or more capture probe modules, wherein each capture probe module comprises a tail sequence and a capture probe sequence capable of hybridizing to a target sequence in a test sample;
   wherein the set of adaptors have passed an adaptor Quality Control (QC) process.
4. A kit comprising:
   a. a set of adaptors, wherein each adaptor comprises an adaptor module;
   b. one or more capture probe modules, wherein each capture probe module comprises a tail sequence and a capture probe sequence capable of hybridizing to a target sequence in a test sample;
   wherein the one or more capture probe modules have passed a probe Quality Control (QC) process.
5. A kit comprising:
   a. a set of adaptors, wherein each adaptor comprises an adaptor module,
   b. one or more capture probe modules, wherein each capture probe module comprises a tail sequence and a capture probe sequence capable of hybridizing to a target sequence in a test sample;

   wherein the set of adaptors have passed an adaptor Quality Control (QC) process;
   wherein the one or more capture probe modules have passed a probe Quality Control (QC) process.
6. The kit of any one of embodiments 1-5, wherein the adaptor QC process comprises a test for adaptor ligation, wherein the test for adaptor ligation comprises:
   a. ligating the set of adaptors to a pre-determined amount of end-repaired DNA fragments to generate a library of adaptor-tagged DNA fragments (LIBS); and
   b. amplifying the LIBS to generate a Library Post Amplification (LPA);
   wherein the set of adaptors is considered to have passed the test for adaptor ligation when the concentration of the LPA is higher than a pre-determined concentration.
7. The kit of embodiment 6, wherein the pre-determined amount of the end-repaired DNA fragments is about 5 ng to about 50 ng and the pre-determined concentration of the LPA is about 1 ng/µL to about 200 ng/µL.
8. The kit of any one of embodiments 6-7, wherein the set of adaptors is considered to have passed the adaptor QC process when it has passed the test for adaptor ligation.
9. The kit of any one of embodiments 1-8, wherein the adaptor QC process comprises a test for adaptor distribution comprising:
   a. ligating the set of adaptors to a pre-determined amount of end-repaired DNA fragments to generate a library of adaptor-tagged DNA fragments (LIBS);
   b. amplifying the LIBS using a primer pair comprising at least one primer comprising an index sequence to generate a Library Post Index Amplification (LPIA); and
   c. performing a quantitative genetic analysis on the LPIA;
   wherein the set of adaptors is considered to have passed the test for adaptor distribution when one or more pre-determined acceptance criteria for the quantitative genetic analysis has been met.
10. The kit of embodiment 9, wherein the pre-determined acceptance criteria comprise one or more of:
   a. Barcode Crosstalk is present in no more than 0.05%-5% of reads;
   b. unknown adaptors are present in no more than 1%-50% of reads;
   c. no more than 10%-80% of unique adaptor sequences have a number of reads that is 0% to 50% of the average number of reads for all unique adaptor sequences;
   d. at least 60%-99.9% of all unique adaptor sequences are present;
   e. no more than 5%-50% of unique adaptor sequences have reads greater than twice the average number of reads for all unique adaptor sequences.
11. The kit of any one of embodiments 9-10, wherein the set of adaptors is considered to have passed the adaptor QC process when it has passed the test for adaptor distribution.
12. The kit of any one of embodiments 9-11, wherein the set of adaptors is considered to have passed the adaptor QC process when it has passed both the test for adaptor ligation and the test for adaptor distribution.
13. The kit of any one of embodiments 1-12, wherein the probe QC process comprises a test for capture probe modules comprising:
   a. ligating a set of adaptors to a DNA sample comprising end-repaired DNA fragments to generate a library of adaptor-tagged DNA fragments (LIBS);
   b. amplifying the LIBS to generate a Library Post Amplification (LPA);
   c. splitting or diluting the LPA to generate a Target Capture LPA (TC LPA) and a Whole-Genome LPA (WG LPA);
   d. amplifying the WG LPA to generate a Whole-Genome Library Amplified (WGLA);
   e. hybridizing the one or more capture probe modules to be tested to the TC LPA to form adaptor-tagged DNA fragment-capture probe module complexes;
   f. isolating the adaptor-tagged DNA fragment-capture probe module complexes to form isolated adaptor-tagged DNA fragment-capture probe module complexes;
   g. enzymatically processing the isolated adaptor-tagged DNA fragment-capture probe module complexes to generate Hybrid Molecules, wherein each Hybrid Molecule comprises the capture probe module and a complement of the adaptor-tagged DNA fragment;
   h. amplifying the Hybrid Molecules to generate a Target Capture Library Amplified (TCLA);
   i. combining the WGLA and the TCLA to form a Sequence-Ready Library (SRL);
   j. performing a quantitative genetic analysis on the SRL;

   wherein the DNA sample comprises a plurality of single nucleotide polymorphisms (SNPs);
   wherein the one or more capture probe modules are considered to have passed the probe QC process if one or more pre-determined acceptance criteria for the quantitative genetic analysis have been met.
14. The kit of embodiment 13, wherein the TC LPA is at least 1 ng/µL to at least 80 ng/µL, the WG LPA is at least 0.1 ng/µL to at least 8 ng/µL, the TCLA is at least 1 ng/µL to at least 80 ng/µL, and the WGLA is at least 1 ng/µL to at least 80 ng/µL.
15. The kit of embodiment 13 or embodiment 14, wherein the pre-determined acceptance criteria comprise one or more of:
   a. at least 60%-99.9% of capture probes have at least 1 total reads;
   b. at least 60%-99.9% of capture probes have at least 10 to at least 200 on-target total reads; and
   c. at least 60%-99.9% of expected SNPs within the DNA sample are detected.
16. The kit of any one of embodiments 12-15, comprising the DNA sample used in the probe QC process.
17. The kit of any one of embodiments 1-16, wherein the set of adaptors has passed an adaptor QC process performed according to the methods of any one of embodiments 174-219.
18. The kit of any one of embodiments 1-17, wherein the one or more capture probe modules have passed a probe QC process performed according to the methods of any one of embodiments 220-240.
19. The kit of any one of embodiments 1-18, comprising a first primer pair comprising a first F primer and a first R primer,
   wherein each adaptor module comprises an amplification region;
   wherein the first F primer comprises an amplification region binding region and a sequencing primer binding region;
   wherein the first R primer comprises a tail sequence binding region and a sequencing primer binding region.
20. The kit of embodiment 19, wherein the first primer pair is used to generate a first modified library.
21. The kit of any one of embodiments 1-20, comprising a second primer pair comprising a second F primer and a second R primer,
   wherein each of the second F primer and the second R primer comprises an amplification region binding region and a sequencing primer binding region.
22. The kit of embodiment 21, wherein the second primer pair is used to generate a second modified library.
23. The kit of any one of embodiments 1-22 comprising:
   (c) a first primer pair comprising a first F primer and a first R primer,
   wherein each adaptor module comprises an amplification region,
   wherein the first F primer comprises an amplification region binding region and a sequencing primer binding region,
   wherein the first R primer comprises a tail sequence binding region and a sequencing primer binding region;
   (d) a second primer pair comprising a second F primer and a second R primer,
   wherein each of the second F primer and the second R primer comprises an amplification region binding region and a sequencing primer binding region.
24. The kit of embodiment 23, wherein the first primer is used to generate a first modified library and the second primer is used to generate a second modified library.
25. The kit of embodiment 24, wherein the first modified library and the second modified library are configured to be combined into a Sequence-Ready Library (SRL).
26. The kit of any one of embodiment 1-25, wherein the tail sequence of each capture probe module comprises a Library Tag.
27. The kit of embodiment 26, comprising:
   (c) a first primer pair comprising a first F primer and a first R primer,
   wherein each adaptor module comprises an amplification region,
   wherein the first F primer comprises an amplification region binding region and a sequencing primer binding region,
   wherein the first R primer comprises a Library Tag binding region and a sequencing primer binding region;
   (d) a second primer pair comprising a second F primer and a second R primer,
   wherein each of the second F primer and the second R primer comprises an amplification region binding region and a sequencing primer binding region,
   wherein none of the primers of the second primer pair bind to the Library Tag.
28. The kit of embodiment 27, wherein the first primer is used to generate a first modified library and the second primer is used to generate a second modified library.
29. The kit of embodiment 28, wherein the first modified library and the second modified library are configured to be combined into a Sequence-Ready Library (SRL).
30. The kit of any one of embodiments 26-29, wherein the Library Tag comprises a nucleic acid sequence or an amino acid sequence.
31. The kit of any one of embodiments 26-30, wherein the Library Tag comprises one or more of a DNA, an RNA, a PNA, or a non-naturally occurring nucleic acid, a synthetic nucleic acid, a modified nucleic acid, a non-naturally occurring amino acid, a synthetic amino acid, and a modified amino acid.
32. The kit of any one of embodiments 26-31, wherein the Library Tag comprises a detectable label.
33. The kit of embodiment 32, wherein the detectable label comprises one or more of a fluorescent moiety, a magnetic or paramagnetic moiety, an enzymatic moiety, a binding moiety, an epitope, and a radioactive moiety.
34. The kit of any one of embodiments 26-33, wherein the Library Tag selectively or specifically binds to a detectable moiety.
35. The kit of embodiment 34, wherein the detectable moiety comprises one or more of a fluorescent moiety, a magnetic or paramagnetic moiety, an enzymatic moiety, a binding moiety, an epitope, and a radioactive moiety.
36. The kit of any one of embodiments 26-35, wherein the Library Tag comprises a unique polynucleotide sequence.
37. The kit of embodiment 36, wherein the unique polynucleotide sequence has no more than 70% sequence identity to any DNA fragment of the test sample.
38. A kit comprising:
   a. a set of adaptors, wherein each adaptor comprises an adaptor module comprising an amplification region;
   b. one or more capture probe modules, wherein each capture probe module comprises a tail sequence and a capture probe sequence capable of hybridizing to a target sequence in a test sample, wherein the tail sequence of each capture probe module comprises a Library Tag;
   c. a first primer pair comprising a first F primer and a first R primer, wherein the first F primer comprises an amplification region binding region and a sequencing primer binding region; wherein the first R primer comprises a Library Tag binding region and a sequencing primer binding region;
   d. a second primer pair comprising a second F primer and a second R primer, wherein each of the second F primer and the second R primer comprises an amplification region binding region and a sequencing primer binding region, wherein none of the primers of the second primer pair bind to the Library Tag.
39. A kit for generating a first modified library and a second modified library, comprising:
   a. a set of adaptors, wherein each adaptor comprises an adaptor module;
   b. one or more capture probe modules, wherein each capture probe module comprises a tail sequence and a capture probe sequence capable of hybridizing to a target sequence in a test sample;

   wherein the first modified library comprises a DNA fragment comprising an adaptor, a capture probe module, and the capture probe module's target sequence;
   wherein the second modified library comprises a DNA fragment comprising an adaptor and at least a portion of a DNA sequence of the test sample.
40. The kit of embodiment 39, wherein the tail sequence of each capture probe module comprises a Library Tag.
41. The kit of embodiment 39 or embodiment 40, wherein both the first modified library and the second modified library are generated from the test sample.
42. The kit of any one of embodiments 39-41, comprising:
   (c) a first primer pair comprising a first F primer and a first R primer,
   wherein each adaptor module comprises an amplification region,
   wherein the first F primer comprises an amplification region binding region and a sequencing primer binding region,
   wherein the first R primer comprises a tail sequence binding region and a sequencing primer binding region; and
   (d) a second primer pair comprising a second F primer and a second R primer,
   wherein each of the second F primer and the second R primer comprises an amplification region binding region and a sequencing primer binding region.
43. The kit of any one of embodiments 39-42, wherein the tail sequence of each capture probe module comprises a Library Tag.
44. The kit of embodiment 43, comprising:
   (c) a first primer pair comprising a first F primer and a first R primer,
   wherein each adaptor module comprises an amplification region,
   wherein the first F primer comprises an amplification region binding region and a sequencing primer binding region,
   wherein the first R primer comprises a Library Tag binding region and a sequencing primer binding region;
   (d) a second primer pair comprising a second F primer and a second R primer,
   wherein each of the second F primer and the second R primer comprises an amplification region binding region and a sequencing primer binding region,
   wherein none of the primers of the second primer pair bind to the Library Tag.
45. The kit of any one of embodiments 38-44, wherein the first primer pair is used to generate a first modified library and the second primer pair is used to generate a second modified library.
46. The kit of embodiment 45, wherein the first modified library and the second modified library are configured to be combined into a Sequence-Ready Library (SRL).
47. The kit of any one of embodiments 39-46,
   wherein the first modified library comprises a first DNA fragment comprising an adaptor, a capture probe module, and the capture probe module's target sequence;
   wherein the second modified library comprises a second DNA fragment comprising an adaptor and a at least a portion of a DNA sequence of the test sample.
48. The kit of any one of embodiments 39-47, wherein the first modified library and the second modified library are configured to be combined into a Sequence-Ready Library (SRL).
49. The kit of any one of embodiments 39-48, wherein each library fragment of the first modified library is an adaptor-tagged DNA fragment comprising an adaptor, a capture probe module, and at least a portion of a DNA sequence of the test sample;
   wherein each library fragment of the second modified library is an adaptor-tagged DNA fragment comprising an adaptor and at least a portion of a DNA sequence of the test sample.
50. The kit of embodiment 49, wherein none of the adaptor-tagged DNA fragments of the second modified library comprises a capture probe module.
51. The kit of any one of embodiments 39-50, wherein the first modified library is a Target Capture Library (TCL) or amplified Target Capture Library (TCLA).
52. The kit of any one of embodiments 39-51, wherein the second modified library is a Whole-Genome Library (WGL) or amplified Whole-Genome Library (WGLA).
53. The kit of any one of embodiments 39-52, wherein the first modified library or the second modified library comprises the Library Tag, wherein the Library Tag is configured to distinguish the first modified library from the second modified library.
54. The kit of any one of embodiments 39-52, wherein the first modified library comprises a first Library Tag and the second modified library comprises a second Library Tag, wherein the first Library Tag and the second Library Tag are not identical.
55. The kit of any one of embodiments 39-54, wherein each library fragment of the first modified library comprises, from 5' to 3':
   a 5' oligonucleotide (A1), a first 5' adaptor module, at least a portion of a DNA sequence of the test sample, and a 3' oligonucleotide (A2), or
   a 5' oligonucleotide (A1), at least a portion of a DNA sequence of the test sample, a first 3' adaptor module, and a 3' oligonucleotide (A2);
   wherein each library fragment of the second modified library comprises, from 5' to 3':
      a 5' oligonucleotide (B1), a second 5' adaptor module, at least a portion of a DNA sequence of the test sample, a second 3' adaptor module, and a 3' oligonucleotide (B2).
56. The kit of embodiment 55, wherein at least one of A1, A2, B1, and B2 contains at least one Library Tag.
57. The kit of embodiment 56, wherein the Library Tag comprises a unique polynucleotide sequence, wherein the unique polynucleotide sequence has 70% identity, or less, to a sequence selected from the list consisting of A1, B1, A2, B2, the first 5' adaptor module, the target sequence, the first 3' adaptor module, the second 5' adaptor module, any DNA fragment of the test sample, and the second 3' adaptor module.
58. The kit of any one of embodiments 39-57,
   wherein each library fragment of the first modified library comprises, from 5' to 3':
   a 5' oligonucleotide (A1), a first 5' adaptor module, at least a portion of a DNA sequence of the test sample, and a 3' oligonucleotide (A2), or
   a 5' oligonucleotide (A1), at least a portion of a DNA sequence of the test sample, a first 3' adaptor module, and a 3' oligonucleotide (A2);
   wherein each library fragment of the second modified library comprises, from 5' to 3': a 5' oligonucleotide (B 1), a second 5' adaptor module, at least a portion of a DNA sequence of the test sample, a second 3' adaptor module, and a 3' oligonucleotide (B2);
   wherein at least one of A1, A2, B1, and B2 comprises the Library Tag;
   wherein the Library Tag comprises a unique polynucleotide sequence, wherein the unique polynucleotide sequence has no more than 70% sequence identity to a sequence selected from the list consisting of A1, B1, A2, B2, the first 5' adaptor module, the target sequence, the first 3' adaptor module, the second 5' adaptor module, any DNA fragment of the test sample, and the second 3' adaptor module.
59. The kit of any one of embodiments 38-58, wherein the Library Tag comprises a nucleic acid sequence or an amino acid sequence.
60. The kit of any one of embodiments 38-59, wherein the Library Tag comprises one or more of a DNA, an RNA, a PNA, or a non-naturally occurring nucleic acid, a synthetic nucleic acid, a modified nucleic acid, a non-naturally occurring amino acid, a synthetic amino acid, and a modified amino acid.
61. The kit of any one of embodiments 38-60, wherein the Library Tag comprises a detectable label.
62. The kit of embodiment 61, wherein the detectable label comprises one or more of a fluorescent moiety, a magnetic or paramagnetic moiety, an enzymatic moiety, a binding moiety, an epitope, and a radioactive moiety.
63. The kit of any one of embodiments 38-62, wherein the Library Tag selectively or specifically binds to a detectable moiety.
64. The kit of embodiment 63, wherein the detectable moiety comprises one or more of a fluorescent moiety, a magnetic or paramagnetic moiety, an enzymatic moiety, a binding moiety, an epitope, and a radioactive moiety.
65. The kit of any one of embodiments 38-64, wherein the Library Tag comprises a unique polynucleotide sequence.
66. The kit of embodiment 65, wherein the unique polynucleotide sequence has no more than 70% sequence identity to any DNA fragment of the test sample.
67. The kit of any one of embodiments 1-66, wherein each adaptor of the set of adaptors comprises a ligation strand oligonucleotide and a non-ligation strand oligonucleotide.
68. The kit of embodiment 67, wherein the non-ligation strand oligonucleotide is capable of hybridizing to a region at the 3' end of the ligation strand oligonucleotide and forming a duplex therewith.
69. The kit of embodiment 67 or embodiment 68, wherein the ligation strand oligonucleotide comprises an adaptor module.
70. The kit of any one of embodiments 67-69, wherein the ligation strand oligonucleotide comprises a dT, dA, dC, or dG overhang at the 3' terminus.
71. The kit of any one of embodiments 67-70, wherein the non-ligation strand oligonucleotide comprises a modification at its 3' terminus that prevents ligation to the 5' end of a dsDNA fragment and/or adaptor dimer formation, wherein the non-ligation strand is configured to be displaced from the duplex.
72. The kit of any one of embodiments 1-71, wherein each adaptor of the set of adaptors comprises an ID region selected from a pool of unique ID regions, wherein the pool is selected from a plurality of pools, and wherein the selected pool is unique to the test sample.
73. The kit of any one of embodiments 1-72, wherein the adaptor module comprises:
   a. an amplification region comprising a primer binding site;
   b. an ID region; and
   c. an anchor region.
74. The kit of embodiment 73, wherein the amplification region comprises a primer binding site, wherein the primer binding site allows for amplification using PCR (polymerase chain reaction), LAMP (loop-mediated isothermal amplification), NASBA (nucleic acid sequence-based amplification), SDA (standard displacement amplification), RCA (rolling circle replication), or LCR (ligase chain reaction).
75. The kit of embodiment 73 or embodiment 74, wherein the amplification region comprises or consists of about 10 to about 50 nucleotides.
76. The kit of embodiment 75, wherein the amplification region comprises or consists of about 20 to about 30 nucleotides.
77. The kit of embodiment 76, wherein the amplification region comprises or consists of 25 nucleotides.
78. The kit of any one of embodiments 73-77, wherein the anchor region comprises an overhang at the 3' terminus.
79. The kit of any one of embodiments 73-78, wherein the anchor region comprises or consists of about 1 to about 50 nucleotides.
80. The kit of embodiment 79, wherein the anchor region comprises or consists of about 5 to about 25 nucleotides.
81. The kit of embodiment 80, wherein the anchor region comprises or consists of 10 nucleotides.
82. The kit of any one of embodiments 73-81, wherein the ID region comprises or consists of about 3 to about 50 nucleotides.
83. The kit of embodiment 82, wherein the ID region comprises or consists of about 3 to about 15 nucleotides.
84. The kit of embodiment 83, wherein the ID region comprises or consists of 8 nucleotides.
85. The kit of any one of embodiments 73-84, wherein the adaptor module further comprises a unique molecule identifier (UMI) multiplier.
86. The kit of embodiment 85, wherein the UMI multiplier is adjacent to or contained within the ID region.
87. The kit of embodiment 85 or embodiment 86, wherein the UMI multiplier comprises or consists of about 1 to about 5 nucleotides.
88. The kit of embodiment 87, wherein the UMI multiplier is 3 nucleotides in length, and comprises a nucleic acid sequence selected from a group of 64 unique nucleotide sequences.
89. The kit of any one of embodiments 72-88, wherein the pool of ID regions comprises about 2 to about 10,000 unique ID region sequences.
90. The kit of embodiment 89, wherein the pool of ID regions comprises about 10 to about 500 unique ID region sequences.
91. The kit of embodiment 90, wherein the pool of ID regions comprises about 50 to about 300 unique ID region sequences.
92. The kit of embodiment 91, wherein the pool of ID regions comprises 60 unique ID region sequences.
93. The kit of any one of embodiments 72-92, wherein each ID region of the pool of ID regions is 8 nucleotides in length.
94. The kit of any one of embodiments 72-94, wherein each ID region sequence is discrete from any other ID region sequence by Hamming distance of at least two.
95. The kit of any one of embodiments 72-94, wherein each ID region is configured to identify the DNA fragment attached thereto.
96. The kit of any one of embodiments 72-95, wherein each adaptor module of the set of adaptor modules is selected from a group consisting of about 64 to about 2,560,000 unique nucleotide sequences.
97. The kit of embodiment 96, wherein each adaptor module of the set of adaptor modules comprises a unique nucleotide sequence selected from 3840 unique nucleotide sequences, wherein each sequence of the 3840 unique nucleotide sequences is discrete from any other sequence by Hamming distance of at least two.
98. The kit of any one of embodiments 73-97, wherein the anchor region of each adaptor of the set of adaptors comprises one of four nucleotide sequences, and wherein each ID region of a given sequence is paired to only one of the four anchor regions of a given sequence.
99. The kit of any one of embodiments 73-98, wherein the amplification region of each adaptor of the set of adaptors comprises an identical primer binding site.
100. The kit of embodiment 99,
   wherein each ID region of the pool of ID regions is 8 nucleotides in length, wherein each ID region sequence is discrete from any other ID region sequence by Hamming distance of at least two;
   wherein each adaptor of the set of adaptors comprises a UMI multiplier that is adjacent to or contained within the ID region, wherein the UMI multiplier of each adaptor of the set of adaptors is three nucleotides in length, and wherein the UMI multiplier of a given sequence is paired to one ID region of a given sequence;
   wherein the anchor region of each adaptor of the set of adaptors comprises one of four nucleotide sequences, and wherein each ID region of a given sequence is paired to only one of the four anchor regions of a given sequence.
101. The kit of any one of embodiments 38-100, wherein the set of adaptors have passed an adaptor Quality Control (QC) process.
102. The kit of embodiment 101, wherein the adaptor QC process comprises a test for adaptor ligation, wherein the test for adaptor ligation comprises:
   a. ligating the set of adaptors to a pre-determined amount of end-repaired DNA fragments to generate a library of adaptor-tagged DNA fragments (LIBS); and
   b. amplifying the LIBS to generate a Library Post Amplification (LPA);
   wherein the set of adaptors is considered to have passed the test for adaptor ligation when the concentration of the LPA is higher than a pre-determined concentration.
103. The kit of embodiment 101, wherein the pre-determined amount of the end-repaired DNA fragments is about 5 ng to about 50 ng and the pre-determined concentration of the LPA is about 1 ng/µL to about 200 ng/µL.
104. The kit of any one of embodiments 101-103, wherein the set of adaptors is considered to have passed the adaptor QC process when it has passed the test for adaptor ligation.
105. The kit of any one of embodiments 101-104, wherein the adaptor QC process comprises a test for adaptor distribution comprising:
   a. ligating the set of adaptors to a pre-determined amount of end-repaired DNA fragments to generate a library of adaptor-tagged DNA fragments (LIBS);
   b. amplifying the LIBS using at least one primer comprising an index sequence to generate a Library Post Index Amplification (LPIA); and
   c. performing a quantitative genetic analysis on the LPIA;
   wherein the set of adaptors is considered to have passed the test for adaptor distribution when one or more pre-determined acceptance criteria for the quantitative genetic analysis has been met.
106. The kit of any one of embodiments 101-105, wherein the pre-determined acceptance criteria comprise one or more of:
   a. Barcode Crosstalk is present in no more than 0.05%-5% of reads;
   b. unknown adaptors are present in no more than 1%-50% of reads;
   c. no more than 10%-80% of unique adaptor sequences have a number of reads that is 0% to 50% of the average number of reads for all unique adaptor sequences;
   d. at least 60%-99.9% of all unique adaptor sequences are present;
   e. no more than 5%-50% of unique adaptor sequences have reads greater than twice the average number of reads for all unique adaptor sequences.
107. The kit of any one of embodiments 105-106, wherein the set of adaptors is considered to have passed the adaptor QC process when it has passed the test for adaptor distribution.
108. The kit of any one of embodiments 102-107, wherein the set of adaptors is considered to have passed the adaptor QC process when it has passed both the test for adaptor ligation and the test for adaptor distribution.
109. The kit of any one of embodiments 101-108, wherein the set of adaptors has passed an adaptor QC process performed according to the methods of any one of embodiments 174-219.
110. The kit of any one of embodiments 1-109, comprising more than one set of adaptor modules.
111. The kit of embodiment 110, comprising about 2 to about100 sets of adaptor modules.
112. The kit of embodiment 111, comprising 48 sets of adaptor modules.
113. The kit of any one of embodiments 110-112, wherein each set of adaptor modules is unique to a given test sample.
114. The kit of any one of embodiments 1-113, comprising one or more reagents for ligating adaptors of the adaptor set to the DNA fragments of the test sample to generate an adaptor-tagged DNA library.
115. The kit of embodiment 114, wherein the one or more reagents for ligating adaptors comprises a DNA ligase.
116. The kit of embodiment 115, wherein the DNA ligase is T4 DNA ligase.
117. The kit of any one of embodiments 1-116, wherein the set of adaptors is configured to ligate to the DNA fragments of the test sample using a method comprising:
   a. ligating the set of adaptors with the DNA fragments to generate a plurality of adaptor/DNA fragment complexes; and
   b. contacting the plurality of adaptor/DNA fragment complexes with one or more enzymes to form an adaptor-tagged DNA library comprising a plurality of adaptor-tagged DNA fragments.
118. The kit of embodiment 117, wherein each adaptor/DNA fragment complex comprises a ligation strand oligonucleotide ligated to each end of the DNA fragment.
119. The kit of embodiment 117 or embodiment 118, wherein the non-ligation strand oligonucleotide is displaced from the adaptor/DNA fragment complex in step b.
120. The kit of any one of embodiments 114-119, comprising one or more reagents for amplifying the adaptor-tagged DNA library to create a Library Post-Amplification (LPA).
121. The kit of embodiment 120, wherein the one or more reagents for amplifying the adaptor-tagged DNA library comprises a DNA polymerase.
122. The kit of any one of embodiments 120-121, wherein one or more reagents for amplifying the adaptor-tagged DNA library comprises one or more primers for PCR.
123. The kit of embodiment 122, wherein the one or more primers comprise a single primer sequence that is complementary to the primer binding site of the adaptor amplification region.
124. The kit of any one of embodiments 120-123, comprising one or more reagents for creating an amplified Whole-Genome Library (WGLA) from the LPA.
125. The kit of embodiment 124, wherein the one or more reagents for creating a WGLA comprises one or more primers that hybridize to the primer binding site of the adaptor amplification region.
126. The kit of embodiment 125, wherein the one or more primers comprise a sequencing adaptor capable of binding to a flow cell.
127. The kit of embodiment 126, wherein the sequencing adaptor comprises a sequencing primer binding site.
128. The kit of any one of embodiments 124-127, wherein the one or more reagents for creating the WGLA comprises a DNA polymerase.
129. The kit of any one of embodiments 124-128, wherein the WGLA is the sequence ready.
130. The kit of any one of embodiments 1-129, wherein the tail sequence of the capture probe module comprises a primer binding site.
131. The kit of any one of embodiments 1-130, wherein the tail sequence comprises a sequencing primer binding site.
132. The kit of any one of embodiments 1-131, wherein the tail sequence is configured to hybridize to a partner oligonucleotide.
133. The kit of any one of embodiments 1-132, wherein each capture probe module is selected from a capture probe panel comprising a plurality of capture probe modules.
134. The kit of embodiment 133, wherein at least one capture probe module is configured to hybridize downstream of a specific DNA target region and at least one capture probe module is configured to hybridize upstream of the specific DNA target region.
135. The kit of embodiment 133 or embodiment 134, wherein each capture probe of the plurality of capture probe modules is configured to hybridize to its target sequence within about 200 bp of any other capture probe.
136. The kit of any one of embodiments 133-135, wherein each capture probe module of the capture probe panel hybridizes to an adaptor-tagged DNA fragment to form a plurality of adaptor-tagged DNA fragment-capture probe module complexes.
137. The kit of any one of embodiments 132-136, wherein the partner oligonucleotide comprises a specific member of a binding pair to enable isolation of the adaptor-tagged DNA fragment-capture probe module complexes.
138. The kit of embodiment 137, wherein the partner oligonucleotide comprises a biotin molecule.
139. The kit of any one of embodiments 1-136, wherein each capture probe module comprises a specific member of a binding pair to enable isolation of the adaptor-tagged DNA fragment-capture probe module complex.
140. The kit of embodiment 139, wherein the capture probe module comprises a biotin molecule.
141. The kit of any one of embodiments 1-140, wherein each capture probe module comprises a capture probe less than 60 nucleotides in length.
142. The kit of embodiment 141, wherein each capture probe module comprises a capture probe that is 40 nucleotides in length.
143. The kit of any one of embodiments 136-142, comprising components for isolating the adaptor-tagged DNA fragment-capture probe module complexes.
144. The kit of embodiment 143, wherein the components for isolating the adaptor-tagged DNA fragment-capture probe module complexes comprises streptavidin.
145. The kit of any one of embodiments 143-144, comprising one of more enzymes for enzymatically processing the isolated adaptor-tagged DNA fragment-capture probe module complexes.
146. The kit of embodiment 145, wherein the one or more enzymes comprise a 5'-3' polymerase configured to extend each capture probe of the adaptor-tagged DNA fragment-capture probe module complexes using the adaptor-tagged DNA fragment as a template to create a plurality of Hybrid Molecules (Target Capture Library), wherein each Hybrid Molecule comprises a capture probe module and a complement of an adaptor-tagged DNA fragment.
147. The kit of embodiment 146, comprising one or more reagents for amplifying the Hybrid Molecules to create a Target Capture Library Amplified (TCLA), wherein the first modified library is the TCLA.
148. The kit of embodiment 147, wherein the one or more reagents for amplifying the Hybrid Molecules comprise one or more primers comprising a sequencing adaptor capable of binding to a flow cell.
149. The kit of embodiment 148, wherein the sequencing adaptor comprises a sequencing primer binding site.
150. The kit of any one of embodiments 147-149, wherein the one or more reagents for amplifying the Hybrid Molecules comprise a DNA polymerase.
151. The kit of any one of embodiments 13-150, wherein the SRL is configured to be sequenced on a single flow cell of a sequencing machine.
152. The kit of any one of embodiments 13-151, comprising components for performing quantitative genetic analysis on the SRL.
153. The kit of embodiment 152, wherein the components for performing quantitative genetic analysis comprises one or more sequencing primers.
154. The kit any one of embodiments 152-153, wherein the quantitative genetic analysis is used to detect a nucleotide transition or transversion, a nucleotide insertion or deletion, a genomic rearrangement, or a change in copy number in the test sample DNA fragments.
155. The kit of any one of embodiments 38-154, wherein the one or more capture probe modules have passed a probe Quality Control (QC) process.
156. The kit of embodiment 155, wherein the probe QC process comprises a test for capture probe modules comprising:
   a. ligating a set of adaptors to a DNA sample comprising end-repaired DNA fragments to generate a library of adaptor-tagged DNA fragments (LIBS);
   b. amplifying the LIBS to generate a Library Post Amplification (LPA);
   c. splitting or diluting the LPA to generate a Target Capture LPA (TC LPA) and a Whole-Genome LPA (WG LPA);
   d. amplifying the WG LPA to generate a Whole-Genome Library Amplified (WGLA);
   e. hybridizing the one or more capture probe modules to be tested to the TC LPA to form adaptor-tagged DNA fragment-capture probe module complexes;
   f. isolating the adaptor-tagged DNA fragment-capture probe module complexes to form isolated adaptor-tagged DNA fragment-capture probe module complexes;
   g. enzymatically processing the isolated adaptor-tagged DNA fragment-capture probe module complexes to generate Hybrid Molecules, wherein each Hybrid Molecule comprises the capture probe module and a complement of the adaptor-tagged DNA fragment;
   h. amplifying the Hybrid Molecules to generate a Target Capture Library Amplified (TCLA);
   i. combining the WGLA and the TCLA to form a Sequence-Ready Library (SRL);
   j. performing a quantitative genetic analysis on the SRL;

   wherein the DNA sample comprises a plurality of single nucleotide polymorphisms (SNPs);
   wherein the one or more capture probe modules are considered to have passed the probe QC process if one or more pre-determined acceptance criteria for the quantitative genetic analysis has been met.
157. The kit of embodiment 156, wherein the TC LPA is at least 1 ng/µL to at least 80 ng/µL, the WG LPA is at least 0.1 ng/µL to at least 8 ng/µL, the TCLA is at least 1 ng/µL to at least 80 ng/µL, and the WGLA is at least 1 ng/µL to at least 80 ng/µL.
158. The kit of embodiment 156 or embodiment 157, wherein the pre-determined acceptance criteria comprise one or more of:
   a. at least 60%-99.9% of capture probes have at least 1 total reads;
   b. at least 60%-99.9% of capture probes have at least 10 to at least 200 on-target total reads; and
   c. at least 60%-99.9% of expected SNPs within the DNA sample are detected.
159. The kit of any one of embodiments 155-158, comprising the DNA sample used in the probe QC process.
160. The kit of any one of embodiments 155-159, wherein the one or more capture probe modules have passed a probe QC process performed according to the methods of any one of embodiments 220-240.
161. The kit of any one of embodiments 1-160, wherein the test sample is obtained from a tissue biopsy.
162. The kit of embodiment 161, wherein the tissue biopsy is obtained from a tumor or a tissue suspected of being a tumor.
163. The kit of embodiment 162, wherein the tissue biopsy is obtained from a malignant tumor or tumor suspected of being a malignant tumor.
164. The kit of any one of embodiments 1-163, wherein the test sample DNA fragments comprise cell free DNA (cfDNA), genomic DNA (gDNA), complementary DNA (cDNA), mitochondrial DNA, methylated DNA, demethylated DNA, or a combination thereof.
165. The kit of any one of embodiments 1-164, wherein the test sample DNA fragments comprise an epigenetic mark.
166. The kit of any one of embodiments 1-165, wherein the test sample DNA fragments are obtained from a library selected from the list consisting of a whole genome library, an amplicon library, a whole exome library, a cDNA library, or a methylated DNA library.
167. The kit of any one of embodiments 1-166, wherein the test sample is obtained from a biological sample selected from the group consisting of an amniotic fluid sample, a blood sample, a skin sample, a hair sample, a hair follicle sample, a saliva sample, a mucous sample, a sweat sample, a tear sample, an epithelial tissue sample, a urine sample, a semen sample, a seminal plasma sample, a serum sample, a prostatic fluid sample, a pre-ejaculatory fluid (Cowper's fluid) sample, an ocular fluid sample, an excreta sample, a biopsy sample, an ascites sample, a cerebrospinal fluid sample, a lymph sample, a tissue extract sample, a stool sample, and a formalin-fixed, paraffin embedded (FFPE) sample.
168. The kit of any one of embodiments 1-167, wherein the DNA fragments of the test sample have been end repaired prior to ligation to adaptors.
169. The kit of embodiment 168, comprising one or more reagents for performing end repair.
170. The kit of embodiment 169, wherein the one or more reagents for performing end repair comprise one or more enzymes selected from a DNA polymerase, a kinase, and a Klenow fragment.
171. The kit of embodiment 170, wherein the one or more reagents for performing end repair comprise a DNA polymerase I, a T4 DNA polymerase, a T4 polynucleotide kinase, and a Klenow fragment.
172. The kit of any one of embodiments 169-171, wherein the one or more reagents for performing end repair comprise an End Repair Buffer.
173. The kit of embodiment 172, wherein the End Repair Buffer comprises MgCl2, NaCl, Tris-HCL, DTT, KCL, and dNTPs.
174. A method for performing an adaptor Quality Control (QC) process on a set of adaptors, wherein each adaptor comprises an adaptor module.
175. The method of embodiment 174, wherein the adaptor QC process comprises a test for adaptor ligation, wherein the test for adaptor ligation comprises:
   a. ligating the set of adaptors to a pre-determined amount of end-repaired DNA fragments to generate a library of adaptor-tagged DNA fragments (LIBS); and
   b. amplifying the LIBS to generate a Library Post Amplification (LPA);
   wherein the set of adaptors is considered to have passed the test for adaptor ligation when the concentration of the LPA is higher than a pre-determined concentration.
176. The method of embodiment 175, wherein the pre-determined amount of the end-repaired DNA fragments is about 5 ng to about 50 ng and the pre-determined concentration of the LPA is about 1 ng/µL to about 200 ng/µL.
177. The method of any one of embodiments 175-176, wherein the set of adaptors is considered to have passed the adaptor QC process when it has passed the test for adaptor ligation.
178. The method of any one of embodiments 174-177, wherein the adaptor QC process comprises a test for adaptor distribution comprising:
   a. ligating the set of adaptors to a pre-determined amount of end-repaired DNA fragments to generate a library of adaptor-tagged DNA fragments (LIBS);
   b. amplifying the LIBS using at least one primer comprising an index sequence to generate a Library Post Index Amplification (LPIA); and
   c. performing a quantitative genetic analysis on the LPIA;
   wherein the set of adaptors is considered to have passed the test for adaptor distribution when one or more pre-determined acceptance criteria for the quantitative genetic analysis has been met.
179. The method of embodiment 178, wherein the pre-determined acceptance criteria comprise one or more of:
   a. Barcode Crosstalk is present in no more than 0.05%-5% of reads;
   b. unknown adaptors are present in no more than 1%-50% of reads;
   c. no more than 10%-80% of unique adaptor sequences have a number of reads that is 0% to 50% of the average number of reads for all unique adaptor sequences;
   d. at least 60%-99.9% of all unique adaptor sequences are present;
   e. no more than 5%-50% of unique adaptor sequences have reads greater than twice the average number of reads for all unique adaptor sequences.
180. The method of any one of embodiments 178-179, wherein the set of adaptors is considered to have passed the adaptor QC process when it has passed the test for adaptor distribution.
181. The method of any one of embodiments 175-180, wherein the set of adaptors is considered to have passed the adaptor QC process when it has passed both the test for adaptor ligation and the test for adaptor distribution.
182. The method of any one of embodiments 174-181, wherein each adaptor of the set of adaptors comprises a ligation strand oligonucleotide and a non-ligation strand oligonucleotide.
183. The method of embodiment 182, wherein the non-ligation strand oligonucleotide is capable of hybridizing to a region at the 3' end of the ligation strand oligonucleotide and forming a duplex therewith.
184. The method of embodiment 182 or embodiment 183, wherein the ligation strand oligonucleotide comprises an adaptor module.
185. The method of any one of embodiments 182-184, wherein the ligation strand oligonucleotide comprises a dT, dA, dC, or dG overhang at the 3' terminus.
186. The method of any one of embodiments 182-186, wherein the non-ligation strand oligonucleotide comprises a modification at its 3' terminus that prevents ligation to the 5' end of a dsDNA fragment and/or adaptor dimer formation, wherein the non-ligation strand is configured to be displaced from the duplex.
187. The method of any one of embodiments 174-186, wherein each adaptor of the set of adaptors comprises an ID region selected from a pool of unique ID regions, wherein the pool is selected from a plurality of pools, and wherein the selected pool is unique to a test sample.
188. The method of any one of embodiments 174-187, wherein the adaptor module comprises:
   a. an amplification region comprising a primer binding site;
   b. an ID region; and
   c. an anchor region.
189. The method of embodiment 188, wherein the amplification region comprises a primer binding site, wherein the primer binding site allows for amplification using PCR (polymerase chain reaction), LAMP (loop-mediated isothermal amplification), NASBA (nucleic acid sequence-based amplification), SDA (standard displacement amplification), RCA (rolling circle replication), or LCR (ligase chain reaction).
190. The method of embodiment 188 or embodiment 189, wherein the amplification region comprises or consists of about 10 to about 50 nucleotides.
191. The method of embodiment 190, wherein the amplification region comprises or consists of about 20 to about 30 nucleotides
192. The method of embodiment 191, wherein the amplification region comprises or consists of 25 nucleotides.
193. The method of any one of embodiments 188-192, wherein the anchor region comprises an overhang at the 3' terminus.
194. The method of any one of embodiments 188-193, wherein the anchor region comprises or consists of about 1 to about 50 nucleotides.
195. The method of embodiment 194, wherein the anchor region comprises or consists of about 5 to about 25 nucleotides.
196. The method of embodiment 195, wherein the anchor region comprises or consists of 10 nucleotides.
197. The method of any one of embodiments 188-196, wherein the ID region comprises or consists of about 3 to about 50 nucleotides.
198. The method of embodiment 197, wherein the ID region comprises or consists of about 3 to about 15 nucleotides.
199. The method of embodiment 198, wherein the ID region comprises or consists of 8 nucleotides.
200. The method of any one of embodiments 188-199, wherein the adaptor module further comprises a unique molecule identifier (UMI) multiplier.
201. The method of embodiment 200, wherein the UMI multiplier is adjacent to or contained within the ID region.
202. The method of embodiment 200 or embodiment 201, wherein the UMI multiplier comprises or consists of about 1 to about 5 nucleotides.
203. The method of embodiment 202, wherein the UMI multiplier is 3 nucleotides in length, and comprises a nucleic acid sequence selected from a group of 64 unique nucleotide sequences.
204. The method of any one of embodiments 187-203, wherein the pool of ID regions comprises about 2 to about 10,000 unique ID region sequences.
205. The method of embodiment 204, wherein the pool of ID regions comprises about 10 to about 500 unique ID region sequences.
206. The method of embodiment 205, wherein the pool of ID regions comprises about 50 to about 300 unique ID region sequences.
207. The method of embodiment 206, wherein the pool of ID regions comprises 60 unique ID region sequences.
208. The method of any one of embodiments 187-207, wherein each ID region of the pool of ID regions is 8 nucleotides in length.
209. The method of any one of embodiments 188 -208, wherein each ID region sequence is discrete from any other ID region sequence by Hamming distance of at least two.
210. The method of any one of embodiments 188-209, wherein each ID region is configured to identify a DNA fragment attached thereto.
211. The method of any one of embodiments 174-210, wherein each adaptor module of the set of adaptor modules is selected from a group consisting of about 64 to about 2,560,000 unique nucleotide sequences.
212. The method of embodiment 211, wherein each adaptor module of the set of adaptor modules comprises a unique nucleotide sequence selected from 3840 unique nucleotide sequences, wherein each sequence of the 3840 unique nucleotide sequences is discrete from any other sequence by Hamming distance of at least two.
213. The method of any one of embodiments 188-212, wherein the anchor region of each adaptor of the set of adaptors comprises one of four nucleotide sequences, and wherein each ID region of a given sequence is paired to only one of the four anchor regions of a given sequence.
214. The method of any one of embodiments 188-213, wherein the amplification region of each adaptor of the set of adaptors comprises an identical primer binding site.
215. The method of embodiment 214,
   wherein each ID region of the pool of ID regions is 8 nucleotides in length,
   wherein each ID region sequence is discrete from any other ID region sequence by Hamming distance of at least two;
   wherein each adaptor of the set of adaptors comprises a UMI multiplier that is adjacent to or contained within the ID region, wherein the UMI multiplier of each adaptor of the set of adaptors is three nucleotides in length, and wherein the UMI multiplier of a given sequence is paired to one ID region of a given sequence;
   wherein the anchor region of each adaptor of the set of adaptors comprises one of four nucleotide sequences, and wherein each ID region of a given sequence is paired to only one of the four anchor regions of a given sequence.
216. The method of any one of embodiments 182-215, comprising:
   a. ligating the set of adaptors with DNA fragments of a test sample to generate a plurality of adaptor/DNA fragment complexes; and
   b. contacting the plurality of adaptor/DNA fragment complexes with one or more enzymes to form an adaptor-tagged DNA library comprising a plurality of adaptor-tagged DNA fragments.
217. The method of embodiment 216, wherein each adaptor/DNA fragment complex comprises a ligation strand oligonucleotide ligated to each end of the DNA fragment.
218. The method of embodiment 216 or embodiment 217, wherein the non-ligation strand oligonucleotide is displaced from the adaptor/DNA fragment complex in step b.
219. The method of any one of embodiments 174-218, wherein the adaptor set is selected from a kit of any one of embodiments 1-173.
220. A method for performing a probe Quality Control (QC) process on one or more capture probe modules, wherein each capture probe module comprises a tail sequence and a capture probe sequence capable of hybridizing to a target sequence in a test sample.
221. The method of embodiment 220, wherein the probe QC process comprises a test for capture probe modules comprising:
   a. ligating a set of adaptors to a DNA sample comprising end-repaired DNA fragments to generate a library of adaptor-tagged DNA fragments (LIBS);
   b. amplifying the LIBS to generate a Library Post Amplification (LPA);
   c. splitting or diluting the LPA to generate a Target Capture LPA (TC LPA) and a Whole-Genome LPA (WG LPA);
   d. amplifying the WG LPA to generate a Whole-Genome Library Amplified (WGLA);
   e. hybridizing the one or more capture probe modules to be tested to the TC LPA to form adaptor-tagged DNA fragment-capture probe module complexes;
   f. isolating the adaptor-tagged DNA fragment-capture probe module complexes to form isolated adaptor-tagged DNA fragment-capture probe module complexes;
   g. enzymatically processing the isolated adaptor-tagged DNA fragment-capture probe module complexes to generate Hybrid Molecules, wherein each Hybrid Molecule comprises the capture probe module and a complement of the adaptor-tagged DNA fragment;
   h. amplifying the Hybrid Molecules to generate a Target Capture Library Amplified (TCLA);
   i. combining the WGLA and the TCLA to form a Sequence-Ready Library (SRL);
   j. performing a quantitative genetic analysis on the SRL;

   wherein the DNA sample comprises a plurality of single nucleotide polymorphisms (SNPs);
   wherein the one or more capture probe modules are considered to have passed the probe QC process if one or more pre-determined acceptance criteria for the quantitative genetic analysis has been met.
222. The method of embodiment 221, wherein the TC LPA is at least 1 ng/µL to at least 80 ng/µL, the WG LPA is at least 0.1 ng/µL to at least 8 ng/µL, the TCLA is at least 1 ng/µL to at least 80 ng/µL, and the WGLA is at least 1 ng/µL to at least 80 ng/µL..
223. The method of embodiment 221 or embodiment 222, wherein the pre-determined acceptance criteria comprise one or more of:
   a. at least 60%-99.9% of capture probes have at least 1 total reads;
   b. at least 60%-99.9% of capture probes have at least 10 to at least 200 on-target total reads; and
   c. at least 60%-99.9% of expected SNPs within the DNA sample are detected.
224. The method of any one of embodiments 220-223, wherein the tail sequence of the capture probe module comprises a primer binding site.
225. The method of any one of embodiments 220-224, wherein the tail sequence comprises a sequencing primer binding site.
226. The method of any one of embodiments 220-225, wherein the tail sequence is configured to hybridize to a partner oligonucleotide.
227. The method of any one of embodiments 220-226, wherein each capture probe module is selected from a capture probe panel comprising a plurality of capture probe modules.
228. The method of embodiment 227, wherein at least one capture probe module is configured to hybridize downstream of a specific DNA target region and at least one capture probe module is configured to hybridize upstream of the specific DNA target region.
229. The method of embodiment 227 or embodiment 228, wherein each capture probe of the plurality of capture probe modules is configured to hybridize to its target sequence within about 200 bp of any other capture probe.
230. The method of any one of embodiments 227-229, wherein each capture probe module of the capture probe panel hybridizes to an adaptor-tagged DNA fragment to form a plurality of adaptor-tagged DNA fragment-capture probe module complexes.
231. The method of any one of embodiments 226-230, wherein the partner oligonucleotide comprises a specific member of a binding pair to enable isolation of the adaptor-tagged DNA fragment-capture probe module complexes.
232. The method of embodiment 231, wherein the partner oligonucleotide comprises a biotin molecule.
233. The method of any one of embodiments 220-232, wherein each capture probe module comprises a specific member of a binding pair to enable isolation of the adaptor-tagged DNA fragment-capture probe module complex.
234. The method of embodiment 233, wherein the capture probe module comprises a biotin molecule.
235. The method of any one of embodiments 220-234, wherein each capture probe module comprises a capture probe less than 60 nucleotides in length.
236. The method of embodiment 235, wherein each capture probe module comprises a capture probe that is 40 nucleotides in length.
237. The method of any one of embodiments 231-236, wherein the binding pair comprises streptavidin.
238. The method of any one of embodiments 221-237, wherein step (g) comprises performing 5'-3' extension of each capture probe of the adaptor-tagged DNA fragment-capture probe module complexes using the adaptor-tagged DNA fragment as a template to create a plurality of Hybrid Molecules (Target Capture Library), wherein each Hybrid Molecule comprises a capture probe module and a complement of an adaptor-tagged DNA fragment.
239. The method of any one of embodiments 221-238, wherein step (j) comprises sequencing the SRL on a single flow cell of a sequencing machine.
240. The method of any one of embodiments 221-239, wherein the quantitative genetic analysis is used to detect a nucleotide transition or transversion, a nucleotide insertion or deletion, a genomic rearrangement, or a change in copy number in the test sample DNA fragments.
241. A method for simultaneous DNA analysis, comprising:
   (a) to a first adaptor-tagged library comprising a first genetic locus, performing a first process to generate a first modified library;
   (b) to a second adaptor-tagged library comprising a second genetic locus, performing a second process to generate a second modified library;
      wherein the first process and the second process are not identical; and
   (c) contacting the first modified library and the second modified library to generate a combined library;
   wherein the first adaptor-tagged library and the second adaptor-tagged library are from the same adaptor-tagged parent library.
242. The method of embodiment 241, the method further comprising, an initial step of splitting the adaptor-tagged parent library into at least two partitions, wherein a first partition is the first adaptor-tagged library and a second partition is the second adaptor-tagged library.
243. The method of embodiment 241 or embodiment 242, wherein the first modified library comprises:
   a first DNA molecule comprising, from 5' to 3', a 5' oligonucleotide (A1), a first 5' adaptor module, a sequence of the first genetic locus, and a 3' oligonucleotide (A2); or
   a first DNA molecule comprising, from 5' to 3', a 5' oligonucleotide (A1), a sequence of the first genetic locus, a first 3' adaptor module, and a 3' oligonucleotide (A2); or
   a first DNA molecule comprising, from 5' to 3', a 5' oligonucleotide (A1), a first 5' adaptor module, a sequence of the first genetic locus, a first 3' adaptor module, and a 3' oligonucleotide (A2);
   wherein the first genetic locus comprises a DNA region;
   wherein a first test sample comprises a DNA molecule having a sequence identical to a sequence of the first genetic locus or a complementary sequence thereof; and
   wherein the first 5' adaptor module, the first 3' adaptor module, or both, are coupled to the first genetic locus in the first adaptor-tagged library;
   wherein the second modified library comprises:
      a second DNA molecule comprising, from 5' to 3', a 5' oligonucleotide (B1), a second 5' adaptor module, a sequence of the second genetic locus, and a 3' oligonucleotide (B2); or
      a second DNA molecule comprising, from 5' to 3', a 5' oligonucleotide (B1), a sequence of the second genetic locus, a second 3' adaptor module and a 3' oligonucleotide (B2); or
      a second DNA molecule comprising, from 5' to 3', a 5' oligonucleotide (B1), a second 5' adaptor module, a sequence of the second genetic locus, a second 3' adaptor module and a 3' oligonucleotide (B2);

   wherein the second genetic locus comprises a DNA region;
   wherein a second test sample comprises a DNA molecule having a sequence identical to a sequence of the second genetic locus or a complementary sequence thereof; and
   wherein the second 5' adaptor module, the second 3' adaptor module, or both, are coupled to the second genetic locus in the second adaptor-tagged library;
   wherein either the first modified library or the second modified library comprises a Library Tag, or
   wherein the first modified library comprises a first Library Tag and the second modified library comprises a second Library Tag, wherein the first and second Library Tags are not identical.
244. The method of embodiment 243, wherein the first test sample and the second test sample are the same sample.
245. The method of embodiment 243, wherein the first test sample and the second test sample are not the same sample.
246. The method of any one of embodiments 243-245, wherein the first genetic locus and the second genetic locus are identical.
247. The method of any one of embodiments 243-245, wherein the first genetic locus and the second genetic locus are not identical.
248. The method of any one of embodiments 243-247, wherein the Library Tag distinguishes the first DNA sequence of the first modified library from the second DNA sequence of the second modified library.
249. The method of any one of embodiments 243-248, wherein at least one of A1, A2, B1, and B2 comprises at least one Library Tag.
250. The method of any one of embodiments 243-249, wherein the Library Tag comprises a nucleic acid sequence or an amino acid sequence.
251. The method of any one of embodiments 243-250, wherein the Library Tag comprises one or more of a DNA, an RNA, a PNA, or a non-naturally occurring nucleic acid, a synthetic nucleic acid, a modified nucleic acid, a non-naturally occurring amino acid, a synthetic amino acid, and a modified amino acid.
252. The method of any one of embodiments 243-251, wherein the Library Tag comprises a detectable label.
253. The method of embodiment 252, wherein the detectable label comprises one or more of a fluorescent moiety, a magnetic or paramagnetic moiety, an enzymatic moiety, a binding moiety, an epitope, and a radioactive moiety.
254. The method of any one of embodiments 243-253, wherein the Library Tag selectively or specifically binds to a detectable moiety.
255. The method of embodiment 254, wherein the detectable moiety comprises one or more of a fluorescent moiety, a magnetic or paramagnetic moiety, an enzymatic moiety, a binding moiety, an epitope, and a radioactive moiety.
256. The method of any one of embodiments 243-255, wherein the Library Tag comprises a unique polynucleotide sequence.
257. The method of embodiment 256, wherein the unique polynucleotide sequence comprises a sequence having 70% identity, or less, to a sequence selected from the list consisting of A1, B1, A2, B2, the first 5' adaptor module, the first genetic locus, the first 3' adaptor module, the second 5' adaptor module, the second genetic locus, and the second 3' adaptor module.
258. The method of any one of embodiments 243-257,
   wherein the first test sample and the second test sample are not the same sample;
   wherein A1, A2, B1, or B2 contains a Library Tag;
   wherein the Library Tag is capable of distinguishing the first DNA sequence of the first modified library from the second DNA sequence of the second modified library;
   wherein the Library Tag comprises a unique polynucleotide sequence; and
   wherein the unique polynucleotide sequence comprises a sequence having 70% identity, or less, to a sequence selected from the list consisting of A1, B1, A2, B2, the first 5' adaptor module, the first genetic locus, the first 3' adaptor module, the second 5' adaptor module, the second genetic locus, and the second 3' adaptor module.
259. The method of any one of embodiments 241-258, wherein the first adaptor-tagged library and the second adaptor-tagged library each comprises an adaptor module.
260. The method of any one of embodiments 241-259, wherein each adaptor comprises a ligation strand oligonucleotide and a non-ligation strand oligonucleotide.
261. The method of embodiment 260, wherein the non-ligation strand oligonucleotide is capable of hybridizing to a region at the 3' end of the ligation strand oligonucleotide and forming a duplex therewith.
262. The method of embodiment 260 or embodiment 261, wherein the ligation strand oligonucleotide comprises an adaptor module.
263. The method of any one of embodiments 260-262, wherein the ligation strand oligonucleotide comprises a dT, dA, dC, or dG overhang at the 3' terminus.
264. The method of any one of embodiments 260-263, wherein the non-ligation strand oligonucleotide comprises a modification at its 3' terminus that prevents ligation to the 5' end of a dsDNA fragment and/or adaptor dimer formation, wherein the non-ligation strand is configured to be displaced from the duplex.
265. The method of any one of embodiments 241-264, wherein each adaptor is selected from a set of adaptors, wherein each adaptor of the set of adaptors comprises an ID region selected from a pool of unique ID regions, wherein the pool is selected from a plurality of pools, and wherein the selected pool is unique to the test sample.
266. The method of any one of embodiments 241-265, wherein each adaptor module comprises:
   a. an amplification region comprising a primer binding site;
   b. an ID region; and
   c. an anchor region.
267. The method of embodiment 266, wherein the amplification region comprises a primer binding site, wherein the primer binding site allows for amplification using PCR (polymerase chain reaction), LAMP (loop-mediated isothermal amplification), NASBA (nucleic acid sequence-based amplification), SDA (standard displacement amplification), RCA (rolling circle replication), or LCR (ligase chain reaction).
268. The method of any one of embodiments 241-265, wherein each adaptor module comprises:
   a. an amplification region comprising a polynucleotide sequence comprising a primer binding site;
   b. an ID region; and
   c. an anchor region.
269. The method of embodiment 268, wherein the amplification region comprises a polynucleotide sequence comprising a primer binding site, wherein the primer binding site allows for amplification of the nucleic acids of the first and/or second adaptor-tagged library using PCR (polymerase chain reaction), LAMP (loop-mediated isothermal amplification), NASBA (nucleic acid sequence-based amplification), SDA (standard displacement amplification), RCA (rolling circle replication), or LCR (ligase chain reaction).
270. The method of any one of embodiments 266-269, wherein the amplification region comprises or consists of between 10 and 50 nucleotides.
271. The method of embodiment 270, wherein the amplification region comprises or consists of between 20 and 30 nucleotides.
272. The method of embodiment 271, wherein the amplification region comprises or consists of 25 nucleotides.
273. The method of any one of embodiments 266-272, wherein the anchor region comprises an overhang at the 3' terminus.
274. The method of any one of embodiments 266-273, wherein the anchor region comprises or consists of between 1 and 50 nucleotides.
275. The method of embodiment 274, wherein the anchor region comprises or consists of between 5 and 25 nucleotides.
276. The method of embodiment 275, wherein the anchor region comprises or consists of 10 nucleotides.
277. The method of any one of embodiments 266-276, wherein the ID region comprises or consists of between 3 and 50 nucleotides.
278. The method of embodiment 277, wherein the ID region comprises or consists of between 3 and 15 nucleotides.
279. The method of embodiment 278, wherein the ID region comprises or consists of 8 nucleotides.
280. The method of any one of embodiments 266-279, wherein each adaptor module further comprises a unique molecule identifier (UMI) multiplier.
281. The method of embodiment 280, wherein the UMI multiplier is adjacent to or contained within the ID region.
282. The method of embodiment 280 or embodiment 281, wherein the UMI multiplier comprises or consists of between 1 and 5 nucleotides.
283. The method of embodiment 282, wherein the UMI multiplier is 3 nucleotides in length, and comprises a nucleic acid sequence selected from a group of 64 unique nucleotide sequences.
284. The method of any one of embodiments 265-283, wherein a plurality of adaptor modules comprises the adaptor module.
285. The method of embodiment 284, wherein each adaptor module of the plurality of adaptor modules is unique.
286. The method of any one of embodiments 284-285, wherein the amplification region of each adaptor module of the plurality of adaptor modules comprises a custom primer binding site.
287. The method of embodiment 286, wherein the custom primer binding site comprises a sequence having 100% sequence identity to a sequence of the custom primer binding site of each adaptor module of the plurality of adaptor modules.
288. The method of any one of embodiments 284-287, wherein the plurality of adaptor modules comprises a first subdivision and a second subdivision.
289. The method of embodiment 288, wherein each adaptor module comprises an ID region comprising a polynucleotide sequence that is distinct from the polynucleotide sequence of the ID region of any other adaptor module.
290. The method of embodiment 289, wherein the first subdivision comprises a first set of unique ID regions and the second subdivision comprises a second set of unique ID regions.
291. The method of any one of embodiments 288-290, wherein each adaptor module further comprises a sample tag comprising a polynucleotide sequence that is not identical to the polynucleotide sequence of the ID region.
292. The method of embodiment 291, wherein each adaptor module in the first subdivision comprises a sample tag comprising a first polynucleotide sequence; wherein each adaptor module in the second subdivision comprises a sample tag comprising a second polynucleotide sequence; wherein the first and second polynucleotide sequences are not identical to each other.
293. The method of any one of embodiments 288-292, wherein the first subdivision of adaptor modules identifies each DNA molecule within the first test sample and the second subdivision of adaptor modules identifies each DNA molecule within the second test sample.
294. The method of any one of embodiments 284-293, wherein the ID region of each adaptor module of the plurality of adaptor modules is selected from a group consisting of between 2 and 10,000 unique nucleotide sequences.
295. The method of embodiment 294, wherein the ID region of each adaptor module of the plurality of adaptor modules is selected from a group consisting of between 50 and 500 unique nucleotide sequences.
296. The method of embodiment 295, wherein the ID region of each adaptor module of the plurality of adaptor modules is selected from a group consisting of between 100 and 400 unique nucleotide sequences.
297. The method of embodiment 296, wherein the ID region of each adaptor module of the plurality of adaptor modules is selected from a group consisting of 60 unique nucleotide sequences.
298. The method of any one of embodiments 284-297, wherein the ID region of each adaptor module of the plurality of adaptor modules is 8 nucleotides in length.
299. The method of any one of embodiments 284-298, wherein each adaptor module of the plurality of adaptor modules is selected from a group consisting of between 64 and 2,560,000 unique nucleotide sequences.
300. The method of embodiment 299, wherein each adaptor module of the plurality of adaptor modules comprises one of 3840 unique nucleotide sequences, and each nucleotide sequence is discrete from any other sequence of the 3840 unique nucleotide sequences by Hamming distance of at least two.
301. The method of any one of embodiments 284-300, wherein the anchor region of each adaptor module of the plurality of adaptor modules is selected from a group consisting of four nucleotide sequences.
302. The method of embodiment 301,
   wherein the amplification regions of each adaptor module of the plurality of adaptor modules comprise a universal primer sequence;
   wherein the ID region of each adaptor module of the plurality of adaptor modules comprises or consists of 8 nucleotides;
   wherein the plurality of adaptor modules is divided into two or more subdivisions, wherein each subdivision of adaptor modules comprises a set of unique ID regions, wherein the polynucleotide sequence of each ID region is discrete from the nucleotide sequence of any other ID regions of the plurality of adaptor modules by Hamming distance of at least two;
   wherein each adaptor module of the plurality of adaptor modules comprises a UMI multiplier that is adjacent to or contained within the ID region, wherein the UMI multiplier of each adaptor module of the plurality of adaptor modules comprises or consists of three nucleotides.
303. The method of any one of embodiments 265-283, wherein the pool of ID regions comprises between 2 and 10,000 unique ID region sequences.
304. The method of embodiment 303, wherein the pool of ID regions comprises 60 unique ID region sequences.
305. The method of any one of embodiments 265-304, wherein each ID region of the pool of ID regions is 8 nucleotides in length.
306. The method of any one of embodiments 265-305, wherein each ID region sequence is discrete from any other ID region sequence by Hamming distance of at least two.
307. The method of any one of embodiments 265-306, wherein each ID region is configured to identify the DNA fragment attached thereto.
308. The method of any one of embodiments 265-307, wherein each adaptor module of the set of adaptor modules is selected from a group consisting of between 64 and 2,560,000 unique nucleotide sequences.
309. The method of embodiment 308, wherein each adaptor module of the set of adaptor modules comprises a unique nucleotide sequence selected from 3840 unique nucleotide sequences, wherein each sequence of the 3840 unique nucleotide sequences is discrete from any other sequence by Hamming distance of at least two.
310. The method of any one of embodiments 265-309, wherein the anchor region of each adaptor of the set of adaptors comprises one of four nucleotide sequences, and wherein each ID region of a given sequence is paired to only one of the four anchor regions of a given sequence.
311. The method of any one of embodiments 265-310, wherein the amplification region of each adaptor of the set of adaptors comprises an identical primer binding site.
312. The method of embodiment 311,
   wherein each ID region of the pool of ID regions is 8 nucleotides in length,
   wherein each ID region sequence is discrete from any other ID region sequence by Hamming distance of at least two;
   wherein each adaptor of the set of adaptors comprises a UMI multiplier that is adjacent to or contained within the ID region, wherein the UMI multiplier of each adaptor of the set of adaptors is three nucleotides in length, and wherein the UMI multiplier of a given sequence is paired to one ID region of a given sequence.
313. The method of any one of embodiments 241-312, wherein the first process comprises:
   (a) contacting the first adaptor-tagged library with one or more capture probes under conditions suitable for hybridization to form one or more capture probe/adaptor-tagged DNA complexes, wherein each capture probe comprises:
      a first region comprising a primer binding site; and
      a second region capable of hybridizing to a target region in the first genetic locus of the first adaptor-tagged library;
   (b) isolating the one or more capture probe/adaptor-tagged DNA complexes from step (a), wherein each isolated capture probe/adaptor-tagged DNA complex comprises a capture probe and an adaptor-tagged DNA molecule; and
   (c) enzymatically processing the one or more isolated capture probe/adaptor-tagged DNA complexes from step (b) to generate one or more adaptor-tagged hybrid nucleic acid molecules (Hybrid Molecules), wherein each Hybrid Molecule comprises:
      (i) at least a portion of the capture probe or a complement thereof; and
      (ii) at least a portion of the adaptor-tagged DNA molecule or a complement thereof.
314. The method of embodiment 313, wherein the first region of the capture probe comprises a sequence having complementarity to a partner oligonucleotide.
315. The method of any one of embodiments 313-314, wherein the enzymatic processing of step (c) comprises performing 5'-3' DNA polymerase extension of the capture probe using the adaptor-tagged DNA molecule in the complex as a template.
316. The method of any one of embodiments 313-315, wherein at least one capture probe hybridizes downstream of the specific region in the target region and at least one capture probe hybridizes upstream of the specific region in the target region.
317. The method of any one of embodiments 313-316, wherein the capture probe comprises a sequencing primer binding site.
318. The method of any one of embodiments 313-317, wherein the Hybrid Molecule comprises at least one Library Tag.
310. The method of any one of embodiments 313-318, wherein the first region of the capture probe comprises a Library Tag.
320. The method of embodiments 318-319, wherein the Library Tag comprises a sequencing primer binding site.
321. The method of any one of embodiments 318-320, further comprising the step of: (d) amplifying the one or more Hybrid Molecules in step (c).
322. The method of embodiment 321, wherein the amplifying comprises a first primer comprising a 5'oligonucleotide (A1) and a second primer comprising a 3' oligonucleotide (A2), and wherein each of the amplified Hybrid Molecule(s) comprise A1 and A2.
323. The method of any one of embodiments 313-322, wherein the target region comprises a genetic lesion.
324. The method of any one of embodiments 313-323, wherein the target region comprises an epigenetic mark.
325. The method of embodiment 324, wherein the capture probe binds to the epigenetic mark.
326. The method of embodiment 325, wherein the epigenetic mark comprises a methylation mark.
327. The method of any one of embodiments 313-326, wherein the first process comprises:
   (a) contacting the first adaptor-tagged library with one or more capture probes under conditions suitable for hybridization to form one or more capture probe/adaptor-tagged DNA complexes, wherein each capture probe comprises:
      a first region comprising:
         a Library Tag comprising a sequencing primer binding site, and
         a sequence having complementarity to a partner oligonucleotide; and
      a second region capable of hybridizing to a target region in the first genetic locus of the first adaptor-tagged library;
   (b) isolating the one or more capture probe/adaptor-tagged DNA complexes from step (a), wherein each isolated capture probe/adaptor-tagged DNA complex comprises a capture probe and an adaptor-tagged DNA molecule;
   (c) enzymatically processing the one or more isolated capture probe/adaptor-tagged DNA complexes from step (b) to generate one or more adaptor-tagged hybrid nucleic acid molecules (Hybrid Molecules),
      wherein the enzymatic processing comprises performing 5'-3' DNA polymerase extension of the capture probe using the adaptor-tagged DNA molecule in the complex as a template;
      wherein each Hybrid Molecule comprises the capture probe and a complement of the adaptor-tagged DNA molecule that is 3' from where the capture probe hybridized to the target region in the first adaptor-tagged library; and
   (d) amplifying the one or more Hybrid Molecules in step (c), wherein the amplifying comprises hybridizing a first primer comprising a 5' oligonucleotide (A1) and a second primer comprising a 3' oligonucleotide (A2) to a Hybrid Molecule, and wherein each of the amplified Hybrid Molecule(s) comprise A1 and A2.
328. The method of any one of embodiments 241-327, wherein the second process comprises amplifying or extending the second genetic locus of the second adaptor-tagged library.
329. The method of embodiment 328, wherein the amplifying or extending comprises hybridizing a first primer comprising a 5' oligonucleotide (B1) and a second primer comprising a 3' oligonucleotide (B2) to an adaptor-tagged DNA molecule, and wherein the amplified or extended second genetic locus comprises B1 and B2.
330. The method of embodiment 329, wherein at least one of B1 and B2 comprises a Library Tag.
331. The method of any one of embodiments 241-330, wherein the second process comprises amplifying or extending the second genetic locus of the second adaptor-tagged library, wherein the amplifying or extending comprises hybridizing a first primer comprising a 5'oligonucleotide (B1) and a second primer comprising a 3' oligonucleotide (B2) to an adaptor-tagged DNA molecule, wherein at least one of B1 and B2 comprises a Library Tag, and wherein the amplified or extended second genetic locus comprises B1 and B2.
332. The method of any one of embodiments 241-331, further comprising a step of genetic analysis.
333. The method of embodiment 332, wherein the genetic analysis comprises detecting the presence of at least one Library Tag in at least one modified library.
334. The method of embodiment 333, wherein the genetic analysis comprises sequencing of the combined library to generate a plurality of sequencing reads and performing bioinformatics analysis on the plurality of sequencing reads.
335. The method of embodiment 334, wherein the first modified library comprises a first Library Tag and the second modified library comprises a second Library Tag.
336. The method of embodiment 335, wherein the first Library Tag generates a first signal and the second Library Tag generates a second signal.
337. The method of embodiment 336, wherein both the first and the second signals are detected or detectable.
338. The method of embodiment 337, wherein the first signal and the second signal are not identical.
339. The method of embodiment 336, wherein the first signal is detected or detectable and the second signal is not detected or detectable.
340. The method of embodiment 336, wherein the second signal is detected or detectable and the first signal is not detected or detectable.
341. The method of embodiment 334, wherein only one of the first modified library and the second modified library comprises a Library Tag.
342. The method of embodiment 341, wherein the Library Tag generates a first signal and the lack of the Library Tag generates a second signal.
343. The method of embodiment 342, wherein both the first and the second signals are detected or detectable.
344. The method of embodiment 343, wherein the first signal and the second signal are not identical.
345. The method of embodiment 341, wherein the first signal is detected or detectable and the second signal is not detected or detectable.
346. The method of embodiment 341, wherein the second signal is detected or detectable and the first signal is not detected or detectable.
347. The method of any one of embodiments 341-346, wherein the first signal is a sequencing read and the second signal is an empty sequencing read.
348. The method of embodiment 347, wherein the first signal is detected or detectable and the second signal is not detected or detectable.
349. The method of embodiment 348, wherein both the first signal and the second signal are detected or detectable.
350. The method of embodiment 349, wherein the empty sequencing read (the second signal) is detected as a series of two or more G nucleotides.
351. The method of any one of embodiments 336-350, wherein
   the detected first signal and the detected second signal can be differentiated from one another; or
   the detected first signal and the non-detected second signal can be differentiated from one another; or
   the non-detected first signal and the detected second signal can be differentiated from one another.
352. The method of any one of embodiments 332-351, wherein the genetic analysis is used to identify one or more genetic lesions.
353. The method of embodiment 352, wherein the one or more genetic lesions comprise a nucleotide transition, a nucleotide transversion, a nucleotide insertion, a nucleotide deletion, a genomic rearrangement, or a change in copy number.
354. The method of any one of embodiments 332-353, wherein the genetic analysis is used to detect one or more genetic lesions that cause or are associated with a genetic disease.
355. The method of embodiment 354, wherein the genetic disease is cancer.
356. The method of embodiment 355, wherein the one or more genetic lesions are chromosomal rearrangements.
357. The method of any one of embodiments 332-356, wherein the first modified library and second modified library are demultiplexed after sequencing by identification of at least one Library Tag or a portion thereof.
358. A composition comprising:
   a first modified library and a second modified library,
   wherein the first modified library comprises:
      a first DNA molecule comprising, from 5' to 3', a 5' oligonucleotide (A1), a first 5' adaptor module, a sequence of the first genetic locus, and a 3' oligonucleotide (A2); or
      a first DNA molecule comprising, from 5' to 3', a 5' oligonucleotide (A1), a sequence of the first genetic locus, a first 3' adaptor module, and a 3' oligonucleotide (A2); or
      a first DNA molecule comprising, from 5' to 3', a 5' oligonucleotide (A1), a first 5' adaptor module, a sequence of the first genetic locus, a first 3' adaptor module, and a 3' oligonucleotide (A2);
      wherein the first genetic locus comprises a DNA region;
      wherein a first test sample comprises a DNA molecule having a sequence identical to a sequence of the first genetic locus or a complementary sequence thereof; and
      wherein the first 5' adaptor module, the first 3' adaptor module, or both, are coupled to the first genetic locus in the first adaptor-tagged library;
      wherein the second modified library comprises:
         a second DNA molecule comprising, from 5' to 3', a 5' oligonucleotide (B1), a second 5' adaptor module, a sequence of the second genetic locus, and a 3' oligonucleotide (B2); or
         a second DNA molecule comprising, from 5' to 3', a 5' oligonucleotide (B1), a sequence of the second genetic locus, a second 3' adaptor module and a 3' oligonucleotide (B2); or
         a second DNA molecule comprising, from 5' to 3', a 5' oligonucleotide (B1), a second 5' adaptor module, a sequence of the second genetic locus, a second 3' adaptor module and a 3' oligonucleotide (B2);
         wherein the second genetic locus comprises a DNA region;
         wherein a second test sample comprises a DNA molecule having a sequence identical to a sequence of the second genetic locus or a complementary sequence thereof; and
         wherein the second 5' adaptor module, the second 3' adaptor module, or both, are coupled to the second genetic locus in the second adaptor-tagged library;
         wherein either the first modified library or the second modified library comprises a Library Tag, or
         wherein the first modified library comprises a first Library Tag and the second modified library comprises a second Library Tag, wherein the first and second Library Tags are not identical.
359. The composition of embodiment 358, wherein the first test sample and the second test sample are the same sample.
360. The composition of embodiment 358, wherein the first test sample and the second test sample are not the same sample.
361. The composition of any one of embodiments 358-360, wherein the first genetic locus and the second genetic locus are identical.
362. The composition of any one of embodiments 358-360, wherein the first genetic locus and the second genetic locus are not identical.
363. The composition of any one of embodiments 358-362, wherein the Library Tag distinguishes the first DNA sequence of the first modified library from the second DNA sequence of the second modified library.
364. The composition of any one of embodiments 358-363, wherein at least one of A1, A2, B1, and B2 contains at least one Library Tag.
365. The composition of any one of embodiments 358-364, wherein the Library Tag comprises a nucleic acid sequence or an amino acid sequence.
366. The composition of any one of embodiments 358-365, wherein the Library Tag comprises one or more of a DNA, an RNA, a PNA, or a non-naturally occurring nucleic acid, a synthetic nucleic acid, a modified nucleic acid, a non-naturally occurring amino acid, a synthetic amino acid, and a modified amino acid.
367. The composition of any one of embodiments 358-366, wherein the Library Tag comprises a detectable label.
368. The composition of embodiment 367, wherein the detectable label comprises one or more of a fluorescent moiety, a magnetic or paramagnetic moiety, an enzymatic moiety, a binding moiety, an epitope, and a radioactive moiety.
369. The composition of any one of embodiments 358-368, wherein the Library Tag selectively or specifically binds to a detectable moiety.
370. The composition of embodiment 369, wherein the detectable moiety comprises one or more of a fluorescent moiety, a magnetic or paramagnetic moiety, an enzymatic moiety, a binding moiety, an epitope, and a radioactive moiety.
371. The composition of any one of embodiments 358-370, wherein the Library Tag comprises a unique polynucleotide sequence.
372. The composition of embodiment 371, wherein the unique polynucleotide sequence comprises a sequence having 70% identity, or less, to a sequence selected from the list consisting of A1, B1, A2, B2, the first 5' adaptor module, the first genetic locus, the first 3' adaptor module, the second 5' adaptor module, the second genetic locus, and the second 3' adaptor module.
373. The composition of embodiment 358,
   wherein the first test sample and the second test sample are not the same sample;
   wherein A1, A2, B1, or B2 contains a Library Tag;
   wherein the Library Tag is capable of distinguishing the first DNA sequence of the first modified library from the second DNA sequence of the second modified library;
   wherein the Library Tag comprises a unique polynucleotide sequence; and
   wherein the unique polynucleotide sequence comprises a sequence having 70% identity, or less, to a sequence selected from the list consisting of A1, B1, A2, B2, the first 5' adaptor module, the first genetic locus, the first 3' adaptor module, the second 5' adaptor module, the second genetic locus, and the second 3' adaptor module
374. The composition of any one of embodiments 358-373, wherein each adaptor module is selected from a set of adaptor modules, wherein each adaptor module of the set of adaptor modules comprises an ID region selected from a pool of unique ID regions, wherein the pool is selected from a plurality of pools, and wherein the selected pool is unique to the test sample.
375. The composition of any one of embodiments 358-374, wherein each adaptor module comprises:
   a. an amplification region comprising a primer binding site;
   b. an ID region; and
   c. an anchor region.
376. The composition of embodiment 375, wherein the amplification region comprises a primer binding site, wherein the primer binding site allows for amplification using PCR (polymerase chain reaction), LAMP (loop-mediated isothermal amplification), NASBA (nucleic acid sequence-based amplification), SDA (standard displacement amplification), RCA (rolling circle replication), or LCR (ligase chain reaction).
377. The composition of any one of embodiments 358-374, wherein each adaptor module comprises:
   a. an amplification region comprising a polynucleotide sequence comprising a primer binding site;
   b. an ID region; and
   c. an anchor region.
378. The composition of embodiment 377, wherein the amplification region comprises a polynucleotide sequence comprising a primer binding site, wherein the primer binding site allows for amplification of the nucleic acids of the first and/or second adaptor-tagged library using for PCR (polymerase chain reaction), LAMP (loop-mediated isothermal amplification), NASBA (nucleic acid sequence-based amplification), SDA (standard displacement amplification), RCA (rolling circle replication), or LCR (ligase chain reaction).
379. The composition of any one of embodiments 377-378, wherein the amplification region comprises or consists of between 10 and 50 nucleotides.
380. The composition of embodiment 379, wherein the amplification region comprises or consists of between 20 and 30 nucleotides.
381. The composition of embodiment 380, wherein the amplification region comprises or consists of 25 nucleotides.
382. The composition of any one of embodiments 377-381, wherein the anchor region comprises an overhang at the 3' terminus.
383. The composition of any one of embodiments 377-382, wherein the anchor region comprises or consists of between 1 and 50 nucleotides.
384. The composition of embodiment 383, wherein the anchor region comprises or consists of between 5 and 25 nucleotides.
385. The composition of embodiment 384, wherein the anchor region comprises or consists of 10 nucleotides.
386. The composition of any one of embodiments 377-385, wherein the ID region comprises or consists of between 3 and 50 nucleotides.
387. The composition of embodiment 386, wherein the ID region comprises or consists of between 3 and 15 nucleotides.
388. The composition of embodiment 387, wherein the ID region comprises or consists of 8 nucleotides.
389. The composition of any one of embodiments 377-388, wherein the adaptor module further comprises a unique molecule identifier (UMI) multiplier.
390. The composition of embodiment 389, wherein the UMI multiplier is adjacent to or contained within the ID region.
391. The composition of embodiment 389 or 390, wherein the UMI multiplier comprises or consists of between 1 and 5 nucleotides.
392. The composition of embodiment 391, wherein the UMI multiplier is 3 nucleotides in length, and comprises a nucleic acid sequence selected from a group of 64 unique nucleotide sequences.
393. The composition of any one of embodiments 377-392, wherein a plurality of adaptor modules comprises the adaptor module.
394. The composition of embodiment 393, wherein each adaptor module of the plurality of adaptor modules is unique.
395. The composition of any one of embodiments 393-394, wherein the amplification region of each adaptor module of the plurality of adaptor modules comprises a custom primer binding site.
396. The composition of embodiment 395, wherein the custom primer binding site comprises a sequence having 100% sequence identity to a sequence of the custom primer binding site of each adaptor module of the plurality of adaptor modules.
397. The composition of any one of embodiments 393-396, wherein the plurality of adaptor modules comprises a first subdivision and a second subdivision.
398. The composition of embodiment 397, wherein each adaptor module comprises an ID region comprising a polynucleotide sequence that is distinct from the polynucleotide sequence of the ID region of any other adaptor module.
399. The composition of embodiment 398, wherein the first subdivision comprises a first set of unique ID regions and the second subdivision comprises a second set of unique ID regions.
400. The composition of any one of embodiments 397-399, wherein each adaptor module further comprises a sample tag comprising a polynucleotide sequence that is not identical to the polynucleotide sequence of the ID region.
401. The composition of embodiment 400, wherein each adaptor module in the first subdivision comprises a sample tag comprising a first polynucleotide sequence and each adaptor module in the second subdivision comprises a sample tag comprising a second polynucleotide sequence; wherein the first and second polynucleotide sequences are not identical to each other.
402. The composition of any one of embodiments 397-401, wherein the first subdivision of adaptor modules identifies each DNA molecule within the first test sample and the second subdivision of adaptor modules identifies each DNA molecule within the second test sample.
403. The composition of any one of embodiments 393-402, wherein the ID region of each adaptor module of the plurality of adaptor modules is selected from a group consisting of between 2 and 10,000 unique nucleotide sequences.
404. The composition of embodiment 403, wherein the ID region of each adaptor module of the plurality of adaptor modules is selected from a group consisting of between 50 and 500 unique nucleotide sequences.
405. The composition of embodiment 404, wherein the ID region of each adaptor module of the plurality of adaptor modules is selected from a group consisting of between 100 and 400 unique nucleotide sequences.
406. The composition of embodiment 405, wherein the ID region of each adaptor module of the plurality of adaptor modules is selected from a group consisting of 60 unique nucleotide sequences.
407. The composition of any one of embodiments 393-406, wherein the ID region of each adaptor module of the plurality of adaptor modules comprises or consists of 8 nucleotides.
408. The composition of any one of embodiments 393-407, wherein each adaptor module of the plurality of adaptor modules is selected from a group consisting of between 64 and 2,560,000 unique nucleotide sequences.
409. The composition of embodiment 408, wherein each adaptor module of the plurality of adaptor modules comprises a unique nucleotide sequence selected from among 3840 unique nucleotide sequences, and each nucleotide sequence is discrete from any other sequence of the 3840 unique nucleotide sequences by Hamming distance of at least two.
410. The composition of any one of embodiments 393-409, wherein the anchor region of each adaptor module of the plurality of adaptor modules is selected from a group consisting of four nucleotide sequences.
411. The composition of embodiment 410, wherein the amplification regions of each adaptor module of the plurality of adaptor modules comprise a universal primer sequence;
   wherein the ID region of each adaptor module of the plurality of adaptor modules comprises or consists of 8 nucleotides;
   wherein the plurality of adaptor modules is divided into two or more subdivisions, wherein each subdivision of adaptor modules comprises a set of unique ID regions, wherein the polynucleotide sequence of each ID region is discrete from the nucleotide sequence of any other ID regions of the plurality of adaptor modules by Hamming distance of at least two;
   wherein each adaptor module of the plurality of adaptor modules comprises a UMI multiplier that is adjacent to or contained within the ID region, wherein the UMI multiplier of each adaptor module of the plurality of adaptor modules comprises or consists of three nucleotides.
412. The composition of any one of embodiments 375-411, wherein the amplification region comprises or consists of between 10 and 50 nucleotides.
413. The composition embodiment 412, wherein the amplification region comprises or consists of 25 nucleotides.
414. The composition of any one of embodiments 375-413, wherein the anchor region comprises an overhang at the 3' terminus.
415. The composition of any one of embodiments 375-414, wherein the anchor region comprises or consists of between 1 and 50 nucleotides.
416. The composition of embodiment 415, wherein the anchor region comprises or consists of 10 nucleotides.
417. The composition of any one of embodiments 375-416, wherein the ID region comprises or consists of between 3 and 50 nucleotides.
418. The composition of embodiment 417, wherein the ID region comprises or consists of 8 nucleotides.
419. The composition of any one of embodiments 375-418, wherein each adaptor module further comprises a unique molecule identifier (UMI) multiplier.
420. The composition of embodiment 419, wherein the UMI multiplier is adjacent to or contained within the ID region.
421. The composition of embodiment 419 or 420, wherein the UMI multiplier comprises or consists of between 1 and 5 nucleotides.
422. The composition of embodiment 421, wherein the UMI multiplier is 3 nucleotides in length, and comprises a nucleic acid sequence selected from a group of 64 unique nucleotide sequences.
423. The composition of any one of embodiments 374-422, wherein the pool of ID regions comprises between 2 and 10,000 unique ID region sequences.
424. The composition of embodiment 423, wherein the pool of ID regions comprises 60 unique ID region sequences.
425. The composition of any one of embodiments 374-424, wherein each ID region of the pool of ID regions is 8 nucleotides in length.
426. The composition of any one of embodiments 374-425, wherein each ID region sequence is discrete from any other ID region sequence by Hamming distance of at least two.
427. The composition of any one embodiments 374-426, wherein each ID region is configured to identify the DNA fragment attached thereto.
428. The composition of any one of embodiments 374-427, wherein each adaptor module of the set of adaptor modules is selected from a group consisting of between 64 and 2,560,000 unique nucleotide sequences.
429. The composition of embodiment 428, wherein each adaptor module of the set of adaptor modules comprises a unique nucleotide sequence selected from 3840 unique nucleotide sequences, wherein each sequence of the 3840 unique nucleotide sequences is discrete from any other sequence by Hamming distance of at least two.
430. The composition of any one of embodiments 375-429, wherein the anchor region of each adaptor of the set of adaptors comprises one of four nucleotide sequences, and wherein each ID region of a given sequence is paired to only one of the four anchor regions of a given sequence.
431. The composition of any one of embodiments 375-430, wherein the amplification region of each adaptor of the set of adaptors comprises an identical primer binding site.
432. The composition of embodiment 431,
   wherein each ID region of the pool of ID regions is 8 nucleotides in length, wherein each ID region sequence is discrete from any other ID region sequence by Hamming distance of at least two;
   wherein each adaptor of the set of adaptors comprises a UMI multiplier that is adjacent to or contained within the ID region, wherein the UMI multiplier of each adaptor of the set of adaptors is three nucleotides in length, and wherein the UMI multiplier of a given sequence is paired to one ID region of a given sequence.
433. The composition of any one of embodiments 358-432, wherein the test sample is a tissue biopsy.
434. The composition of embodiment 433, wherein the tissue biopsy is obtained from a tumor or a tissue suspected of being a tumor.
435. The composition of embodiment 434, wherein the tissue biopsy is obtained from a malignant tumor or tumor suspected of being a malignant tumor.
436. The composition of any one of embodiments 358-435, wherein the DNA molecule is cell free DNA (cfDNA), genomic DNA (gDNA), complementary DNA (cDNA), mitochondrial DNA, methylated DNA, or demethylated DNA.
437. The composition of embodiment 436, wherein the DNA molecule comprises an epigenetic mark.
438. The composition of any one of embodiments 358-437, wherein the DNA molecule is obtained from a library selected from the list consisting of a whole genome library, an amplicon library, a whole exome library, a cDNA library, or a methylated DNA library.
439. The composition of any one of embodiments 358-438, wherein the DNA molecule is isolated or generated from the test sample.
440. The composition of embodiment 439, wherein the test sample comprises a biological sample selected from the group consisting of an amniotic fluid sample, a blood sample, a skin sample, a hair sample, a hair follicle sample, a saliva sample, a mucous sample, a sweat sample, a tear sample, an epithelial tissue sample, a urine sample, a semen sample, a seminal plasma sample, a serum sample, a prostatic fluid sample, a pre-ejaculatory fluid (Cowper's fluid) sample, an ocular fluid sample, an excreta sample, a biopsy sample, an ascites sample, a cerebrospinal fluid sample, a lymph sample, a tissue extract sample, a stool sample, and a formalin-fixed, paraffin embedded (FFPE) sample.
441. The composition of any one of embodiments 358-440, wherein the DNA molecule is obtained by the steps comprising:
   (a) isolating cellular DNA from the test sample; or
   (b) fragmenting the cellular DNA to obtain the genomic DNA fragment.
442. The composition of embodiment 441, wherein step (b) is performed by contacting the cellular DNA with at least one digestion enzyme.
443. The composition of embodiment 441, wherein step (b) is performed by applying mechanical stress to the cellular DNA.
444. The composition of embodiment 443, wherein the mechanical stress is applied by sonicating the cellular DNA.
445. The composition of embodiment 441, wherein step (b) is performed by contacting the cellular DNA with one or more compounds to chemically disrupt one or more bonds of the cellular DNA.
446. A kit comprising reagents, wherein the kit can be used in genetic analysis comprising the method of any one of embodiments 241-357.

### Additional Definitions

Unless otherwise defined in the disclosure, scientific and technical terms used in this application shall have the meanings that are commonly understood by those of ordinary skill in the art. Generally, nomenclature used in connection with, and techniques of, chemistry, molecular biology, cell and cancer biology, immunology, microbiology, pharmacology, and protein and nucleic acid chemistry, described in the disclosure, are those well-known and commonly used in the art.

As used in the disclosure, the following terms have the meanings ascribed to them unless specified otherwise.

The articles "a, " "an, " and "the" are used in the disclosure to refer to one or to more than one (*i.e.,* to at least one) of the grammatical object of the article. By way of example, "an element" means one element or more than one element.

The use of the alternative (*e.g*., "or") should be understood to mean either one, both, or any combination thereof of the alternatives.

The term "and/or" should be understood to mean either one, or both of the alternatives.

As used in the disclosure, the term "about" or "approximately" refers to a quantity, level, value, number, frequency, percentage, dimension, size, amount, weight or length that varies by as much as 15%, 10%, 9%, 8%, 7%, 6%, 5%, 4%, 3%, 2% or 1% to a reference quantity, level, value, number, frequency, percentage, dimension, size, amount, weight or length. In one embodiment, the term "about" or "approximately" refers a range of quantity, level, value, number, frequency, percentage, dimension, size, amount, weight or length ± 15%, ± 10%, ± 9%, ± 8%, ± 7%, ± 6%, ± 5%, ± 4%, ± 3%, ± 2%, or ± 1% about a reference quantity, level, value, number, frequency, percentage, dimension, size, amount, weight or length.

As used in the disclosure, the term "isolated" means material that is substantially or essentially free from components that normally accompany it in its native state. In some embodiments, the term "captured", "obtained" or "derived" is used synonymously with isolated.

A "subject," "individual," or "patient" as used in the disclosure, includes any animal that exhibits a symptom of a condition that can be detected or identified with compositions of the disclosure. Suitable subjects include laboratory animals (such as mouse, rat, rabbit, hamster, or guinea pig), farm animals (such as horses, cows, sheep, pigs), and domestic animals or pets (such as a cat or dog). In some embodiments, the subject is a mammal. In certain embodiments, the subject is a non-human primate, and, in some embodiments, the subject is a human.

While the present invention has been described in conjunction with the specific embodiments set forth above, many alternatives, modifications and other variations thereof will be apparent to those of ordinary skill in the art. All such alternatives, modifications and variations are intended to fall within the spirit and scope of the present invention.

Furthermore, it is intended that any method described in the disclosure may be rewritten into Swiss-type format for the use of any agent described in the disclosure, for the manufacture of a medicament, in treating any of the disorders described in the disclosure. Likewise, it is intended for any method described in the disclosure to be rewritten as a compound for use claim, or as a use of a compound claim.

All publications, patents, and patent applications described in the disclosure are hereby incorporated by reference in their entireties.

### EXAMPLES

The disclosure is further illustrated by the following examples, which are not to be construed as limiting this disclosure in scope or spirit to the specific procedures described in the disclosure. It is to be understood that the examples are provided to illustrate certain embodiments and that no limitation to the scope of the disclosure is intended thereby. It is to be further understood that resort may be had to various other embodiments, modifications, and equivalents thereof which may suggest themselves to those skilled in the art without departing from the spirit of the present disclosure.

### EXAMPLE 1 - FEASIBILITY STUDIES FOR SAMPLE DE-MULTIPLEXING

Initial experiments as proof-of-concept for sample de-multiplexing and gene deletion identification were carried out using contrived cell line DNA samples. Two different cell lines were blended with cell line NA12878 at known frequencies: NA09596, which contains an ATM deletion, and NA02718, which contains a BRCA2 deletion. Amplified adaptor-tagged libraries (LPA) were prepared using the "kinase/ligase ligation method" described above.

50 ng of LPA were amplified for 6-8 cycles using Forward Primer and Reverse Primer 63 (Table 1). Final LPWG libraries were quantified by Qubit, following the manufacturer's instructions, and sequenced on Illumina NextSeq550, following manufacturer's instructions, using custom primers, Forward Primer and Reverse Primer 62.

**Table 1 Primer Sequences**

| Amplification Primers: | | SEQ ID NO |
|---|---|---|
| **Forward Primer:** | AATGATACGGCGACCACCGAGATCTACACGTCATGCAGGACCAG AGAATTCGAATACA | 7 |
| **Reverse Primer 63:** | CAAGCAGAAGACGGCATACGAGATGTGACTGGCACGGGACCAGA GAATTCGAATACA | 8 |
| **Reverse Primer LPWG_AMP _R:** | CAAGCAGAAGACGGCATACGAGATGTGACTGGTGCAGGACCAGA GAATTCGAATACA | 9 |

| Sequencing Primers: | | |
|---|---|---|
| **Forward Primer:** | CAAGCAGAAGACGGCATACGAGATGTGACTGGCACGGGACCAGA GAATTCGAATACA | 10 |
| **Reverse Primer 62:** | GTGACTGGCACGGGACCAGAGAATTCGAATACA | 11 |
| **Reverse Primer LPWG_SEQ _R:** | GTGACTGGTGCAGGACCAGAGAATTCGAATACA | 12 |

| qPCR Primers : | | |
|---|---|---|
| **Oligo 1:** | AATGATACGGCGACCACCGA | 13 |
| **Oligo 2:** | CAAGCAGAAGACGGCATACGA | 14 |
| **Oligo 1c:** | TCGGTGGTCGCCGTATCATT | 15 |
| **Oligo 2c:** | TCGTATGCCGTCTTCTGCTTG | 16 |
| **Library Primer:** | TGCAGGACCAGAGAATTCGAATACA | 5 |

All samples were successfully sequenced, and gene deletions and amplifications detected as expected, suggesting that LPWG sequencing is useful for detection of CNVs.

While LPWG libraries could be successfully sequenced, libraries loaded at 4nM typically under-clustered. Quantification of the libraries by either Qubit, which measures all dsDNA, or by qPCR using the KAPA Library Quantification kit, which measures libraries with both a P5 and P7 Illumina sequencing adapter, showed a notable discrepancy between total dsDNA and libraries capable of generating clusters for LPWG libraries but not for target capture libraries, as shown in Table 2.

**Table 2: Measured Concentration of LPWG libraries or target capture libraries from NA02718 blended with NA12878**

| **LPA pool** | **Sequencing Run** | **Library Type** | **Library Conc by Qubit (ng/uL)** | **Library Conc by qPCR (ng/uL)** | **Cluster Density (K/mm²)** |
|---|---|---|---|---|---|
| 1 | 3 | LPWG | 3.34 | 0.37 | 33 |
| | 12 | Target Capture | 56.0 | 57.34 | 208 |
| 2 | 4 | LPWG | 2.65 | 1.20 | 108 |
| | 13 | Target Capture | 13.7 | 15.48 | 194 |

The feasibility of amplifying either libraries (LIBs) or libraries post-amplification (LPAs) for whole genome sequencing was tested. Wild type cfDNA was processed using the "kinase/ligase ligation method" described above. 2 µL pooled LIBs or LPA (8 ng and 5 ng respectively) were amplified with Forward primer and Primer 63 and a 69°C annealing temperature for 8, 12, or 16 cycles. LPWG amplified library were eluted from Agencourt AMPure XP magnetic beads (following manufacturer's suggestions) in 25 µL TEZ and measured by Qubit.

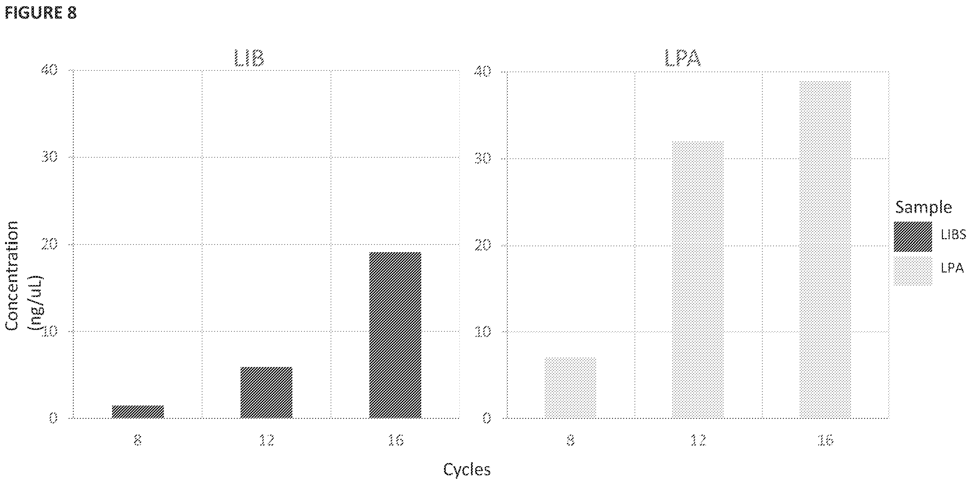

The resulting concentrations of LPWG libraries, using either LIBS or LPA, are shown in FIGURE 8. Both LIBS and LPA can be amplified with the LPWG protocol such that there is sufficient library material to be sequenced. Further development was carried out using this diluted LPA as a template for the LPWG amplification.

### EXAMPLE 2 - OPTIMIZATION OF AMPLIFICATION CONDITIONS

### Annealing Temperature

The amplification efficiency was investigated with primer annealing temperatures of either 58°C or 69°C using Forward Primer and Reverse Primer 63. LPA from sheared NA12878 cell line was amplified for 8, 12, or 16 cycles. LPWG library concentrations, as measured by Qubit or qPCR, are shown in **FIGURE 9****.** All qPCR reactions were carried out using a Roche Lightcycler 480 qPCR instrument, following the manufacturer's recommendation:
Initial denature 95°C 5 min 1 cycle.
Denaturation 95°C 30 sec 35 cycles.
Annealing/Extension/Data acquisition 60°C 45 sec 35 cycles.

Slightly more product was seen after 8 cycles with a primer annealing temperature of 58°C compared with an annealing temperature of 69°C. However, this difference disappeared by 12 cycles. The PCR primer annealing temperature did not appear to have a large impact on the quantity of PCR product generated between 12 and 16 cycles. An annealing temperature of 65°C was also used for further development of library amplification for LPWG sequencing.

### Amplification Cycle Number

While previous feasibility experiments demonstrated that LPWG libraries could be successfully sequenced, the loading typically resulted in under-clustering of the libraries on the sequencer. Library measurements by Qubit and by qPCR (using the KAPA Library Quantification kit) showed discrepant values (see **Table 2),** suggesting that the dsDNA that was measured in the library pool after 6-8 cycles consists of a mixture of libraries, some capable of generating clusters and other libraries not capable of generating clusters. To determine whether this observation was affected by amplification cycle number, the effects of cycle number on LPWG library composition were tested.

In five independent experiments, 50 ng of LPA pooled from wild type samples were amplified while using a 65°C annealing temperature and Forward Primer with either "primer 63" (n=3) or "LPWG_AMP_R" (n=4) reverse primers (described in Table 1). Reactions were allowed to proceed for 8, 12, 14, or 16 cycles of amplification and the resulting libraries quantified by either Qubit or by qPCR using standards using the KAPA Library Quantification kit. In a subsequent experiment (n=1), 50 ng of pooled LPA from wild type (WT) cfDNA samples were amplified for 4, 6, 8, or 16 cycles. To further analyze the components in the PCR products, qPCR was performed with either P5 and P7 primers (which quantify libraries capable of forming sequencing clusters), P5 only, P7 only, P5 with a P5 reverse compliment primer, or P7 with a P7 reverse compliment primer. Libraries were also amplified with Primer Mix 1S ("ACA") and quantified with LPA standards at known concentrations to determine whether the PCR product consists of tagged LPA.

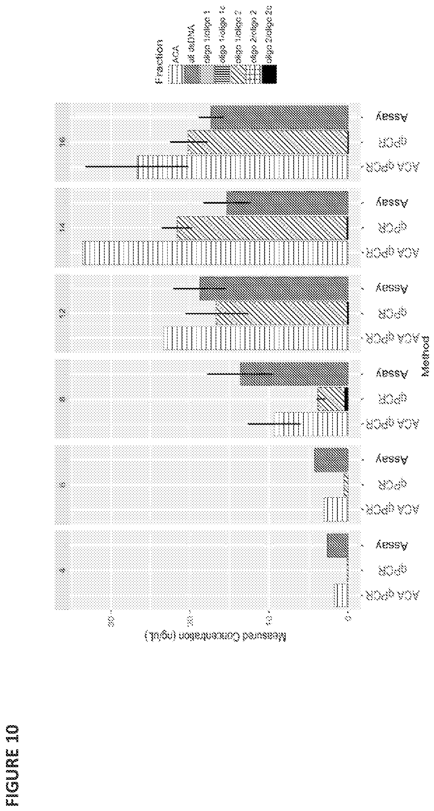

The average measured amplicon concentrations after 8, 12, 14, and 16 cycles of amplification, as well as the measured amplicon concentrations after 4 or 6 cycles, are shown in **FIGURE 10****.**

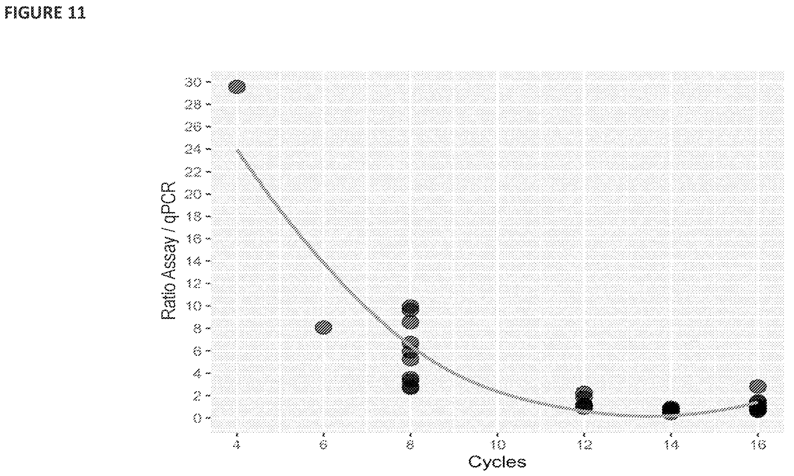

A discrepancy between concentration values determined by qPCR or by Qubit can be seen after 4 to 12 cycles of amplification. After 8 cycles, the measured concentration by Qubit varies from approximately 2.5-fold greater to 10-fold greater than that measured by qPCR. Interestingly, this discrepancy was not due to product with P5/P5 or P7/P7 adapters. The ratio of amplification products between Qubit and qPCR measurements was as high as 30 after 4 cycles and decreased until 14 cycles, with 14-16 cycles consistently showing a ratio value closer to 1, as shown in **FIGURE 11****.**

For LPWG sequencing to be an effective, reproducible, assay it is important that the measured library concentration reflect the quantity of libraries capable of generating clusters. As seen in **Table 3,** cluster density was low after 6-8 PCR cycles unless a high loading concentration was targeted. This is likely because the LPWG libraries consisted of a mixture of libraries, some of which were capable of generating clusters and others were not. Loading was calculated from the Qubit measurement which measured DNA mass but will not differentiate between DNA library sequences. Cluster densities consistently passed the QC metrics of 150 - 270 K/mm² when the libraries have been amplified for 12-16 cycles.

**Table 3: Cluster density in LPWG sequencing runs.**

| (Loading was calculated based on Qubit quantification measurements) | | | | | | |
|---|---|---|---|---|---|---|
| **Run Number** | **Sample Type** | **PCR Cvcles** | **Est. Lib. Size (bp)** | **Target Loading Conc (nM)** | **Cluster Density (K/mm²)** | **Cluster Density Pass/Fail** |
| 1 | gDNA | 7 | 300 | 3.6 | 57.3 | Fail |
| 2 | cfDNA | 7 | 300 | 15 | 168.1 | Pass |
| 3 | gDNA | 6 | 396 | 4 | 33 | Fail |
| 4 | gDNA | 8 | 396 | 4 | 108.3 | Fail |
| 5 | cfDNA | 12 | 330 | 4 | 176.8 | Pass |
| 6 | cfDNA | 14 | 330 | 4 | 208.0 | Pass |
| 7 | cfDNA | 14 | 330 | 4 | 243.5 | Pass |
| 8 | cfDNA | 14 | 330 | 4 | 190.0 | Pass |
| 9 | gDNA | 16 | 420 | 4 | 204.5 | Pass |
| 10 | gDNA | 16 | 420 | 4 | 196.8 | Pass |
| 11 | gDNA | 16 | 420 | 4 | 192.0 | Pass |

### Bioanalyzer Analysis of LPWG Libraries Amplified for Varying Number of Cycles

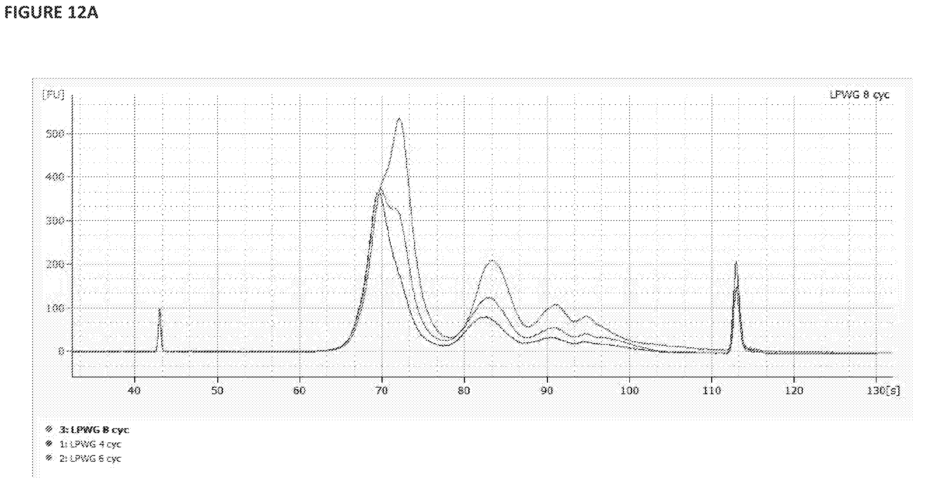

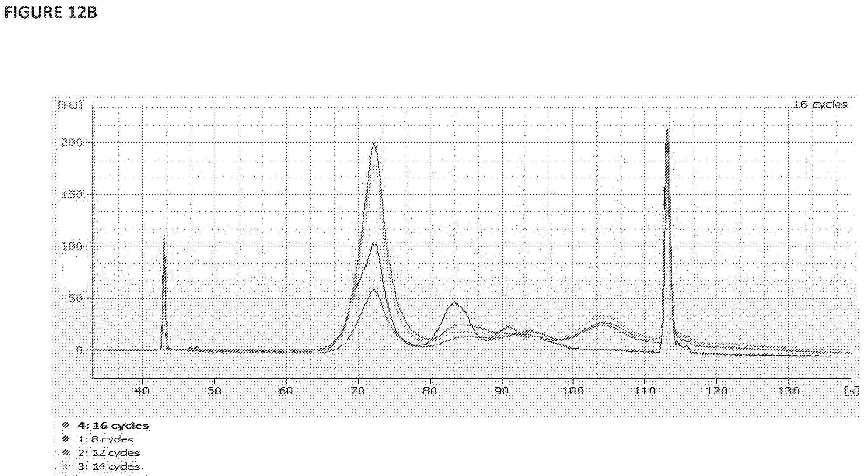

Libraries that had been PCR amplified for 4, 6, 8, 12, 14, or 16 cycles using Forward Primer and LPWG_AMP_R primers in the LPWG protocol were loaded onto a high sensitivity DNA Bioanalyzer chip (Agilent, part no. 5067-4627) after being diluted 10-fold. Libraries were separated by electrophoresis using the Agilent Bioanalyzer, as described in the "Agilent High Sensitivity DNA Kit Guide". Bioanalyzer (BA) traces from LPWG libraries were overlaid and are shown in **FIGURE 12A** **and** **FIGURE 12B****.**

After 4 cycles of amplification, a dominant peak was seen at ~300 bp. A second 330 bp peak appeared after 6 cycles suggesting at least two different products can be found in this library. The larger of these products appeared to be to be preferentially amplified in subsequent cycles and aligned with the dominant peak seen after 12-16 cycles suggesting that this larger product was amplifiable with P5 and P7 primers while the smaller product was not. Interestingly, after 16 cycles there was a relative decrease in 330 bp cfDNA libraries and increase in larger-sized libraries. One possible explanation is that these might represent "daisy chains" of mis-annealed DNA generated from over-amplification.

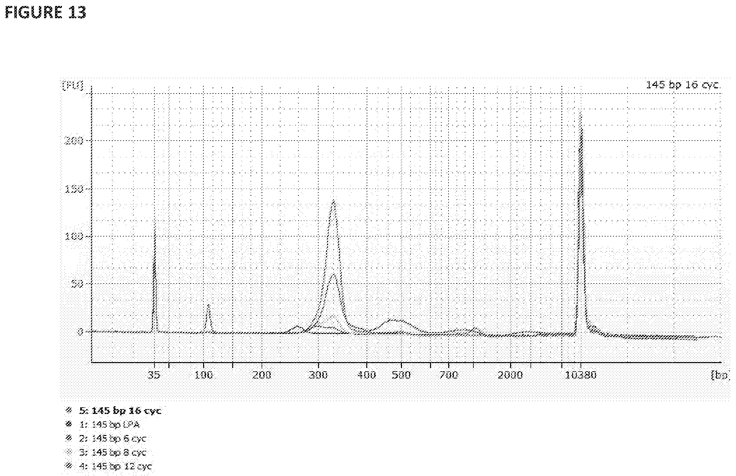

In order to differentiate between 2-nucleosome cfDNA products and potential "daisy chains", libraries from 145 bp fragments of DNA isolated by Pippin size-selection were constructed and amplified with LPWG primers for varying cycle numbers. As seen in **FIGURE 13****,** after 6 cycles of amplification, two products were apparent: one ~35-40 bp larger than the LPA template and one ~ 70 bp larger than the LPA template. This larger product was preferentially amplified over the following 10 cycles. After 16 cycles, there was a reduction in the quantity of the 330 bp product and an increase in the 470 bp product. This larger product was not seen in the LPA template, indicating that it was a product of over-amplification. Although over-amplification of the libraries did not appear to affect either cluster density **(Table 3)** or sequencing quality, 14 cycles of PCR appeared to balance accurate library quantification by Qubit with over-amplification.

### EXAMPLE 3 - OPTIMIZATION OF LIBRARY INPUT

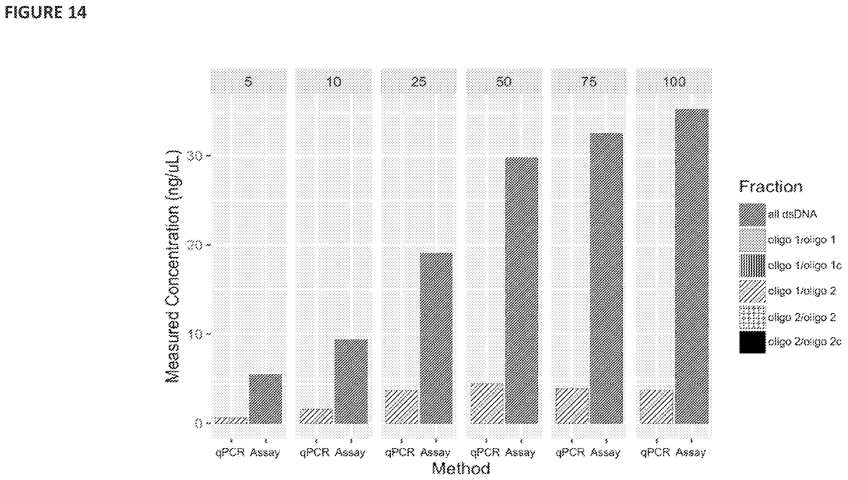

LPWG libraries were prepared using LPA from wild type cfDNA in two independent experiments: one performed with 5, 10, 25, 50, 75, or 100 ng of LPA input into the reaction and amplified for 8 cycles with Forward Primer and Primer 63 as the reverse primer, and one performed with 10, 20, 50, 100, or 200 ng of LPA input into the reaction and amplified for 16 cycles with Forward Primer and LPWG_AMP_R as the reverse primer. Whole genome sequencing library concentrations were quantified by Qubit and/or by qPCR using P5, P7, P5c and P7c primers listed in **Table 1.** The concentration of PCR product after 8 cycles of amplification are shown in **FIGURE 14****.**

After 8 cycles, there was a large discrepancy between the measured library concentration at all input amounts, with an 8-fold higher concentration measured by Qubit than by qPCR. Libraries with both P5 and P7 adapters appeared to plateau after approximately 50 ng input into the amplification reaction, suggesting that, while increasing the input into the PCR results in more dsDNA after 8 cycles, it did not result in more libraries capable of generating sequencing clusters.

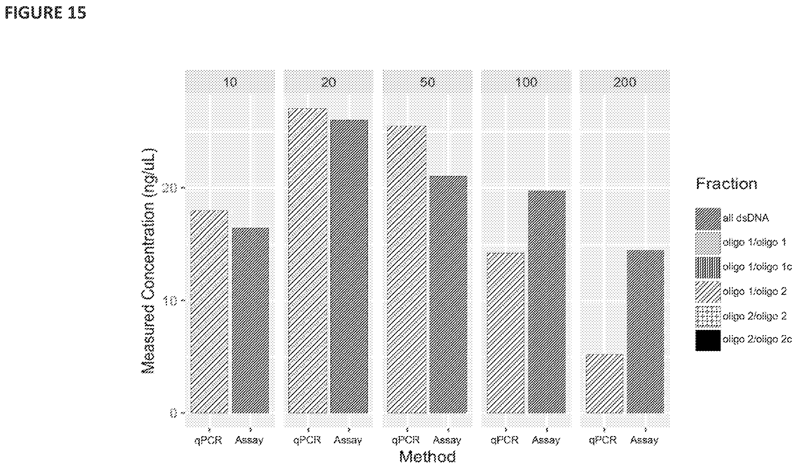

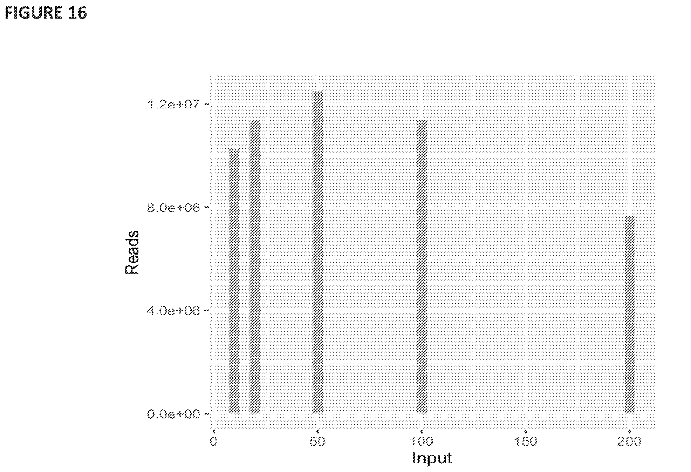

As seen in **FIGURE 15****,** there was little difference in concentration values measured by either Qubit or by qPCR after 16 PCR cycles when between 10 and 100 ng were added into the LPWG qPCR reaction. When 200 ng were added, the Qubit measurement value was approximately 3-fold higher than that measured by qPCR. When these LPWG libraries were normalized for loading onto the flow cell using the Qubit measurements, resulting read count suggested that the 200 ng input sample was under-loaded, as shown in **FIGURE 16****.**

It was feasible that inputs as low as 2 ng (2 µL of a 1/10 dilution of a 10 ng/ µL sample) could be used as input into the LPWG amplification. Therefore, the number of reads when 2 ng were amplified for LPWG then loaded onto a flow cell with a maximum number of capture libraries was determined.

Eight LPAs from wild type samples were amplified for low pass whole genome sequencing. Two replicates each with 1 ng, 2 ng, 10 ng, or 15 ng input material were pooled then amplified for 16 cycles with a 65°C annealing temperature. LPWG amplified libraries were purified with Ampure beads, quantified by Qubit, then co-loaded with wild type samples. LPWG libraries were loaded to a target coverage of 0.5x, as described in the calculation of Example 5.

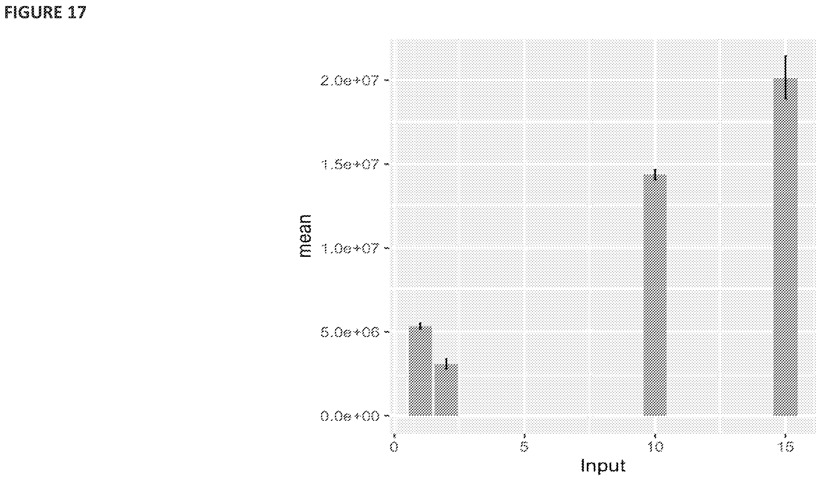

As seen in **FIGURE 17****,** increasing the input for sequencing resulted in an increase in sequencing reads, although 1ng and 2 ng of cfDNA resulted in approximately 5 million and 3 million reads per sample respectively.

A sample with 2 ng that is added to a LPWG amplification pool of more concentrated samples will therefore likely generate fewer than the required minimum reads necessary for analysis, resulting in under-sequencing of the sample. To prevent under- or over-sequencing of samples, it was found necessary to either normalize sample input when pooling into the LPWG amplification reaction or to amplify samples individually and normalize when loading onto the sequencing flow cell.

### EXAMPLE 4 - OPTIMIZATION OF REACTION CONDITIONS

### Reaction Volume

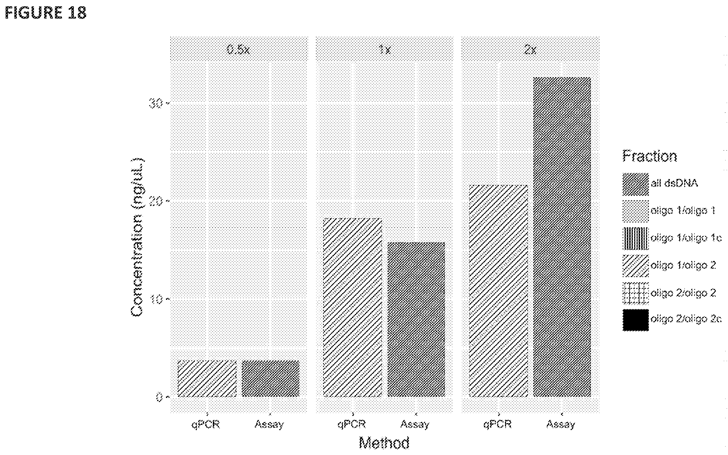

LPAs from four WT cfDNA were pooled to equal mass and added into LPWG amplification reactions at 50 ng total DNA. "LPWG_AMP _R" primers were used in the PCR reaction, with an annealing temperature of 65°C and 16 cycles. Reaction volumes of 100 µL (1x), 200 µL (2x), or 50 µL (0.5x) were tested. Resulting PCR product was purified with Ampure beads and concentrations determined by Qubit and by qPCR. The concentration of PCR product in 0.5x, 1x, or 2x reaction volumes are shown in **FIGURE 18****.**

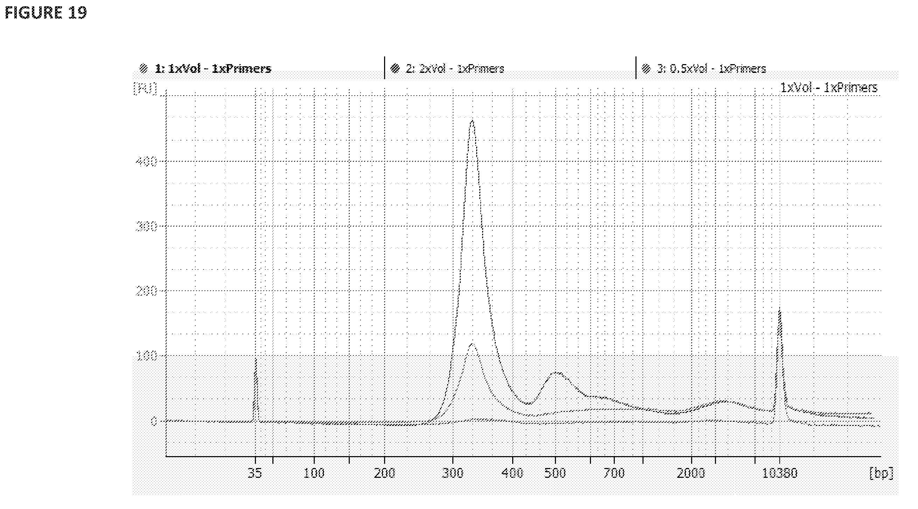

Increasing the reaction volume increased the amount of PCR product. The increase in PCR product was greater in the Qubit measurement compared with the qPCR measurement and was confirmed in the Bioanalyzer traces **(****FIGURE 19****).**

### Primer Concentration

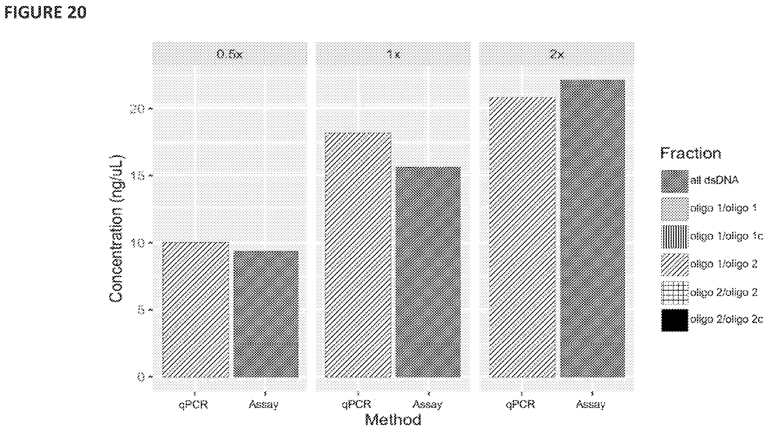

LPA from four samples of WT cfDNA was pooled and added into the LPWG amplification reaction at 50 total ng. Libraries were amplified with Forward Primer and LPWG_AMP_R primers for 16 cycles of PCR. Primer concentrations at 1 µM (1x), 2 µM (2x), or 0.5 µM (0.5x) were tested. The resulting PCR products were purified with Ampure beads and the concentrations determined by Qubit and by qPCR. The concentration of PCR products amplified with 0.5 µM (0.5x), 1 µM (1x), or 2 µM (2x) reaction volumes are shown in **FIGURE 20****.**

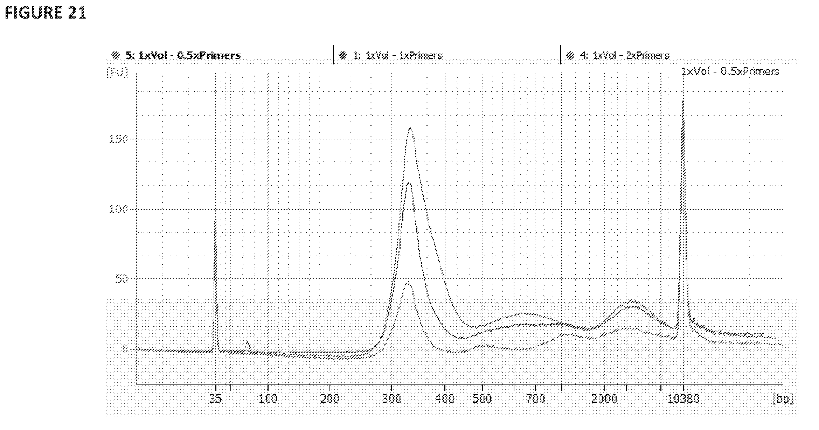

As shown in **FIGURE 20****,** increasing the concentration of PCR primers 2-fold, up to 2 µM, provided a 42% and 14% increase in LPWG library product when measured by Qubit or by qPCR respectively. Interestingly, increasing the primer concentration did not appear to decrease the "daisy chains" which were thought to result from primer deficiency during over-amplification, as shown by the electropherograms in **FIGURE 21****.**

### Polymerase

LPAs from four samples of WT cfDNA were pooled and added into the LPWG amplification reaction at 50 total ng. Forward Primer and LPWG_ANP_R primers were used in the reaction, with an annealing temperature of 65°C and the template was amplified for 8, 12, 14, or 16 cycles with either the HiFi Q5 Polymerase (NEB) or with KAPA HiFi polymerase from Roche (cat # KK2600). The resulting PCR product was purified with Ampure beads and concentrations determined by Qubit and by qPCR. Product was qualitatively evaluated with the Agilent Bioanalyzer.

As shown in **FIGURE 22****,** after 12-14 PCR cycles similar concentration values were found by either Qubit or qPCR when KAPA HiFi Hotstart was used in the PCR reaction. However, after 16 cycles there was an ~1.6X difference between Qubit values and qPCR values which could be due in part to a larger portion of the product forming "daisy chains. While an increase in "daisy chains" can be seen on the Bioanalyzer traces in **FIGURE 23****,** the same electropherogram traces also showed an overall decrease in yield suggesting that the qPCR quantification might be over-measuring DNA product.

PCR amplification using the KAPA HiFi Hotstart polymerase provide between an 85 and 500% increase in LPWG library product, as shown in **Table 4.** The optimal cycle number for PCR with KAPA HiFi Hotstart polymerase lies between 8 and 12 cycles.

**Table 4: Percent increase in library concentration when amplified with KAPA polymerase compared with Q5 polymerase.**

| | % **Increase** | | |
|---|---|---|---|
| **Cycles** | **Qubit** | **qPCR P5/P7** | **qPCR ACA** |
| 8 | 86 | 85 | 86 |
| 12 | 282 | 302 | 304 |
| 14 | 335 | 316 | 223 |
| 16 | 312 | 506 | 525 |

### EXAMPLE 5 - EVALUATION OF TARGET COVERAGE

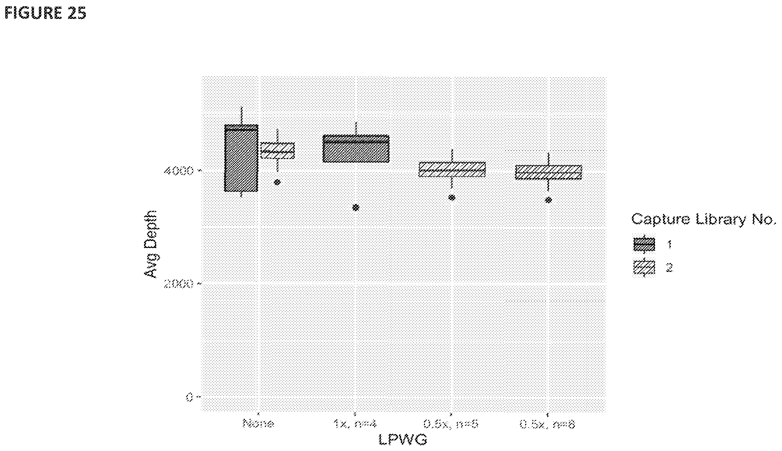

As shown in **FIGURE 25****,** LPWG libraries can be accurately loaded to a target coverage using the calculations described above. Samples that were amplified in individual reactions for 16 cycles as described in Example 4 ("Individual") and samples normalized into a pooled amplification reaction as described in Example 3 ("Pooled Normalized") showed the least variability in coverage, while samples in which inputs ranging from 2-15 ng into a pooled PCR showed higher variability in coverage.

To carry out the experiment there were three sequencing runs in which WT libraries amplified for LPWG were co-loaded with WT libraries. Library loading was calculated as described above. Coverage was calculated from the number of reads generated for each barcoded sample.

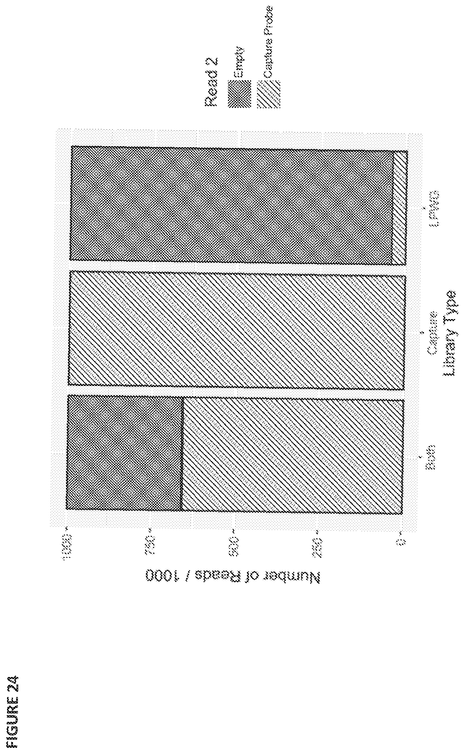

**FIGURE 24** shows calculated coverage for wild type LPWG libraries, co-loaded with target capture libraries. Red boxplots show libraries loaded to a target of 0.5x coverage while the blue boxplot shows libraries targeted to 1x coverage.

### EXAMPLE 6 - EVALUATION OF CAPTURE LIBRARY DEPTH IN CO-LOADED LIBRARIES

As shown in **FIGURE 25** and summarized in **Table 6,** including LPWG libraries in the sequencing run along with target capture libraries resulted in a decrease in the average depth in the target capture libraries. This loss in depth for the targeted library increased as the depth of LPWG coverage increased, as shown in **Table 5.** This shows that the depth of the libraries can be varied, allowing optimization of the target capture library reads necessary for the desired assay sensitivity.

To carry out this experiment, there were three sequencing runs in which WT libraries amplified for LPWG and co-loaded with target capture libraries were evaluated: No. 14, No. 15, and No. 16. To determine the potential loss of depth of the capture libraries when LPWG libraries were loaded on the same flow cell, the average depth of each co-loaded capture library was compared to the average depth observed when the same library was analyzed alone (NB552199_0026 and NB551744_0099).

**Table 5: Mean percent loss in average depth for 5 WT samples in two capture libraries.**

| **Capture Library** | **Sample** | **Target coverage and no. LPWG libraries** | | |
|---|---|---|---|---|
| | | **1x, n=4** | **0.5x, n=8** | **0.5x, n=5** |
| 2 | WT R537995 | | 8.35 | 7.23 |
| 2 | WT R538001 | | 8.20 | 7.21 |
| 2 | WT R538005 | | 8.53 | 7.39 |
| 1 | WT R537984 | 6.72 | | |
| 1 | WT R537989 | 5.39 | | |
| 1 | WT R537995 | 5.24 | | |

### EXAMPLE 7 - OPTIMIZATION OF NORMALIZATION METHODS

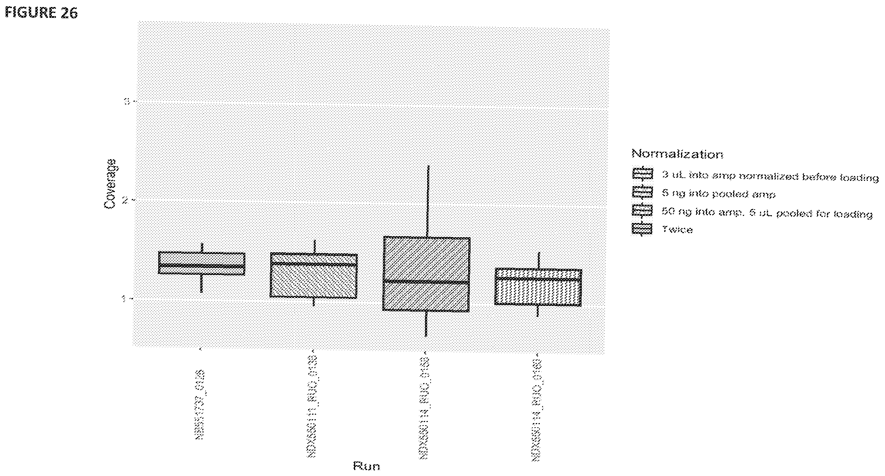

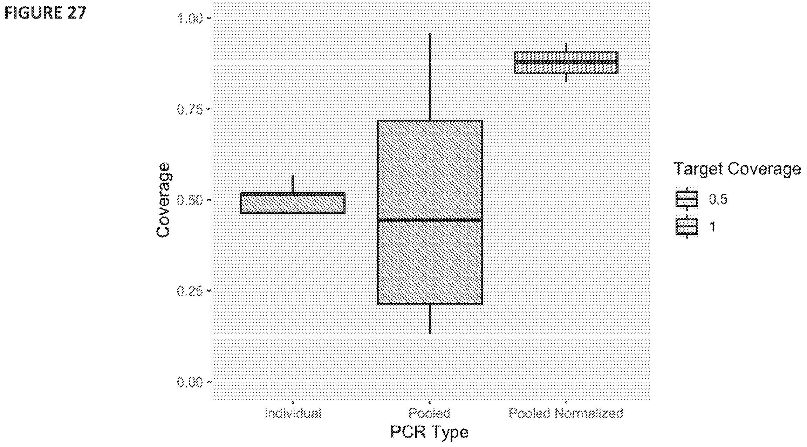

As shown in **FIGURE 26****,** of the four normalization processes investigated, variability in coverage was the least when normalization was carried out both going into PCR amplification and before going into the flow cell after pooling amplicon. In these experiments, the greatest variability in coverage was seen when samples were pooled into a single amplification reaction then normalized. However, in previous experiments, pooling into a single amplification showed less variability, as seen in **FIGURE 27****.** Taken together, even coverage across sequenced samples was observed when samples were individually amplified then normalized and pooled.

Clinical cfDNA from 10 patients and from 6 wild type samples was converted to LPA and diluted. Samples were normalized in one of the following four ways and the variability in sequencing coverage determined:
1. 50 ng each diluted LPA was used as input into the LPWG amplification. Each sample was amplified in a separate reaction and, following bead cleanup, the concentration was determined. 100 ng of each product was pooled, and 4 nM was loaded onto the flow cell. Run number: 6.
2. 5 ng of each diluted LPA was pooled into a single amplification reaction. The concentration of the resulting PCR product was determined following bead cleanup and 4 nM was loaded onto the flow cell. Run number: 7.
3. 3 µL of each diluted LPA was amplified in a separate reaction and, following bead cleanup, the concentration was determined. 100 ng of each product was pooled, and 4 nM was loaded onto the flow cell. Run number: 17.
4. 50 ng each diluted LPA was used as input into the LPWG amplification. Each sample was amplified in a separate reaction and, following bead cleanup, 5 µL from each reaction was pooled. The concentration of the pooled LPWG product was determined and 4 nM was loaded onto the flow cell. Run number: 8.

### EXAMPLE 8 - SIMULTANEOUS CO-SEQUENCING OF TARGETED AND WHOLE GENOME LIBRARIES

Adaptor-tagged genomic libraries were prepared as shown in **FIGURE 4****.** The adaptor-tagged libraries were split into two parts after amplification, with one part processed for sequencing of the low pass whole genome (LPWG) library and the other part was separately enriched by target capture then processed for sequencing of the targeted libraries. Both library types were then pooled and co-sequenced on the same flow cell. In this example, libraries were demultiplexed after 17 cycles of sequencing through the Read 2 sequence, as shown in **FIGURE 28****.** Results clearly demonstrated the effectiveness of this novel strategy.

Genomic libraries were prepared from cfDNA that was extracted from wild type plasma samples using a QIAmp DSP Circulating NA kit (Qiagen Cat. No. 61504). For each sample, 30 ng of cell free DNA (cfDNA) was end-repaired by treatment with Shrimp Alkaline Phosphatase (New England Biolabs/NEB Cat. No. M0371B)) followed by T4 DNA Polymerase (NEB Cat No. M0203B)) and PreCR Repair Mix (NEB Cat. No. M0309B)). The resulting blunt-ended DNA was coupled to a barcode oligonucleotide and made doublestranded using Taq ligase (NEB Cat No. M0208B), T4 Polynucleotide Kinase (NEB Cat No. M0201B), and Bst Polymerase (NEB Cat No. M0328B). The barcode coupling step was mediated through an anchor oligo that was ligated to the cfDNA fragment using an Ultra II Ligation kit (NEB Cat No. E7648B) followed by purification with Ampure XP beads (Beckman Coulter Cat. No. A63882). The barcode oligonucleotides were comprised of barcode sequences for sample identification (ID region) and primer binding sequences for amplification (amplification region). Following purification with Ampure XP beads, genomic libraries were amplified by PCR (8 cycles of 98°C for 30s, 65°C for 30s, 72°C for 30s) using the NEBNext HF PCR mix (NEB Cat. No. M0541B) and a genomic library PCR primer (1 µM).

Targeted enrichment was carried out on 2 genomic libraries using a Lung panel of capture probes. The Lung capture probe panel comprised biotinylated capture probes that targeted the following genes: AKT1, ALK, ALS, B2M, BRAF, EGFR, ERBB2, FGFR1, FGFR2, FGFR3, KEAP1, KRAS, MAP2K1, MET, MYC, NRAS, NTRK1, PIK3CA, PTEN, RET, RICTOR, ROS1, STK11, and TP53. The probe panel and tagged genomic libraries were hybridized overnight at 65°C in a hybridization buffer. After hybridization, the targeted tagged genomic libraries were isolated using streptavidin coated paramagnetic microspheres (Thermo Fisher Scientific Cat. No. 6500), then washed with wash buffer to remove unbound tagged genomic libraries.

The isolated capture probes were extended with NEBNext HF PCR mix using the tagged genomic library fragment as a template to produce a hybrid molecule comprising the capture probe, the reverse complement of the cfDNA fragment that is 3' of where the capture probe binds to the cfDNA fragment, and the barcode tag (ID region + amplification region). The hybrid molecule was then PCR amplified using Hybrid PCR Primers (0.4 µM) for 5 cycles, as follows:
Extension: 1 cycle of 60°C for 30s, 72°C for 2 min, 98°C for 30 s, followed by
Amplification: 5 cycles of 98°C for 30s, 65°C for 30s, 72°C for 30s.

100 µL of the resulting product was eluted from the Streptavidin paramagnetic beads, combined with 100 µL NEBNext Ultra II Q5 master mix, and amplified for 12 more cycles. The final product was purified with Ampure XP beads.

Low pass whole genome amplification was performed on two libraries as follows, one of which was also in the target capture pool: 12.5 ng of each genomic library was pooled and amplified using Q5 polymerase (NEB cat #M0492) with LPWG PCR primers (0.4 µM of Forward Primer and Primer 63). Libraries were amplified for 14 cycles, with an annealing temperature of 65°C, and the resulting product was purified with Ampure XP beads.

Target coverage for LPWG sequencing was 1x. To achieve this, libraries were blended for sequencing such that the pooled LPWG libraries consisted of 22% of the final sequencing library pool and the target capture libraries consisted of 78% of the pool. Final libraries were sequenced with a NextSeq 500/550 Sequencer (Illumina), for 151 cycles for Read 1, 17 Cycles for Read 2, and custom sequencing primers for both reads.

The first one thousand reads were selected and analyzed from each of three samples: B716256-A, which was amplified for LPWG sequencing only; B716171-A, which underwent both target capture and LPWG amplification; and B716052-A which underwent target capture only. The number of 'empty' reads, *i.e.* those that include "GGGGGGGGGGGGGGGGG" (SEQ ID NO: 17), were tallied for each sample and summarized in **Table 6.** The results indicated that empty reads can be successfully used to demultiplex the different library types, differentiating LPWG sequences from targeted sequences.

**Table 6: Sequencing Results for LPWG and Targeted Libraries**

| **Sample** | **Library Type** | **Total Reads** | **Number Empty Reads** | **Number Non-Empty Reads** | **Percent Empty Reads** |
|---|---|---|---|---|---|
| B716256-A | LPWG | 1000 | 960 | 40 | 96 |
| B716171-A | LPWG and Targeted | 1000 | 344 | 656 | 34.4 |
| B716052-A | Targeted | 1000 | 3 | 997 | 0.3 |

### EXAMPLE 9 - SNV/INDEL CALLS FROM CO-/SEQUENCING RUNS

Libraries were constructed from cfDNA that was extracted from the plasma sample of a single wild-type individual as follows:
1) 50 ng of cfDNA that had been extracted using a QIAmp DSP Circulating NA kit (Qiagen Cat. No. 61504) was end-repaired and A-tailed using the NEBNext Ultra II End Repair/dA-Tailing kit (NEB Cat. No. E7546).
2) Adaptors were added to a final concentration of 250 nM in the reaction and ligated to cfDNA fragments using the Ultra II Ligation kit (NEB Cat No. E7648) for 30 min at 20°C.
3) Following purification with Ampure XP beads (Beckman Coulter Cat. No. A63882), the resulting genomic libraries were amplified with the Library Primer (1 µM) using the NEBNext Ultra II Q5 Master Mix (NEB Cat. No. M0544)). Reactions were run on the following cycling program:
   a. Extension: 1 cycle of 60°C for 30s, 72°C for 2 min, 98°C for 30 s, followed by
   b. Amplification: 8 cycles of 98°C for 30s, 65°C for 30s, 72°C for 30s.
   The amplified genomic libraries were then purified with Ampure XP beads.
4) Amplified tagged genomic libraries from different samples were pooled and hybridized overnight at 65°C with a biotinylated HRD capture probe panel in a hybridization buffer. (The HRD capture probe panel targets 12 genes: AR, ATM, BRCA1, BRCA2, BRIP1, CDK12, CDKN2A, CHEK2, FANCA, HDAC2, PALB2, and TP53) Targeted libraries were isolated with paramagnetic Streptavidin beads (Thermo Fisher Scientific Cat. No. 65002) and washed at 45°C for 5 minutes with a wash buffer.
5) The isolated capture probes were extended with NEBNext Ultra II Q5 Master Mix using the tagged genomic library fragment as a template to produce a hybrid molecule comprising the capture probe, the reverse complement of the cfDNA fragment that is 3' of where the capture probe binds to the cfDNA fragment, and the adaptor module. The hybrid molecule was then PCR amplified using Forward and Reverse Hybrid PCR Primers (0.4 µM) for 5 cycles, as follows:
   a. Extension: 1 cycle of 60°C for 30s, 72°C for 2 min, 98°C for 30 s, followed by
   b. Amplification: 5 cycles of 98°C for 30s, 65°C for 30s, 72°C for 30s. 100 µL of the resulting product was eluted from the Streptavidin paramagnetic beads, combined with 100 µL NEBNext Ultra II Q5 master mix, and amplified for 12 more cycles. The final product was purified with Ampure XP beads.

Separately, Low Pass Whole Genome amplification was performed on 4 tagged genomic libraries. 50 ng of each library was amplified using NEBNext Ultra II Q5 Master Mix with Low Pass Whole Genome primers (Forward Primer and LPWG_AMP_R primers in Table 1) (0.4 µM). Libraries were amplified for 12 cycles of 98°C for 30s, 65°C for 30s, 72°C for 30s.The resulting product was purified with Ampure XP beads.

Target capture libraries and LPWG libraries were pooled such that each LPWG library would be sequenced to 1x coverage. Final libraries were sequenced with a NextSeq 500/550 Sequencer (Illumina), for 151 cycles for Read 1, 17 Cycles for Read 2, using custom primers for both reads.

Results indicated that SNV/Indel calls from targeted capture libraries were not influenced by co-sequencing with LPWG libraries. This experiment also demonstrated that co-sequencing is amenable to using different library preparation methods.

The results from demultiplexing sequencing reads are shown in **Table** 7, which indicated that LPWG libraries can be reproducibly demultiplexed (the % fraction of LPWG reads was consistent between samples) and differentiated from the targeted libraries carrying the same sample-identifying adaptor modules (the estimated coverage, 1, is close to the realized coverage).

**Table 7: Sequence Coverage for LPWG Libraries from Pools of LPWG + Targeted Amplicon**

| **Replicate** | **Number of Demuxed LPWG Reads** | **LPWG Fraction of Reads** | **Coverage** |
|---|---|---|---|
| 1 | 19745521 | 16% | 0.781 |
| 2 | 17946984 | 16% | 0.712 |
| 3 | 17286025 | 16% | 0.685 |
| 4 | 15855059 | 16% | 0.628 |

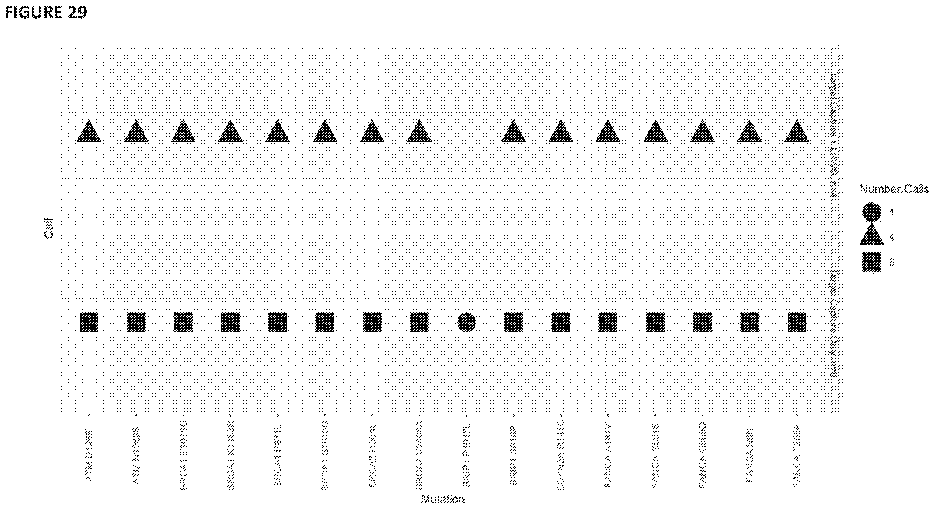

Variant calls resulting from sequencing the targeted libraries are shown in **FIGURE** 29. The 16 variants listed in **FIGURE 29** were called for all 8 samples where targeted libraries were sequenced alone (black squares) and in all 4 targeted libraries that were co-sequenced along with Low Pass Whole Genome libraries (black triangles). One variant, BRIP1 P1017L, was called in only one library; this variant was just below the limit of detection for the assay. Results indicated that including LPWG sequencing with target capture did not affect the identification of mutations.

### EXAMPLE 10 - FEASIBILITY OF CO-LOADING LPWG AND CAPTURE LIBRARIES

Coverage depth for WT R538001 with 8NNN-47 and WT R538001 with 8NNN-48 (LPWG samples that do not share sequencing space with targeted libraries) were calculated to be 0.93x and 0.82x respectively, suggesting that coverage can be accurately targeted with the calculations outlined below:
1. Calculate the proportion of the flow cell to load with LPWG libraries (where n is the no. samples in the library; PL = Proportion of Low Pass Library; PC = Proportion of Capture Library; VL = Volume of Low Pass Library; VC = Volume of Capture Library; V = Total Volume):

| | | |
|---|---|---|
| for 1x coverage: | n * 0.056 | = PL |
| for 0.5x coverage: | n * 0.028 | = PL |
| 1 - PL | | = PC |

| | | |
|---|---|---|
| *For multiple LPWG libraries, this will be: | | |

| | |
|---|---|
| 1-SUM(PL) | = PC |

2. Calculate the Volume of each to add for 100 ng

| | |
|---|---|
| PL * 100 / library conc. | VL = |
| PC * 100 / library conc. | = VC |

3. Calculate the final library volume

| | |
|---|---|
| VL+VC | = V |

4. Calculate the final library concentration:

| | |
|---|---|
| 100 / V | = Final library conc. |

As shown in the library distributions in **FIGURE 24****,** examining 1000 reads from each of three samples (one LPWG, one capture, and one with both library types) established proof-of-concept for co-loading LPWG and Target Capture libraries from the sample onto the same sequencing flow cell and that the libraries can be successfully demultiplexed using Read 2 results.

LPWG libraries from wild type samples were co-loaded with wild type samples that had been processed as described in Example 9. Four LPAs were pooled and amplified for the LPWG library: two with barcodes that overlapped with the capture library samples and two with barcodes that did not. LPAs (12.5 ng) from each wild type were pooled then amplified for 16 cycles with a 65°C annealing temperature, then purified with Ampure beads and quantified by Qubit. Low pass whole genome libraries were loaded to a target coverage of 1x, as described in the calculation above. Forward and reverse sequencing primers were those described in Table 1, such that Read 1 will sequence both the target captured (TCLA) and LPWG libraries, while Read 2 will sequence only the target captured libraries. Empty sequences from LPWG libraries were seen as 17 "G"s in Read 2. Read 2 can therefore be used to differentiate the capture library sequences from the LPWG library sequences. Total number of reads for each barcoded sample (ID region identifier) are listed in **Table 8.**

**Table 8: Total reads per sample from co-loaded LPWG and capture libraries.**

| **Barcode** | **Sample** | **Library Type** | **Reads** |
|---|---|---|---|
| 8NNN-41 | WT R537984 | LPWG + Capture | 53717504 |
| 8NNN-42 | WT R537984 | LPWG + Capture | 55803131 |
| 8NNN-37 | WT R537984 | Capture | 33782985 |
| 8NNN-40 | WT R537984 | Capture | 35254861 |
| 8NNN-38 | WT R537989 | Capture | 43717435 |
| 8NNN-39 | WT R537989 | Capture | 41882025 |
| 8NNN-34 | WT R537989 | Capture | 42191765 |
| 8NNN-33 | WT R537989 | Capture | 42594803 |
| 8NNN-35 | WT R537995 | Capture | 47287483 |
| 8NNN-36 | WT R537995 | Capture | 45628161 |
| 8NNN-43 | Lung8 Pos Control | Capture | 19031726 |
| 8NNN-44 | NTC | Capture | 8880 |
| 8NNN-47 | WT R538001 | LPWG | 20474208 |
| 8NNN-48 | WT R538001 | LPWG | 18130947 |

### EXAMPLE 11-METHODS FOR PREPARING AN ADAPTOR-TAGGED PARENT LIBRARY

Illustrative methods of sample preparation, end repair, and adaptor ligation during the preparation of an adaptor-tagged Parent Library are provided below. These methods may be used in any quality control (QC) process of the disclosure, with any of the disclosed kit components, or in any of the disclosed methods. One of skill in the art would recognize that the reagents below are for illustrative purposes, *i.e.,* the amount of the reagents below may be changed to any suitable amount and the specific reagents may be interchangeable with other appropriate reagents.

### Sample Preparation

Each DNA sample was prepared to a suitable concentration (*e.g.,* 10 ng/µL) and diluted with an appropriate buffer such as a TEZ Buffer (TRIS-EDTA).

### End Repair

Commercially available enzymes and buffers were used to end repair the DNA fragments. 5 µL of an End Repair Master Mix was added to each DNA sample in a single tube reaction mixture. End Repair Master Mix was prepared by combining a commercially available End Repair Enzyme (*e.g.,* NEBNext Ultra II End Prep Enzyme Mix^{®} with an appropriate End Repair Buffer (*e.g.,* NEBNext Ultra II End Prep Reaction Buffer^{®}). The reaction mixture was incubated in a thermocycler under the following reaction conditions: 20°C for 15 min and then at 70°C for 10 min.

### Adaptor Ligation

One unique set of adaptors comprising a plurality of adaptor modules was assigned to each end-repaired sample. In some embodiments, the set of adaptors has passed an adaptor QC process of the disclosure. 5 µL of a set of adaptors was added to its assigned end-repaired sample, after which 30 µL of a Ligation Mix comprising a DNA ligase (*e.g.,* NEB Ultra II Ligation Mix^{®} ) was added. The reaction mixture was incubated at 20°C for 30 min to generate adaptor-tagged DNA fragments.

Each ligation strand of the adaptor modules was 47 nt in length, and comprised (from 5' -> 3') an amplification region (AMP, 25 nt), a multifunctional ID region (8 nt) capable of identifying both the sample and the unique fragment, a UMI multiplier (3 nt), an anchor (10 nt), and a 3' dT overhang. Exemplary adaptor structures are provided in **Table** 9. Exemplary adaptor ligation strands are provided in **Table 10.**

The set of the adaptor modules was prepared such that each adaptor set contained equimolar amounts of adaptors modules comprising four types of anchor regions, where each anchor type had a 3' terminal nucleotide selected from A, T, C, and G.

**Table 9: Illustrative Adaptor structures**

| Adaptor name | Description/Sequence |
|---|---|
| Ligation Strand/ Anchor Region 1 (16-1) | AMP-ID Region/UMI Multiplier-ACGTATGCCA (SEQ ID NO: 1)-3'dT |
| Ligation Strand/ Anchor Region 2 (16-2) | AMP-ID Region/UMI Multiplier-CTAGCGTTAC (SEQ ID NO: 2)-3'dT |
| Ligation Strand/ Anchor Region 3 (16-3) | AMP-ID Region/UMI Multiplier-GATCGACATG (SEQ ID NO: 3)-3'dT |
| Ligation Strand/ Anchor Region 4 (16-4) | AMP-ID Region/UMI Multiplier-TGCATCAGGT (SEQ ID NO: 4)-3'dT |
| Non-ligation strand/Anchor Region 1 (16_1) | ***TGGCATACGT*** (SEQ ID NO: 18) |
| Non-ligation strand/Anchor Region 2 (16_2) | ***GTAACGCTAG*** (SEQ ID NO: 19) |
| Non-ligation strand/Anchor Region 3 (16_3) | ***CATGTCGATC*** (SEQ ID NO: 20) |
| Non-ligation strand/Anchor Region 4 (16_4) | ***ACCTGATGCA*** (SEQ ID NO: 21) |

**Table 10: Illustrative Adaptor Ligation Strand Sequences**

| **Adaptor Module ligation strands with 3' dT overhangs** | **SEQ ID NO** |
|---|---|
| TGCAGGACCAGAGAATTCGAATACAAAAATCCTNNNACGTATGCCAT | 22 |
| TGCAGGACCAGAGAATTCGAATACAAATGATCTNNNACGTATGCCAT | 23 |
| TGCAGGACCAGAGAATTCGAATACAAGTAATAGNNNACGTATGCCAT | 24 |
| TGCAGGACCAGAGAATTCGAATACACACCTCCGNNNACGTATGCCAT | 25 |
| TGCAGGACCAGAGAATTCGAATACACGCCCCATNNNACGTATGCCAT | 26 |
| TGCAGGACCAGAGAATTCGAATACACTACCAAGNNNACGTATGCCAT | 27 |
| TGCAGGACCAGAGAATTCGAATACACTGTCGTTNNNACGTATGCCAT | 28 |
| TGCAGGACCAGAGAATTCGAATACAGCAAATGGNNNACGTATGCCAT | 29 |
| TGCAGGACCAGAGAATTCGAATACAGCTCGAGCNNNACGTATGCCAT | 30 |
| TGCAGGACCAGAGAATTCGAATACAGTCCACAANNNACGTATGCCAT | 31 |
| TGCAGGACCAGAGAATTCGAATACAGTTACCCTNNNACGTATGCCAT | 32 |
| TGCAGGACCAGAGAATTCGAATACATAGTTTTCNNNACGTATGCCAT | 33 |
| TGCAGGACCAGAGAATTCGAATACATCTCAGAGNNNACGTATGCCAT | 34 |
| TGCAGGACCAGAGAATTCGAATACATGACCTTCNNNACGTATGCCAT | 35 |
| TGCAGGACCAGAGAATTCGAATACATTACGGCANNNACGTATGCCAT | 36 |
| TGCAGGACCAGAGAATTCGAATACAAACAAAACNNNTGCATCAGGTT | 37 |
| TGCAGGACCAGAGAATTCGAATACAACACTGCANNNTGCATCAGGTT | 38 |
| TGCAGGACCAGAGAATTCGAATACAATCGCGATNNNTGCATCAGGTT | 39 |
| TGCAGGACCAGAGAATTCGAATACAATGGTGGANNNTGCATCAGGTT | 40 |
| TGCAGGACCAGAGAATTCGAATACACAACTCTCNNNTGCATCAGGTT | 41 |
| TGCAGGACCAGAGAATTCGAATACACGCCCGAANNNTGCATCAGGTT | 42 |
| TGCAGGACCAGAGAATTCGAATACACGTATGACNNNTGCATCAGGTT | 43 |
| TGCAGGACCAGAGAATTCGAATACAGAAACGACNNNTGCATCAGGTT | 44 |
| TGCAGGACCAGAGAATTCGAATACAGACTCTGANNNTGCATCAGGTT | 45 |
| TGCAGGACCAGAGAATTCGAATACAGTCACTCTNNNTGCATCAGGTT | 46 |
| TGCAGGACCAGAGAATTCGAATACATACTGGACNNNTGCATCAGGTT | 47 |
| TGCAGGACCAGAGAATTCGAATACATGCGATACNNNTGCATCAGGTT | 48 |
| TGCAGGACCAGAGAATTCGAATACATGTTAATGNNNTGCATCAGGTT | 49 |
| TGCAGGACCAGAGAATTCGAATACATTGTACTTNNNTGCATCAGGTT | 50 |
| TGCAGGACCAGAGAATTCGAATACATTTGGCTCNNNTGCATCAGGTT | 51 |
| TGCAGGACCAGAGAATTCGAATACAAACGCCTANNNGATCGACATGT | 52 |
| TGCAGGACCAGAGAATTCGAATACAAAGTTTCANNNGATCGACATGT | 53 |
| TGCAGGACCAGAGAATTCGAATACAACAGCGAANNNGATCGACATGT | 54 |
| TGCAGGACCAGAGAATTCGAATACAAGCGCCTGNNNGATCGACATGT | 55 |
| TGCAGGACCAGAGAATTCGAATACACAACCCTTNNNGATCGACATGT | 56 |
| TGCAGGACCAGAGAATTCGAATACACAGAATAANNNGATCGACATGT | 57 |
| TGCAGGACCAGAGAATTCGAATACACGGACACCNNNGATCGACATGT | 58 |
| TGCAGGACCAGAGAATTCGAATACAGCCTATTCNNNGATCGACATGT | 59 |
| TGCAGGACCAGAGAATTCGAATACAGCGTCCAGNNNGATCGACATGT | 60 |
| TGCAGGACCAGAGAATTCGAATACAGGTACAAGNNNGATCGACATGT | 61 |
| TGCAGGACCAGAGAATTCGAATACATAACCCTCNNNGATCGACATGT | 62 |
| TGCAGGACCAGAGAATTCGAATACATAGGAGTGNNNGATCGACATGT | 63 |
| TGCAGGACCAGAGAATTCGAATACATCCGCATTNNNGATCGACATGT | 64 |
| TGCAGGACCAGAGAATTCGAATACATGCGTCAANNNGATCGACATGT | 65 |
| TGCAGGACCAGAGAATTCGAATACATTGGTAATNNNGATCGACATGT | 66 |
| TGCAGGACCAGAGAATTCGAATACAAATAGCTTNNNCTAGCGTTACT | 67 |
| TGCAGGACCAGAGAATTCGAATACAAGAGAGAGNNNCTAGCGTTACT | 68 |
| TGCAGGACCAGAGAATTCGAATACACAACCTGANNNCTAGCGTTACT | 69 |
| TGCAGGACCAGAGAATTCGAATACACATATGGCNNNCTAGCGTTACT | 70 |
| TGCAGGACCAGAGAATTCGAATACACCATATCCNNNCTAGCGTTACT | 71 |
| TGCAGGACCAGAGAATTCGAATACACGAGGTCCNNNCTAGCGTTACT | 72 |
| TGCAGGACCAGAGAATTCGAATACACGTCAATGNNNCTAGCGTTACT | 73 |
| TGCAGGACCAGAGAATTCGAATACACTTATCATNNNCTAGCGTTACT | 74 |
| TGCAGGACCAGAGAATTCGAATACAGCATTGACNNNCTAGCGTTACT | 75 |
| TGCAGGACCAGAGAATTCGAATACAGGAGGTATNNNCTAGCGTTACT | 76 |
| TGCAGGACCAGAGAATTCGAATACATAACAGTTNNNCTAGCGTTACT | 77 |
| TGCAGGACCAGAGAATTCGAATACATCGAACACNNNCTAGCGTTACT | 78 |
| TGCAGGACCAGAGAATTCGAATACATGCATAATNNNCTAGCGTTACT | 79 |
| TGCAGGACCAGAGAATTCGAATACATGTCATAANNNCTAGCGTTACT | 80 |
| TGCAGGACCAGAGAATTCGAATACATTGCGCGGNNNCTAGCGTTACT | 81 |

| | |
|---|---|
| *NNN in the sequences of Table 10 represents a 3-nucleotide UMI multiplier wherein each N may be selected from any one of A, G, C, T. | |

After ligation, 100 µL of DNA purification beads (*e.g.,* Ampure XP^{®}; Beckman^{®}) were added to each sample. The reaction mixture was incubated at room temperature for 2 min. The beads were washed two times with 200 µL of 80% ethanol/water (v/v) while on a magnet, air-dried, then eluted with 25 µL of TRIS-EDTA (TEZ). The eluted clarified supernatant, about 25 µL containing the adaptor-tagged DNA fragments, was transferred to a fresh PCR tube or microtiter plate well for amplification to generate the adaptor-tagged DNA library. In some embodiments, DNA libraries at this stage may be referred to as LIBS.

### Library Amplification

Following adaptor ligation, 75 µL of a master mix containing a Library PCR Mix comprising PCR reagents, a high-fidelity DNA polymerase enzyme (*e.g.,* NEBNext^{®} Ultra II Q5^{®} Master Mix; New England Biolabs^{®}), and a Library Primer was added to each sample. The reaction mixture was amplified using the following PCR parameters:
60°C for 30 sec, 72°C for 2 min, 98°C for 30 sec;
8 cycles of: 98°C for 30 sec, 65°C for 30 sec and 72°C for 30 sec.

The Library Primer is a single amplification primer having the sequence TGCAGGACCAGAGAATTCGAATACA (SEQ ID NO: 5).

The initial 3 min incubation cycle was performed to form a plurality of contiguous adaptor-tagged dsDNA fragments by ligation-strand-templated extension, followed by an 8 cycle PCR amplification of the contiguous adaptor-tagged dsDNA fragments to form an amplified tagged DNA library containing adaptor-tagged DNA fragment molecules.

After amplification, 120 µL of DNA purification beads were added to the ligation mix. The reaction mixture was incubated at room temperature for 2 min. The beads were washed two times with 200 µL of 80% ethanol/water (v/v) while on a magnet, air-dried, then eluted with 20 µL of TEZ Buffer. Clarified supernatant containing the amplified tagged DNA library was transferred to a fresh PCR tube.

Libraries at this stage may be referred to as the adaptor-tagged Parent Library or Library Post-Amplification (LPA). The LPA was split into at least two portions to generate the Target Capture Library Post-Amplification (TC LPA) and the Whole-Genome Library Post-Amplification (WG LPA). The TC LPA was directly generated by aliquoting a portion of the LPA. The WG LPA was generated by making a 1:10 dilution of the LPA or TC LPA.

In some embodiments, the TC LPA and WG LPA may be subject to a Quality Control (QC) process. The concentration of the LPA was determined using a Qubit^{™} dsDNA HS Assay Kit following manufacturer's instructions. The concentration of the LPA was also the concentration of the TC LPA. In cases where the WG LPA was created by making a 1:10 dilution of the LPA or TC LPA, the concentration of the WG LPA was first be determined, and the TC LPA concentration was calculated by 10 x [concentration of the WG LPA]. In some embodiments, only a TC LPA with a concentration of at least 1 ng/µL to at least 80 ng/µL was used in downstream processes. In some embodiments, only a WG LPA with a concentration of at least 0.1 ng/µL to at least 8 ng/µL was used in downstream processes.

### EXAMPLE 12-METHODS FOR PREPARING A TARGET CAPTURE LIBRARY (TCL) OR TARGET CAPTURE LIBRARY AMPLIFIED (TCLA)

In order to capture and enrich genetic loci (target regions) of interest, a portion of the adaptor-tagged Parent Library prepared as described in Example 11 can be multiplexed and hybridized to a pool of capture probe modules specific for a target gene panel. The target gene panel may be a collection of genes specific of a disease. For example, the capture probe modules may be specific for one or more lung cancer genes as described in **Table 11** below. In some embodiments, a set of capture probe modules specific for lung cancer genes are referred to as Lung Probes. In some embodiments, a specific set of capture probes may be used in a QC process of kit components, for example, a set of capture probes that are designed to be homologous recombination deficient (HRD Probes). An illustrative set of target genes for HRD Probes include one or more genes selected from *AR, ATM, ATR, BARD1, BRCA1, BRCA2, BRIP1, CDK12, CHEK1, CHEK2, ERBB2, ESR1, FANCA, FANCL, HDAC2, KRAS, MYC, PALB2, PIK3CA, PTEN, RAD50, RAD51, RAD51B, RAD51C, RAD54L, RB1, SRY,* and *TP53.*

**Table 11: Illustrative target genes for lung cancer**

| **Variant type** | **No. of genes** | **Gene names** |
|---|---|---|
| SNV(single-nucleotide variation) /Indel | 27 | *AKT1, ALK, BRM, BRAF, CD274, CDK2NA, EGFR, ERBB2, FGFR1, FGFR2, FGFR3, KEAP1, KRAS, MAP2KI, MET, MLHI, MSH2, MSH6, MYC, NRAS, PIK3CA, PMS2, PTEN, RET, ROS1, STK11, TP53* |
| Fusion/Rearrangement | 8 | *ALK, BRAF, EGFR, FGFR2, FGFR3, NTRKI, RET, ROSI* |
| CNV (copy number variation) | 23 | *AKTI, B2M, BRAF, CD274, CDKN2A, EGFR, ERBB2, FGFR1, FGFR2, FGFR3, KRAS, MET, MYC, NRAS, NTRK1, PIK3CA, PMS2, PTEN, RET, RICTOR, ROS1, STK11, TP53* |
| Control | 1 | *SRY* |

A portion of the LPA as prepared according to Example 11 was used as the starting material for target capture. Libraries at this stage (*e.g.,* a portion of the LPA) may also be referred to as Target Capture Library Post Amplification (TC LPA).

The TC LPA was combined with capture probe modules in a hybridization reaction mixture. The amount of capture probe modules may be any suitable amount. In this example, the volume of capture probe modules was ½ of the total volume of the TC LPA.

The hybridization reaction mixture was denatured in a thermocycler for 2 minutes at 98 °C. The hybridization reaction mixture was then lowered to 60 °C and transferred to a precooled PCR rack on ice. A hybridization buffer was added, and each hybridization reaction mixture was denatured in a thermocycler for 1 minute at 98 °C. The temperature was then lowered to 65 °C and the hybridization reaction was allowed to proceed for 12 to 24 hours.

After the hybridization reaction was complete, 30 µL of streptavidin-coated beads (Dynabeads MyOne C1) in TEZ buffer was combined with each hybridization reaction and allowed to stand at room temperature for 20 min. The beads were collected on a magnet and washed once with 200 µL of TEZ buffer. The washed beads were re-suspended in 20 µL of TEZ buffer. 80 µL of a suitable wash buffer was added to the resuspended beads, and the mixture was incubated for 5 min at 45 °C. The beads were then separated using a magnet and washed with 200 µL of TEZ buffer. The washed beads were re-suspended in 20 µL of TEZ buffer.

Following hybridization, primer extension of the capture probe was used to copy the captured genomic sequences, the A/T overhang at the junction of the DNA fragment, and the attached adaptor module to form a library of Hybrid Molecules. The extended capture probes, *i.e.,* Hybrid Molecules, comprise the DNA fragment flanked by the capture probe module on one end and the adaptor module on the other end. On-bead probe extension was performed by adding a POST-CAP master mix to the hybridization reaction mixture (*i.e.,* the re-suspended washed beads above). The POST-CAP master mix comprises a Post Amp PCR Mix and a Post Hyb Primer mix. The Post Hyb Primer mix was used to incorporate sequencing adaptors, e.g., Illumina^{®} sequencing adaptors, to the Hybrid Molecules. In some embodiments, the Post Amp PCR mix comprised a high fidelity DNA polymerase, *e.g.,* the NEBNext Ultra II Q5 master mix. In some embodiments, 80 µL of the POST-CAP master mix was added to each hybridization reaction mixture.

In some embodiments, the Post Hyb Primer mix comprises:
a Forward Primer: 
AATGATACGGCGACCACCGAGATCTACACGTCATGCAGGACCAGAGAATTCGAA
TACA (SEQ ID NO: 7)
 and
a Reverse Primer:

CAAGCAGAAGACGGCATACGAGATGTGACTGGCACGGGAGTTGATCCTGGTTTT
CAC (SEQ ID NO: 82).

The on-bead probe extension reaction was performed on a thermal cycler according to the following program: 60°C for 30 sec; 72°C for 30 sec; 98°C for 30 sec; 5 cycles of 98°C for 30 sec; 65°C for 30 sec; 72°C for 30 sec.

The reaction mixture comprising washed beads at this stage may be referred to as the Target Capture Library (TCL).

Next, the beads were separated from the reaction mixture on a magnet. The supernatant was transferred to a fresh PCR tube and combined with a TC AMP master mix comprising the Post AMP PCR Mix and the Post Hyb Primer mix. In some embodiments, 100 µL of TC AMP master mix was added to each PCR tube comprising the supernatant. The resulting mixture comprising the supernatant was amplified on a thermal cycler using the following amplification program:
12 cycles of: 98°C for 30 sec; 65°C for 30 sec; 72°C for 30 sec.

Following amplification, 120 µL of DNA purification beads (*e.g.,* Ampure XP^{®}; Beckman^{®}) was added to each PCR tube. The reaction mixture was incubated at room temperature for 2 min. The beads were washed two times with 200 µL of 80% ethanol/water (v/v) while on a magnet, air-dried, then eluted with 50 µL of TRIS-EDTA (TEZ). The eluted clarified supernatant, about 50 µL containing the amplified Hybrid Molecules, was transferred to a fresh PCR tube. DNA libraries at this stage may be referred to as Target Capture Library Amplified (TCLA) or amplified Target Capture Library. In some embodiments, the amplified Hybrid Molecules within the TCLA are "sequence ready" in that they contain sequencing primer binding sites at the two ends of the molecule, as shown in FIGURE 28.

### EXAMPLE 13-METHODS FOR PREPARING A WHOLE-GENOME LIBRARY AMPLIFIED (WGLA)

WG LPA libraries were generated according to Example 11. Each WG LPA may be quantified using a Qubit^{™} dsDNA HS Assay Kit following manufacturer's instructions.

Prior of amplification of the WG LPA, a sample input normalization step was performed because more than one test sample was analyzed. For each test sample, one WG LPA was generated with a uniquely assigned adaptor set and each WG LPA was pooled into a "Transfer Pool". A suitable amount of the Transfer Pool was transferred into a new tube and the sample volume was brought to 40 µL with TEZ buffer, creating a normalized WG LPA pool ready for amplification.

To each WG LPA pool, a WG AMP master mix was added to create a WG LPA amplification mixture. The WG AMP master mix comprises the Post AMP PCR Mix and a Genomic Mix. The Genomic Mix is a primer mixture comprising:
Genomic Mix Primer F: 
AATGATACGGCGACCACCGAGATCTACACGTCATGCAGGACCAGAGAATTCGAA
TACA
(SEQ ID NO: 7)
Genomic Mix Primer R: 
CAAGCAGAAGACGGCATACGAGATGTGACTGGTGCAGGACCAGAGAATTCGAAT
ACA
(SEQ ID NO: 9).
60 µL of WG AMP master mix was added to each WG LPA pool.

The WG LPA amplification mixture was amplified on a thermal cycler using the following amplification program:
12 cycles of: 98°C for 30 sec; 65°C for 30 sec; 72°C for 30 sec.

Following amplification, 120 µL of DNA purification beads (*e.g.,* Ampure XP^{®}; Beckman^{®}) was added to each PCR tube. The reaction mixture was incubated at room temperature for 2 min. The beads were washed two times with 200 µL of 80% ethanol/water (v/v) while on a magnet, air-dried, then eluted with 50 µL of TRIS-EDTA (TEZ). The eluted clarified supernatant, about 50 µL containing the amplified library, was transferred to a fresh PCR tube. DNA libraries at this stage may be referred to as Whole-genome Library Amplified (WGLA) or a low pass whole genome (LPWG) library.

### EXAMPLE 14-METHODS FOR SEQUENCING THE COMBINED LIBRARY

The TCLA and WGLA generated according to Examples 11-13 were combined to create a Sequence-Ready Library (SRL).

In some embodiments, prior to combination, the TCLA and WGLA were subject to a Quality Control (QC) process. The concentration of the TCLA and the WGLA were determined using a Qubit^{™} dsDNA HS Assay Kit following manufacturer's instructions. In some embodiments, only a TCLA with a concentration of at least 1 ng/µL to at least 80 ng/µL was used in downstream processes; and only a WGLA with a concentration of at least 1 ng/µL to at least 80 ng/µL was used in downstream processes.

The SRL was created by combining the TCLA and WGLA at suitable volumes. The resulting SRL was quantified using a Qubit^{™} dsDNA HS Assay Kit following manufacturer's instructions prior to sequencing.

The SRL was sequenced using an Illumina^{®} sequencer following manufacturer instructions, *e.g.,* the NextSeq 550. Sequencing primers comprising the sequences below were used:
FWD Seq Primer: 
CAAGCAGAAGACGGCATACGAGATGTGACTGGCACGGGACCAGAGAATTCGAAT
ACA (SEQ ID NO: 10)
REV Seq Primer:
   GTGACTGGCACGGGACCAGAGAATTCGAATACA (SEQ ID NO: 11).

### EXAMPLE 15-QUALITY CONTROL OF KIT REAGENTS AND PRIMERS

In some embodiments, compositions of the disclosure are assembled into a kit comprising one or more reagents and/or primers selected from an End Repair Buffer, an End Repair Enzyme, a Ligation Mix, a Library PCR Mix, a Library Primer, a Post Amp PCR Mix, a Post Hyb Primer mix, a Genomic Mix, a FWD Seq Primer and a REV Seq Primer. Each of these reagents and primers may be the corresponding composition described elsewhere in the disclosure, or as described in Examples 11-14.

To ensure high performance of a kit of the disclosure, one or more of the above reagents and primers of the kit may be subject to a Quality Control (QC) process prior to use. This QC process may use a sample comprising sheared genomic DNA from a blend of cell lines, NA09596/NA12878 at 50:50 ratio (cell line gDNA is sourced from Coriell Institute). This sample may also be referred to as the 50:50 blended sample.

In this illustrative example, at least two test samples each comprising a suitable amount of the 50:50 blended sample were prepared in TEZ buffer. A unique adaptor set was assigned to each test sample. In some embodiments, the set of adaptors has passed an adaptor QC process of the disclosure. For each test sample, end repair, adaptor ligation, and library amplification were performed according to the methods of Example 11 to generate an LPA. After generation of the LPA, a TC LPA and a WG LPA were created according to the methods of Example 11. A QC process on the TC LPA and WG LPA was performed and libraries that passed QC were selected for downstream use. For QC of the reagents and primers in this example, a TC LPA with a concentration of at least 1 ng/µL to at least 80 ng/µL was used in downstream processes; and a WG LPA with a concentration of at least 0.1 ng/µL to at least 8 ng/µL was used in downstream processes.

The TC LPA of each test sample was used to create a TCL using HRD Probes according to the methods of Example 12, and a TCLA was subsequently created according to the methods of Example 12. The WG LPA of each test sample was used to create a WGLA according to the methods of Example 13. A QC process was performed on each of the TCLA and WGLA generated, where each TCLA that passed QC had a concentration of at least 1 ng/µL to at least 80 ng/µL and each WGLA that passed QC had a concentration of at least 1 ng/µL to at least 80 ng/µL. The TCLA and WGLA of each test sample that have passed QC were combined and sequenced according to the methods of Example 14.

After sequencing analysis, one or more acceptance criteria determined whether the reagents and primers in this example met the quality requirements to enable high performance of the kit. One or more acceptance criteria were set for QC of the Reagents and Primers. An illustrative set of acceptance criteria is described in **Table 12.**

**Table 12: Illustrative acceptance criteria for Reagents and Primers of a kit**

| **Test Description** | **Sample** | **Acceptance Criterion** |
|---|---|---|
| SNP (single-nucleotide polymorphism) Detection | 50:50 blended sample | At least 90% of the SNPs per Table 16 are detected |
| Copy Number Loss | 50:50 blended sample | At least one copy number loss even is detected |
| Homodel Ran (Low Pass Amplification) | 50:50 blended sample | At least 100,000 to 2,000,000 reads are detected for a given sample |
| Coverage | 50:50 blended sample | At least 90% of the region of interest has coverage to enable detection of variants at 3% AF (allele frequency) |
| Off-Target Reads | 50:50 blended sample | No more than 20% of total sequencing reads |

### EXAMPLE 16-QUALITY CONTROL OF KIT ADAPTOR SETS

In some embodiments, compositions of the disclosure are assembled into a kit comprising one or more adaptor sets. These adaptor sets may comprise any of the adaptor modules described in the disclosure. To ensure high performance of the adaptors, each of adaptor sets may be subject to a Quality Control (QC) process prior to inclusion in the kit.

### QC for adaptor ligation efficiency.

In this example, an adaptor QC process to test adaptor ligation efficiency was performed using the 50:50 blended sample described above.

At least two test samples each comprising a suitable amount of the 50:50 blended sample were prepared in TEZ buffer. A unique adaptor set was assigned to each test sample. For each test sample, end repair, adaptor ligation, and library amplification were performed according to the methods of Example 11 to generate an LPA.

A QC process was performed on the LPA to gauge the efficiency of adaptor attachment in each adaptor set. As described above, the efficiency of adaptor attachment refers to the conversion rate or percentage of input DNA fragments to adaptor-tagged DNA library molecules. One or more acceptance criteria were set for the QC of adaptor attachment efficiency. In this example, the acceptance criterion for adaptor attachment efficiency was the LPA had a concentration of at least at least 1 ng/µL to 200 ng/µL with 5 ng to 50 ng input DNA.

### OC for Adaptor distribution and sequence identification

A QC process to test adaptor distribution and sequence identification was performed using a wild type (WT) cfDNA sample. The WT cfDNA sample had a concentration of at least 0.2 ng/µL and was obtained from a healthy human or a number of healthy humans.

At least two test samples each comprising a suitable amount of WT cfDNA were prepared in TEZ buffer. A unique adaptor set was assigned to each test sample. In some embodiments, the set of adaptors has passed an adaptor QC process of the disclosure. For each test sample, end repair, and adaptor ligation were performed according to the methods of Example 11 to generate a collection of adaptor-tagged DNA fragments (LIBS).

Amplification of the LIBS of each test sample was performed using Index Primers, where one or more unique Index Primers were assigned to each the LIBS of each test sample. In this example, the Index Primers comprise Illumina^{®} P5 and/or P7 sequences. In some embodiments, the Index Primers comprise the sequences of SEQ ID NO: 13 or SEQ ID NO: 14. An appropriate Index Primer, a Library PCR Mix (as described in Example 11) and a Primer F comprising the sequence of SEQ ID NO: 7 were added to each LIBS to create an amplification mixture. The resulting mixture was amplified on a thermal cycler following the program below:
60°C for 30 sec, 72°C for 2 min, 98°C for 30 sec;
12 cycles of: 98°C for 30 sec, 65°C for 30 sec and 72°C for 30 sec.

After amplification, 120 µL of DNA purification beads (*e.g.,* AMPure beads) were added to the ligation mix. The reaction mixture was incubated at room temperature for 2 min. The beads were washed two times with 200 µL of 80% ethanol/water (v/v) while on a magnet, air-dried, then eluted with 20 µL of TEZ Buffer. Clarified supernatant containing the amplified tagged DNA library was transferred to a fresh PCR tube.

Libraries at this stage may be referred to as the Library Post Index Amplification (LPIA). The concentration of the LPIA was determined using a Qubit^{™} dsDNA HS Assay Kit following manufacturer's instructions.

Prior to sequencing, the LPIA of each test sample was pooled by combining each LPIA into a new PCR tube. The combined LPIA pool was brought to a suitable concentration using TEZ buffer. The concentration of the combined LPIA pool was determined using a Qubit^{™} dsDNA HS Assay Kit following manufacturer's instructions.

The LPIA pool was sequenced using an Illumina^{®} sequencer following manufacturer instructions, *e.g.,* the NextSeq 550. Sequencing primers comprising the sequences below were used:
FWD Seq Primer: 
CAAGCAGAAGACGGCATACGAGATGTGACTGGCACGGGACCAGAGAATTCGAAT
ACA (SEQ ID NO: 10);
REV Seq Primer:
   GTGACTGGCACGGGACCAGAGAATTCGAATACA (SEQ ID NO: 11);
and an Index Sequencing Primer comprising the sequence of the Index Primer used during amplification.

After sequencing analysis, one or more acceptance criteria determined whether the adaptor sets met the quality requirements to enable high performance of the kit. One or more acceptance criteria were set for QC of the adaptor distribution and sequence identification. Illustrative acceptance criteria for QC of the adaptor distribution and sequence identification used in this example are listed in Table 13.

Cross-contamination of adaptor sets was also analyzed. Cross-contamination of adaptor sets may occur when an adaptor that is not assigned to a given sample appears in the sequencing reads of this sample. In this example, the acceptance criteria for QC of adaptor set cross-contamination include:
a. for each sample, no more than 0.05%-2% of "Barcode Crosstalk";
b. for each sample, no more than 1%-20% of reads are from unknown adaptors (*i.e.,* not included in the adaptor set).
Barcode Crosstalk as used herein refers to the reads of one barcode (adaptor) being mislabeled as adaptor A when they are actually generated from adaptor B.

An illustrative set of acceptance criteria for adaptor distribution and sequence identification is presented in **Table 13.**

**Table 13: Illustrative acceptance criteria for adaptor distribution and sequence identification**

| **Test Description** | **Sample** | **Acceptance Criterion** |
|---|---|---|
| Barcode Crosstalk | WT cfDNA | No more than 0.05%-2% of reads |
| Unknown adaptors | WT cfDNA | No more than 1%-20% of reads |
| Adaptor distribution | WT cfDNA | no more than 10%-80% of unique adaptor sequences have a number of reads that is 0% to 50% of the average number of reads for all unique adaptor sequences |
| UMI coverage | WT cfDNA | At least 80%-99.9% of all unique adaptor sequences within an adaptor set are present in the test sample |
| UMI distribution | WT cfDNA | No more than 5%-50% of unique adaptor sequences have reads greater than twice the average number of reads for all unique adaptor sequences |

### EXAMPLE 17-QUALITY CONTROL OF KIT POSITIVE CONTROL SAMPLE

In some embodiments, compositions of the disclosure are assembled into a kit comprising a positive control DNA sample. In some embodiments, the positive control DNA sample of a given kit is specific to the capture probe modules of the same kit. To ensure high performance of the kit, the positive control DNA sample may be subject to a Quality Control (QC) process prior to inclusion in the kit.

In this example, the positive control DNA sample comprises sheared genomic DNA from a blend of cell lines: 8% NA18511 in NA12878 (cell line gDNA is sourced from Coriell Institute). To test the positive control sample, an internal control sample comprising the 50:50 blended sample described above was used.

At least two test samples comprising different concentrations of the positive control sample were prepared in TEZ buffer, and an internal control sample comprising the 50:50 blended sample was prepared in TEZ buffer. A unique adaptor set was assigned to each test sample and internal control sample. In some embodiments, the set of adaptors has passed an adaptor QC process of the disclosure. For each test sample and internal control sample, end repair, adaptor ligation, and library amplification were performed according to the methods of Example 11 to generate an LPA.

After generation of the LPA, a TC LPA and a WG LPA were created according to the methods of Example 11. A QC process on the TC LPA and WG LPA was performed and libraries that passed QC were selected for downstream use. For this example, a TC LPA with a concentration of at least 1 ng/µL to at least 80 ng/µL was used in downstream processes; and a WG LPA with a concentration of at least 0.1 ng/µL to at least 8 ng/µL was used in downstream processes.

The TC LPA of each test sample and internal control sample were used to create a TCL using HRD Probes according to the methods of Example 12. A corresponding TCLA was subsequently created according to the methods of Example 12. The WG LPA of each test sample internal control sample was used to create a WGLA according to the methods of Example 13. A QC process was performed on each of the TCLA and WGLA generated, where each TCLA that passed QC had a concentration of at least 1 ng/µL to at least 80 ng/µL and each WGLA that passed QC had a concentration of at least 1 ng/µL to at least 80 ng/µL. The TCLA and WGLA of each test sample that have passed QC were combined and sequenced according to the methods of Example 14.

After sequencing analysis, one or more acceptance criteria determined whether the positive control DNA sample met the quality requirements to enable high performance of the kit. One or more acceptance criteria were set for QC of the positive control DNA sample. Illustrative acceptance criteria for QC of the positive control DNA sample are provided in **Table 14.**

**Table 14: Illustrative acceptance criteria for the Positive Control DNA sample**

| **Test Description** | **Sample** | **Acceptance Criterion** |
|---|---|---|
| SNP Detection | Internal control sample (50:50 blended sample) | At least 90% of the SNPs per Table 16 |
| SNP Detection | Positive Control DNA sample at various input amounts | At least 90% of expected SNPs per Table 17 detected between 1% and 20% inclusive |
| Copy Number Loss | Internal control sample (50:50 blended sample) | At least one copy number loss event detected |
| Blending | Positive Control DNA sample at various input amounts | Mean AF of expected SNPs (listed in Table 17) between 0.1% and 10% inclusive |
| Contamination | Positive Control DNA sample at various input amounts | Less than 10-50 unexpected SNPs (listed in Table 18) detected at ≥1%. |
| Coverage | Internal control sample (50:50 blended sample) | At least 90% of the region of interest has coverage to enable detection of variants at 3% AF (allele frequency) |
| Coverage | Positive Control DNA sample at lowest input amount | At least 90% of the region of interest has coverage to enable detection of variants at 4% AF (allele frequency) |
| Coverage | Positive Control DNA sample at highest input amount | At least 90% of the region of interest has coverage to enable detection of variants at 4% AF (allele frequency) |

### EXAMPLE 18-QUALITY CONTROL OF KIT CAPTURE PROBE MODULES

In some embodiments, compositions of the disclosure are assembled into a kit comprising one or more capture probe modules. A set of capture probe modules designed to target a specific collection of genes, *e.g.,* a set of lung cancer genes as described in Table 11, may be referred to as a capture probe panel. These capture probe modules or capture probe panels may comprise any capture probe module of the disclosure. To ensure high performance of the kit, each of the capture probe modules or capture probe panels may be subject to a Quality Control (QC) process prior to inclusion in the kit.

A QC process to test performance of a capture probe panel was performed using a positive control DNA sample. In this example, the positive control sample comprises sheared genomic DNA from a blend of cell lines: 8% NA18511 in NA12878 (cell line gDNA is sourced from Coriell Institute). The positive control DNA sample has passed the QC process described in Example 17.

At least two test samples each comprising the positive control DNA sample were prepared in TEZ buffer. A unique adaptor set was assigned to each test sample. In some embodiments, the set of adaptors has passed an adaptor QC process of the disclosure. For each test sample, end repair, adaptor ligation, and library amplification were performed according to the methods of Example 11 to generate an LPA. After generation of the LPA, a TC LPA and a WG LPA were created according to the methods of Example 11. A QC process on the TC LPA and WG LPA was performed and libraries that passed QC were selected for downstream use. For this example, a TC LPA with a concentration of at least 1 ng/µL to at least 80 ng/µL was used in downstream processes; and a WG LPA with a concentration of at least 0.1 ng/µL to at least 8 ng/µL was used in downstream processes.

The TC LPA of each test sample was used to create a TCL using a specific capture probe panel according to the methods of Example 12, and a TCLA was subsequently created according to the methods of Example 12. The specific capture probe panel comprised a set of Lung Probes. In some embodiments, each capture probe module within the set of Lung Probes binds to a specific target sequence in a gene associated with lung cancer. In some embodiments, each capture probe module within the set of Lung Probes binds to a specific target sequence in a gene listed in Table 11.

The WG LPA of each test sample was used to create a WGLA according to the methods of Example 13. A QC process was performed on each of the TCLA and WGLA generated, where each TCLA that passed QC had a concentration of at least 1 ng/µL to at least 80 ng/µL and each WGLA that passed QC had a concentration of at least 1 ng/µL to at least 80 ng/µL The TCLA and WGLA of each test sample that passed QC were combined and sequenced according to the methods of Example 14.

After sequencing analysis, one or more acceptance criteria determined whether the capture probe modules met the quality requirements to enable high performance of the kit. One or more acceptance criteria were set for QC of the capture probe modules. An illustrative set of acceptance criteria for capture probe modules is presented in **Table 15.**

**Table 15: Illustrative acceptance criteria for capture probe modules**

| **Test Description** | **Sample** | **Acceptance Criterion** |
|---|---|---|
| Probe Presence | Positive Control DNA sample | At least 90%-99.9% of probes have ≥1 total reads |
| On-target Reads | Positive Control DNA sample | At least 90% of probes have ≥70 on-target total reads |
| Off-Target Reads | Positive Control DNA sample | The combined off-target targeted reads and unaligned targeted reads for a positive control sample are equal to or less than 20% of the total targeted reads for that sample |
| SNP Detection | Positive Control DNA sample | At least 90% of expected SNPs (listed in Table 19) detected |
| Coverage | Positive Control DNA sample | At least 90% of the region of interest has coverage to enable detection of variants at 3% AF (allele frequency) |

### EXAMPLE 19-ADDITIONAL KITS

In some embodiments, a kit of the disclosure comprises one or more capture probe modules, wherein each capture probe module comprises a tail sequence and a capture probe sequence capable of hybridizing to a target sequence in a test sample.

In some embodiments, a kit of the disclosure comprises one or more capture probe modules, wherein each capture probe module comprises a tail sequence and a capture probe sequence capable of hybridizing to a target sequence in a test sample, wherein the one or more capture probe modules have passed a probe QC process performed according to Example 18.

In some embodiments, a kit of the disclosure comprises a set of adaptors, wherein each adaptor comprises an adaptor module.

In some embodiments, a kit of the disclosure comprises a set of adaptors, wherein each adaptor comprises an adaptor module, wherein the set of adaptors have passed an adaptor QC process performed according to Example 16.

In some embodiments, a kit of the disclosure comprises (a) one or more capture probe modules, wherein each capture probe module comprises a tail sequence and a capture probe sequence capable of hybridizing to a target sequence in a test sample; and (b) a set of adaptors, wherein each adaptor comprises an adaptor module.

In some embodiments, a kit of the disclosure comprises (a) one or more capture probe modules, wherein each capture probe module comprises a tail sequence and a capture probe sequence capable of hybridizing to a target sequence in a test sample; and (b) a set of adaptors, wherein each adaptor comprises an adaptor module, wherein the set of adaptors have passed an adaptor QC process performed according to Example 16.

In some embodiments, a kit of the disclosure comprises (a) one or more capture probe modules, wherein each capture probe module comprises a tail sequence and a capture probe sequence capable of hybridizing to a target sequence in a test sample, wherein the one or more capture probe modules have passed a probe QC process performed according to Example 18; and (b) a set of adaptors, wherein each adaptor comprises an adaptor module.

In some embodiments, a kit of the disclosure comprises (a) one or more capture probe modules, wherein each capture probe module comprises a tail sequence and a capture probe sequence capable of hybridizing to a target sequence in a test sample, wherein the one or more capture probe modules have passed a probe QC process performed according to Example 18; and (b) a set of adaptors, wherein each adaptor comprises an adaptor module, wherein the set of adaptors have passed an adaptor QC process performed according to Example 16.

In some embodiments, a kit of the disclosure comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; and (c) a first primer pair comprising a first F primer and a first R primer, wherein the first F primer comprises an amplification region binding region and a sequencing primer binding region, wherein the first R primer comprises a tail sequence binding region and a sequencing primer binding region.
In some embodiments, a kit of the disclosure comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; and (c) a first primer pair comprising a first F primer and a first R primer, wherein the first F primer comprises an amplification region binding region and a sequencing primer binding region, wherein the first R primer comprises a tail sequence binding region and a sequencing primer binding region; wherein the capture probe modules have passed a probe QC process performed according to Example 18.

In some embodiments, a kit of the disclosure comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; and (c) a first primer pair comprising a first F primer and a first R primer, wherein the first F primer comprises an amplification region binding region and a sequencing primer binding region, wherein the first R primer comprises a tail sequence binding region and a sequencing primer binding region; wherein the set of adaptors has passed an adaptor QC process performed according to Example 16.

In some embodiments, a kit of the disclosure comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; and (c) a first primer pair comprising a first F primer and a first R primer, wherein the first F primer comprises an amplification region binding region and a sequencing primer binding region, wherein the first R primer comprises a tail sequence binding region and a sequencing primer binding region; wherein the set of adaptors has passed an adaptor QC process performed according to Example 16 and the capture probe modules have passed a probe QC process performed according to Example 18.

In some embodiments, a kit of the disclosure comprises (a) a set of adaptors, wherein each adaptor comprises an adaptor module comprising an amplification region; and (b) a second primer pair comprising a second F primer and a second R primer, wherein each of the second F primer and the second R primer comprises an amplification region binding region and a sequencing primer binding region.

In some embodiments, a kit of the disclosure comprises (a) a set of adaptors, wherein each adaptor comprises an adaptor module comprising an amplification region; and (b) a second primer pair comprising a second F primer and a second R primer, wherein each of the second F primer and the second R primer comprises an amplification region binding region and a sequencing primer binding region; wherein the set of adaptors has passed an adaptor QC process performed according to Example 16.

In some embodiments, a kit of the disclosure comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a tail sequence binding region and a sequencing primer binding region; and (d) a second primer pair comprising a second F primer and a second R primer, wherein each of the second F primer and the second R primer comprises an amplification region binding region and a sequencing primer binding region.

In some embodiments, a kit of the disclosure comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a tail sequence binding region and a sequencing primer binding region; and (d) a second primer pair comprising a second F primer and a second R primer, wherein each of the second F primer and the second R primer comprises an amplification region binding region and a sequencing primer binding region; wherein the capture probe modules have passed a probe QC process performed according to Example 18.

In some embodiments, a kit of the disclosure comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a tail sequence binding region and a sequencing primer binding region; and (d) a second primer pair comprising a second F primer and a second R primer, wherein each of the second F primer and the second R primer comprises an amplification region binding region and a sequencing primer binding region; wherein the set of adaptors has passed an adaptor QC process performed according to Example 16.

In some embodiments, a kit of the disclosure comprises (a) one or more capture probe modules, wherein each capture probe module comprises a tail sequence and a capture probe sequence capable of hybridizing to a target sequence in a test sample; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a tail sequence binding region and a sequencing primer binding region; and (d) a second primer pair comprising a second F primer and a second R primer, wherein each of the second F primer and the second R primer comprises an amplification region binding region and a sequencing primer binding region; wherein the set of adaptors has passed an adaptor QC process performed according to Example 16 and the capture probe modules have passed a probe QC process performed according to Example 18.

In some embodiments, a kit of the disclosure comprises (a) one or more capture probe modules, wherein each capture probe module comprises a tail sequence and a capture probe sequence capable of hybridizing to a target sequence in a test sample, wherein the tail sequence of each capture probe module comprises a Library Tag; and (b) a set of adaptors, wherein each adaptor comprises an adaptor module comprising an amplification region.

In some embodiments, a kit of the disclosure comprises (a) one or more capture probe modules, wherein each capture probe module comprises a tail sequence and a capture probe sequence capable of hybridizing to a target sequence in a test sample, wherein the tail sequence of each capture probe module comprises a Library Tag; and (b) a set of adaptors, wherein each adaptor comprises an adaptor module comprising an amplification region; wherein the set of adaptors has passed an adaptor QC process performed according to Example 16.

In some embodiments, a kit of the disclosure comprises (a) one or more capture probe modules, wherein each capture probe module comprises a tail sequence and a capture probe sequence capable of hybridizing to a target sequence in a test sample, wherein the tail sequence of each capture probe module comprises a Library Tag; and (b) a set of adaptors, wherein each adaptor comprises an adaptor module comprising an amplification region; wherein the one or more capture probe modules have passed a probe QC process performed according to Example 18.

In some embodiments, a kit of the disclosure comprises (a) one or more capture probe modules, wherein each capture probe module comprises a tail sequence and a capture probe sequence capable of hybridizing to a target sequence in a test sample, wherein the tail sequence of each capture probe module comprises a Library Tag; and (b) a set of adaptors, wherein each adaptor comprises an adaptor module comprising an amplification region; wherein the one or more capture probe modules have passed a probe QC process performed according to Example 18 and the set of adaptors has passed an adaptor QC process performed according to Example 16.

In some embodiments, a kit of the disclosure comprises (a) one or more capture probe modules, wherein each capture probe module comprises a tail sequence and a capture probe sequence capable of hybridizing to a target sequence in a test sample, wherein the tail sequence of each capture probe module comprises a Library Tag; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a Library Tag binding region and a sequencing primer binding region; and (d) a second primer pair comprising a second F primer and a second R primer, wherein each of the second F primer and the second R primer comprises an amplification region binding region and a sequencing primer binding region, and wherein none of the primers of the second primer pair bind to the Library Tag.

In some embodiments, a kit of the disclosure comprises (a) one or more capture probe modules, wherein each capture probe module comprises a tail sequence and a capture probe sequence capable of hybridizing to a target sequence in a test sample, wherein the tail sequence of each capture probe module comprises a Library Tag; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a Library Tag binding region and a sequencing primer binding region; and (d) a second primer pair comprising a second F primer and a second R primer, wherein each of the second F primer and the second R primer comprises an amplification region binding region and a sequencing primer binding region, and wherein none of the primers of the second primer pair bind to the Library Tag; wherein the one or more capture probe modules have passed a probe QC process performed according to Example 18.

In some embodiments, a kit of the disclosure comprises (a) one or more capture probe modules, wherein each capture probe module comprises a tail sequence and a capture probe sequence capable of hybridizing to a target sequence in a test sample, wherein the tail sequence of each capture probe module comprises a Library Tag; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a Library Tag binding region and a sequencing primer binding region; and (d) a second primer pair comprising a second F primer and a second R primer, wherein each of the second F primer and the second R primer comprises an amplification region binding region and a sequencing primer binding region, and wherein none of the primers of the second primer pair bind to the Library Tag; wherein the set of adaptors has passed an adaptor QC process performed according to Example 16.

In some embodiments, a kit of the disclosure, the kit comprises (a) one or more capture probe modules, wherein each capture probe module comprises a tail sequence and a capture probe sequence capable of hybridizing to a target sequence in a test sample, wherein the tail sequence of each capture probe module comprises a Library Tag; (b) a set of adaptors, wherein each adaptor comprises an adaptor module comprising an amplification region; (c) a first primer pair comprising a first F primer and a first R primer, wherein the first F primer comprises an amplification region binding region and a sequencing primer binding region, wherein the first R primer comprises a Library Tag binding region and a sequencing primer binding region; and (d) a second primer pair comprising a second F primer and a second R primer, wherein each of the second F primer and the second R primer comprises an amplification region binding region and a sequencing primer binding region, and wherein none of the primers of the second primer pair bind to the Library Tag; wherein the one or more capture probe modules have passed a probe QC process performed according to Example 18 and the set of adaptors has passed an adaptor QC process performed according to Example 16.

In some embodiments, a kit of the disclosure comprises (a) a set of adaptors, wherein each adaptor comprises an adaptor module comprising an amplification region; (b) one or more capture probe modules, wherein each capture probe module comprises a tail sequence and a capture probe sequence capable of hybridizing to a target sequence in a test sample, wherein the tail sequence of each capture probe module comprises a Library Tag; (c) a first primer pair comprising a first F primer and a first R primer, wherein the first F primer comprises an amplification region binding region and a sequencing primer binding region; wherein the first R primer comprises a Library Tag binding region and a sequencing primer binding region; and (d) a second primer pair comprising a second F primer and a second R primer, wherein each of the second F primer and the second R primer comprises an amplification region binding region and a sequencing primer binding region, wherein none of the primers of the second primer pair bind to the Library Tag.

**Table 16: Cell Line Variants**

| **sample** | **coord** | **TP/FN** | **score** | **af** | **depth** | **DP4** | **type** |
|---|---|---|---|---|---|---|---|
| B413010-A | chr13:32912299:T:C | TP | 186.4 | 0.0602 | 1097 | 319;712;28;38 | HET |
| B413010-A | chr16:23646295:T:C | TP | 197.9 | 0.0511 | 1448 | 842;532;48;26 | HET |
| B413010-A | chr16:23646857:A:G | TP | 194.1 | 0.0536 | 1305 | 769;466;47;23 | HET |
| B413010-A | chr17:7578210:T:C | TP | 200.4 | 0.0442 | 1695 | 943;677;34;41 | HET |
| B413010-A | chr17:41245471:C:T | TP | 185.7 | 0.045 | 1511 | 808;635;35;33 | HET |
| B413010-A | chr17:7417663:C:T | TP | 558.3 | 0.1257 | 1400 | 642;581;79;97 | HOM |
| B413010-A | chr17:7348625:A:G | TP | 530.9 | 0.1183 | 1471 | 652;645;92;82 | HOM |
| B413010-A | chr17:7342148:T:A | TP | 527 | 0.1155 | 1472 | 703;599;102;68 | HOM |
| B413010-A | chr17:60137085:T:C | TP | 514.8 | 0.1282 | 1271 | 519;589;84;79 | HOM |
| B413010-A | chr13:32504939:A:C | TP | 484.2 | 0.1246 | 1228 | 572;502;80;73 | HOM |
| B413010-A | chr17:60188441:G:A | TP | 457.3 | 0.1184 | 1233 | 535;552;80;66 | HOM |
| B413010-A | chr16:89328477:A:G | TP | 452.5 | 0.1106 | 1320 | 564;610;68;78 | HOM |
| B413010-A | chr6:114084492:A:G | TP | 442.7 | 0.1134 | 1252 | 552;558;72;70 | HOM |
| B413010-A | chr17:60376908:G:A | TP | 415.8 | 0.1117 | 1200 | 573;492;80;54 | HOM |
| B413010-A | chr17:7411447:C:T | TP | 408.4 | 0.0999 | 1331 | 645;553;68;65 | HOM |
| B413010-A | chr16:23143876:A:G | TP | 390.1 | 0.1047 | 1222 | 555;538;57;71 | HOM |
| B413010-A | chr17:7342682:C:T | TP | 376.8 | 0.1002 | 1227 | 626;477;75;48 | HOM |
| B413010-A | chr17:7537792:T:C | TP | 366.1 | 0.0913 | 1336 | 642;572;63;59 | HOM |
| B413010-A | chr6:114554866:A:G | TP | 358.2 | 0.11 | 1055 | 510;429;60;56 | HOM |
| B413010-A | chr17:7402600:T:C | TP | 355 | 0.0904 | 1316 | 581;616;61;58 | HOM |
| B413010-A | chr16:89081075:A:G | TP | 264.7 | 0.0617 | 1508 | 708;707;51;42 | HET |
| B413010-A | chr6:114409122:C:T | TP | 264.6 | 0.0728 | 1236 | 536;610;41;49 | HET |
| B413010-A | chr22:29540920:C:T | TP | 259.6 | 0.0719 | 1251 | 654;507;49;41 | HET |
| B413010-A | chr6:113975118:C:T | TP | 259.4 | 0.0694 | 1283 | 592;602;44;45 | HET |
| B413010-A | chr13:32811607:G:A | TP | 257.5 | 0.0655 | 1359 | 624;646;48;41 | HET |
| B413010-A | chr11:107542394:C:T | TP | 255.8 | 0.073 | 1191 | 563;541;43;44 | HEM |
| B413010-A | chr16:23724753:G:A | TP | 254.8 | 0.0644 | 1381 | 690;600;47;42 | HET |
| B413010-A | chr11:107707070:G:T | TP | 250.7 | 0.0685 | 1271 | 540;640;36;51 | HEM |
| B413010-A | chr11:107655949:A:G | TP | 249.7 | 0.0725 | 1213 | 632;492;44;44 | HEM |
| B413010-A | chr16:23080634:C:A | TP | 249.4 | 0.0636 | 1369 | 617;665;39;48 | HET |
| B413010-A | chr13:33526366:A:C | TP | 246 | 0.0593 | 1484 | 728;668;41;47 | HET |
| B413010-A | chr17:41735120:T:C | TP | 245.6 | 0.0652 | 1320 | 537;695;36;50 | HET |
| B413010-A | chr16:90028106:T:G | TP | 243.7 | 0.0716 | 1187 | 512;590;37;48 | HET |
| B413010-A | chr17:41726325:A:G | TP | 243.5 | 0.0733 | 1146 | 555;507;43;41 | HET |
| B413010-A | chr17:7400815:T:C | TP | 241 | 0.0594 | 1447 | 676;685;44;42 | HET |
| B413010-A | chr17:7461343:G:A | TP | 231.6 | 0.0551 | 1505 | 624;797;35;48 | HET |
| B413010-A | chr11:108905350:C:T | TP | 228.1 | 0.0646 | 1223 | 534;610;39;40 | HEM |
| B413010-A | chr6:114228368:C:T | TP | 226.1 | 0.0644 | 1226 | 568;579;41;38 | HET |
| B413010-A | chr11:107729106:C:T | TP | 225.7 | 0.0625 | 1264 | 568;616;45;34 | HEM |
| B413010-A | chr17:40849842:A:G | TP | 224.7 | 0.0607 | 1335 | 622;631;38;43 | HET |
| B413010-A | chr11:107474795:A:G | TP | 223.4 | 0.0625 | 1263 | 530;654;31;48 | HEM |
| B413010-A | chr17:7514544:C:T | TP | 222.2 | 0.0553 | 1446 | 621;745;41;39 | HET |
| B413010-A | chr6:114168573:G:T | TP | 222.2 | 0.0577 | 1368 | 650;639;43;36 | HET |
| B413010-A | chr16:90024206:A:G | TP | 221.5 | 0.0606 | 1288 | 557;653;32;46 | HET |
| B413010-A | chr17:41710104:C:T | TP | 218.9 | 0.0554 | 1407 | 624;704;30;48 | HET |
| B413010-A | chr6:114361180:G:A | TP | 217.8 | 0.0564 | 1365 | 625;661;36;41 | HET |
| B413010-A | chr17:60789405:T:C | TP | 216.7 | 0.0564 | 1383 | 617;686;35;43 | HET |
| B413010-A | chr13:32833878:A:G | TP | 216.6 | 0.0586 | 1315 | 618;620;36;41 | HET |
| B413010-A | chr16:23195646:T:C | TP | 210.8 | 0.0583 | 1287 | 542;670;32;43 | HET |
| B413010-A | chr17:60769803:A:G | TP | 208.9 | 0.0597 | 1307 | 627;602;37;41 | HET |
| B413010-A | chr16:89038880:G:T | TP | 208.3 | 0.0531 | 1412 | 684;653;50;25 | HET |
| B413010-A | chr13:32826186:G:A | TP | 207.8 | 0.0618 | 1181 | 572;536;38;35 | HET |
| B413010-A | chr17:60795074:T:G | TP | 207.6 | 0.0502 | 1493 | 647;771;37;38 | HET |
| B413010-A | chr11:108858032:A:C | TP | 207.1 | 0.0709 | 1002 | 484;447;36;35 | HEM |
| B413010-A | chr16:23074431:T:C | TP | 206.7 | 0.0614 | 1188 | 574;541;32;41 | HET |
| B413010-A | chr17:60470547:C:T | TP | 206.4 | 0.0601 | 1214 | 563;577;33;40 | HET |
| B413010-A | chr17:41131645:C:A | TP | 204.5 | 0.0536 | 1361 | 639;649;32;41 | HET |
| B413010-A | chr6:114361189:C:T | TP | 204.3 | 0.054 | 1353 | 629;650;35;38 | HET |
| B413010-A | chr11:107673628:T:C | TP | 201.8 | 0.052 | 1403 | 598;732;27;46 | HEM |
| B413010-A | chr17:41692606:G:T | TP | 201.4 | 0.0575 | 1234 | 577;586;38;33 | HET |
| B413010-A | chr17:7453505:C:A | TP | 201.2 | 0.0495 | 1475 | 727;675;42;31 | HET |
| B413010-A | chr17:40897470:T:C | TP | 199.6 | 0.0556 | 1277 | 594;612;31;40 | HET |
| B413010-A | chr17:7458795:A:G | TP | 197.9 | 0.052 | 1385 | 595;718;35;37 | HET |
| B413010-A | chr13:32965310:G:A | TP | 196.8 | 0.0684 | 994 | 537;389;41;27 | HET |
| B413010-A | chr11:107496966:A:G | TP | 196.1 | 0.059 | 1170 | 619;482;45;24 | HEM |
| B413010-A | chr13:32979138:T:C | TP | 195.4 | 0.0649 | 1079 | 486;522;38;32 | HET |
| B413010-A | chr6:114028139:C:T | TP | 194.7 | 0.0618 | 1100 | 496;535;35;33 | HET |
| B413010-A | chr17:40507980:A:C | TP | 194.2 | 0.0583 | 1184 | 521;593;32;37 | HET |
| B413010-A | chr13:32547131:T:C | TP | 194 | 0.058 | 1190 | 511;610;34;35 | HET |
| B413010-A | chr11:107663103:C:G | TP | 193.1 | 0.0562 | 1228 | 496;662;28;41 | HEM |
| B413010-A | chr6:114568687:C:T | TP | 192.1 | 0.0558 | 1218 | 591;559;25;43 | HET |
| B413010-A | chr6:113886419:T:A | TP | 191.9 | 0.0531 | 1299 | 653;577;35;34 | HET |
| B413010-A | chr16:23077748:A:G | TP | 191.2 | 0.0539 | 1279 | 686;524;34;35 | HET |
| B413010-A | chr11:108464209:T:C | TP | 190.1 | 0.053 | 1303 | 620;614;35;34 | HEM |
| B413010-A | chr16:90114833:T:C | TP | 189.4 | 0.0554 | 1227 | 515;644;36;32 | HET |
| B413010-A | chr6:114226445:C:T | TP | 189.3 | 0.0568 | 1180 | 552;561;35;32 | HET |
| B413010-A | chr11:108256859:C:T | TP | 189 | 0.0569 | 1177 | 603;507;30;37 | HEM |
| B413010-A | chr17:40658533:C:T | TP | 188.6 | 0.0497 | 1367 | 697;602;38;30 | HET |
| B413010-A | chr16:24030162:A:C | TP | 186.9 | 0.0529 | 1266 | 586;613;33;34 | HET |
| B413010-A | chr16:90110950:C:T | TP | 184.9 | 0.0523 | 1282 | 611;603;33;34 | HET |
| B413010-A | chr11:108384207:C:T | TP | 184.7 | 0.0549 | 1203 | 580;556;30;36 | HEM |
| B413010-A | chr6:114242899:T:C | TP | 184.2 | 0.0539 | 1225 | 581;578;35;31 | HET |
| B413010-A | chr6:114410689:A:G | TP | 183.5 | 0.0553 | 1193 | 557;570;29;37 | HET |
| B413010-A | chr17:41173086:G:A | TP | 183.4 | 0.0609 | 1084 | 413;605;29;37 | HET |
| B413010-A | chr6:114077332:T:C | TP | 183.2 | 0.0649 | 1033 | 510;456;34;33 | HET |
| B413010-A | chr16:90027838:G:A | TP | 182.7 | 0.049 | 1346 | 611;667;31;35 | HET |
| B413010-A | chr16:23754561:A:G | TP | 181.1 | 0.0518 | 1274 | 612;595;33;33 | HET |
| B413010-A | chr11:107706832:C:T | TP | 179.9 | 0.0542 | 1181 | 534;580;33;31 | HEM |
| B413010-A | chr22:29710360:T:C | TP | 178.8 | 0.0498 | 1304 | 619;620;28;37 | HET |
| B413010-A | chr13:32683151:A:G | TP | 175.4 | 0.0548 | 1150 | 598;489;30;33 | HET |
| B413010-A | chr13:32821976:T:C | TP | 175 | 0.0533 | 1183 | 604;516;37;26 | HET |
| B413010-A | chr13:32707663:A:G | TP | 174.8 | 0.0561 | 1123 | 559;501;39;24 | HET |
| B413010-A | chr17:60755746:T:G | TP | 174.2 | 0.0517 | 1218 | 521;633;28;35 | HET |
| B413010-A | chr11:107481518:C:T | TP | 174.1 | 0.057 | 1087 | 542;483;33;29 | HEM |
| B413010-A | chr13:32565539:T:C | TP | 171.7 | 0.0518 | 1216 | 592;561;30;33 | HET |
| B413010-A | chr6:114348520:T:C | TP | 171.6 | 0.0545 | 1138 | 574;502;29;33 | HET |
| B413010-A | chr13:32986219:A:G | TP | 170.2 | 0.05 | 1239 | 573;604;33;29 | HET |
| B413010-A | chr11:108425790:T:C | TP | 169.2 | 0.0538 | 1134 | 573;500;39;22 | HEM |
| B413010-A | chr6:114233646:G:A | TP | 168 | 0.0544 | 1103 | 490;553;30;30 | HET |
| B413010-A | chr22:29630337:G:A | TP | 167.5 | 0.0503 | 1213 | 558;593;29;32 | HET |
| B413010-A | chr11:107625409:T:C | TP | 167.3 | 0.0652 | 889 | 360;471;29;29 | HEM |
| B413010-A | chr22:29361159:A:G | TP | 165.5 | 0.0487 | 1253 | 559;633;26;35 | HET |
| B413010-A | chr13:32983250:G:A | TP | 165.3 | 0.0476 | 1260 | 596;604;26;34 | HET |
| B413010-A | chr13:32864488:G:C | TP | 164.7 | 0.0502 | 1214 | 604;549;28;33 | HET |
| B413010-A | chr11:108437928:T:G | TP | 163.7 | 0.0539 | 1095 | 496;540;26;33 | HEM |
| B413010-A | chr17:41145527:C:T | TP | 163.3 | 0.0516 | 1143 | 573;511;32;27 | HET |
| B413010-A | chr13:32653377:A:G | TP | 163.3 | 0.051 | 1196 | 583;552;29;32 | HET |
| B413010-A | chr13:32857769:A:C | TP | 163.3 | 0.0654 | 872 | 433;382;20;37 | HET |
| B413010-A | chr16:24029774:C:A | TP | 161.9 | 0.0517 | 1121 | 571;492;28;30 | HET |
| B413010-A | chr11:107478586:G:C | TP | 161.8 | 0.0541 | 1091 | 518;514;31;28 | HEM |
| B413010-A | chr11:107623767:G:T | TP | 158.4 | 0.0568 | 1003 | 445;501;29;28 | HEM |
| B413010-A | chr11:107858027:G:C | TP | 157 | 0.0542 | 1070 | 517;492;31;27 | HEM |
| B413010-A | chr11:108038477:A:G | TP | 156.7 | 0.0537 | 1061 | 513;491;29;28 | HEM |
| B413010-A | chr11:107956771:T:C | TP | 156.5 | 0.0557 | 1006 | 461;488;29;27 | HEM |
| B413010-A | chr6:114691511:G:A | TP | 156.1 | 0.0482 | 1182 | 561;564;24;33 | HET |
| B413010-A | chr22:29640019:C:T | TP | 155.1 | 0.0451 | 1286 | 589;639;26;32 | HET |
| B413010-A | chr16:23970677:T:C | TP | 154.5 | 0.0483 | 1180 | 542;581;30;27 | HET |
| B413010-A | chr13:32707287:A:G | TP | 153.3 | 0.0507 | 1105 | 497;552;26;30 | HET |
| B413010-A | chr16:23076410:C:T | TP | 153.2 | 0.0462 | 1211 | 519;634;24;32 | HET |
| B413010-A | chr17:7448288:C:G | TP | 152.5 | 0.0473 | 1227 | 556;613;32;26 | HET |
| B413010-A | chr17:41056245:A:G | TP | 151.9 | 0.0453 | 1235 | 663;516;30;26 | HET |
| B413010-A | chr6:114625448:C:A | TP | 150.2 | 0.0522 | 1034 | 481;498;28;26 | HET |
| B413010-A | chr13:32545856:G:A | TP | 149.7 | 0.0511 | 1056 | 465;535;19;35 | HET |
| B413010-A | chr16:23098422:A:G | TP | 148.5 | 0.054 | 982 | 597;332;38;15 | HET |
| B413010-A | chr16:89471246:A:C | TP | 148.4 | 0.0438 | 1257 | 599;603;30;25 | HET |
| B413010-A | chr6:113989441:G:A | TP | 147.5 | 0.0516 | 1028 | 550;424;33;20 | HET |
| B413010-A | chr11:108383676:A:G | TP | 146 | 0.0479 | 1128 | 491;583;31;23 | HEM |
| B413010-A | chr16:90032255:C:G | TP | 145.6 | 0.0444 | 1215 | 560;601 ;15;39 | HET |
| B413010-A | chr16:23737679:A:G | TP | 143.5 | 0.0456 | 1163 | 554;554;27;26 | HET |
| B413010-A | chr11:108887701:G:A | TP | 142.5 | 0.0469 | 1108 | 507;549;25;27 | HEM |
| B413010-A | chr11:108509754:A:G | TP | 140.2 | 0.0461 | 1128 | 505;571;27;25 | HEM |
| B413010-A | chr6:114229352:A:G | TP | 139.4 | 0.0389 | 1362 | 645;664;22;31 | HET |
| B413010-A | chr17:60264240:G:T | TP | 139 | 0.0428 | 1214 | 565;596;25;27 | HET |
| B413010-A | chr6:114316305:T:C | TP | 137.1 | 0.0465 | 1096 | 613;432;37;14 | HET |
| B413010-A | chr22:29495373:C:T | TP | 137.1 | 0.0443 | 1150 | 581;517;25;26 | HET |
| B413010-A | chr6:114437276:C:T | TP | 135.4 | 0.045 | 1110 | 575;482;30;20 | HET |
| B413010-A | chr11:107485515:G:A | TP | 135.2 | 0.0447 | 1119 | 545;521;24;26 | HEM |
| B413010-A | chr17:40858841:T:C | TP | 132.1 | 0.0422 | 1160 | 638;473;31;18 | HET |
| B413010-A | chr17:41738823:G:A | TP | 131.1 | 0.0507 | 946 | 571;327;30;18 | HET |
| B413010-A | chr16:23718623:G:A | TP | 125.7 | 0.0354 | 1355 | 645;662;26;22 | HET |
| B413010-A | chr17:60264211:C:T | TP | 125.2 | 0.0393 | 1197 | 514;636;21;26 | HET |
| B413010-A | chr6:114398669:A:G | TP | 118.5 | 0.0421 | 1045 | 495;506;17;27 | HET |
| B413010-A | chr11:108849093:C:T | TP | 116 | 0.0417 | 1032 | 492;497;17;26 | HEM |

**Table 17: List of expected SNPs**

| **rs** | **alt** | **cds_effect** | **chr** | **gene** | **pos** | **ref** |
|---|---|---|---|---|---|---|
| rs10083789 | A | NA | chr16 | PALB2 | 23080634 | C |
| rs1024375 | A | NA | chr13 | BRCA2 | 32710920 | G |
| rs1043284 | T | NA | chr17 | BRCA1 | 41145527 | C |
| rs1048149 | T | NA | chr16 | FANCA | 90110950 | C |
| rs10509544 | G | NA | chr10 | PTEN | 90168074 | T |
| rs10852891 | A | NA | chr17 | TP53 | 7490810 | G |
| rs10890742 | G | NA | chr11 | ATM | 107474795 | A |
| rs11078685 | T | NA | chr17 | TP53 | 7411447 | C |
| rs11078708 | C | NA | chr17 | TP53 | 7720091 | G |
| rs11078716 | T | NA | chr17 | TP53 | 7820183 | G |
| rs11079387 | G | NA | chr17 | BRCA1 | 41510850 | A |
| rs11202435 | G | NA | chr10 | PTEN | 89295958 | T |
| rs11212320 | T | NA | chr11 | ATM | 107542394 | C |
| rs112632517 | A | NA | chr17 | BRCA1 | 41394329 | G |
| rs11616892 | A | NA | chr13 | BRCA2 | 32545856 | G |
| rs11862875 | T | NA | chr16 | FANCA | 89145541 | A |
| rs11865549 | A | NA | chr16 | FANCA | 89099226 | G |
| rs11865731 | C | NA | chr16 | PALB2 | 24030162 | A |
| rs11868590 | C | NA | chr17 | BRCA1 | 40672922 | T |
| rs12100085 | T | NA | chr13 | BRCA2 | 33471737 | C |
| rs12250975 | G | NA | chr10 | PTEN | 89282145 | A |
| rs12290206 | G | NA | chr11 | ATM | 107761113 | A |
| rs12293030 | A | NA | chr11 | ATM | 107700502 | T |
| rs12416046 | G | NA | chr10 | PTEN | 89861218 | A |
| rs12576509 | T | NA | chr11 | ATM | 107494405 | G |
| rs12864812 | G | NA | chr13 | BRCA2 | 32653377 | A |
| rs12927285 | A | NA | chr16 | PALB2 | 23589483 | G |
| rs12948043 | C | NA | chr17 | BRCA1 | 41615830 | T |
| rs13056977 | C | NA | chr22 | CHEK2 | 29710360 | T |
| rs1320304 | C | NA | chr17 | BRCA1 | 41673990 | T |
| rs146186955 | C | NA | chr13 | RB1 | 48622938 | T |
| rs1529370 | T | NA | chr13 | RB1 | 49478028 | G |
| rs1544724 | G | NA | chr17 | TP53 | 7621777 | T |
| rs1553468 | G | NA | chr17 | BRCA1 | 40849842 | A |
| rs161626 | G | NA | chr17 | BRCA1 | 41059795 | A |
| rs16965228 | G | NA | chr16 | FANCA | 89059879 | A |
| rs16972944 | T | NA | chr16 | PALB2 | 23899609 | C |
| rs17077321 | C | NA | chr13 | BRCA2 | 32814974 | T |
| rs17882163 | T | NA | chr17 | BRCA1 | 40481170 | A |
| rs187406 | G | NA | chr16 | PALB2 | 23737679 | A |
| rs1963626 | G | NA | chr11 | ATM | 107693120 | A |
| rs1984518 | G | NA | chr17 | BRCA1 | 41521666 | T |
| rs2038158 | C | NA | chr22 | CHEK2 | 28368473 | T |
| rs205010 | A | NA | chr13 | BRCA2 | 32735718 | G |
| rs206319 | G | NA | chr13 | BRCA2 | 32986219 | A |
| rs206340 | A | NA | chr13 | BRCA2 | 32965310 | G |
| rs2074439 | T | NA | chr17 | BRCA1 | 41692606 | G |
| rs2228129 | C | NA | chr17 | TP53 | 7402600 | T |
| rs2239815 | C | NA | chr22 | CHEK2 | 29192670 | T |
| rs2269738 | G | NA | chr16 | PALB2 | 23143876 | A |
| rs2287499 | G | NA | chr17 | TP53 | 7592168 | C |
| rs2302764 | C | NA | chr17 | TP53 | 7360110 | T |
| rs2303153 | C | NA | chr16 | PALB2 | 23390201 | G |
| rs2593595 | G | NA | chr17 | BRCA1 | 41056245 | A |
| rs2846412 | T | NA | chr11 | ATM | 108384207 | C |
| rs2846418 | C | NA | chr11 | ATM | 108476461 | T |
| rs28758793 | T | NA | chr11 | ATM | 107707070 | G |
| rs2887481 | G | NA | chr16 | PALB2 | 23307915 | A |
| rs3132818 | C | NA | chr11 | ATM | 107478586 | G |
| rs3178915 | G | NA | chr22 | CHEK2 | 29453027 | A |
| rs323495 | A | NA | chr17 | BRCA1 | 41074487 | G |
| rs34241435 | A | NA | chr16 | PALB2 | 23313185 | G |
| rs34539888 | T | NA | chr13 | BRCA2 | 33016603 | G |
| rs35537731 | G | NA | chr13 | RB1 | 48746364 | T |
| rs357680 | C | NA | chr13 | RB1 | 49493436 | T |
| rs378609 | C | NA | chr13 | BRCA2 | 33553549 | A |
| rs3809682 | A | NA | chr16 | PALB2 | 23724753 | G |
| rs383 | C | NA | chr13 | BRCA2 | 32504939 | A |
| rs3904629 | T | NA | chr13 | BRCA2 | 33530851 | C |
| rs4151120 | A | NA | chr17 | TP53 | 7342148 | T |
| rs4383207 | G | NA | chr17 | TP53 | 7936282 | A |
| rs472817 | A | NA | chr13 | BRCA2 | 32983250 | G |
| rs4785648 | G | NA | chr16 | FANCA | 89328477 | A |
| rs4788423 | G | NA | chr16 | PALB2 | 24018863 | A |
| rs4793011 | G | NA | chr17 | BRCA1 | 41726325 | A |
| rs4823006 | G | NA | chr22 | CHEK2 | 29451671 | A |
| rs4934378 | C | NA | chr10 | PTEN | 89867693 | T |
| rs493770 | A | NA | chr11 | ATM | 107529940 | G |
| rs524611 | T | NA | chr11 | ATM | 107531320 | C |
| rs541504918 | G | NA | chr16 | PALB2 | 23582278 | A |
| rs55901083 | G | NA | chr13 | RB1 | 48856336 | T |
| rs566238 | T | NA | chr11 | ATM | 107706832 | C |
| rs56872996 | G | NA | chr17 | TP53 | 7928924 | A |
| rs5723 | G | NA | chr16 | PALB2 | 23226787 | C |
| rs5728 | G | NA | chr16 | PALB2 | 23227362 | A |
| rs5752793 | G | NA | chr22 | CHEK2 | 29160314 | A |
| rs5752867 | A | NA | chr22 | CHEK2 | 29513729 | G |
| rs5762430 | G | NA | chr22 | CHEK2 | 28378472 | A |
| rs5762788 | A | NA | chr22 | CHEK2 | 29174770 | C |
| rs5762795 | A | NA | chr22 | CHEK2 | 29182500 | C |
| rs57752647 | G | NA | chr17 | TP53 | 7381256 | T |
| rs58258018 | C | NA | chr11 | ATM | 107679779 | G |
| rs58465745 | G | NA | chr16 | PALB2 | 23389816 | A |
| rs59001570 | C | NA | chr16 | FANCA | 89997947 | G |
| rs593925 | T | NA | chr11 | ATM | 107492199 | G |
| rs59602687 | T | NA | chr13 | RB1 | 49377156 | A |
| rs59806584 | G | NA | chr16 | FANCA | 89660509 | A |
| rs60034915 | T | NA | chr10 | PTEN | 89872699 | C |
| rs6005863 | G | NA | chr22 | CHEK2 | 29156448 | A |
| rs6005907 | A | NA | chr22 | CHEK2 | 29227971 | G |
| rs6005915 | C | NA | chr22 | CHEK2 | 29233262 | T |
| rs60646729 | G | NA | chr16 | FANCA | 89102240 | A |
| rs607506 | G | NA | chr11 | ATM | 107655949 | A |
| rs62031942 | C | NA | chr16 | PALB2 | 23254025 | T |
| rs62059833 | T | NA | chr17 | TP53 | 7514544 | C |
| rs647756 | A | NA | chr11 | ATM | 107535758 | G |
| rs6761 | T | NA | chr17 | TP53 | 7417663 | C |
| rs693963 | C | NA | chr13 | BRCA2 | 32979138 | T |
| rs701893 | A | NA | chr10 | PTEN | 90144950 | G |
| rs7069968 | T | NA | chr10 | PTEN | 90043824 | C |
| rs7070980 | T | NA | chr10 | PTEN | 89855608 | C |
| rs7077182 | G | NA | chr10 | PTEN | 90216244 | C |
| rs7092284 | A | NA | chr10 | PTEN | 89451838 | G |
| rs7092392 | A | NA | chr10 | PTEN | 89451853 | G |
| rs7097903 | C | NA | chr10 | PTEN | 90049968 | G |
| rs7103534 | C | NA | chr11 | ATM | 107911652 | T |
| rs7116354 | G | NA | chr11 | ATM | 107622778 | A |
| rs7192878 | A | NA | chr16 | FANCA | 89342733 | G |
| rs7198060 | C | NA | chr16 | FANCA | 89115822 | T |
| rs7200084 | C | NA | chr16 | FANCA | 89089220 | T |
| rs7201172 | A | NA | chr16 | FANCA | 89293525 | G |
| rs7210527 | T | NA | chr17 | BRCA1 | 41409959 | C |
| rs7213700 | G | NA | chr17 | BRCA1 | 41056260 | T |
| rs7220372 | G | NA | chr17 | BRCA1 | 40656043 | T |
| rs72815600 | C | NA | chr16 | FANCA | 89136182 | T |
| rs7287918 | A | NA | chr22 | CHEK2 | 29281710 | G |
| rs732098 | T | NA | chr11 | ATM | 107764164 | G |
| rs7324259 | T | NA | chr13 | BRCA2 | 33509030 | G |
| rs73245806 | A | NA | chr17 | TP53 | 7947908 | T |
| rs73256250 | C | NA | chr16 | FANCA | 89038114 | G |
| rs73314948 | C | NA | chr17 | BRCA1 | 41471691 | T |
| rs7335609 | G | NA | chr13 | BRCA2 | 33055431 | T |
| rs7336874 | C | NA | chr13 | BRCA2 | 33396698 | T |
| rs73480207 | C | NA | chr13 | RB1 | 49337306 | T |
| rs73540963 | T | NA | chr16 | PALB2 | 24009879 | C |
| rs73548783 | G | NA | chr11 | ATM | 108414373 | A |
| rs73550205 | C | NA | chr16 | PALB2 | 23714311 | T |
| rs7359598 | C | NA | chr17 | BRCA1 | 40897470 | T |
| rs737173 | T | NA | chr11 | ATM | 107824091 | A |
| rs738453 | T | NA | chr22 | CHEK2 | 28301743 | C |
| rs738748 | C | NA | chr22 | CHEK2 | 28993183 | T |
| rs739769 | C | NA | chr17 | BRCA1 | 41735120 | T |
| rs74033443 | G | NA | chr16 | FANCA | 89294044 | A |
| rs743920 | G | NA | chr22 | CHEK2 | 29621128 | C |
| rs76092147 | G | NA | chr16 | PALB2 | 23865984 | T |
| rs767664 | G | NA | chr11 | ATM | 108437928 | T |
| rs77687672 | A | NA | chr11 | ATM | 107432154 | G |
| rs780169 | G | NA | chr16 | PALB2 | 23754561 | A |
| rs78790828 | A | NA | chr17 | TP53 | 7830280 | G |
| rs7895494 | C | NA | chr10 | PTEN | 89379732 | A |
| rs7904229 | A | NA | chr10 | PTEN | 90221561 | C |
| rs7947466 | A | NA | chr11 | ATM | 107771148 | G |
| rs7993336 | C | NA | chr13 | RB1 | 48433801 | T |
| rs8000270 | C | NA | chr13 | RB1 | 49412235 | T |
| rs8001409 | C | NA | chr13 | BRCA2 | 33510213 | T |
| rs8057712 | G | NA | chr16 | PALB2 | 23403744 | A |
| rs8068764 | C | NA | chr17 | BRCA1 | 41490161 | T |
| rs8136754 | C | NA | chr22 | CHEK2 | 28197815 | T |
| rs868045 | G | NA | chr16 | FANCA | 90102835 | A |
| rs9526401 | T | NA | chr13 | RB1 | 48420828 | A |
| rs9535120 | A | NA | chr13 | RB1 | 49539246 | G |
| rs9568668 | G | NA | chr13 | BRCA2 | 33522655 | A |
| rs9590896 | A | NA | chr13 | BRCA2 | 32877696 | G |
| rs9594995 | G | NA | chr13 | BRCA2 | 32707663 | A |
| rs9595159 | A | NA | chr13 | BRCA2 | 32798953 | G |
| rs9595469 | A | NA | chr13 | BRCA2 | 32984336 | T |
| rs9595932 | C | NA | chr13 | RB1 | 49158816 | T |
| rs9595958 | T | NA | chr13 | RB1 | 49242249 | C |
| rs9596011 | G | NA | chr13 | RB1 | 49368949 | A |
| rs9596042 | A | NA | chr13 | RB1 | 49472485 | G |
| rs9596539 | C | NA | chr13 | BRCA2 | 33498938 | A |
| rs9620804 | A | NA | chr22 | CHEK2 | 28919200 | G |
| rs9625679 | G | NA | chr22 | CHEK2 | 29456699 | A |
| rs9806913 | G | NA | chr16 | FANCA | 89953773 | A |
| rs9890403 | G | NA | chr17 | BRCA1 | 41458614 | A |
| rs9891119 | C | NA | chr17 | BRCA1 | 40507980 | A |
| rs9901673 | A | NA | chr17 | TP53 | 7484101 | C |
| rs990324 | G | NA | chr13 | BRCA2 | 33387246 | A |
| rs9903378 | C | NA | chr17 | TP53 | 7588006 | A |
| rs9912587 | A | NA | chr17 | BRCA1 | 41173086 | G |
| rs9915640 | C | NA | chr17 | BRCA1 | 41603882 | T |
| rs9921637 | A | NA | chr16 | FANCA | 89146714 | G |
| rs9922448 | T | NA | chr16 | PALB2 | 23236090 | C |

**Table 18: List of unexpected SNPs (indicating contamination if ≥ 1%)**

| **rs** | **alt** | **cds_effect** | **chr** | **gene** | **pos** | **ref** |
|---|---|---|---|---|---|---|
| rs10153241 | C | NA | chr17 | BRCA1 | 40493337 | T |
| rs10431058 | T | NA | chr11 | ATM | 107481518 | C |
| rs10492397 | A | NA | chr13 | BRCA2 | 32976358 | G |
| rs10492790 | T | NA | chr16 | PALB2 | 23076410 | C |
| rs10502107 | A | NA | chr11 | ATM | 108893765 | C |
| rs10509408 | A | NA | chr10 | PTEN | 89310281 | G |
| rs10509539 | T | NA | chr10 | PTEN | 89998283 | G |
| rs1053005 | C | NA | chr17 | BRCA1 | 40465910 | T |
| rs10749570 | G | NA | chr10 | PTEN | 90038033 | T |
| rs10887818 | T | NA | chr10 | PTEN | 90183102 | C |
| rs11078697 | T | NA | chr17 | TP53 | 7469229 | C |
| rs11078710 | A | NA | chr17 | TP53 | 7777041 | C |
| rs11078711 | T | NA | chr17 | TP53 | 7777214 | G |
| rs111785947 | T | NA | chr13 | RB1 | 49210785 | A |
| rs111789652 | CA | NA | chr13 | RB1 | 48800978 | - |
| rs111916330 | C | NA | chr17 | TP53 | 7721917 | T |
| rs11202441 | G | NA | chr10 | PTEN | 89301014 | A |
| rs11202528 | T | NA | chr10 | PTEN | 89483062 | C |
| rs11202691 | T | NA | chr10 | PTEN | 90005048 | C |
| rs11202754 | T | NA | chr10 | PTEN | 90226590 | A |
| rs11212509 | C | NA | chr11 | ATM | 107956771 | T |
| rs11212704 | C | NA | chr11 | ATM | 108425790 | T |
| rs11212837 | G | NA | chr11 | ATM | 108864444 | A |
| rs1123547 | C | NA | chr17 | TP53 | 7619861 | T |
| rs112402962 | AC | NA | chr13 | RB1 | 48856333 | - |
| rs112465465 | A | NA | chr10 | PTEN | 90043769 | - |
| rs112467246 | C | NA | chr13 | BRCA2 | 33513982 | T |
| rs11389505 | A | NA | chr13 | RB1 | 48556635 | - |
| rs11406289 | A | NA | chr13 | RB1 | 49412212 | - |
| rs114126369 | G | NA | chr13 | RB1 | 48657979 | A |
| rs114539386 | A | NA | chr10 | PTEN | 89427833 | C |
| rs11619762 | T | NA | chr13 | RB1 | 49499087 | A |
| rs116469311 | A | NA | chr10 | PTEN | 89879673 | G |
| rs11648589 | C | NA | chr16 | PALB2 | 23295974 | T |
| rs11653545 | A | NA | chr17 | TP53 | 7512074 | G |
| rs116600817 | G | NA | chr17 | TP53 | 7458795 | A |
| rs117385313 | C | NA | chr10 | PTEN | 90168094 | G |
| rs117836982 | T | NA | chr10 | PTEN | 90109809 | C |
| rs118044350 | C | NA | chr13 | RB1 | 49478033 | G |
| rs11868946 | G | NA | chr17 | TP53 | 7673605 | A |
| rs12157905 | A | NA | chr22 | CHEK2 | 28192423 | G |
| rs12162045 | A | NA | chr16 | PALB2 | 23331743 | G |
| rs12226290 | G | NA | chr11 | ATM | 107496966 | A |
| rs12274468 | T | NA | chr11 | ATM | 108905350 | C |
| rs12357948 | T | NA | chr10 | PTEN | 90202273 | C |
| rs12444767 | T | NA | chr16 | PALB2 | 23200107 | A |
| rs12446463 | A | NA | chr16 | PALB2 | 23387393 | G |
| rs12449814 | T | NA | chr17 | TP53 | 7911709 | G |
| rs12453250 | A | NA | chr17 | TP53 | 7761512 | C |
| rs12485095 | G | NA | chr22 | CHEK2 | 29540198 | T |
| rs12572813 | T | NA | chr10 | PTEN | 90069353 | C |
| rs12574049 | C | NA | chr11 | ATM | 107858027 | G |
| rs12600401 | G | NA | chr17 | BRCA1 | 41465867 | A |
| rs12628787 | T | NA | chr22 | CHEK2 | 29495373 | C |
| rs12709089 | A | NA | chr16 | FANCA | 89630311 | G |
| rs12793497 | T | NA | chr11 | ATM | 108599993 | C |
| rs12877579 | G | NA | chr13 | BRCA2 | 33321045 | A |
| rs12924133 | A | NA | chr16 | FANCA | 89638290 | C |
| rs12930806 | T | NA | chr16 | FANCA | 89203073 | C |
| rs12935297 | T | NA | chr16 | PALB2 | 24031478 | C |
| rs12941509 | G | NA | chr17 | TP53 | 7448288 | C |
| rs12943692 | A | NA | chr17 | TP53 | 7332930 | G |
| rs12946015 | T | NA | chr17 | BRCA1 | 41478455 | C |
| rs13058031 | A | NA | chr22 | CHEK2 | 29273966 | G |
| rs132280 | T | NA | chr22 | CHEK2 | 29540920 | C |
| rs13332145 | G | NA | chr16 | FANCA | 89081075 | A |
| rs13333659 | T | NA | chr16 | FANCA | 89038880 | G |
| rs13339649 | G | NA | chr16 | PALB2 | 23098422 | A |
| rs134195 | C | NA | chr22 | CHEK2 | 28656691 | T |
| rs1359393 | A | NA | chr13 | RB1 | 48383104 | G |
| rs1359688 | T | NA | chr13 | RB1 | 49146072 | A |
| rs138125275 | A | NA | chr10 | PTEN | 89579689 | G |
| rs141184053 | A | NA | chr10 | PTEN | 89451879 | - |
| rs141670949 | A | NA | chr13 | RB1 | 49350399 | - |
| rs142703354 | G | NA | chr10 | PTEN | 89579697 | T |
| rs144848 | C | NA | chr13 | BRCA2 | 32906729 | A |
| rs1460819 | C | NA | chr13 | BRCA2 | 32857769 | A |
| rs1468087 | G | NA | chr13 | BRCA2 | 32683151 | A |
| rs147173441 | A | NA | chr10 | PTEN | 89295976 | - |
| rs148550648 | C | NA | chr13 | RB1 | 48592100 | T |
| rs1557670 | C | NA | chr16 | PALB2 | 23292173 | A |
| rs16940016 | A | NA | chr16 | PALB2 | 23322445 | G |
| rs16956822 | A | NA | chr17 | TP53 | 7499349 | G |
| rs16968177 | G | NA | chr17 | BRCA1 | 41080815 | A |
| rs16985575 | C | NA | chr22 | CHEK2 | 28217319 | T |
| rs16986280 | C | NA | chr22 | CHEK2 | 28695642 | T |
| rs17077875 | G | NA | chr13 | BRCA2 | 33376118 | A |
| rs17078082 | G | NA | chr13 | BRCA2 | 33485961 | A |
| rs17085 | G | NA | chr13 | RB1 | 48414487 | A |
| rs17098687 | C | NA | chr10 | PTEN | 89579599 | G |
| rs17107183 | A | NA | chr11 | ATM | 107452482 | G |
| rs17199565 | G | NA | chr16 | PALB2 | 23273704 | A |
| rs17256755 | A | NA | chr16 | PALB2 | 23336365 | C |
| rs17431184 | C | NA | chr10 | PTEN | 89720251 | T |
| rs17451544 | G | NA | chr22 | CHEK2 | 29553039 | A |
| rs1753621 | T | NA | chr13 | RB1 | 49499060 | C |
| rs17834230 | G | NA | chr13 | RB1 | 49377189 | A |
| rs17856697 | G | NA | chr17 | TP53 | 7348625 | A |
| rs17881556 | C | NA | chr17 | TP53 | 7570869 | T |
| rs17882626 | G | NA | chr17 | TP53 | 7306727 | A |
| rs183078 | G | NA | chr13 | RB1 | 49513352 | A |
| rs1834456 | C | NA | chr11 | ATM | 108858032 | A |
| rs1884816 | C | NA | chr22 | CHEK2 | 29106733 | T |
| rs191994935 | T | NA | chr13 | RB1 | 48878279 | C |
| rs1935580 | C | NA | chr10 | PTEN | 90197181 | T |
| rs195993 | T | NA | chr16 | PALB2 | 24017961 | C |
| rs196014 | T | NA | chr16 | PALB2 | 23989600 | G |
| rs1974953 | A | NA | chr11 | ATM | 108887701 | G |
| rs201071425 | GT | NA | chr13 | RB1 | 48433748 | - |
| rs201404152 | C | NA | chr13 | RB1 | 48461537 | T |
| rs2025416 | C | NA | chr13 | BRCA2 | 32821976 | T |
| rs205013 | A | NA | chr13 | BRCA2 | 32807586 | T |
| rs206321 | C | NA | chr13 | BRCA2 | 32990780 | A |
| rs2076505 | A | NA | chr22 | CHEK2 | 28424507 | G |
| rs2077873 | T | NA | chr10 | PTEN | 89315395 | C |
| rs2078326 | C | NA | chr13 | BRCA2 | 32859674 | A |
| rs2086824 | C | NA | chr16 | FANCA | 89471246 | A |
| rs2089115 | C | NA | chr17 | BRCA1 | 40858841 | T |
| rs2128243 | G | NA | chr11 | ATM | 108509754 | A |
| rs2143180 | A | NA | chr22 | CHEK2 | 28298336 | G |
| rs2213645 | G | NA | chr22 | CHEK2 | 29361159 | A |
| rs2228128 | C | NA | chr17 | TP53 | 7400815 | T |
| rs2287357 | A | NA | chr16 | FANCA | 89250145 | G |
| rs2287498 | T | NA | chr17 | TP53 | 7592560 | C |
| rs2290294 | T | NA | chr11 | ATM | 107904379 | C |
| rs2292954 | G | NA | chr16 | FANCA | 89613123 | A |
| rs2292957 | C | NA | chr16 | FANCA | 89653173 | T |
| rs2433340 | G | NA | chr10 | PTEN | 90160023 | A |
| rs2488273 | C | NA | chr10 | PTEN | 90116871 | T |
| rs2575384 | T | NA | chr16 | PALB2 | 23837694 | C |
| rs2596242 | C | NA | chr13 | RB1 | 49505805 | T |
| rs2640738 | C | NA | chr11 | ATM | 108464209 | T |
| rs2640779 | T | NA | chr11 | ATM | 108381247 | C |
| rs2640784 | G | NA | chr11 | ATM | 108409442 | A |
| rs2640785 | A | NA | chr11 | ATM | 108409784 | T |
| rs2673813 | T | NA | chr10 | PTEN | 89801899 | G |
| rs2673822 | C | NA | chr10 | PTEN | 89793086 | G |
| rs2806634 | A | NA | chr13 | BRCA2 | 32826186 | G |
| rs2806638 | G | NA | chr13 | BRCA2 | 32833878 | A |
| rs2806639 | A | NA | chr13 | BRCA2 | 32811607 | G |
| rs3092989 | A | NA | chr13 | BRCA2 | 32889363 | G |
| rs34140758 | A | NA | chr10 | PTEN | 89726794 | C |
| rs34598902 | T | NA | chr17 | TP53 | 7915912 | C |
| rs34704253 | C | NA | chr13 | RB1 | 49417493 | T |
| rs34870602 | G | NA | chr10 | PTEN | 89705371 | C |
| rs35264225 | G | NA | chr16 | FANCA | 89643464 | C |
| rs35743999 | C | NA | chr13 | RB1 | 49417531 | - |
| rs35749740 | C | NA | chr16 | FANCA | 90114833 | T |
| rs35758127 | A | NA | chr17 | BRCA1 | 41727924 | G |
| rs36047689 | A | NA | chr16 | PALB2 | 23717833 | G |
| rs365873 | C | NA | chr13 | BRCA2 | 32547131 | T |
| rs3742318 | C | NA | chr13 | BRCA2 | 33017043 | T |
| rs3744258 | G | NA | chr17 | TP53 | 7623394 | A |
| rs3751688 | A | NA | chr16 | FANCA | 89634439 | G |
| rs3785361 | T | NA | chr16 | PALB2 | 23363399 | A |
| rs3785414 | T | NA | chr16 | FANCA | 89250399 | C |
| rs3812996 | G | NA | chr16 | PALB2 | 23077748 | A |
| rs3950176 | A | NA | chr22 | CHEK2 | 29630337 | G |
| rs399622 | C | NA | chr13 | BRCA2 | 33212785 | A |
| rs4073930 | C | NA | chr16 | PALB2 | 23195646 | T |
| rs4151122 | T | NA | chr17 | TP53 | 7342682 | C |
| rs4287627 | A | NA | chr17 | TP53 | 7932788 | G |
| rs4411548 | T | NA | chr17 | BRCA1 | 40658533 | C |
| rs447529 | G | NA | chr16 | PALB2 | 23633423 | C |
| rs4554861 | A | NA | chr11 | ATM | 107839211 | G |
| rs4577093 | T | NA | chr16 | PALB2 | 23234654 | C |
| rs470097 | A | NA | chr22 | CHEK2 | 28373744 | C |
| rs472826 | T | NA | chr11 | ATM | 107623767 | G |
| rs4753808 | A | NA | chr11 | ATM | 107465180 | G |
| rs478839 | G | NA | chr10 | PTEN | 89731870 | A |
| rs488512 | T | NA | chr11 | ATM | 107729106 | C |
| rs492661 | T | NA | chr11 | ATM | 107567754 | C |
| rs4941698 | G | NA | chr13 | BRCA2 | 33485053 | A |
| rs4943021 | G | NA | chr13 | BRCA2 | 33514547 | T |
| rs4943043 | C | NA | chr13 | BRCA2 | 33534926 | T |
| rs4968031 | A | NA | chr16 | PALB2 | 23765774 | G |
| rs4968044 | C | NA | chr16 | PALB2 | 23074431 | T |
| rs516091 | G | NA | chr11 | ATM | 107663103 | C |
| rs517118 | G | NA | chr13 | BRCA2 | 32970586 | A |
| rs533531826 | AGGA AGGA GGGG AGAG CAGG TAGG AGGG AGGA GC | NA | chr10 | PTEN | 89879755 | - |
| rs556153 | C | NA | chr11 | ATM | 107673628 | T |
| rs55645971 | G | NA | chr13 | BRCA2 | 33189589 | C |
| rs564444 | C | NA | chr11 | ATM | 107625409 | T |
| rs5752851 | G | NA | chr22 | CHEK2 | 29398459 | A |
| rs5752866 | A | NA | chr22 | CHEK2 | 29498118 | G |
| rs5762497 | G | NA | chr22 | CHEK2 | 28546346 | T |
| rs5762930 | A | NA | chr22 | CHEK2 | 29378708 | C |
| rs5762981 | C | NA | chr22 | CHEK2 | 29480954 | T |
| rs57985356 | G | NA | chr17 | TP53 | 7735063 | T |
| rs58040082 | G | NA | chr17 | BRCA1 | 41555780 | A |
| rs597788 | T | NA | chr11 | ATM | 108256859 | C |
| rs5997320 | G | NA | chr22 | CHEK2 | 28270372 | T |
| rs5997443 | T | NA | chr22 | CHEK2 | 29521631 | C |
| rs60030426 | C | NA | chr16 | FANCA | 90068417 | T |
| rs6006075 | T | NA | chr22 | CHEK2 | 29640019 | C |
| rs60543910 | A | NA | chr17 | TP53 | 7928643 | G |
| rs60791058 | G | NA | chr22 | CHEK2 | 29515642 | A |
| rs61227616 | G | NA | chr10 | PTEN | 89445636 | T |
| rs61348941 | T | NA | chr13 | BRCA2 | 33401101 | C |
| rs62045818 | C | NA | chr16 | FANCA | 89047654 | T |
| rs62059804 | A | NA | chr17 | TP53 | 7453505 | C |
| rs62070781 | T | NA | chr16 | FANCA | 89361222 | C |
| rs6503369 | C | NA | chr17 | BRCA1 | 41463821 | G |
| rs652243 | G | NA | chr11 | ATM | 107470916 | A |
| rs6561461 | A | NA | chr13 | RB1 | 49118569 | G |
| rs6561487 | G | NA | chr13 | RB1 | 49521542 | T |
| rs6586129 | T | NA | chr10 | PTEN | 90137045 | C |
| rs667131 | C | NA | chr13 | RB1 | 49485613 | A |
| rs683763 | T | NA | chr10 | PTEN | 89807680 | G |
| rs701848 | C | NA | chr10 | PTEN | 89726745 | T |
| rs7069120 | A | NA | chr10 | PTEN | 90101723 | G |
| rs7076715 | G | NA | chr10 | PTEN | 89459041 | A |
| rs7086211 | G | NA | chr10 | PTEN | 90056152 | A |
| rs7100988 | G | NA | chr10 | PTEN | 89339054 | A |
| rs7127880 | T | NA | chr11 | ATM | 107600570 | C |
| rs71327329 | G | NA | chr22 | CHEK2 | 29327347 | A |
| rs71475111 | A | NA | chr11 | ATM | 108885698 | G |
| rs7184655 | C | NA | chr16 | FANCA | 89601554 | T |
| rs7197490 | T | NA | chr16 | FANCA | 89691798 | C |
| rs7220814 | G | NA | chr17 | TP53 | 7290695 | A |
| rs7222675 | C | NA | chr17 | BRCA1 | 41705105 | T |
| rs7224790 | C | NA | chr17 | BRCA1 | 41748964 | G |
| rs72820004 | A | NA | chr10 | PTEN | 90096580 | G |
| rs72820016 | T | NA | chr10 | PTEN | 90136998 | C |
| rs72820018 | C | NA | chr10 | PTEN | 90144956 | T |
| rs72842820 | A | NA | chr17 | TP53 | 7329134 | G |
| rs730517 | T | NA | chr22 | CHEK2 | 29550379 | G |
| rs73184603 | G | NA | chr13 | RB1 | 49453685 | C |
| rs73191734 | C | NA | chr13 | RB1 | 48868999 | T |
| rs7319930 | T | NA | chr13 | BRCA2 | 33311005 | G |
| rs73233606 | G | NA | chr17 | TP53 | 7750936 | A |
| rs73235718 | A | NA | chr17 | TP53 | 7837228 | G |
| rs73237655 | G | NA | chr17 | TP53 | 7911911 | A |
| rs732508 | G | NA | chr17 | BRCA1 | 41526526 | A |
| rs7337786 | G | NA | chr13 | BRCA2 | 33479823 | T |
| rs73481288 | A | NA | chr13 | RB1 | 48689348 | G |
| rs73540400 | A | NA | chr16 | PALB2 | 23287060 | G |
| rs73550211 | A | NA | chr16 | PALB2 | 23718623 | G |
| rs73557760 | G | NA | chr11 | ATM | 107493106 | C |
| rs73882474 | C | NA | chr22 | CHEK2 | 29523092 | T |
| rs73977765 | G | NA | chr17 | TP53 | 7728764 | A |
| rs74150466 | G | NA | chr10 | PTEN | 89260712 | A |
| rs74150467 | T | NA | chr10 | PTEN | 89260724 | A |
| rs74406212 | G | NA | chr10 | PTEN | 89926667 | A |
| rs74765359 | T | NA | chr17 | BRCA1 | 41088292 | C |
| rs74899331 | A | NA | chr16 | FANCA | 89267096 | C |
| rs75022285 | A | NA | chr10 | PTEN | 89980912 | G |
| rs75128833 | T | NA | chr13 | RB1 | 48527317 | C |
| rs75263440 | C | NA | chr16 | FANCA | 89421388 | A |
| rs75318090 | T | NA | chr10 | PTEN | 89861266 | A |
| rs75764178 | T | NA | chr13 | RB1 | 48592112 | G |
| rs75967297 | C | NA | chr13 | BRCA2 | 33526366 | A |
| rs76154312 | C | NA | chr11 | ATM | 108639173 | T |
| rs76523936 | T | NA | chr13 | RB1 | 48711833 | C |
| rs76682226 | C | NA | chr13 | RB1 | 48505462 | T |
| rs76730553 | T | NA | chr17 | TP53 | 7771462 | C |
| rs76933266 | T | NA | chr10 | PTEN | 89980900 | C |
| rs77057175 | A | NA | chr17 | BRCA1 | 41643970 | G |
| rs77077633 | C | NA | chr13 | BRCA2 | 33437517 | T |
| rs77495014 | T | NA | chr13 | RB1 | 49448209 | C |
| rs77661220 | G | NA | chr11 | ATM | 108597413 | T |
| rs78048465 | C | NA | chr13 | RB1 | 49485591 | T |
| rs78208831 | A | NA | chr16 | FANCA | 89087705 | G |
| rs78236738 | G | NA | chr11 | ATM | 108584854 | C |
| rs78430727 | A | NA | chr10 | PTEN | 90116882 | G |
| rs78579751 | T | NA | chr16 | FANCA | 89198762 | C |
| rs78744936 | A | NA | chr17 | TP53 | 7461343 | G |
| rs7895674 | A | NA | chr10 | PTEN | 90096625 | G |
| rs7897058 | G | NA | chr10 | PTEN | 89787293 | A |
| rs79037983 | A | NA | chr22 | CHEK2 | 28459665 | G |
| rs7912780 | T | NA | chr10 | PTEN | 90096642 | A |
| rs79201073 | A | NA | chr16 | FANCA | 89611199 | G |
| rs79207621 | A | NA | chr13 | RB1 | 48584933 | G |
| rs792212 | T | NA | chr10 | PTEN | 90174153 | C |
| rs792224 | G | NA | chr10 | PTEN | 90109816 | C |
| rs79230416 | C | NA | chr16 | FANCA | 89517502 | G |
| rs79278399 | A | NA | chr13 | BRCA2 | 32794568 | T |
| rs7934423 | T | NA | chr11 | ATM | 108849093 | C |
| rs79375168 | T | NA | chr13 | BRCA2 | 32880009 | C |
| rs7937558 | T | NA | chr11 | ATM | 108495541 | C |
| rs79401250 | G | NA | chr16 | PALB2 | 23871457 | T |
| rs79651004 | G | NA | chr13 | BRCA2 | 32707287 | A |
| rs79750876 | T | NA | chr17 | BRCA1 | 41710104 | C |
| rs79833791 | C | NA | chr16 | FANCA | 90012737 | T |
| rs79840905 | G | NA | chr10 | PTEN | 89323144 | A |
| rs798980 | A | NA | chr13 | BRCA2 | 32750571 | C |
| rs799524 | C | NA | chr13 | BRCA2 | 32523928 | T |
| rs799923 | A | NA | chr17 | BRCA1 | 41251931 | G |
| rs80201685 | A | NA | chr13 | BRCA2 | 32820039 | G |
| rs80333342 | C | NA | chr22 | CHEK2 | 28728983 | T |
| rs8051733 | G | NA | chr16 | FANCA | 90024206 | A |
| rs8052423 | T | NA | chr16 | FANCA | 89269178 | G |
| rs8054767 | A | NA | chr16 | PALB2 | 24029774 | C |
| rs8055868 | A | NA | chr16 | PALB2 | 23348251 | G |
| rs8062311 | A | NA | chr16 | FANCA | 90027838 | G |
| rs872345 | T | NA | chr17 | TP53 | 7720858 | A |
| rs920679 | G | NA | chr11 | ATM | 108038477 | A |
| rs9534076 | C | NA | chr13 | BRCA2 | 32864488 | G |
| rs9534154 | A | NA | chr13 | BRCA2 | 32886213 | G |
| rs9566949 | C | NA | chr13 | BRCA2 | 32565539 | T |
| rs9568672 | C | NA | chr13 | BRCA2 | 33528456 | G |
| rs9568679 | T | NA | chr13 | BRCA2 | 33536160 | C |
| rs9590684 | A | NA | chr13 | BRCA2 | 32570500 | G |
| rs9595227 | G | NA | chr13 | BRCA2 | 32836633 | A |
| rs9595871 | T | NA | chr13 | RB1 | 48794101 | C |
| rs9666351 | G | NA | chr11 | ATM | 108468768 | A |
| rs9895916 | A | NA | chr17 | TP53 | 7950394 | G |
| rs9898682 | A | NA | chr17 | BRCA1 | 41738823 | G |
| rs990253 | A | NA | chr11 | ATM | 108494855 | G |
| rs9902697 | C | NA | chr17 | BRCA1 | 41682852 | G |
| rs9905358 | G | NA | chr17 | BRCA1 | 41555474 | A |
| rs9908405 | C | NA | chr17 | TP53 | 7467461 | A |
| rs9914497 | T | NA | chr17 | BRCA1 | 41009445 | C |
| rs9923604 | C | NA | chr16 | FANCA | 90068980 | G |
| rs9940479 | C | NA | chr16 | PALB2 | 23964004 | T |

**Table 19: List of expected SNPs in Positive Control DNA sample detected using Lung Probes**

| **rs** | **alt** | **cds_effect** | **chr** | **gene** | **pos** | **ref** |
|---|---|---|---|---|---|---|
| rs10509544 | G | NA | chr10 | PTEN | 90168074 | T |
| rs10852891 | A | NA | chr17 | TP53 | 7490810 | G |
| rs11078685 | T | NA | chr17 | TP53 | 7411447 | C |
| rs11078708 | C | NA | chr17 | TP53 | 7720091 | G |
| rs11078716 | T | NA | chr17 | TP53 | 7820183 | G |
| rs11079387 | G | NA | chr17 | BRCA1 | 41510850 | A |
| rs11202435 | G | NA | chr10 | PTEN | 89295958 | T |
| rs12250975 | G | NA | chr10 | PTEN | 89282145 | A |
| rs12416046 | G | NA | chr10 | PTEN | 89861218 | A |
| rs1320304 | C | NA | chr17 | BRCA1 | 41673990 | T |
| rs 1544724 | G | NA | chr17 | TP53 | 7621777 | T |
| rs 1553468 | G | NA | chr17 | BRCA1 | 40849842 | A |
| rs1984518 | G | NA | chr17 | BRCA1 | 41521666 | T |
| rs2228129 | C | NA | chr17 | TP53 | 7402600 | T |
| rs2287499 | G | NA | chr17 | TP53 | 7592168 | C |
| rs2302764 | C | NA | chr17 | TP53 | 7360110 | T |
| rs2846412 | T | NA | chr11 | ATM | 108384207 | C |
| rs4151120 | A | NA | chr17 | TP53 | 7342148 | T |
| rs4383207 | G | NA | chr17 | TP53 | 7936282 | A |
| rs472817 | A | NA | chr13 | BRCA2 | 32983250 | G |
| rs4934378 | C | NA | chr10 | PTEN | 89867693 | T |
| rs566238 | T | NA | chr11 | ATM | 107706832 | C |
| rs56872996 | G | NA | chr17 | TP53 | 7928924 | A |
| rs57752647 | G | NA | chr17 | TP53 | 7381256 | T |
| rs60034915 | T | NA | chr10 | PTEN | 89872699 | C |
| rs62059833 | T | NA | chr17 | TP53 | 7514544 | C |
| rs6761 | T | NA | chr17 | TP53 | 7417663 | C |
| rs693963 | C | NA | chr13 | BRCA2 | 32979138 | T |
| rs701893 | A | NA | chr10 | PTEN | 90144950 | G |
| rs7069968 | T | NA | chr10 | PTEN | 90043824 | C |
| rs | alt | cds_effect | chr | gene | pos | ref |
| rs7077182 | G | NA | chr10 | PTEN | 90216244 | C |
| rs7092284 | A | NA | chr10 | PTEN | 89451838 | G |
| rs7092392 | A | NA | chr10 | PTEN | 89451853 | G |
| rs7097903 | C | NA | chr10 | PTEN | 90049968 | G |
| rs73245806 | A | NA | chr17 | TP53 | 7947908 | T |
| rs7359598 | C | NA | chr17 | BRCA1 | 40897470 | T |
| rs78790828 | A | NA | chr17 | TP53 | 7830280 | G |
| rs7895494 | C | NA | chr10 | PTEN | 89379732 | A |
| rs7904229 | A | NA | chr10 | PTEN | 90221561 | C |
| rs8068764 | C | NA | chr17 | BRCA1 | 41490161 | T |
| rs9891119 | C | NA | chr17 | BRCA1 | 40507980 | A |
| rs9901673 | A | NA | chr17 | TP53 | 7484101 | C |
| rs9903378 | C | NA | chr17 | TP53 | 7588006 | A |

The invention will now be defined by reference to the following clauses:
1. A kit comprising one or more capture probe modules,
   wherein each capture probe module comprises a tail sequence and a capture probe sequence capable of hybridizing to a target sequence in a test sample;
   wherein the one or more capture probe modules have passed a probe Quality Control (QC) process.
2. A kit comprising a set of adaptors,
   wherein each adaptor comprises an adaptor module;
   wherein the set of adaptors have passed an adaptor Quality Control (QC) process.
3. The kit of clause 1, further comprising a set of adaptors, wherein each adaptor comprises an adaptor module.
4. The kit of clause 2, further comprising one or more capture probe modules,
   wherein each capture probe module comprises a tail sequence and a capture probe sequence capable of hybridizing to a target sequence in a test sample.
5. The kit of clause 3, wherein the set of adaptors have passed an adaptor Quality Control (QC) process.
6. The kit of any one of clauses 2-5, wherein the adaptor QC process comprises a test for adaptor ligation, wherein the test for adaptor ligation comprises:
   (a) ligating the set of adaptors to a pre-determined amount of end-repaired DNA fragments to generate a library of adaptor-tagged DNA fragments (LIBS); and
   (b) amplifying the LIBS to generate a Library Post Amplification (LPA);
   wherein the set of adaptors is considered to have passed the test for adaptor ligation when the concentration of the LPA is higher than a pre-determined concentration.
7. The kit of any one of clauses 2-6, wherein the adaptor QC process comprises a test for adaptor distribution comprising:
   (a) ligating the set of adaptors to a pre-determined amount of end-repaired DNA fragments to generate a library of adaptor-tagged DNA fragments (LIBS);
   (b) amplifying the LIBS using a primer pair comprising at least one primer comprising an index sequence to generate a Library Post Index Amplification (LPIA); and
   (c) performing a quantitative genetic analysis on the LPIA;
   wherein the set of adaptors is considered to have passed the test for adaptor distribution when one or more pre-determined acceptance criteria for the quantitative genetic analysis has been met.
8. The kit of clause 7, wherein the pre-determined acceptance criteria comprise one or more of:
   (a) Barcode Crosstalk is present in no more than 0.05%-5% of reads;
   (b) unknown adaptors are present in no more than 1%-50% of reads;
   (c) no more than 10%-80% of unique adaptor sequences have a number of reads that is 0% to 50% of the average number of reads for all unique adaptor sequences;
   (d) at least 60%-99.9% of all unique adaptor sequences are present; and
   (e) no more than 5%-50% of unique adaptor sequences have reads greater than twice the average number of reads for all unique adaptor sequences.
9. The kit of any one of clauses 6-8, wherein the set of adaptors is considered to have passed the adaptor QC process when it has passed the test for adaptor ligation and/or the test for adaptor distribution.
10. The kit of any one of clauses 1-9, wherein the probe QC process comprises a test for capture probe modules comprising:
   (a) ligating a set of adaptors to a DNA sample comprising end-repaired DNA fragments to generate a library of adaptor-tagged DNA fragments (LIBS);
   (b) amplifying the LIBS to generate a Library Post Amplification (LPA);
   (c) splitting or diluting the LPA to generate a Target Capture LPA (TC LPA) and a Whole-Genome LPA (WG LPA);
   (d) amplifying the WG LPA to generate a Whole-Genome Library Amplified (WGLA);
   (e) hybridizing the one or more capture probe modules to be tested to the TC LPA to form adaptor-tagged DNA fragment-capture probe module complexes;
   (f) isolating the adaptor-tagged DNA fragment-capture probe module complexes to form isolated adaptor-tagged DNA fragment-capture probe module complexes;
   (g) enzymatically processing the isolated adaptor-tagged DNA fragment-capture probe module complexes to generate Hybrid Molecules, wherein each Hybrid Molecule comprises the capture probe module and a complement of the adaptor-tagged DNA fragment;
   (h) amplifying the Hybrid Molecules to generate a Target Capture Library Amplified (TCLA);
   (i) combining the WGLA and the TCLA to form a Sequence-Ready Library (SRL); and
   (j) performing a quantitative genetic analysis on the SRL;

   wherein the DNA sample comprises a plurality of single nucleotide polymorphisms (SNPs);
   wherein the one or more capture probe modules are considered to have passed the probe QC process if one or more pre-determined acceptance criteria for the quantitative genetic analysis have been met.
11. The kit of clause 10, wherein the pre-determined acceptance criteria comprise one or more of:
   (a) at least 60%-99.9% of capture probes have at least 1 total reads;
   (b) at least 60%-99.9% of capture probes have at least 10 to at least 200 on-target total reads; and
   (c) at least 60%-99.9% of expected SNPs within the DNA sample are detected.
12. The kit of any one of clauses 1-11, comprising a first primer pair comprising a first F primer and a first R primer,
   wherein each adaptor module comprises an amplification region;
   wherein the first F primer comprises an amplification region binding region and a sequencing primer binding region;
   wherein the first R primer comprises a tail sequence binding region and a sequencing primer binding region.
13. The kit of any one of clauses 1-12, comprising a second primer pair comprising a second F primer and a second R primer,
   wherein each adaptor module comprises an amplification region;
   wherein each of the second F primer and the second R primer comprises an amplification region binding region and a sequencing primer binding region.
14. The kit of any one of clauses 1-13, wherein the tail sequence of each capture probe module comprises a Library Tag.
15. A kit comprising:
   (a) a set of adaptors, wherein each adaptor comprises an adaptor module comprising an amplification region;
   (b) one or more capture probe modules, wherein each capture probe module comprises a tail sequence and a capture probe sequence capable of hybridizing to a target sequence in a test sample, wherein the tail sequence of each capture probe module comprises a Library Tag;
   (c) a first primer pair comprising a first F primer and a first R primer, wherein the first F primer comprises an amplification region binding region and a sequencing primer binding region;
      wherein the first R primer comprises a Library Tag binding region and a sequencing primer binding region;
   (d) a second primer pair comprising a second F primer and a second R primer, wherein each of the second F primer and the second R primer comprises an amplification region binding region and a sequencing primer binding region, wherein none of the primers of the second primer pair bind to the Library Tag.
16. The kit of clause 14 or clause 15, wherein the Library Tag comprises a nucleic acid sequence or an amino acid sequence.
17. The kit of any one of clauses 12-16, wherein the first primer pair is used to generate a first modified library and the second primer pair is used to generate a second modified library, wherein the first modified library and the second modified library are configured to be combined into a Sequence-Ready Library (SRL).
18. The kit of clause 17, wherein both the first modified library and the second modified library are generated from the test sample.
19. The kit of clause 17 or clause 18, wherein the first modified library or the second modified library comprises the Library Tag, wherein the Library Tag is configured to distinguish the first modified library from the second modified library.
20. The kit of any one of clauses 17-19,
   wherein each library fragment of the first modified library is an adaptor-tagged DNA fragment comprising an adaptor, a capture probe module, and at least a portion of a DNA sequence of the test sample;
   wherein each library fragment of the second modified library is an adaptor-tagged DNA fragment comprising an adaptor and at least a portion of a DNA sequence of the test sample,
   wherein none of the adaptor-tagged DNA fragments of the second modified library comprises a capture probe module.

## Claims

1. A method for simultaneous DNA analysis, the method comprising:
a) to a first adaptor-tagged library comprising a first genetic locus, performing a first process to generate a first modified library;
b) to a second adaptor-tagged library comprising a second genetic locus, performing a second process to generate a second modified library;
wherein the first process and the second process are not identical; and
wherein the first modified library is a Target Capture Library (TCL) or amplified Target Capture Library (TCLA) and the second modified library is a Whole-Genome Library (WGL) or Whole-Genome Library Amplified (WGLA); and
c) contacting the first modified library and the second modified library to generate a combined library;
wherein the first adaptor-tagged library and the second adaptor-tagged library are from the same adaptor-tagged parent library.

2. The method of claim 1, wherein the first process comprises contacting the first adaptor-tagged library with a capture probe and the second process comprises amplifying or extending a DNA fragment of the second adaptor-tagged library.

3. The method of claim 1 or claim 2, wherein a targeted genetic analysis and a broad-coverage whole genome analysis are performed on the combined library.

4. The method of claim 3, wherein the targeted genetic analysis and the broad-coverage whole genome analysis are performed on the results of one sequencing run.

5. The method of any one of claims 1-4, wherein the adaptor-tagged parent library is split into the first adaptor-tagged library and the second adaptor-tagged library, optionally wherein the method further comprises an initial step of splitting the adaptor-tagged parent library into at least two partitions, wherein a first partition is the first adaptor-tagged library and a second partition is the second adaptor-tagged library.

6. The method of claim 5, wherein the adaptor-tagged parent library is split into more than two adaptor-tagged libraries.

7. The method of any one of claims 1-6, wherein the first adaptor-tagged library and the second adaptor-tagged library each comprises an adaptor module;
and/or wherein each adaptor of the first adaptor-tagged library and the second adaptor-tagged library comprises a ligation strand oligonucleotide and a non-ligation strand oligonucleotide, wherein the non-ligation strand oligonucleotide is capable of hybridizing to a region at the 3' end of the ligation strand oligonucleotide and forming a duplex therewith;
and/or wherein each adaptor of the first adaptor-tagged library and the second adaptor-tagged library is selected from a set of adaptors, wherein each adaptor of the set of adaptors comprises an ID region selected from a pool of unique ID regions, wherein the pool is selected from a plurality of pools, and wherein the selected pool is unique to a test sample;
and/or wherein each adaptor of the first adaptor-tagged library and the second adaptor-tagged library comprises an adaptor module comprising:
a) an amplification region comprising a primer binding site;
b) an ID region; and
c) an anchor region; optionally wherein the ID region identifies both a sample and a DNA fragment.

8. The method of any one of claims 1-7, wherein the first process comprises:
a) contacting the first adaptor-tagged library with one or more capture probes under conditions suitable for hybridization to form one or more capture probe/adaptor-tagged DNA complexes, wherein each capture probe comprises:
a first region comprising a primer binding site; and
a second region capable of hybridizing to a target region in the first genetic locus of the first adaptor-tagged library;
b) isolating the one or more capture probe/adaptor-tagged DNA complexes from step (a), wherein each isolated capture probe/adaptor-tagged DNA complex comprises a capture probe and an adaptor-tagged DNA molecule; and
c) enzymatically processing the one or more isolated capture probe/adaptor-tagged DNA complexes from step (b) to generate one or more adaptor-tagged hybrid nucleic acid molecules (Hybrid Molecules), wherein each Hybrid Molecule comprises:
i) at least a portion of the capture probe or a complement thereof; and
ii) at least a portion of the adaptor-tagged DNA molecule or a complement thereof.

9. The method of any one of claims 1-8, wherein the second process comprises amplifying or extending the second genetic locus of the second adaptor-tagged library; and/or wherein the second process comprises amplifying or extending the second genetic locus of the second adaptor-tagged library, wherein the amplifying or extending comprises hybridizing a first primer comprising a 5' oligonucleotide (B1) and a second primer comprising a 3' oligonucleotide (B2) to an adaptor-tagged DNA molecule, wherein at least one of B1 and B2 comprises a Library Tag, and wherein the amplified or extended second genetic locus comprises B1 and B2.

10. The method of any one of claims 1-9, further comprising performing genetic analysis.

11. The method of claim 10, wherein the genetic analysis comprises sequencing of the combined library to generate a plurality of sequencing reads and performing bioinformatics analysis on the plurality of sequencing reads.

12. The method of any one of claims 1-11, wherein the combined library is a Sequence-Ready Library.

13. The method of any one of claims 1-12, further comprising ligating adaptors to DNA fragments of a test sample to generate a plurality of adaptor/DNA fragment complexes; and contacting the plurality of adaptor/DNA fragment complexes with one or more enzymes to form an adaptor-tagged DNA library comprising a plurality of adaptor-tagged DNA fragments;

14. The method of claim 13, wherein said plurality of adaptor-tagged DNA fragments provides said adaptor-tagged parent library.

15. Use of a kit in a method according to any one of claims 1 to 14, the kit comprising:
a) a set of adaptors, wherein each adaptor comprises an adaptor module comprising an amplification region;
b) one or more capture probe modules, wherein each capture probe module comprises a tail sequence and a capture probe sequence capable of hybridizing to a target sequence in a test sample, wherein the tail sequence of each capture probe module comprises a Library Tag;
c) a first primer pair comprising a first F primer and a first R primer, wherein the first F primer comprises an amplification region binding region and a sequencing primer binding region; wherein the first R primer comprises a Library Tag binding region and a sequencing primer binding region; and
d) a second primer pair comprising a second F primer and a second R primer, wherein each of the second F primer and the second R primer comprises an amplification region binding region and a sequencing primer binding region, wherein none of the primers of the second primer pair bind to the Library Tag.
